# EUROPEAN PATENT APPLICATION

(11) **EP 4 686 495 A1**
(43) Date of publication of application: **04.02.2026**
(21) Application number: 24192658.3
(22) Date of filing: 02.08.2024
(51) Int. Cl.: A61P 3/00, A61P 11/06, A61P 25/00, C07D 498/08, A61K 31/438

(54) **MACROCYCLIC LIGANDS FOR FK506-BINDING PROTEINS FOR TREATMENT OF DISEASES WITH IMPROVED CHARACTERISTICS**

(71) Applicant: Technische Universität Darmstadt, 64289 Darmstadt (DE)
(72) Inventor: Spiske, Moritz, 64289 Darmstadt (DE); Hausch, Felix, 64289 Darmstadt (DE); Meyners, Christian, 64289 Darmstadt (DE); Bauder, Michael, 64289 Darmstadt (DE)
(74) Representative: Fuchs Patentanwälte Partnerschaft mbB

(57) **Abstract**

The present invention relates to macrocyclic FKBP51-binding compounds having improved characteristics, as well as solvates, hydrates and/or pharmaceutically acceptable salts of these compounds as well as pharmaceutical compositions containing at least one of these compounds together with pharmaceutically acceptable carrier, excipient and/or diluents. Said compounds have been identified as especially potent ligands of FK506 binding proteins (FKBPs also called MIPs in bacteria), especially human FKBP51 and are useful for treatment of diseases such as psychiatric, metabolic and neurological disorders as well as pain and cancers. Said substituted derivatives may further be used as agents for inducing protein-protein interactions (PPIs) acting as molecular glues (Figure 10).

## Description

The present invention relates to macrocyclic FKBP51-binding compounds having improved characteristics, as well as solvates, hydrates and/or pharmaceutically acceptable salts of these compounds as well as pharmaceutical compositions containing at least one of these compounds together with pharmaceutically acceptable carrier, excipient and/or diluents. Said compounds have been identified as especially potent ligands of FK506 binding proteins (FKBPs also called MIPs in bacteria), especially human FKBP51 and are useful for treatment of diseases such as psychiatric, metabolic and neurological disorders as well as pain and cancers. Said substituted derivatives may further be used as agents for inducing protein-protein interactions (PPIs) acting as molecular glues.

### Background of the invention

The FK506-binding protein (FKBP) family, a group of immunophilins, comprises proteins featuring diverse protein-protein interaction domains and multifunctional cellular roles. Their bacterial counterparts, known as macrophage infectivity potentiators (Mip), are highly conserved and interact with immunosuppressive drugs like FK506 and rapamycin. These proteins exhibit peptidyl prolyl isomerase (PPlase) activity, akin to that seen in cyclophilins and parvulins. FKBP12, a protein weighing 12 kDa, is the most extensively researched member of this family.

The immunosuppressant drugs FK506, rapamycin, and cyclosporin are notable for their potent T-cell specific immunosuppressive properties, effective against conditions like autoimmunity, transplant rejection, inflammation, allergic responses, and other autoimmune or immune-mediated diseases. FK506 and rapamycin, in addition to binding FKBP12, also inhibit calcineurin (CaN) and mTOR, respectively, thereby exerting their immunosuppressive effects.

The high molecular weight homologs FKBP51 and FKBP52 function as cochaperones for heat shock protein 90 (Hsp90), influencing the signal transduction of steroid hormone receptors, including the glucocorticoid receptor, by participating in the Hsp90-steroid hormone receptor complex.

In this complex, FKBP51 and FKBP52 modulate the binding competence and signalling of steroid hormone receptors, thereby regulating cellular responses to circulating hormone levels. This regulatory role has been substantiated by cellular studies and knockout mice experiments, which have demonstrated the critical influence of FKBP51 and FKBP52 on the activity of glucocorticoid, progesterone, and androgen receptors. Furthermore, polymorphisms in the FKBP51 gene in psychiatric patients have been linked to various stress-related psychiatric disorders, diabetes, obesity, and chronic pain states. Both proteins have been found to be overexpressed in several cancers.

Further research has led to the development of α-ketoamide analogues of FK506 that lack immunosuppressive activity. Historically, FKBP inhibitors have been described, such as bicyclic aza-amide derivatives and their stereoisomers detailed in WO/2014/015993. These bicyclic aza-amide derivatives have been identified as specific FKBP inhibitors, including FKBP51, and are promising for treating psychiatric disorders, neurodegenerative diseases, vision disorders, memory impairment, and for promoting hair growth and treating alopecia. WO2015/110271 describes Diazabicyclo-[4.3.1]-decane derivatives as potent inhibitors of FKBP-function, with binding to certain members of FKBP-family, such as FKBP51, for example.

WO2022/049005 describes high affinity macrocyclic FKBP51-lnhibitors (HAM-FKBP51-Inhibitors), which enable the selective inhibition of FKPB51 with a macrocyclic scaffold with improved physicochemical and pharmacokinetic characteristics.

However, the inhibitors known in the prior art still show sub-optimal physicochemical and pharmacokinetic characteristics, which need to be improved. Moreover, at the same time any modification of the FKBP-inhibitors bears the risk of a reduction of selectivity and/or affinity to the target molecule, which needs to be avoided.

Therefore, it is the object of the present invention to provide compounds and/or pharmaceutically acceptable salts thereof, which inhibits FKBP51 more efficiently than their linear precursors while having lower molecular weights and lower lipophilicity compared to prior art molecules.

Another aspect of the invention is to provide compounds and/or pharmaceutically acceptable salts thereof which can be used as pharmaceutically active agents, especially for the treatment of psychiatric, metabolic, infective, neurological and haematological disorders as well as pain diseases and cancers, as well as compositions comprising at least one of those compounds and/or pharmaceutically acceptable salts thereof as pharmaceutically active ingredients.

A further aspect of the invention is to provide methods for preparing said compounds.

The object of the present invention is solved by the teaching of the independent claims. Further advantageous features, aspects and details of the invention are evident from the dependent claims, the description, and the examples of the present application.

### Description of the invention

In a first aspect, the compound of the present invention may be represented by general formula 1: 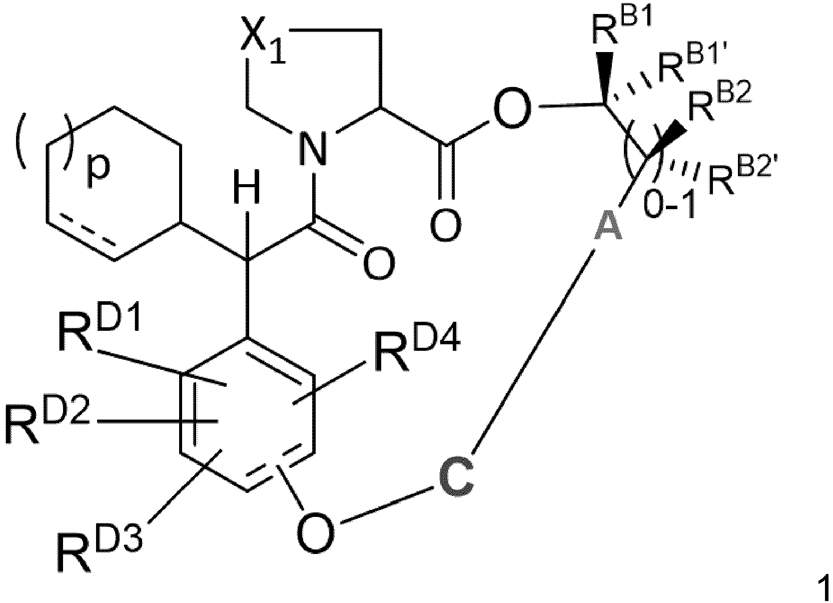 wherein
**X1** represents -CH₂-, -CH₂CH₂-, -CH=CH-, -CH₂-S-, or -S-CH₂-;
**p** is an integer of 0 or 1;
" 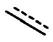 " represents a C=C bond or a C-C bond;
wherein **C** represents 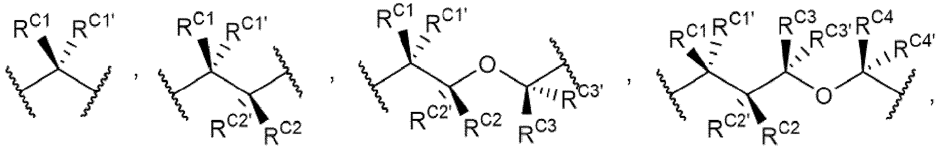 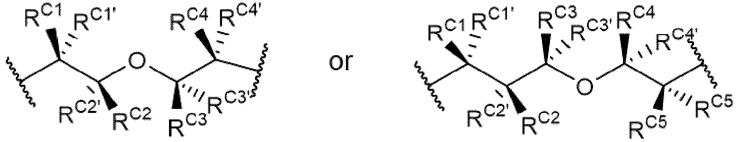
wherein R^{C1}, R^{C1'}, R^{C2}, R^{C2'}, R^{C3}, R^{C3'}, R^{C4}, R^{C4'}, R^{C5}, R^{C5'}, represent independently from each other: H, F, Me, C₂H₅, C₃H₇, CH₂CH=CH₂, CH=CH₂, C=CH, cyclo-C₃H₅, OH, OMe, CH₂OH, CH₂OMe, NH₂, NH-Me, NH-Me₂, or CN;
wherein A represents 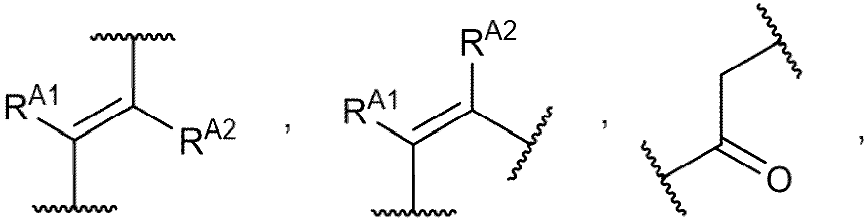 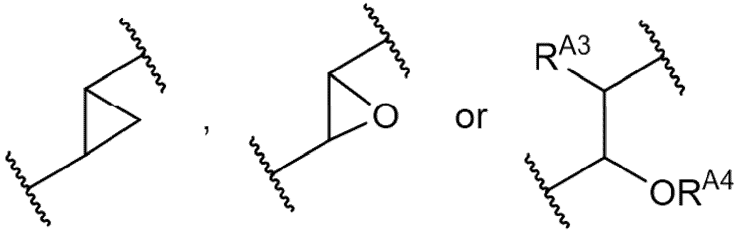
wherein R^{A1}, R^{A2} represent independently from each other: H, F, Me, or CH₂OH; and
wherein R^{A3} represents H, F, OH, O-Me, Me, CN, NH2, NH-Me, N-Me2, SH, or S-Me; and
wherein R^{A4} represents H, or Me.
wherein R^{B1}, R^{B1'}, R^{B2}, R^{B2'} represent independently from each other: H, F, Me, C₂H₅, C₃H₇, CH₂CH=CH₂, CH=CH₂, C=CH, cyclo-C₃H₅, OH, O-Me, CH₂OH, CH₂O-Me, NH₂, NH-Me, NH-Me₂, or CN;
wherein R^{D1}, R^{D2}, R^{D3}, R^{D4} represent independently from each other: -H, -OH, -OCH₃, - OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OH, -C₂H₄-OH, -C₃H₆-OH, -CH(OH)-CH₂-OH, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, - CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I, - P(O)(OH)₂, -P(O)(OCH₃)₂, -P(O)(OC₂H₅)₂, -P(O)(OCH(CH₃)₂)₂, -C(OH)[P(O)(OH)₂]₂, - Si(CH₃)₂(C(CH₃)₃), -Si(C₂H₅)₃, -Si(CH₃)₃, -N₃, -CN, -OCN, -NCO, -SCN, -NCS, -CHO, - COCH₃, -COC₂H₅, -COC₃H₇, -CO-cyclo-C₃H₅, -COCH(CH₃)₂, -COC(CH₃)₃, -COOH, - COCN, -COOCH₃, -COOC₂H₅, -COOC₃H₇, -COO-cyclo-C₃H₅, -COOCH(CH₃)₂, - COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CH₂-CONH₂, -CONHCH₃, -CONHC₂H₅, -CONHC₃H₇, -CONH-cyclo-C₃H₅, - CONH[CH(CH₃)_{2]}, -CONH[C(CH₃)_{3]}, -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, - S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂] -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cy-clo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₅H₉, -cyclo-C₆H₁₁, -CH₂-cyclo-C₆H₁₁, -CH₂-CH₂-cyclo-C₆H₁₁, -cyclo-C₇H₁₃, -cyclo-C₈H₁₅, -Ph, -CH₂-Ph, -CH₂-CH₂-Ph, -CH=CH-Ph, - CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, - CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, - C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C=CH, -CH(CH₃)-CH=CH-CH=CH₂, -CH=CH-CH₂-C(CH₃)=CH₂, -CH(CH₃)-C=C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, -cyclo-C₃H₅, - cyclo-C₄H₇, -O-CHF₂, -O-CF₂-CH=CH₂, -O-CF₂-C₂H₅, -NH-COOCH₃, -N(CH₃)-COCH₃
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts.

In one embodiment the residues R^{B1}, R^{B1'}, R^{B2}, R^{B2'} are independently selected from -H, -CH₃, - C₂H₅, -OH, -OCH₃, -F, -Cl, or -NH₂.

In another embodiment the compound may be represented by formula 2:

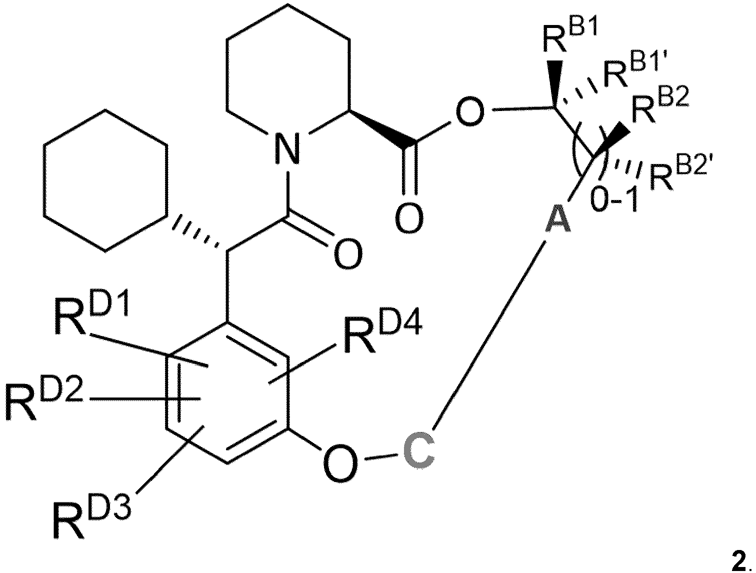

In another embodiment the compound may be represented by formula 3:

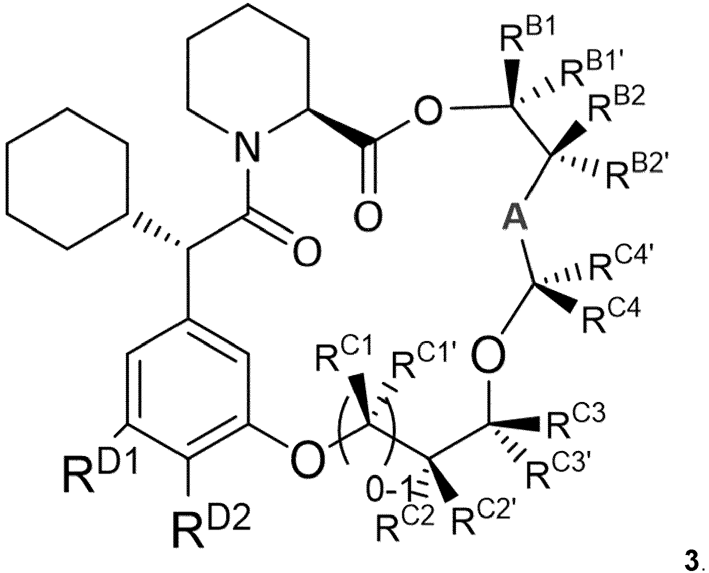

In another embodiment the compound may be represented by any of the formula 1-3, wherein at least one of the substituents R^{B1}, R^{B1'}, R^{B2}, R^{B2'}, R^{C1}, R^{C1'}, R^{C2}, R^{C2'}, R^{C3}, R^{C3'}, R^{C4}, R^{C4'}, R^{C5}, or R^{C5'} is not H.

In another embodiment the compound has a K_{d} [nM] for human FKBP51 of 5000 nM or less, of 2000 nM or less, of 1000 nM or less, of 700 nM or less, of 500 nM or less, of 400 nM or less, of 300 nM or less, of 200 nM or less, or of 100 or less. In another embodiment the compound has a K_{d} [nM] to FKBP51 of 10 nM or more, of 20 nM or more, of 30 nM or more, of 40 nM or more, or of 50 nM or more. In yet another embodiment the compound has a K_{d} [nM] to FKBP51 of from 20 nM to 400 nM, of from 30 nM to 350 nM, of from 40 nM to 200 nM, or of from 50 nM to 150 nM.

In another embodiment the compound has an IC50 [nM] measured by nanoBRET to FKBP51 of 5000 or less, of 2000 or less, of 1000 or less, of 700 or less, of 500 or less, or of 400 or less. In another embodiment the compound has an IC50 [nM] measured by nanoBRET to human FKBP51 of 50 or more, of 100 or more, of 150 or more, of 200 or more, of 250 or more, or of 300 or more. In yet another embodiment the compound has an IC50 [nM] measured by nanoBRET to human FKBP51 of from 50 to 2000, of from 100 to 1500, of from 150 to 1000, of from 175 to 900, of from 200 to 800, or of from 250 to 750.

In another embodiment the compound has a selectivity for human FKBP51 over human FKBP52 (K_{d}^{FKBP51}_{/} K_{d}^{FKBP52}) of 50 or more, of 500 or more, or of 5000 or more. In another embodiment the compound has a selectivity for human FKBP51 over human FKBP12 or human FKBP12.6 (K_{d}^{FKBP51}_{/} K_{d}^{FKBP12} or K_{d}^{FKBP51}_{/} K_{d}^{FKBP12.6}) of 5 or more, of 10 or more, or of 25 or more.

In another embodiment the compound has a molecular weight of 650g/mol or less, 600g/mol or less, 550g/mol or less, 500g/mol or less, or 450g/mol or less.

In a second aspect the disclosure pertains to a pharmaceutical composition comprising at least one compound as disclosed hereinunder together with at least one pharmaceutically acceptable carrier, solvent or excipient or together with at least one pharmaceutically acceptable carrier, solvent or excipient and at least one active agent selected from the group consisting of an anti-depressant and other psychotropic drugs, as well as diabetes drugs, pain drugs and/or antibiotics.

In another embodiment the pharmaceutical composition may further comprise at least one active agent selected from the group consisting of an anti-depressant and other psychotropic drugs. In one embodiment the anti-depressant may be selected from amitriptyline, amioxide clomipramine, doxepine, duloxetine, imipramine trimipramine, mirtazapine, reboxetine, citaloprame, fluoxetine, moclobemide and sertraline.

In a third aspect the disclosure pertains to a compound as disclosed hereinunder for use as pharmaceutically active agent in medicine.

In one embodiment the compounds of the present disclosure (1) can be compared to the linear form of the same type of compounds (1'):

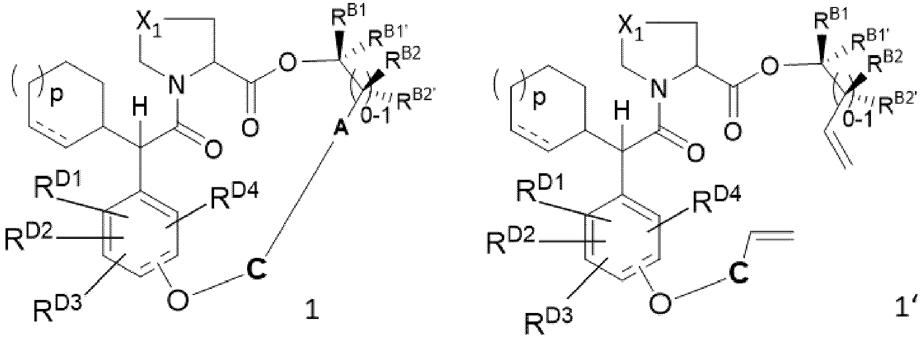

Furthermore, cyclic compounds of the formula 2 can be compared to linear compounds of the formula 2' and cyclic compounds of the formula 3 can be compared to linear compounds of the formula 3': 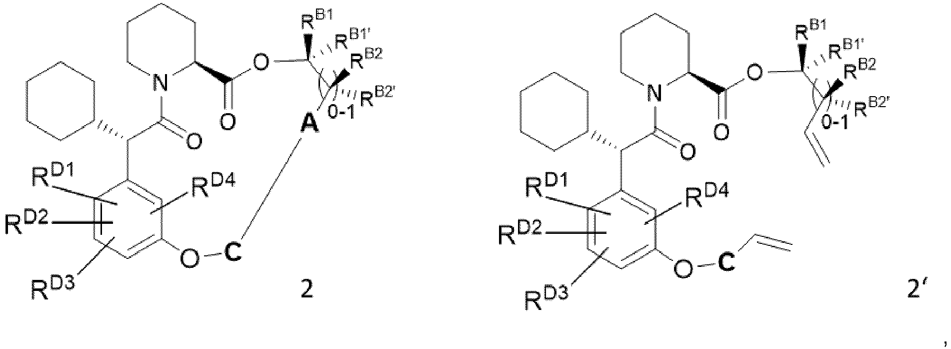 and

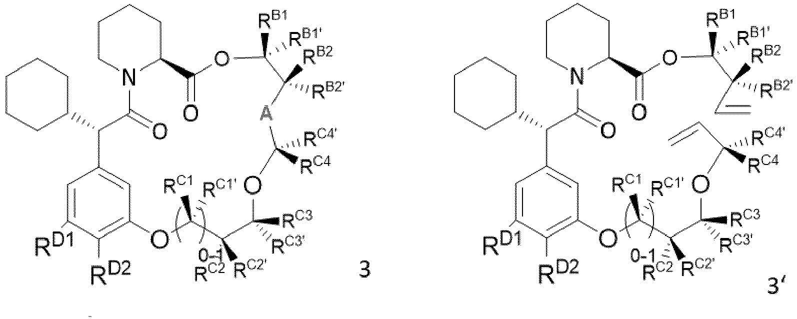

It could be shown that the cyclic compounds (1, 2 or 3) are advantageous in terms of their affinity for FKBP51 or physicochemical and pharmacokinetic characteristics.

Specifically, the affinity for human FKBP51 of the cyclic compound (K_{d}^{FKBP51}(cyclic)) compared to the affinity for human FKBP51 of the corresponding linear compound (K_{d}^{FKBP51}(linear)) is greater than 3, greater than 5, greater than 10, or greater than 20.

### Pharmaceutical Composition

The present invention also comprises pharmaceutically acceptable salts of the compounds according to the general formula **1,** all stereoisomeric forms of the compounds according to the general formula **1** as well as solvates, especially hydrates or prodrugs thereof.

In case, the inventive compounds bear basic and/or acidic substituents, they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples for suitable inorganic or organic bases are, for example, NaOH, KOH, NH₄OH, tetraalkyl-ammonium hydroxide, lysine or arginine and the like. Salts may be prepared in a conventional manner using methods well known in the art, for example by treatment of a solution of the compound of the general formula 1 with a solution of an acid, selected out of the group mentioned above.

Some of the compounds of the present invention may be crystallised or re-crystallised from solvents such as aqueous and organic solvents. In such cases solvates may be formed. This invention includes within its scope stoichiometric solvates including hydrates as well as compounds containing variable amounts of water that may be produced by processes such as lyophilisation.

Certain compounds of the general formula **1** may exist in the form of optical isomers if substituents with at least one asymmetric center are present, e.g. diastereoisomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses. Where a compound according to the general formula **1** contains an alkene moiety, the alkene can be presented as a cis- or trans-isomer or a mixture thereof. When an isomeric form of a compound of the invention is provided substantially free of other isomers, it will preferably contain less than 5% w/w, more preferably less than 2% w/w and especially less than 1% w/w of the other isomers.

Therefore, one aspect of the present invention is that the compounds according to the general formula 1 are suitable for use as ligand of FK506-binding proteins (FKBP), especially FKBP51.

FKBP51 has been implicated in numerous in human diseases. Consequently, FKBP51 is a target which is addressed in order to prevent and/or treat the diseases disclosed in the afore-mentioned literature.

Thus, FKBP51-binding compounds of the present invention can be used as pharmaceutically active agent in medicine.

Preferred, the FKBP51 ligand compounds of the present invention can be used for treatment, or for the preparation of a pharmaceutical formulation for prophylaxis and/or treatment of these FKBP51-associated diseases such as depression, obesity or chronic pain.

The inventive compound of any one of formula **1,** subformula **2** and/or subformula **3** is used in the manufacture of a medicine or of a pharmaceutical composition for the treatment and/or prevention of FKBP--associated diseases.

Another aspect of the present invention relates to a method of treating FKBP- -associated diseases comprising administration a therapeutically effective amount of at least one inventive compound or a pharmaceutical composition comprising at least one inventive compound.

These FKBP- -associated diseases include psychiatric and neurodegenerative diseases, disorders and conditions, for metabolic diseases such as localized adiposity or obesity, for sleep disorders, neuroprotection or neuroregeneration, for the treatment of neurological disorders, for the treatment of diseases relating to neurodegeneration, for the treatment of cancers such as malignant melanoma, multiple myeloma, or acute lymphoblastic leukaemia and especially steroid-hormone dependent cancers such as prostate cancer, for the treatment of glucocorticoid hyposensitivity syndromes and for peripheral glucocorticoid resistance, for asthma, especially steroid-resistant asthma, and for the treatment of infectious diseases, for stimulating neurite growth or neuroregeneration, for neuroprotection, for facial infiltrating lipomatosis, for the use as wound healing agents for treating wounds resulting from injury or surgery; for the use in limiting or preventing haemorrhage or neovascularization for treating macular degeneration, and psychiatric disorders (such as depression or post-traumatic stress disorder), metabolic disorders (such as obesity or diabetes), neurological disorders (such as Alzheimer's diseases or Parkinson's diseases) and haematological disorders (such as hereditary haemorrhagic telangiectasia or pulmonary arterial hypertension) as well as pain diseases (such as chronic neuropathic pain) and cancers (such as prostate cancer, melanoma, multiple myeloma, or glioblastoma).

The FKBP51 ligand compounds of the present invention are preferably suitable for treatment, or for the preparation of a pharmaceutical formulation for prophylaxis and treatment of psychiatric diseases. It is especially preferred if these psychiatric diseases are an affective disorder (ICD-10 classification: F30-F39) or an anxiety disorder.

Affective disorder is a mental disorder characterized by dramatic changes or extremes of mood.

The affective disorder according to the invention is selected from the group comprising or consisting of depression, bipolar disorder, mania, substance induced mood disorder and seasonal affective disorder (SAD). Among the psychiatric diseases and disorders, the most preferred is depression, the most commonly diagnosed psychiatric disorder.

The anxiety disorder according to the invention is selected from the group comprising or consisting of generalized anxiety disorder, panic disorder, panic disorder with agoraphobia, phobias, obsessive-compulsive disorder, post-traumatic stress disorder, separation anxiety and childhood anxiety disorders.

Among the hundreds of different neurodegenerative disorders, the attention has been given only to a handful, including Alzheimer's Disease, Parkinson's Disease, and amyotrophic lateral sclerosis.

Among the glucocorticoid hyposensitivity syndromes, the attention has been given to the group of related diseases enclosing resistant asthma, eosinophilic esophagitis, AIDS, rheumatoid arthritis, hypertension and diabetes, metabolic syndrome or obesity.

Among the cancers, the attention has been given to malignant melanoma, acute lymphoblastic leukaemia, gliomas, idiopathic myelofibrosis, pancreatic and breast cancers, steroid-hormone dependent cancers or prostate cancer, multiple myeloma.

Among the metabolic disorders, attention has been given to obesity and type 2 diabetes.

Among the neurological disorders, attention has been given to neuropathic pain and fibromyalgia.

Among the haematological disorders, attention has been given to hereditary haemorrhagic telangiectasia or pulmonary arterial hypertension.

Said pharmaceutical compositions may further comprise at least one FKBP ligand of the general formula **1.**

The pharmaceutical compositions may further comprise at least one further active agent. It is preferred if this active agent is selected from the group consisting of anti-depressant and other psychotropic drugs. It is further preferred if the anti-depressant is selected from amitriptyline, aminoxide clomipramine, doxepin, duloxetine, imipramine trimipramine, mirtazapine, reboxetine, citalopram, fluoxetine, moclobemide and sertraline.

### Molecular Glues

A further aspect of the invention is to provide compounds suitable as molecular glues. Molecular glues work by binding to a specific site on one protein, which then creates or enhances a binding surface for a second protein. This interaction can lead to a functional consequence, such as protein degradation, stabilization, or modulation of protein activity.

### Definitions

FKPB (FK506-binding protein) ligands are a class of organic compounds that bind to FKBPs or inhibit the activity of FKPB proteins. FKPB proteins are involved in various cellular processes, including immune response regulation. Enantiomers of FKPB ligands may have different effects on the binding affinity and activity of these proteins due to their mirrored structures, potentially resulting in differences in biological activity, pharmacokinetics, and toxicity. Therefore, it is important to consider the stereochemistry of FKPB ligands when studying their properties and effects.

Stereoisomeric forms of an organic compound, including FKPB ligands, refer to different spatial arrangements of atoms within molecules that result in distinct structural forms but have the same molecular formula and connectivity of atoms. Stereoisomers arise due to differences in the spatial orientation of atoms or groups around one or more stereo-centres within a molecule.

In the case of FKPB ligands, which are organic compounds designed to inhibit FK506-binding proteins, stereoisomeric forms may include:
An "enantiomer", in the context of the present disclosure, refers to one of a pair of molecules that are mirror images of each other but cannot be superimposed onto one another. Enantiomers have identical physical and chemical properties in an achiral environment but can exhibit different interactions in chiral environments, such as biological systems. This is because biological systems often contain chiral molecules or receptors, and enantiomers can interact differently with these systems due to their mirrored structures.

"Diastereoisomers" as disclosed herein are a type of stereoisomers that are not mirror images of each other and exist when a molecule has two or more chiral centres but differs in configuration at some, but not all, of these centres. In other words, diastereoisomers have different spatial arrangements of atoms around at least one chiral centre but share the same configuration at others.

"Anomers" as disclosed herein are a specific type of stereoisomers that occur in cyclic forms of carbohydrates, particularly when they undergo intramolecular cyclization to form hemiacetals or hemiketals. Anomers differ from one another in the configuration around the anomeric carbon, which is the carbon atom that becomes a new chiral centre during the cyclization process. Thus, if an FKPB-ligand were to undergo cyclization reactions involving a hemiacetal or hemiketal formation, it might produce anomeric forms if it contained a chiral centre at the site of cyclization.

"Prodrugs" are defined herein as inactive or less active forms of drugs that are designed to undergo metabolic or chemical transformation in the body to become active pharmacological agents. They are typically synthesized by chemically modifying the parent drug molecule to render it inactive or less active. Once administered, prodrugs undergo specific enzymatic or chemical reactions, such as hydrolysis, oxidation, or reduction, to release the active drug moiety.

In the context of FKPB ligands, prodrugs could be designed to improve pharmacokinetic properties, enhance bioavailability, or reduce toxicity. For example, if a particular FKPB ligands has poor oral bioavailability due to rapid metabolism or poor absorption, a prodrug form could be developed that is more readily absorbed or metabolized into the active form in vivo. Similarly, prodrugs could be designed to target specific tissues or cells more effectively or to prolong the duration of action of the active drug.

Overall, prodrugs offer a strategy to optimize the therapeutic properties of FKPB ligands and other organic compounds by modifying their chemical structure to improve pharmacokinetics, bioavailability, and target specificity.

In the context of this disclosure, "deoxy-forms" typically refer to molecules that lack one or more oxygen atoms compared to their corresponding fully oxygenated counterparts. The prefix "deoxy-" indicates the removal or absence of oxygen. It includes modifications to the structure of the ligand involving the removal of oxygen-containing functional groups in order to alter the pharmacological properties of the compound, such as its potency, selectivity, or metabolic stability.

In the context of this disclosure, a "racemate" (or racemic mixture) refers to a mixture of equal amounts of two enantiomers of a chiral compound. Enantiomers are mirror-image isomers of each other that cannot be superimposed onto each other. When a chiral compound exists as a racemate, it means that both enantiomers are present in equal proportions, resulting in no overall optical activity (no net rotation of plane-polarized light). In the context of FKBP ligands or any other organic compound, if the compound contains one or more chiral centres and can exist as enantiomers, a racemate would consist of an equal mixture of both enantiomers. Racemates are commonly encountered in organic chemistry, and they may exhibit properties that are intermediate between those of the individual enantiomers. However, it's essential to note that racemates may also have different pharmacological properties compared to their individual enantiomers due to interactions with chiral biological receptors or enzymes.

In this disclosure, a "tautomer" refers to one of two or more isomeric forms of a compound that readily interconvert through a chemical reaction known as tautomerization. Tautomerization involves the migration of a proton and the rearrangement of bonds within the molecule, resulting in a structural change. The two primary types of tautomers are keto-enol tautomers and aldehyde-ketone tautomers, although other types also exist. Keto-enol tautomerization involves the reversible interconversion between a keto form (containing a carbonyl group) and an enol form (containing a hydroxyl group attached to a carbon-carbon double bond). Aldehyde-ketone tautomerization involves the reversible interconversion between an aldehyde and a ketone, typically involving an intramolecular proton transfer. Tautomerism can affect the chemical and biological properties of FKPB ligands, potentially influencing factors such as solubility, reactivity, and binding affinity to target proteins.

In this context, a "solvate" refers to a type of solid compound formed when solvent molecules are incorporated into the crystal lattice of a solid organic molecule. Solvates are formed through the interaction between solvent molecules and the solute molecules during the crystallization process. Solvates can be categorized based on the type of interaction between the solvent molecules and the solute molecules. Common types of solvates include:
Solvates with solvent molecules physically trapped within the crystal lattice without any significant chemical interaction with the solute molecules. These solvates are often referred to as nonstoichiometric solvates.

Solvates where solvent molecules form specific interactions (such as hydrogen bonds or dipoledipole interactions) with functional groups on the solute molecules. In these cases, the solvate may contain a defined ratio of solvent molecules to solute molecules, resulting in a stoichiometric solvate.

"Pharmaceutically acceptable salts" refer herein to salts of organic compounds that are suitable for pharmaceutical use. In the context of FKBP-ligands or any other pharmaceutical compound, these salts are formed by reacting the active pharmaceutical ingredient (API) with an appropriate acid or base to produce a salt that is pharmaceutically acceptable.

The choice of counterion (the ion that pairs with the charged API to form the salt) is critical in producing a pharmaceutically acceptable salt. Common counterions used to form salts include inorganic acids (e.g., hydrochloric acid, sulfuric acid) for basic drugs and inorganic bases (e.g., sodium hydroxide, potassium hydroxide) for acidic drugs. However, organic acids and bases can also be used to form salts.

The formation of pharmaceutically acceptable salts can offer several advantages, including:
- Improved solubility: Salt formation can enhance the solubility of the API, which can be particularly beneficial for poorly water-soluble compounds, thereby improving bioavailability.
- Stability: Salt forms of APIs can exhibit improved stability, both in solid-state and in solution, compared to the free base or acid forms.
- Dosing flexibility: Different salts of the same API may have different physicochemical properties, allowing for flexibility in formulation and dosing.
- Taste masking: Some salts may have a more favourable taste profile than the parent compound, making them more palatable for oral administration.

"Molecular glue" as used herein refers to the small molecules of the present disclosure that possess the ability to promote interactions between proteins or other biomolecules that do not typically interact under normal cellular conditions. These molecules act as bridges or facilitators, bringing together proteins that may not naturally bind to each other, thereby influencing various cellular processes. Molecular glues have potential applications in modulating protein-protein interactions (PPI) and developing novel therapeutic agents. They can be utilized to target specific proteins involved in diseases such as cancer, neurodegenerative disorders, and infectious diseases.

In one embodiment any designated substituent, such as R¹, R², R³, ... (or any other number) can only be one specific and selected substituent within in one compound. Thus, R¹ cannot represent in the same compound two different substituents such as -CH₃ and -COOH.

### Description of the Figures

**Figure 1****-** Linear starting point with a binding affinity for FKBP51 of 2.8 nM.
**Figure 2****-** Generic scheme for macrocycle assembling.
**Figure 3** - Overview of the synthesized A building blocks A1-A11
**Figure 4** Overview of the used B building blocks B1-B18. B3 and B4 were synthesized according to literature: Bauder et al: J. Med. Chem. 2021, 64, 3320-3349.

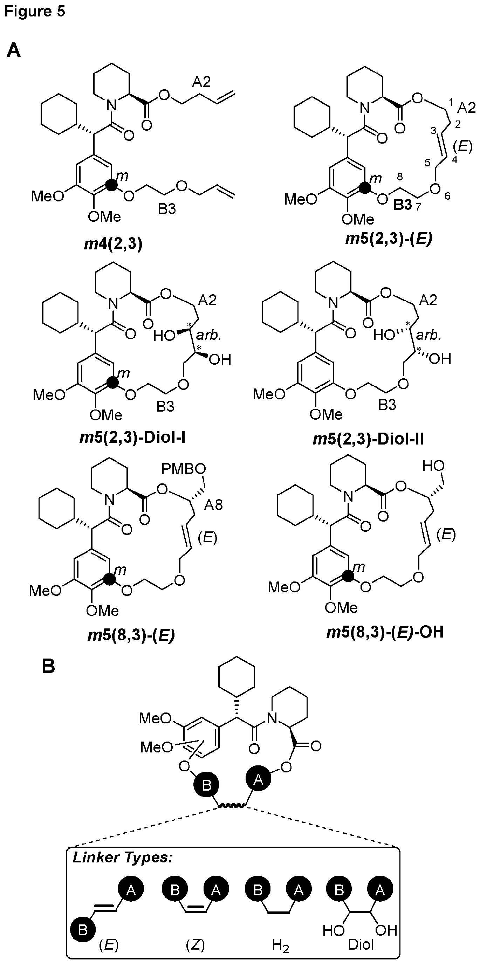

**Figure 5** **A** Examples for compound nomenclature and linker atom numbering based on macrocycles with functionalized and non-functionalized linkers and for an exemplary acyclic precursor. The names of macrocycles and intermediates start with the used exit vector (para: p; meta: m) followed by an arabic number referring to the stage in the reaction sequence (e.g. m5). The building blocks used are specified in the brackets with the number of the A building block (e.g. A2) first and the number of the used B building block (e.g. B3) second (e.g. (m4(2,3)). If the described compound is a macrocycle the last part of its name comes from the respective linker type (e.g. m5(2,3)-(E)). Diastereomers obtained from dihydroxylation were indicated additional roman numbers (e.g. m5(2,3)-Diol-I and II, stereochemistry arbitrarily assigned.). For deprotected alcohols, the name is extended by OH (e.g. m5(8,3)-(E) and m5(8,3)-(E)-OH). **B:** Summary of the linker types based on double bond configuration or post-macrocyclic derivatization.

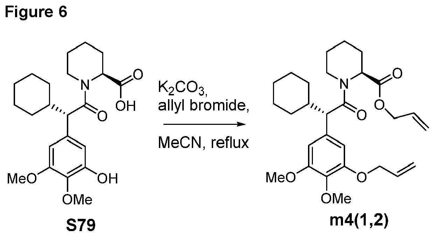

**Figure 6****:** Synthesis of m4(1,1) by an alternative route starting from S79, which was synthesized according to literature: Bauder et al: J. Med. Chem. 2021, 64, 3320-3349.

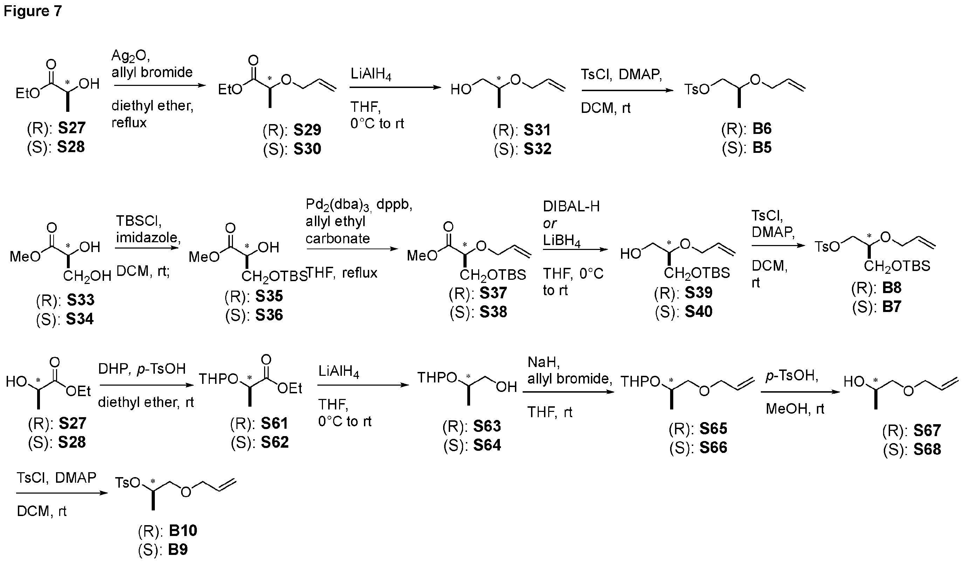

**Figure 7** Scheme for the synthesis of the functionalized linker building blocks B5-B10 for the formation of the bottom part of the macrocyclic linker moiety in macrocycles with 8-membered linkers.

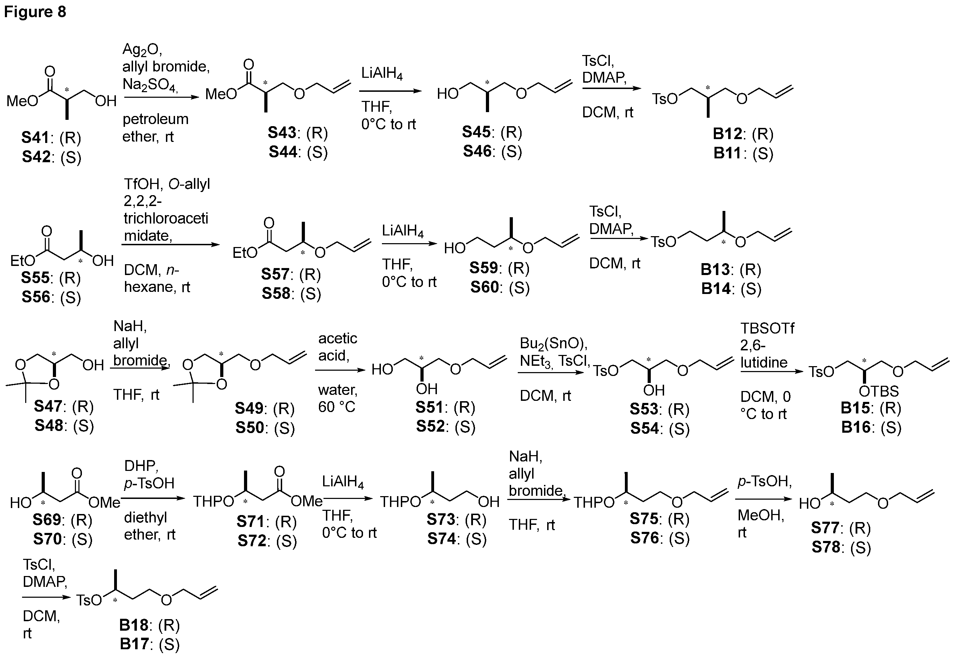

**Figure 8** Scheme for the synthesis of the functionalized linker building blocks B11-B18 for the formation of the bottom part of the macrocyclic linker moiety in macrocycles with 9-membered linkers.

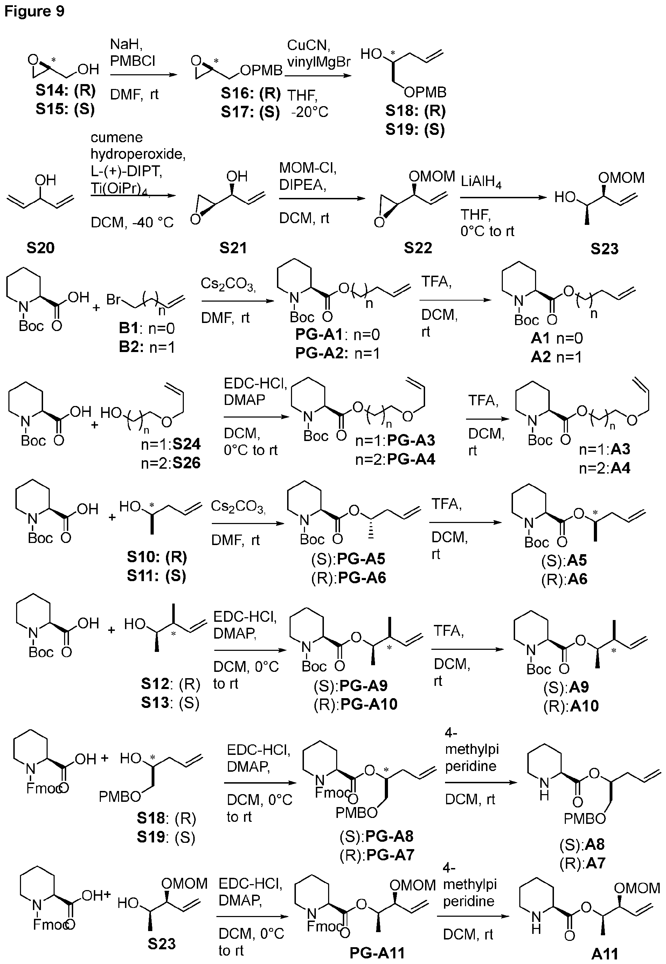

**Figure 9** Scheme for the synthesis of the unfunctionalized linker building blocks A1-4 and the functionalized linker building blocks A5-11 for the formation of the top part of the macrocyclic linker moiety in macrocycles with 8- and 9-membered linkers.

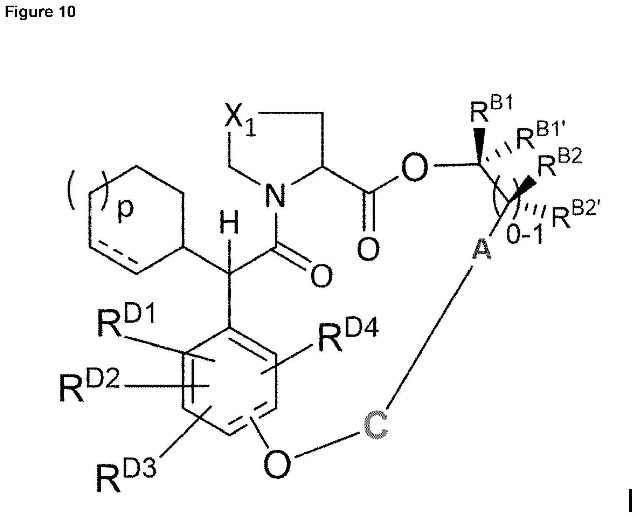

**Figure 10** Markush-formula (formula I) of new compounds according to the disclosure.

### Examples

### Example 1: New Linker Design

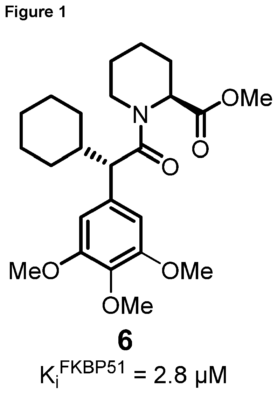

As a starting point compound **6** which represents the minimal scaffold for selective FKBP51 binding (Figure 1).

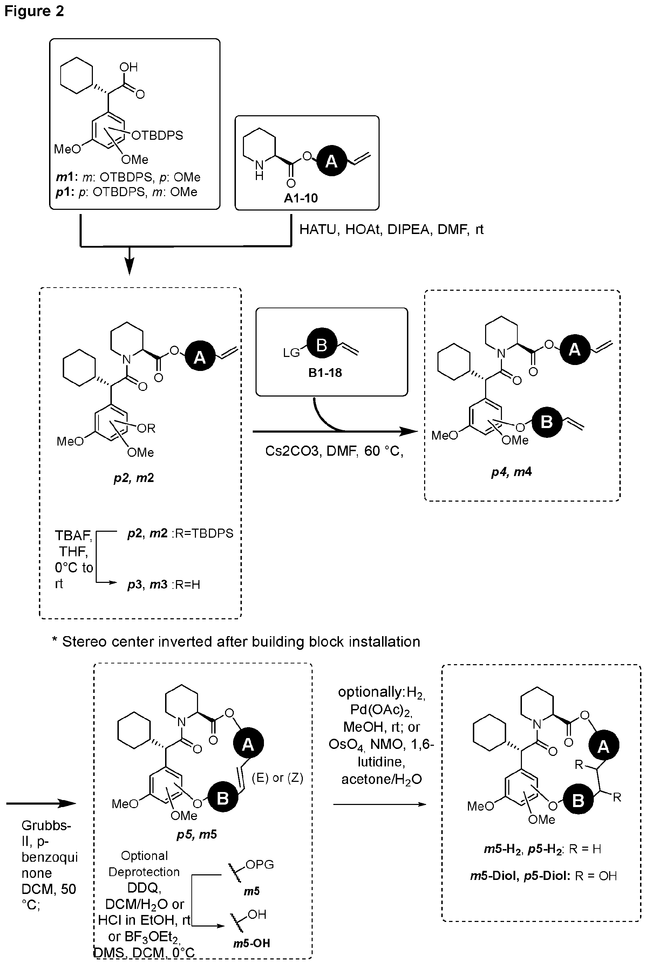

To systematically explore the linker, a build-couple-pair strategy was applied (Figure 2) which allowed the efficient incorporation of linkers differing in length, rigidity, and polarity. This approach was based on two core building blocks (para: p-1 and meta: m-1 (for m1 synthesis shown in scheme below) that differed in the position of the TBDPS-protected phenol and provided two different exit vectors for the attachment of the linker. 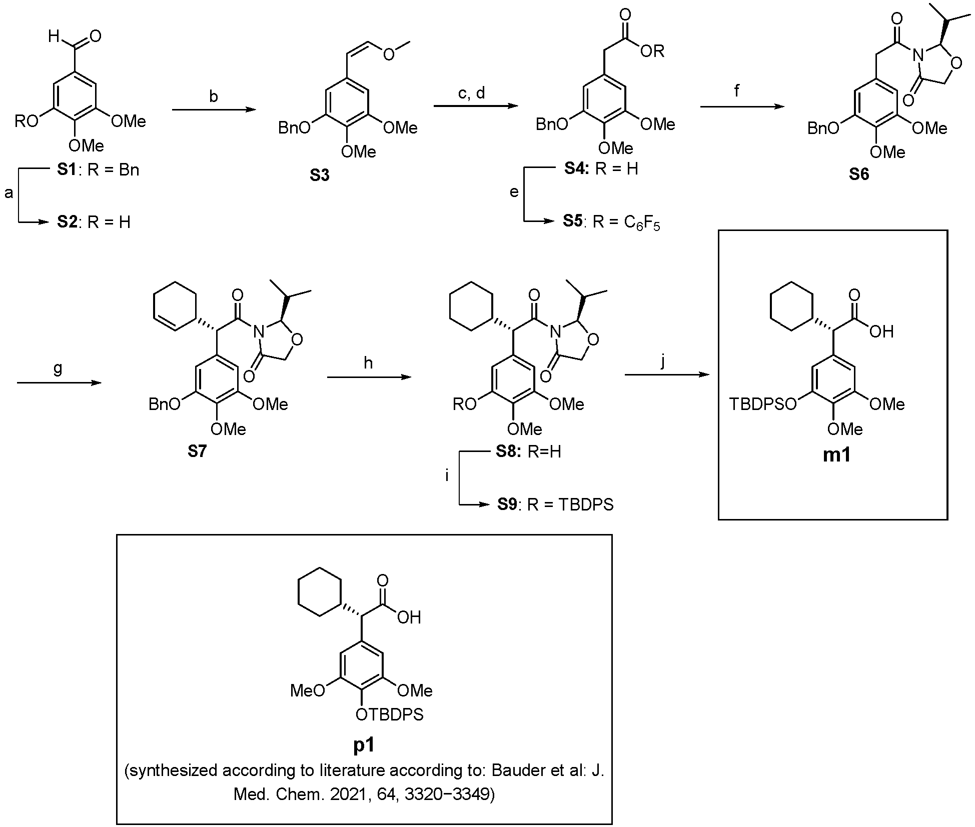 *^{a} reagents and conditions:(a) BnBr, MeCN, reflux,* 96 %; *(b) Ph₃P*=*CH(OMe), t-BuOK, THF 0* °C *to rt, 83 %; (c) (COCl)₂, H₂O, HCl, rt, no purification (d) Jones reagent, acetone, 0°C,* 75 % *over two steps; (e) pentafluorophenol, EDC-HCl, DMAP, DCM, rt, 77* %; *(f) (S)-4-isopropylyloxazolidin-2-one, n-BuLi, THF, 0* °C - *rt, 82 %; (g) 3-bromocyclohexene, NaHMDS, THF,* -78 °C - *rt, 68%; (h) H₂ [Pd*/*C], THF*/*MeOH, rt (quant.); (i) TBDPSCI, imidazole, DCM*/*DMF, rt,* 88 %; *(j) LiOH, H₂O₂, THF*/*H₂O, rt,* %; 51 % + 15 % *recovered starting material.*

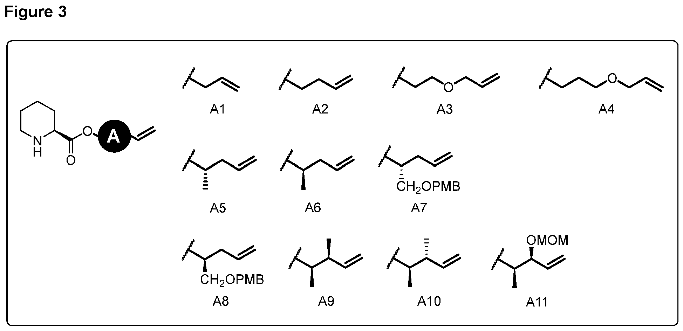

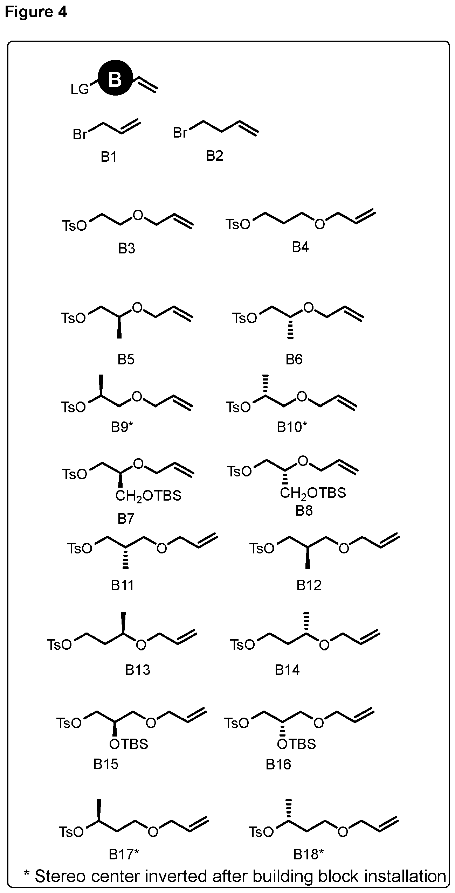

In the first step, the respective core structures were coupled to an A building block represented by a pipecolic acid ester bearing a terminal alkene (A1-10). After TBAF-mediated deprotection of the phenol a B building block (B1-14) was installed by a SN reaction. For both A and B building blocks several derivatives with a variety of small substituents were synthetically amenable (Figure 3 and Figure 4).

After the coupling phase the macrocycles were formed by ring-closing metathesis (RCM) giving preferably the (E)-isomer. For a subseries the resulting double bond was further modified by hydrogenation or dihydroxylation to obtain more polar or less polar and more flexible analogs, respectively. For macrocycles with a pre-installed protected alcohol, a subsequent deprotection step was included.

### Example 2a: Synthesis - General Information Chemistry

All chemicals and solvents from commercial sources (Sigma Aldrich, abcr, BLD Pharm, Carl Roth, Fluorochem, aaron chemicals, ChemScene) were used without further purification. If not otherwise noted, all chemical reactions were performed in dry glassware under an argon atmosphere and with anhydrous solvents unless water was present in the reaction mixture. Reactions were monitored by thin-layer chromatography (TLC), analytical high performance liquid chromatography mass spectrometry (LC-MS)

Analytical LC-MS measurements were conducted using an Agilent 1260 Infinity II system, which includes a 1260 Infinity II flexible pump, a vial sampler, and a multicolumn thermostat equipped with either a Poroshell 120 EC-C18 column (3 mm × 150 mm, 2.7 µm) or a Poroshell 120 EC-C18 column (50 mm × 2.1 mm, 1.9 µm). The system also features a diode array detector connected to a 6125B MSD single quadrupole detector.

For all analytical and preparative chromatographic methods, the detailed parameters are described in the analytical section of the individual compounds.

¹H spectra were recorded at the NMR facility at the department of chemistry at Technische Universität Darmstadt (TUD) using a Bruker AC 300, AR300 or DRX500. Chemical shifts for ¹H and ¹³C are reported in ppm (*δ*). Deuterated chloroform (CDCl₃) or acetonitrile (MeCN-*d*₃) were used as solvents, and the spectra were calibrated to their respective peaks. Multiplicities are abbreviated as follows: singlet (s), doublet (d), triplet (t), quartet (q), doublet of doublets (dd), doublet of doublets (ddd), doublet of triplets (dt), multiplet (m). Only the major peaks were reported in the case of rotamers and diastereomers.

High-resolution mass spectra were recorded by the Mass Spectrometry Department of the Technical University of Darmstadt on a Bruker Daltonics Impact II mass spectrometer (quadrupole time-of-flight).

### Biochemistry

### Fluorescence polarization assay (FP assay)

The competitive fluorescence polarization assay was conducted as described by Kozany *et al.*¹

### NanoBRET assay

The NanoBRET assay was performed as described by Gnatzy *et al*.²

### Example 2b: Synthesis Procedures General Procedures

### General Procedure A (amide coupling)

To a 0.1 M solution of the carboxylic acid (1.0 eq.) in DMF were added HATU (1.3 eq.) and HOAt (1.3 eq.). While stirring, DIPEA (3.0 eq.) was added followed by the amine (1.3 eq.). The reaction mixture was stirred overnight. The reaction solution was diluted with ethyl acetate and the organic phase is washed with 1M HCl, then three times with brine (3x). Combined organic layers were dried with MgSO₄ and volatiles were removed under reduced pressure. The crude product was purified by flash column chromatography.

### General Procedure B (TBDPS deprotection)

A 0.1 M solution of the TBDPS-protected phenol (1.0 eq.) in anhydrous THF. While stirring, TBAF (1.1 eq.) was added at 0°C. The solution was stirred at room temperature for 15 minutes. The reaction solution was quenched by the addition of water. The aqueous phase was extracted with ethyl acetate. Combined organic layers were washed with brine, dried with MgSO₄ and volatiles were removed under reduced pressure. The crude product was purified by flash column chromatography.

### General Procedure C (phenol alkylation)

To a solution of the phenol in DMF or acetonitrile (0.1 M). While stirring, cesium carbonate or potassium carbonate (3.0 eq) was added, followed by the bromide or to sylate and the reaction mixture was either heated to 60 °C (DMF as solvent) or to reflux (acetonitrile as solvent) and stirred overnight. Solids were filtered off, washed with ethyl acetate and volatiles were removed under reduced pressure. The crude product was purified by flash column chromatography or preparative HPLC.

### General Procedure D (ring-closing metathesis)

The dialkene substrate was dissolved in anhydrous DCM (5 mM). After the addition of Grubbs II catalyst (0.1 eq.) and *p*-benzoquinone (0.1 eq.) were added to the reaction mixture was heated to reflux (50 °C) and stirred for 1 h at this temperature. The blue reaction solution is quenched by the addition of Tris(hydroxymethyl)phosphine (THMP) (1 M in *i*-PrOH, 5.0 eq.) and stirred at room temperature for 30 minutes. The organic phase is washed with brine and the aqueous phase is extracted with DCM. Combined organic layers were dried with MgSO₄ and volatiles were removed under reduced pressure. The crude product was purified by flash column chromatography or preparative HPLC.

### General Procedure E (dihydroxylation)

The alkene was dissolved in acetone. After cooling to 0°C, *N*-methylmorpholine *N*-oxide (NMO) was added followed by 1,6-lutidine (optional), osmiumtetroxide solution (2.5 wt% in *t*-BuOH) and water. The reaction mixture was then allowed to reach ambient temperature and was stirred at room temperature overnight. The reaction solution is quenched by the addition of sat. Thiosulfate solution. DCM is added and the aqueous phase is extracted with DCM. Combined organic layers were dried with MgSO₄ and volatiles were removed under reduced pressure. The crude product was purified by flash column chromatography or preparative HPLC.

### General Procedure F (alkene hydrogenation 1)

A stirred solution of the alkene in a mixture of THF and MeOH (v/v = 1:1, 2 mL) was degassed by sparging with argon for 10 min. After the addition of Wilkinson's catalyst (RhCl(PPh₃)₃, 0.5 eq.) the solution was sparged with hydrogen for 15 min and was then stirred under a hydrogen atmosphere at room temperature. After completion the solvent was removed under reduced pressure. The crude product was purified by flash column chromatography or preparative HPLC.

### General Procedure G (alkene hydrogenation 2)

Charcoal (90 wt%/Pd) and Pd(OAc)₂ (0.05 M in THF) were added to a stirred solution of the alkene in MeOH. The solution was purged with hydrogen for 10 minutes and was then stirred under a hydrogen atmosphere at room temperature. After completion the reaction mixture was filtered and concentrated under reduced pressure. The crude product was purified by flash column chromatography or preparative HPLC.

### General Procedure H (TBS deprotection)

The TBS-protected alcohol was dissolved in EtOH. 6 M aq. HCl was added. The resulting solution was stirred at room temperature overnight. Volatiles were removed under reduced pressure and the resulting crude product was purified by flash column chromatography or preparative HPLC.

### General Procedure I (PMB deprotection)

The PMB-protected alcohol was dissolved in a mixture of DCM and water (v/v = 100:2), then DDQ was added and the resulting mixture was stirred at room temperature. The reaction was quenched by the addition of saturated aqueous NaHCO₃ solution. The aqueous phase was extracted with DCM. Combined organic layers were dried with MgSO₄, filtered and concentrated under reduced pressure. The crude product was purified by flash column chromatography or preparative HPLC.

### General Procedure J (MOM deprotection)

To the MOM-protected alcohol was dissolved in DCM dimethyl sulfide (DMS) and boron trifluoride diethyl etherate were added at 0 °C. The reaction mixture was stirred at 0 °C. After completion the reaction was quenched with saturated aqueous NaHCO₃ solution. Phases were separated and the aqueous phase was extracted with DCM. Combined organic layers were washed with brine, dried with MgSO₄ filtered and concentrated under reduced pressure. The crude product was purified by preparative HPLC.

### General Procedure K (ester coupling)

The alcohol (1.0 eq.), the carboxylic acid (1.2 eq.) and DMAP (0.4 eq) were dissolved in anhydrous DCM (c(alcohol)=0.1 M). The resulting solution was cooled to 0°C and stirred for 10 minutes at this temperature. EDC-HCl (1.5 eq.) was added portion wise at 0°C and the reaction mixture was stirred for 60 minutes at 0°C before it was allowed to reach ambient temperature overnight. The reaction was quenched by the addition of brine. The aqueous phase was extracted with DCM three times. Combined organic layers were washed with saturated aqueous NH₄Cl solution, then with saturated NaHCO₃ solution, dried with MgSO₄, filtered and concentrated under reduced pressure. The title compound was obtained after flash column chromatography.

### General Procedure L (Boc deprotection)

A solution of the Boc-protected amine(1.0 eq.) in DCM (0.1 M) was treated with TFA (15.0 eq). The resulting solution was stirred at room temperature for 1 h. Saturated NaHCO₃ was added to the reaction mixture until a pH of >7 was reached. The aqueous phase was extracted with DCM. Combined organic layers are washed with saturated aqueous NH₄Cl solution, then with saturated NaHCO₃ solution, dried with MgSO₄, filtered and concentrated under reduced pressure.

### General Procedure M (Fmoc deprotection)

A solution of the Fmoc-protected amine (1.0 eq.) was dissolved in a mixture of 4-methylpiperidine and DCM (v/v =1:10, 0.1 M), and the mixture was stirred at room temperature for 2 h. The solvent was removed under reduced pressure and the crude product directly subjected to flash column chromatography.

### General Procedure N (tosylation)

Triethylamine (3.0 eq.), DMAP (0.20 eq.) and tosyl chloride (.6 eq.) were added sequentially to a solution of a 0.1 M solution of an alcohol (1.0 eq.) in anhydrous DCM. The resulting mixture was stirred at room temperature overnight. The reaction was quenched by the addition 3-(dimethylamino)-1-propylamine (2.0 eq.) and stirred for further 30 minutes. The mixture was diluted with DCM and washed three times with 1M HCl, sat. NaHCO₃, and brine. Organic layer was dried with MgSO₄, filtered and concentrated under reduced pressure. The crude product was purified by flash column chromatography.

### Example 2c: Synthesis of A-Building Blocks

### 3-(allyloxy)propan-1-ol (S26)

### Synthesized as previously reported by Bauder et al.³

### (R)-2-(((4-methoxybenzyl)oxy)methyl)oxirane (S16)

To a suspension of NaH (60% in mineral oil, 713 mg, 17.8 mmol, 1.1 eq.) in DMF (32 mL) cooled to 0 °C was added *p*-methoxybenzyl chloride (2.50 mL, 17.8 mmol, 1.1 eq.) dropwise. The mixture was stirred for 10 min at 0 °C after which (S)-glycidol (1.20 g, 16.2 mmol) was added dropwise via syringe. The mixture was then allowed to warm to room temperature and stirred overnight. The reaction mixture was poured into a separating funnel containing NH₄Cl and ethyl acetate. The layers were separated and the organic layer washed with 10% aqueous NaHCO₃ and water. The combined aqueous layers were then further extracted with ethyl acetate and the combined organic layers dried with MgSO₄ and concentrated *in vacuo.* The title compound was obtained after flash column chromatography (0 - 50 % EA in CH) as a colorless oil (2.07 g, 66 %). **¹H NMR** (500 MHz, CDCl₃): δ 7.34 - 7.27 (m, 2H), 6.93 - 6.87 (m, 2H), 4.56 (d, J = 11.5 Hz, 1H), 4.51 (d, J = 11.5 Hz, 1H), 3.85 - 3.80 (m, 3H), 3.75 (ddd, J = 11.5, 3.1, 1.0 Hz, 1H), 3.43 (ddd, J = 11.5, 5.8, 1.1 Hz, 1H), 3.19 (ddtd, J = 5.7, 4.0, 2.9, 1.0 Hz, 1H), 2.81 (ddd, J = 5.2, 4.1, 1.1 Hz, 1H), 2.62 (ddd, J = 5.1, 2.7, 1.1 Hz, 1H) ppm. **MS** (m/z): (ESI⁺) calculated for: C₁₁H₁₄O₃N₈ [M+Na]⁺: 217.1, found: 217.2

### (S)-2-(((4-methoxybenzyl)oxy)methyl)oxirane (S17)

Synthesis as shown for **S16** with (R)-glycidol as the substrate. **Yield:** 2.17 g, 55 %, colorless oil. **¹H NMR** (500 MHz, CDCl₃): δ 7.32 - 7.22 (m, 2H), 6.91 - 6.85 (m, 2H), 4.55 (d, J = 11.5 Hz, 1H), 4.48 (d, J = 11.5 Hz, 1H), 3.80 (s, 3H), 3.75 - 3.70 (m, 1H), 3.41 (ddd, J = 11.4, 5.9, 1.0 Hz, 1H), 3.22 - 3.13 (m, 1H), 2.83 - 2.76 (m, 1H), 2.64 - 2.56 (m, 1H) ppm. **MS** (m/z): (ESI⁺) calculated for: C₁₁H₁₄O₃Na [M+Na]⁺: 217.1, found: 217.0

### (R)-1-((4-methoxybenzyl)oxy)pent-4-en-2-ol (S18)

A suspension of epoxide **S16** (2.07 g, 10.7 mmol, 1.0 eq.) and CuCN (193 mg, 2.13 mmol, 0.2 eq) in dry THF (105 mL, 0.1 M) was cooled to -20°C and vinylmagnesium bromide (1 M in THF, 16.0 mL, 16.0 mmol, 1.5 eq) was added dropwise. After 1 h at -20°C, the reaction was quenched by addition of saturated aqueous solution of NH₄Cl and resulting layers were separated. Aqueous layer was extracted with diethyl ether. Combined organic layers were washed with brine, dried with MgSO₄ and evaporated under reduced pressure. The title compound was obtained after flash column chromatography (40 - 100 % EA in CH) as a yellowish oil (2.28 g, 98 %). **¹H NMR** (300 MHz, CDCl₃): δ 7.32 - 7.23 (m, 2H), 6.98 - 6.79 (m, 2H), 5.82 (ddt, J = 17.2, 10.2, 7.1 Hz, 1H), 5.17 - 5.03 (m, 2H), 4.48 (s, 2H), 3.87 (ddt, J = 10.3, 6.7, 3.3 Hz, 1H), 3.80 (d, J = 0.7 Hz, 3H), 3.48 (ddd, J = 9.4, 3.5, 0.7 Hz, 1H), 3.34 (dd, J = 9.5, 7.5 Hz, 1H), 2.39 (s, 1H), 2.25 (t, J = 6.8 Hz, 2H) ppm. **MS** (m/z): (ESI⁺) calculated for: C₁₃H₁₈O₃Na [M+Na]⁺: 245.1, found: 245.2.

### (R)-1-((4-methoxybenzyl)oxy)pent-4-en-2-ol (S19)

Synthesis as shown for **S18** with **S17** as substrate. **Yield:** 2.23 g, 90%, yellowish oil. **¹H NMR** (300 MHz, CDCl₃): δ 7.33 - 7.24 (m, 2H), 6.94 - 6.87 (m, 2H), 5.93 - 5.75 (m, 1H), 5.17 - 5.06 (m, 2H), 4.51 (s, 2H), 3.88 (s, 1H), 3.83 (s, 3H), 3.55 - 3.47 (m, 1H), 3.41 - 3.33 (m, 1H), 2.45 (s, 1H), 2.32 -2.23 (m, 2H) ppm. **MS** (m/z): (ESI⁺) calculated for: C₁₃H₁₈O₃Na [M+Na]⁺: 245.1, found: 245.2

### (1S)-1-(oxiran-2-yl)prop-2-en-1-ol (S21)

### Synthesis according to literature procedure.³

### 2-((S)-1-(methoxymethoxy)allyl)oxirane (S22)

MOMCI (2.66 mL, 31.5 mmol, 5.0 eq.) was added at 0 °C to a stirred solution of **S21** (631 mg, 6.30 mmol, 1.0 eq.) and DIPEA (6.65 mL, 37.8 mmol, 6.0 eq.) in anhydrous DCM (42 mL). The reaction mixture was allowed to reach ambient temperature and was stirred overnight. The reaction was quenched by the addition of saturated aqueous NH₄Cl solution. The aqueous phase was extracted with DCM. Combined organic layers were washed with brine, dried with MgSO₄, filtered and concentrated under reduced pressure. The crude product was purified by flash column chromatography (0 - 40 % diethyl ether in pentane). The title compound was obtained as a colorless oil (700 mg, 77 %). **¹H NMR** (300 MHz, CDCl₃): δ 5.77 (ddd, J = 17.4, 10.4, 7.2 Hz, 1H), 5.41 - 5.26 (m, 2H), 4.70 - 4.65 (m, 1H), 4.62 (d, J = 6.7 Hz, 1H), 4.06 (dd, J = 7.3, 4.2 Hz, 1H), 3.37 (d, J = 0.6 Hz, 3H), 3.06 (td, J = 4.1, 2.7 Hz, 1H), 2.78 (dd, J = 5.2, 3.9 Hz, 1H), 2.71 (dd, J = 5.2, 2.6 Hz, 1H). ppm.

### (2R,3S)-3-(methoxymethoxy)pent-4-en-2-ol (S23)

Lithium aluminium hydride (4 M in Et₂O, 1.18 mL, 4.72 mmol, 1.0 eq) was added to a solution of **S24** (680 mg, 4.72 mmol, 1.0 eq.) in anhydrous Et₂O (31 mL) at 0°C. The mixture was left to stir at 0 °C for 10 minutes. The reaction mixture was diluted with diethyl ether and saturated aqueous Rochelle salt was added. The aqueous phase was extracted with diethyl ether. Combined organic layers were dried with MgSO₄, filtered and concentrated under reduced pressure. The title compound was obtained as a colorless oil (680 mg, 99 %) without further purification. **¹H NMR** (300 MHz, CDCl₃): δ 5.86 - 5.67 (m, 1H), 5.40 - 5.24 (m, 2H), 4.72 (dd, J = 6.7, 0.6 Hz, 1H), 4.60 (d, J = 6.7 Hz, 1H), 3.97 - 3.91 (m, 1H), 3.90 - 3.82 (m, 1H), 3.39 (s, 3H), 2.09 (s, 1H), 1.15 (d, J = 6.4 Hz, 3H) ppm.

### (S)-2-allyl 1-tert-butyl piperidine-1,2-dicarboxylate (PG-A1)

A solution of (*S*)-*N*-Boc-pipecolic acid (2.0 g, 8.73 mmol, 1.0 eq.) and Cs₂CO₃ (3.4 g, 10.5 mmol, 1.2 eq) in DMF (15 mL) was treated with allyl bromide (1.28 g, 10.5 mmol, 1.2 eq.) and the mixture was stirred at room temperature. The mixture was diluted with ethyl acetate, and the organic phase was washed with water and brine, dried with MgSO₄, filtered, and concentrated under reduced pressure. The title compound was obtained without further purification as a yellow oil (2.24 g, 95 %). **¹H NMR** (500 MHz, CDCl₃, mixture of rotamers, major rotamer reported): δ 5.89 (ddt, J = 16.5, 10.8, 5.7 Hz, 1H), 5.31 (d, J = 17.2 Hz, 1H), 5.22 (d, J = 10.5 Hz, 1H), 4.82 (d, J = 86.9 Hz, 1H), 4.68 - 4.58 (m, 2H), 4.10 - 3.86 (m, 1H), 3.10 - 2.82 (m, 1H), 2.46 - 2.07 (m, 1H), 1.76 - 1.53 (m, 3H), 1.53 - 1.31 (m, 10H), 1.29 - 1.16 (m, 1H) ppm.

### (S)-2-but-3-en-1-yl 1-tert-butyl piperidine-1,2-dicarboxylate (PG-A2)

A solution of (*S*)-*N*-Boc-pipecolic acid (1.00 g, 4.36 mmol, 1.0 eq.) and Cs₂CO₃ (1.72 g, 5.24 mmol, 1.2 eq.) in DMF (10 mL) was treated with 4-bromo-1-butene (457 µL, 2.24 mmol, 1.2 eq.) and the mixture was stirred at room temperature. The mixture was diluted with ethyl acetate, and the organic phase was washed with water and brine, dried with MgSO₄, filtered, and concentrated under reduced pressure. The title compound was obtained without further purification as a yellow oil (1.17 g, 95 %). **¹H NMR** (500 MHz, CDCl₃, mixture of rotamers, major rotamer reported): δ 5.84 - 5.71 (m, 1H), 5.20 - 5.00 (m, 2H), 4.99 - 4.62 (m, 1H), 4.33 - 4.08 (m, 2H), 4.08 - 3.85 (m, 1H), 2.91 (dt, J = 50.3, 13.3 Hz, 1H), 2.49 - 2.33 (m, 2H), 2.25 - 2.12 (m, 1H), 1.70 - 1.54 (m, 3H), 1.45 (d, J = 13.6 Hz, 10H), 1.29 - 1.13 (m, 1H) ppm. **MS** (m/z): (ESI⁺) calculated for: C₁₅H₂₅NO₄Na [M+Na]⁺: 306.2, found: 306.2.

### (S)-2-(2-(allyloxy)ethyl) 1-tert-butyl piperidine-1,2-dicarboxylate (PG-A3)

General procedure K was applied to (allyloxy)ethanol and (*S*)-*N*-Boc-pipecolic acid. **Yield:** 1.13 g, 93%, colorless oil.**¹H NMR** (500 MHz, CDCl₃, mixture of rotamers, major rotamer reported): δ 6.05 - 5.81 (m, 1H), 5.33 - 5.26 (m, 1H), 5.23 - 5.17 (m, 1H), 5.03 - 4.68 (m, 1H), 4.37 - 4.26 (m, 2H), 4.10 - 3.86 (m, 3H), 3.66 (t, J = 4.9 Hz, 2H), 3.21 -2.74 (m, 1H), 2.24 (t, J = 16.2 Hz, 1H), 1.74-1.56 (m, 3H), 1.55-1.35 (m, 10H), 1.33-1.19 (m, 1H) ppm.

### (S)-2-(3-(allyloxy)propyl) 1-tert-butyl piperidine-1,2-dicarboxylate (PG-A4)

General procedure K was applied to **S26** and (*S*)-N-Boc-pipecolic acid. **Yield** 1.75 g, 78%, colorless oil. **¹H NMR** (500 MHz, CDCl₃, mixture of rotamers, major rotamer reported): δ 5.91 (ddt, J = 16.4, 10.8, 5.6 Hz, 1H), 5.28 (dt, J = 17.2, 1.7 Hz, 1H), 5.19 (dd, J = 10.4, 1.6 Hz, 1H), 4.81 (d, J = 83.4 Hz, 1H), 4.26 (t, J = 6.6 Hz, 2H), 3.99 - 3.96 (m, 2H), 3.51 (t, J = 6.2 Hz, 2H), 2.92 (dt, J = 52.9, 12.9 Hz, 1H), 1.94 (p, J = 6.4 Hz, 2H), 1.79 - 1.60 (m, 4H), 1.56 - 1.38 (m, 12H) ppm.

### (S)-1-tert-butyl 2-((S)-pent-4-en-2-yl) piperidine-1,2-dicarboxylate (PG-A5)

General procedure K was applied to (*S*)-pent-4-en-2-ol and (*S*)-*N*-Boc-pipecolic acid. **Yield:** 921 mg, 78 %, colorless oil. **¹H NMR** (500 MHz, CDCl₃, mixture of rotamers, major rotamer reported): δ 5.88 - 5.63 (m, 1H), 5.14 - 4.95 (m, 3H), 4.92 - 4.59 (m, 1H), 4.11 - 3.84 (m, 1H), 3.04 - 2.80 (m, 1H), 2.38 - 2.23 (m, 2H), 2.22 - 2.09 (m, 1H), 1.72 - 1.55 (m, 3H), 1.50 - 1.39 (m, 10H), 1.26 - 1.21 (m, 4H) ppm.

### (S)-1-tert-butyl 2-((R)-pent-4-en-2-yl) piperidine-1,2-dicarboxylate (PG-A6)

General procedure K was applied to (*S*)-pent-4-en-2-ol and (*S*)-*N*-Boc-pipecolic acid. **Yield:** 908 mg, 72 %, colorless oil. **¹H NMR** (500 MHz, CDCl₃, mixture of rotamers, major rotamer reported): δ 5.85 - 5.67 (m, 1H), 5.17 - 4.92 (m, 3H), 4.65 (d, J = 5.8 Hz, 1H), 4.03 - 3.95 (m, 1H), 2.85 (t, J = 13.2 Hz, 1H), 2.43 - 2.24 (m, 2H), 2.24 - 2.11 (m, 1H), 1.71 - 1.60 (m, 3H), 1.48 - 1.31 (m, 11H), 1.25 - 1.15 (m, 4H) ppm. **MS** (m/z): (ESI⁺) calculated for: C₁₆H₂₇NO₄Na [M+Na]⁺: 320.2, found: 320.2

### (S)-1-((9H-fluoren-9-yl)methyl) 2-((R)-1-((4-methoxybenzyl)oxy)pent-4-en-2-yl) piperidine-1,2-dicarboxylate (PG-A7)

General procedure K was applied to **S18** and (*S*)-*N*-Fmoc-pipecolic acid. **Yield:** 1.21 g, 94%. **¹H NMR** (500 MHz, CDCl₃, mixture of rotamers, major rotamer reported): δ 7.77 (t, J = 6.5 Hz, 2H), 7.62 (t, J = 6.7 Hz, 2H), 7.45 - 7.14 (m, 6H), 6.86 (d, J = 8.4 Hz, 2H), 5.79 - 5.60 (m, 1H), 5.24 - 5.12 (m, 1H), 5.10 - 4.95 (m, 3H), 4.52 - 4.01 (m, 6H), 3.78 (s, 3H), 3.55 - 3.47 (m, 2H), 3.25 - 3.12 (m, 1H), 2.35 (ddt, J = 28.4, 20.5, 10.7 Hz, 3H), 1.76 - 1.60 (m, 3H), 1.37 (d, J = 39.5 Hz, 2H) ppm. **MS** (m/z): (ESI⁺) calculated for: C₂₄H₃₅NO₆Na [M+Na]⁺: 456.2, found: 456.2

### (S)-1-((9H-fluoren-9-yl)methyl) 2-((S)-1-((4-methoxybenzyl)oxy)pent-4-en-2-yl) piperidine-1,2-dicarboxylate (PG-A8)

General procedure K was applied to **S19** and (*S*)-*N*-Fmoc-pipecolic acid. **Yield:** 1.83 g, 73 %, colorless oil. **¹H NMR** (300 MHz, CDCl₃, mixture of rotamers, major rotamer reported): δ 7.82 - 7.73 (m, 2H), 7.64 - 7.48 (m, 2H), 7.45 - 7.36 (m, 2H), 7.36 - 7.27 (m, 2H), 7.24 - 7.13 (m, 2H), 6.87 - 6.78 (m, 2H), 5.84 - 5.64 (m, 1H), 5.24 - 5.14 (m, 1H), 5.13 - 5.00 (m, 2H), 4.98 (d, J = 5.3 Hz, 0H), 4.52 - 4.00 (m, 8H), 3.78 (s, 3H), 3.54 - 3.39 (m, 2H), 3.18 (t, J = 12.8 Hz, 0H), 2.37 (dtd, J = 28.0, 13.8, 5.2 Hz, 3H), 1.80 - 1.61 (m, 3H), 1.41 (p, J = 14.9, 14.1 Hz, 2H) ppm. **MS** (m/z): (ESI⁺) calculated for: C₃₄H₃₇NO₆Na [M+Na]⁺: 578.3, found: 578.2

### (S)-1-tert-butyl 2-((2R,3S)-3-methylpent-4-en-2-yl) piperidine-1,2-dicarboxylate (PG-A9)

General procedure K was applied to (2*R*,3*S*)-3-methylpent-4-en-2-ol and (*S*)-*N*-Boc-pipecolic acid. **Yield:** 1.14 g, quant., colorless oil. **¹H NMR** (500 MHz, CDCl₃, mixture of rotamers, major rotamer reported): δ 5.71 (ddd, *J* = 16.0, 10.9, 8.2 Hz, 1H), 5.07 - 5.01 (m, 2H), 4.92 (dt, *J* = 12.6, 6.3 Hz, 1H), 4.76 (dd, *J* = 85.0, 5.6 Hz, 1H), 4.06 - 3.87 (m, 1H), 2.92 (dt, *J* = 33.6, 13.2 Hz, 1H), 2.38-2.29 (m, 1H), 2.26 - 2.13 (m, 1H), 1.73-1.56 (m, 4H), 1.50 - 1.33 (m, 10H), 1.17-1.12 (m, 3H), 1.02 (d, *J* = 6.9 Hz, 3H) ppm.

### (S)-1-tert-butyl 2-((2R,3R)-3-methylpent-4-en-2-yl) piperidine-1,2-dicarboxylate (PG-A10)

General procedure K was applied to (2*R*,3)-3-methylpent-4-en-2-ol and (*S*)-*N*-Boc-pipecolic acid. **Yield:** 1.72 g, 85 %, colorless oil. **¹H NMR** (300 MHz, CDCl₃, mixture of rotamers, major rotamer reported): δ 5.74 (ddd, J = 17.5, 10.6, 7.5 Hz, 1H), 5.11 - 5.00 (m, 2H), 4.94 - 4.63 (m, 2H), 3.96 (dd, J = 36.7, 13.4 Hz, 1H), 2.92 (dt, J = 24.2, 13.6 Hz, 1H), 2.44-2.29 (m, 1H), 2.19 (d, J = 13.0 Hz, 1H), 1.74 - 1.54 (m, 3H), 1.52 - 1.20 (m, 10H), 1.16 (d, J = 6.4 Hz, 4H), 1.02 (d, J = 6.8 Hz, 3H) ppm.

### 1-((9H-fluoren-9-yl)methyl) 2-((2R,3S)-3-(methoxymethoxy)pent-4-en-2-yl) (S)-piperidine-1,2-dicarboxylate (PG-A11)

General procedure K was applied to (2*R*,3)-3-methylpent-4-en-2-ol and (*S*)-*N*-Fmoc-pipecolic acid. **Yield:** 1.82 g, 82 %, colorless oil. **¹H NMR** (500 MHz, CDCl₃, mixture of rotamers, major rotamer reported): δ 7.76 (t, J = 6.9 Hz, 2H), 7.65 - 7.53 (m, 2H), 7.43 - 7.28 (m, 4H), 5.79 - 5.64 (m, 1H), 5.34 - 5.25 (m, 2H), 5.13 - 5.03 (m, 1H), 4.99 - 4.95 (m, 1H), 4.69 - 4.66 (m, 1H), 4.57 (d, J = 6.7 Hz, 1H), 4.47 - 4.33 (m, 2H), 4.28 (t, J = 7.2 Hz, 1H), 4.09 - 4.01 (m, 2H), 3.36 (s, 2H), 3.17 (td, J = 13.0, 3.0 Hz, 1H), 1.70 (td, J = 13.7, 6.7 Hz, 5H), 1.36-1.15 (m, 5H) ppm. **MS** (m/z): (ESI⁺) calculated for: C₂₈H₃₃NO₆Na [M+Na]⁺: 502.3, found: 502.2

### (S)-allyl piperidine-2-carboxylate (A1)

General procedure L was applied to **PG-A1. Yield:** 278 mg, 98 %, yellow oil. **¹H NMR** (500 MHz, CDCl₃): δ 5.91 (ddt, J = 17.1, 10.3, 5.8 Hz, 1H), 5.43 - 5.14 (m, 2H), 4.81 - 4.46 (m, 2H), 3.54 - 3.31 (m, 1H), 3.28 - 3.06 (m, 1H), 2.81 - 2.54 (m, 1H), 2.25 (s, 1H), 2.12 - 1.87 (m, 1H), 1.70 - 1.34 (m, 4H) ppm. **MS** (m/z): (ESI⁺) calculated for: C₁₉H₁₆NO₂ [M+H]⁺: 170.2, found: 170.2.

### (S)-(R)-pent-4-en-2-yl piperidine-2-carboxylate (A2)

General procedure L was applied to **PG-A2. Yield:** 323 mg, quant., yellow oil. **¹H NMR** (500 MHz, CDCl₃): δ 6.09 (s, 1H), 5.79 - 5.68 (m, 1H), 5.18 - 5.01 (m, 2H), 4.28 - 4.08 (m, 2H), 3.55 (dd, J = 10.7, 3.4 Hz, 1H), 3.40 - 3.25 (m, 1H), 2.90 - 2.72 (m, 1H), 2.52 - 2.33 (m, 2H), 2.06 (dq, J = 13.5, 4.0 Hz, 1H), 1.92 - 1.77 (m, 1H), 1.77 - 1.56 (m, 3H), 1.54 - 1.42 (m, 1H) ppm. **MS** (m/z): (ESI⁺) calculated for: C₁₀H₁₈NO₂ [M+H]⁺: 184.2, found: 184.2.

### (S)-2-(allyloxy)ethyl piperidine-2-carboxylate (A3)

General procedure L was applied to **PG-A3. Yield:** 595 mg, 78 %, yellow oil.

**¹H NMR** (500 MHz, CDCl₃): δ 5.88 (ddd, J = 17.2, 10.7, 5.3 Hz, 1H), 5.32 - 5.24 (m, 1H), 5.21 - 5.16 (m, 1H), 4.30 - 4.25 (m, 2H), 4.01 (dt, J = 5.6, 1.4 Hz, 2H), 3.64 (t, J = 4.8 Hz, 2H), 3.40 (dd, J = 9.9, 3.2 Hz, 1H), 3.09 (dt, J = 12.1, 3.7 Hz, 1H), 2.66 (ddd, J = 12.0, 10.3, 3.2 Hz, 1H), 2.26 (s, 1H), 2.01 - 1.94 (m, 1H), 1.77 (tt, J = 7.1, 3.8 Hz, 1H), 1.61 - 1.51 (m, 2H), 1.50 - 1.38 (m, 2H) ppm.

### (S)-3-(allyloxy)propyl piperidine-2-carboxylate (A4)

General procedure L was applied to **PG-A4. Yield:** 850 mg, 94 %, yellow oil.

**¹H NMR** (500 MHz, CDCl₃): δ 5.98 - 5.79 (m, 1H), 5.32 - 5.20 (m, 1H), 5.20 - 5.11 (m, 1H), 4.22 (qt, J = 10.8, 6.4 Hz, 2H), 3.95 (dt, J = 5.7, 1.5 Hz, 2H), 3.49 (t, J = 6.2 Hz, 2H), 3.37 (dd, J = 9.9, 3.3 Hz, 1H), 3.15 - 3.04 (m, 1H), 2.72 - 2.60 (m, 1H), 2.49 (s, 1H), 2.00 - 1.85 (m, 3H), 1.84 - 1.67 (m, J = 5.3 Hz, 1H), 1.64 - 1.50 (m, 2H), 1.50 - 1.39 (m, 2H) ppm.

### (S)-(S)-pent-4-en-2-yl piperidine-2-carboxylate (A5)

General procedure L was applied to **PG-A5. Yield:** 343 mg, quant., yellow oil. **¹H NMR** (500 MHz, CDCl₃): δ 5.72 (ddt, J = 17.3, 10.3, 7.0 Hz, 1H), 5.11 - 5.03 (m, 2H), 5.00 (p, J = 6.3 Hz, 1H), 3.45 (s, 1H), 3.37 (dd, J = 10.2, 3.3 Hz, 1H), 3.14 (dd, J = 12.4, 4.2 Hz, 1H), 2.69 (ddd, J = 12.5, 10.5, 3.1 Hz, 1H), 2.30 (h, J = 7.3 Hz, 2H), 1.97 (dt, J = 13.1, 4.1 Hz, 1H), 1.78 (dq, J = 12.2, 4.3, 3.9 Hz, 1H), 1.51 (m, 4H), 1.22 (d, J = 6.4 Hz, 3H) ppm. MS (m/z): (ESI⁺) calculated for: C₁₁H₂₀NO₂ [M+H]⁺: 198.2, found: 198.2.

### (S)-(R)-pent-4-en-2-yl piperidine-2-carboxylate (A6)

General procedure L was applied to **PG-A6. Yield:** 278 mg, 98 %, yellow oil. **¹H NMR** (500 MHz, CDCl₃): δ 5.79 - 5.67 (m, 1H), 5.10 - 5.04 (m, 2H), 5.04 - 4.92 (m, 1H), 3.55 (dd, *J* = 9.9, 3.6 Hz, 1H), 3.33 (dt, *J* = 12.5, 4.3 Hz, 1H), 2.88 - 2.80 (m, 1H), 2.39 - 2.33 (m, 1H), 2.32 - 2.25 (m, 1H), 2.06 (dt, *J* = 12.8, 4.3 Hz, 1H), 1.83 - 1.74 (m, 2H), 1.74 - 1.66 (m, 2H), 1.56 - 1.45 (m, 1H), 1.23 (d, *J* = 6.4 Hz, 3H) ppm. **MS** (m/z): (ESI⁺) calculated for: C₁₁H₂₀NO₂ [M+H]⁺: 198.2, found: 198.2.

### (S)-(R)-1-((4-methoxybenzyl)oxy)pent-4-en-2-yl piperidine-2-carboxylate (A7)

General procedure M was applied to **PG-A7. Yield:** 260 mg, 87 %, yellow oil. **¹H NMR** (500 MHz, CDCl₃): 7.25 - 7.21 (m, 2H), 6.88 - 6.84 (m, 2H), 5.78 - 5.65 (m, 1H), 5.17 - 5.02 (m, 3H), 4.48 (d, J = 11.6 Hz, 1H), 4.42 (d, J = 11.8 Hz, 1H), 3.80 (s, 4H), 3.53 - 3.46 (m, 2H), 3.36 (dd, J = 9.7, 3.3 Hz, 1H), 3.11 - 3.05 (m, 1H), 2.70 - 2.62 (m, 1H), 2.45 - 2.30 (m, 2H), 1.98 - 1.92 (m, 1H), 1.78 - 1.72 (m, 1H), 1.63 - 1.33 (m, 4H) **ppm.MS** (m/z): (ESI⁺) calculated for: C₁₉H₂₈NO₄ [M+H]⁺: 334.2, found: 334.2.

### (S)-(S)-1-((4-methoxybenzyl)oxy)pent-4-en-2-yl piperidine-2-carboxylate (A8)

General procedure M was applied to **PG-A8. Yield:** 242 mg, 81 %, yellow oil. **¹H NMR** (300 MHz, CDCl₃): δ 7.27 - 7.21 (m, 2H), 6.89 - 6.84 (m, 2H), 5.72 (ddt, J = 17.1, 10.1, 7.1 Hz, 1H), 5.18 - 5.01 (m, 3H), 4.48 (d, J = 11.6 Hz, 1H), 4.41 (d, J = 11.7 Hz, 1H), 3.80 (s, 3H), 3.49 (d, J = 5.0 Hz, 2H), 3.35 (dd, J = 9.4, 3.2 Hz, 1H), 3.06 (dt, J = 11.8, 3.8 Hz, 1H), 2.70 - 2.59 (m, 1H), 2.48 - 2.27 (m, 2H), 2.16 (s, 1H), 1.99 - 1.88 (m, 1H), 1.81 - 1.70 (m, 1H), 1.62 - 1.36 (m, 4H) ppm. MS (m/z): (ESI⁺) calculated for: C₁₉H₂₈NO₄ [M+H]⁺: 334.2, found: 334.2.

### (S)-(2R,3S)-3-methylpent-4-en-2-yl piperidine-2-carboxylate (A9)

General procedure L was applied to **PG-A9. Yield:** 740 mg, 96 %, yellow oil. **¹H NMR** (500 MHz, CDCl₃): δ 5.77 - 5.66 (m, 1H), 5.07 - 5.01 (m, 2H), 4.94 - 4.87 (m, 1H), 3.41 (dd, *J* = 10.2, 3.4 Hz, 1H), 3.17 (dtd, *J* = 12.4, 3.9, 1.3 Hz, 1H), 2.72 (ddd, *J* = 12.3, 10.3, 3.2 Hz, 1H), 2.41 - 2.30 (m, 1H), 2.02 - 1.94 (m, 1H), 1.79 (ddd, *J* = 12.8, 5.7, 3.3 Hz, 1H), 1.65 - 1.41 (m, 4H), 1.16 (d, *J* = 6.4 Hz, 3H), 1.01 (d, *J* = 6.9 Hz, 3H) ppm. **MS** (m/z): (ESI⁺) calculated for: C₁₂H₂₂NO₂ [M+H]⁺: 212.2, found: 212.2.

### (S)-(2R,3R)-3-methylpent-4-en-2-yl piperidine-2-carboxylate (A10)

General procedure L was applied to **PG-A10. Yield:** 740 mg, 96 %, yellow oil. **¹H NMR** (300 MHz, CDCl₃): δ 5.72 (ddd, J = 16.9, 10.6, 7.5 Hz, 1H), 5.09 - 5.00 (m, 2H), 4.89 - 4.77 (m, 1H), 3.32 (dd, J = 9.5, 3.2 Hz, 1H), 3.15 - 3.01 (m, 1H), 2.71 - 2.57 (m, 1H), 2.43 - 2.28 (m, 1H), 2.14 (s, 1H), 1.99 - 1.89 (m, 1H), 1.85 - 1.66 (m, 1H), 1.62 - 1.33 (m, 4H), 1.15 (d, J = 6.3 Hz, 3H), 0.99 (d, J = 6.9 Hz, 3H) ppm. **MS** (m/z): (ESI⁺) calculated for: C₁₂H₂₂NO₂ [M+H]⁺: 212.2, found: 212.2.

### (2R,3S)-3-(methoxymethoxy)pent-4-en-2-yl (S)-piperidine-2-carboxylate (A11)

General procedure M was applied to **PG-A11. Yield:** 889 mg, 92 %, yellow oil. **¹H NMR** (300 MHz, CDCl₃): δ 7.27 - 7.21 (m, 2H), 6.89 - 6.84 (m, 2H), 5.72 (ddt, J = 17.1, 10.1, 7.1 Hz, 1H), 5.18 - 5.01 (m, 3H), 4.48 (d, J = 11.6 Hz, 1H), 4.41 (d, J = 11.7 Hz, 1H), 3.80 (s, 3H), 3.49 (d, J = 5.0 Hz, 2H), 3.35 (dd, J = 9.4, 3.2 Hz, 1H), 3.06 (dt, J = 11.8, 3.8 Hz, 1H), 2.70 - 2.59 (m, 1H), 2.48 - 2.27 (m, 2H), 2.16 (s, 1H), 1.99 - 1.88 (m, 1H), 1.81 - 1.70 (m, 1H), 1.62 - 1.36 (m, 4H) ppm. MS (m/z): (ESI⁺) calculated for: C₁₃H₂₄NO₄ [M+H]⁺: 258.2, found: 258.2.

### Example 2d: Synthesis of B-Building Blocks

### 2-(allyloxy)ethyl 4-methylbenzenesulfonate (B3)

Synthesized according to literature procedure.⁴

### 3-(allyloxy)propyl 4-methylbenzenesulfonate (B4)

Synthesized according to literature procedure.⁴

### (S)-ethyl 2-(allyloxy)propanoate (S29)

Ag₂O (6.96 g, 30.0 mmol, 1.0 eq.) was added portion wise, under vigorous stirring, to a solution of ethyl-2-(S)-lactate (3.50 g, 30.0 mmol, 1.0 eq.) and allyl bromide (5.45 g, 45.0 mmol, 1.5 wq.) in anhydrous diethyl ether (60 mL) at room temperature. The mixture was refluxed for 2 h. The reaction was filtered through a pad celite and concentrated *in vacuo.* Flash column chromatography (0 - 20 % Et₂O in pentane) on silica gel afforded the title compound as a colorless oil (3.54 g, 76 %). **¹H NMR** (300 MHz, CDCl₃): δ 5.99 - 5.80 (m, 1H), 5.33 - 5.24 (m, 1H), 5.22 - 5.16 (m, 1H), 4.27 - 4.09 (m, 3H), 4.06 - 3.88 (m, 2H), 1.41 (d, *J* = 6.9 Hz, 3H), 1.28 (t, *J* = 7.2 Hz, 3H) ppm.

### (R)-ethyl 2-(allyloxy)propanoate (S30)

Synthesized as shown for **S29** with ethyl-2-(S)-lactate as the substrate. **Yield:** 3.82 g, 78 %, colorless oil. **¹H NMR** (300 MHz, CDCl₃): δ 5.92 (dddd, *J* = 17.2, 10.3, 6.1, 5.5 Hz, 1H), 5.34 - 5.15 (m, 2H), 4.25-4.10 (m, 3H), 4.04 - 3.89 (m, 2H), 1.41 (d, *J =* 6.8 Hz, 3H), 1.28 (t, *J* = 7.1 Hz, 3H) ppm.

### (R)-2-(allyloxy)propan-1-ol (S31)

LiAlH₄ (14.2 mL of a 2 M solution in THF, 28.4 mmol, 1.1 eq.) was added to a solution of **S30** (3.72 g, 25.8 mmol, 1.0 eq.) in THF (86 mL) at 0 °C. The mixture was stirred at room temperature for 10 minutes. The reaction was quenched by the addition of ethyl acetate. Then saturated aqueous Rochelle salt was added and the resulting slurry was vigorously stirred until a clear biphasic mixture was obtained. The organic layer was dried with MgSO₄, filtered and concentrated under reduced pressure. The title compound was obtained as a colorless oil (2.62 g, 87 %) without further purification. **¹H NMR** (300 MHz, CDCl₃): 6.02 - 5.85 (m, 1H), 5.38 - 5.14 (m, 2H), 4.23 - 4.06 (m, 1H), 4.02 - 3.91 (m, 1H), 3.68 - 3.55 (m, 2H), 3.53 - 3.40 (m, 1H), 1.14 (d, *J* = 6.1 Hz, 3H) ppm.

### (S)-2-(allyloxy)propan-1-ol (S32)

Synthesized as shown for **S31** with **S29** as substrate. Yield: 391 mg, 97 %, colorless oil. **¹H NMR** (300 MHz, CDCl₃): δ 5.92 (ddt, *J* = 17.2, 10.4, 5.7 Hz, 1H), 5.36 - 5.21 (m, 1H), 5.17 (ddt, *J* = 10.3, 1.8, 1.2 Hz, 1H), 4.11 (ddt, *J* = 12.9, 5.7, 1.5 Hz, 1H), 3.96 (ddt, *J* = 12.6, 5.8, 1.4 Hz, 1H), 3.78 - 3.67 (m, 2H), 3.66 - 3.53 (m, 2H), 3.51 - 3.40 (m, 1H), 1.12 (d, *J* = 6.0 Hz, 3H) ppm.

### (S)-2-(allyloxy)propyl 4-methylbenzenesulfonate (B5)

General procedure N was applied to **S32. Yield:** 2.97 g, 81 %, colorless oil. **¹H NMR** (300 MHz, CDCl₃): δ 5.99 - 5.80 (m, 1H), 5.33 - 5.24 (m, 1H), 5.22 - 5.16 (m, 1H), 4.27 - 4.09 (m, 3H), 4.06 - 3.88 (m, 2H), 1.41 (d, *J* = 6.9 Hz, 3H), 1.28 (t, *J* = 7.2 Hz, 3H) ppm. **MS** (m/z): (ESI⁺) calculated for: C₁₃H₁₉O₄S [M+H]⁺: 270.1, found: 271.0

### (R)-2-(allyloxy)propyl 4-methylbenzenesulfonate (B6)

General procedure N was applied to **S31. Yield:** 3.14 g, 60 %, yellowish oil. **¹H NMR** (300 MHz, CDCl₃): δ 7.85 - 7.76 (m, 2H), 7.38 - 7.31 (m, 2H), 5.92 - 5.74 (m, 1H), 5.29 - 5.09 (m, 2H), 4.02 - 3.90 (m, 4H), 3.77 - 3.64 (m, 1H), 2.45 (s, 3H), 1.13 (d, *J* = 6.4 Hz, 3H) ppm. **MS** (m/z): (ESI⁺) calculated for: C₁₃H₁₉O₄S [M+H]⁺: 270.1, found: 271.0

### (R)-methyl 3-((tert-butyldimethylsilyl)oxy)-2-hydroxypropanoate (S35)

To a solution of (*R*)-Methyl-2,3-dihydroxy propanoate (4.00 g, 33.3 mmol, 1.0 eq.) in DCM (90 mL) was added imidazole (6.87 g, 99.9 mmol, 3.0 eq.) under an atmosphere of argon. After stirring for 5 minutes, TBSCI (5.83 g, 38.3 mmol, 2.5 eq.) was added and the reaction mixture was stirred overnight. The reaction was quenched by the addition saturated aqueous NH₄Cl solution. The aqueous layer was extracted with DCM. Combined organic layers were dried with MgSO₄, filtered and concentrated under reduced pressure. The crude product was purified by flash column chromatography (0 - 20 % EA in CH). The title compound was obtained as a colourless oil (5.12 g, 66 %). **¹H NMR** (300 MHz, CDCl₃): 4.26 - 4.21 (m, 1H), 3.96 - 3.84 (m, 2H), 3.79 (s, 3H), 0.90 - 0.84 (m, 9H), 0.07 (s, 3H), 0.05 (s, 3H) ppm. **MS** (m/z): (ESI⁺) calculated for: C₁₀H₂₃O₄Si [M+H]⁺: 235.1, found: 235.2

### (S)-methyl 3-((tert-butyldimethylsilyl)oxy)-2-hydroxypropanoate (S36)

Synthesis as shown for **S35** with substrate (*S*)-Methyl-2,3-dihydroxy propanoate. **Yield:** 2.62 g, 64 %, colorless oil. **¹H NMR** (300 MHz, CDCl₃): 4.26 - 4.21 (m, 1H), 3.96 - 3.84 (m, 2H), 3.77, 3. (s, 3H), 3.04 (s, 1H) 0.86 (s, 9H), 0.04 (s, 3H), 0.03 (s, 3H) ppm. **MS** (m/z): (ESI⁺) calculated for: C₁₀H₂₃O₄Si [M+H]⁺: 235.1, found: 235.2

### (S)-methyl 2-(allyloxy)-3-((tert-butyldimethylsilyl)oxy)propanoate⁵ (S37)

**S36** (2.38 g, 10.1 mmol, 1.0 eq.) was dissolved in anhydrous THF (50 mL) in flame dried glassware under an atmosphere of argon. Pd₂(dba)₃ (56 mg. 0.06 mmol, 0.6 mol%), 1,4-bis(diphe-nylphosphino)-butane (111 mg, 0.26 mmol, 2.6 mol%) and allyl ethyl carbonate (2.64 g, 20.3 mmol, 2.0 eq.) were added. The reaction mixture was then heated to reflux and stirred for 2 h. After filtration over a pad of celite volatiles were evaporated and the crude product was purified by flash column chromatography to give the title compound as a yellowish oil (1.32 g, 48 %). **¹H NMR** (300 MHz, CDCl₃): δ 6.02 - 5.78 (m, 1H), 5.40 - 5.14 (m, 2H), 4.24 - 4.13 (m, 1H), 4.05 - 3.95 (m, 2H), 3.87 (d, *J* = 5.2 Hz, 1H), 3.74 (s, 2H), 0.87 (s, 9H), 0.05 (s, 6H) ppm. **MS** (m/z): (ESI⁺) calculated for: C₁₃H₂₇O₄Si [M+H]⁺: 275.2, found: 275.2

### (R)-methyl 2-(allyloxy)-3-((tert-butyldimethylsilyl)oxy)propanoate⁵ (S38)

Synthesis as shown for **S37** with substrate **S35.** Yield: 2.90 g, 83 %, yellowish oil. **¹H NMR** (300 MHz, CDCl₃): 6.02 - 5.78 (m, 1H), 5.40 - 5.14 (m, 2H), 4.24 - 4.13 (m, 1H), 4.05 - 3.95 (m, 2H), 3.87 (d, *J* = 5.2 Hz, 1H), 3.74 (s, 2H), 0.87 (s, 9H), 0.05 (s, 6H) ppm. **MS** (m/z): (ESI⁺) calculated for: C₁₃H₂₇O₄Si [M+H]⁺: 275.2, found: 275.2

### (R)-2-(allyloxy)-3-((tert-butyldimethylsilyl)oxy)propan-1-ol (S39)

Under an atmosphere of argon DIBAL-H (1 M in DCM, 11.5 mL, 11.5 mmol, 2.5 eq.) was added to a solution of **S37** (1.26 g, 4.59 mmol, 1.0 eq.) in anhydrous THF (50 mL) at 0°C. The resulting mixture was then stirred at this temperature for 30 minutes. The reaction was quenched with saturated aqueous solution of Rochelle salt. The queous phase was extracted with diethyl ether. The combined organic extracts were dried over MgSO₄ and concentrated under reduced pressure. Flash column chromatography (0 - 15 % EA in CH) afforded the title compound as a colorless oil (527 mg, 47 %). **¹H NMR** (300 MHz, CDCl₃): δ 5.92 (ddt, *J* = 17.1, 10.3, 5.7 Hz, 1H), 5.34 - 5.12 (m, 2H), 4.21 - 4.04 (m, 2H), 3.80 - 3.42 (m, 5H), 2.12 (s, 1H), 0.89 (s, 9H), 0.06 (s, 6H) ppm. **MS** (m/z): (ESI⁺) calculated for: C₁₂H₂₇O₃Si [M+H]⁺: 247.2, found: 247.2

### (S)-2-(allyloxy)-3-((tert-butyldimethylsilyl)oxy)propan-1-ol (S40)

Synthesis as shown for **S39** with substrate **S38. Yield:** 1.34 g, quant., colorless oil. **¹H NMR** (300 MHz, CDCl₃): δ 6.01 - 5.83 (m, 1H), 5.34 - 5.14 (m, 2H), 4.21 - 4.04 (m, 2H), 3.80 - 3.42 (m, 5H), 0.89 (s, 9H), 0.07 (s, 6H) ppm. **MS** (m/z): (ESI⁺) calculated for: C₁₂H₂₇O₃Si [M+H]⁺: 247.2, found: 247.2

### (S)-2-(allyloxy)-3-((tert-butyldimethylsilyl)oxy)propyl 4-methylbenzenesulfonate (B7)

General procedure N was applied to **S39. Yield:** 657 mg, 77 %, yellowish oil. **¹H NMR** (300 MHz, CDCl₃): δ 7.79 (d, *J* = 8.3 Hz, 2H), 7.33 (d, *J* = 8.1 Hz 2H), 5.81 (ddt, *J* = 17.1, 10.3, 5.6 Hz, 1H), 5.24-5.18 (m, 1H), 5.15-5.10 (m, 1H), 4.17 (dd, *J* = 10.4, 3.6 Hz 1H), 4.05-4.01 (m, 2H), 3.62 (dd, *J* = 9.4, 4.2 Hz, 1H), 3.59 - 3.52 (m, 2H), 2.44 (s, 3H), 0.83 (s, 9H), 0.01 (s, 3H), 0.00 (s, 3H) ppm. **MS** (m/z): (ESI⁺) calculated for: C₁₉H₃₃O₅Si [M+H]⁺: 401.2, found: 401.2

### (R)-2-(allyloxy)-3-((tert-butyldimethylsilyl)oxy)propyl 4-methylbenzenesulfonate (B8)

General procedure N was applied to **S40. Yield:** 206 mg, 66 %, yellowish oil. **¹H NMR** (300 MHz, CDCl₃): δ 7.82 - 7.77 (m, 2H), 7.36 - 7.30 (m, 2H), 5.86 - 5.74 (m, 1H), 5.24 - 5.18 (m, 1H), 5.15 - 5.10 (m, 1H), 4.21 - 4.15 (m, 1H), 4.05 - 4.01 (m, 3H), 3.62 (dd, J = 9.4, 4.1 Hz, 1H), 3.59 - 3.52 (m, 2H), 2.44 (s, 3H), 0.83 (s, 9H), 0.01 (s, 3H), 0.00 (s, 3H) ppm. **MS** (m/z): (ESI⁺) calculated for: C₁₉H₃₃O₅Si [M+H]⁺: 401.2, found: 401.2

### (R)-methyl 3-(allyloxy)-2-methylpropanoate (S43)

In a flask wrapped with aluminum foil and under argon, (*R*)-Roche ester (5.0 g, 4.3 mL, 42.3 mmol, 1.0 eq.) was added to a suspension of Na₂SO₄ (6.1 g, 42.3 mmol, 1.0 eq.) and Ag₂O (12.9 g, 55.0 mmol 1.3 eq.) in petroleum ether (200 mL). After the system had been stirred for 1 h at room temperature, the flask was cooled in an ice bath, and allyl bromide (5.5 mL, 63.5 mmol, 1.5 eq.) was slowly added. After 30 min, a second fraction of Ag₂O was added (10.5 g, 45.3 mmol 1.1 eq.), and the mixture was stirred at room temperature overnight. The the mixture was filtered through a pad of celite and concentrated under reduced pressure. Flash column chromatography on silica gel (0 - 20 % diethyl ether in pentane) afforded the title compound as colorless oil (4.10 g, 61 %). **¹H NMR** (500 MHz, CDCl₃): δ 5.88 (ddt, *J* = 17.3, 10.4, 5.6 Hz, 1H), 5.29 - 5.22 (m, 1H), 5.19 - 5.15 (m, 1H), 4.00 - 3.95 (m, 2H), 3.70 (s, 3H), 3.63 (dd, *J* = 9.2, 7.3 Hz, 1H), 3.46 (dd, J = 9.2, 5.9 Hz, 1H), 2.76 (pd, *J* = 7.1, 5.8 Hz, 1H), 1.18 (d, *J* = 7.1 Hz, 3H) ppm.

### (S)-methyl 3-(allyloxy)-2-methylpropanoate (S44)

Synthesis as shown for **S43** with (S)-Roche ester as substrate. **Yield:** 3.16 g, 47 %, colorless oil. **¹H NMR** (300 MHz, CDCl₃): δ 5.92 - 5.81 (m, 1H), 5.27 - 5.12 (m, 2H), 3.97 (dt, *J* = 5.5, 1.4 Hz, 2H), 3.69 (s, 3H), 3.66 - 3.57 (m, 1H), 3.50 - 3.42 (m, 1H), 2.85 - 2.69 (m, 1H), 1.20 - 1.14 (m, 3H) ppm.

### (S)-3-(allyloxy)-2-methylpropan-1-ol (S46)

A solution of LiAlH₄ (28.4 mL of 1 M solution in THF, 28.4 mmol, 1.1 eq.) was added to a solution of **S43** (4.08 g, 25.8 mmol, 1.0 eq.) in THF (129 mL) at 0 °C. The mixture was stirred at room temperature. The reaction was quenched by the careful addition of ethyl acetate. Then saturated aqueous Rochelle salt was added, and the resulting slurry was vigorously stirred until a clear biphasic mixture was obtained. The organic layer was dried with MgSO₄, filtered and concentrated under reduced pressure. **Yield:** 2.90 g, 86 %, colorless oil. **¹H NMR** (300 MHz, CDCl₃): δ 5.99 - 5.80 (m, 1H), 5.32 - 5.12 (m, 2H), 3.98 (dt, *J* = 5.6, 1.5 Hz, 2H), 3.66 - 3.47 (m, 3H), 3.44 - 3.32 (m, 1H), 2.32 (d, *J* = 32.1 Hz, 1H), 2.13 - 1.96 (m, 1H), 0.87 (d, *J =* 6.9 Hz, 3H) ppm.

### (S)-3-(allyloxy)-2-methylpropyl 4-methylbenzenesulfonate (B11)

A solution of LiAlH₄ (11.0 mL of 2 M solution in THF, 22.0 mmol, 1.1 eq.) was added to a solution of **S44** (3.16 g, 20.0 mmol, 1.0 eq.) in THF (67 mL) at 0 °C. The mixture was stirred at room temperature. The reaction was quenched by the careful addition of ethyl acetate. Then saturated aqueous solution of Rochelle salt was added and the resulting slurry was vigorously stirred until a clear biphasic mixture was obtained. The organic layer was dried with MgSO₄, filtered and concentrated under reduced pressure. Then general procedure N was applied. **Yield:** 3.45 g, 61 % over 2 steps, colorless oil. **¹H NMR** (300 MHz, CDCl₃): δ 7.80 - 7.76 (m, 2H), 7.37 - 7.32 (m, 2H), 5.89 - 5.74 (m, 1H), 5.28 - 5.08 (m, 2H), 4.02 (dd, *J* = 9.4, 5.6 Hz, 1H), 3.96 (dd, *J* = 9.4, 5.8 Hz, 1H), 3.85 (dt, *J* = 5.6, 1.4 Hz, 2H), 3.28 (qd, *J* = 9.3, 6.0 Hz, 2H), 2.44 (s, 3H), 2.12 - 2.02 (m, 1H), 0.93 (d, *J* = 6.9 Hz, 3H) ppm. **MS** (m/z): (ESI⁺) calculated for: C₁₄H₂₁O₄S [M+H]⁺: 285.1, found: 285.2

### (R)-3-(allyloxy)-2-methylpropyl 4-methylbenzenesulfonate (B12)

General procedure N was applied to **S46. Yield:** 4.75 g, 76 **%. ¹H NMR** (300 MHz, CDCl₃): δ 7.84 - 7.74 (m, 2H), 7.39 - 7.30 (m, 2H), 5.80 (ddt, *J* = 17.3, 10.7, 5.5 Hz, 1H), 5.44 - 5.03 (m, 2H), 4.02 (dd, *J* = 9.4, 5.6 Hz, 1H), 3.96 (dd, *J* = 9.4, 5.7 Hz, 1H), 3.85 (dt, *J* = 5.8, 1.6 Hz, 2H), 3.34 - 3.19 (m, 2H), 2.44 (s, 3H), 2.12 - 2.02 (m, 1H), 0.93 (d, *J* = 6.9 Hz, 3H) ppm. **MS** (m/z): (ESI⁺) calculated for: C₁₄H₂₁O₄S [M+H]⁺: 285.1, found: 285.2

### (R)-4-((allyloxy)methyl)-2,2-dimethyl-1,3-dioxolane (S49)

To a suspension of NaH (60%, in mineral oil, 3.0 g, 75.7 mmol, 2.0 eq) in anhydrous THF (50 mL) was added (*R*)-solketal (5.0 g, 37.8 mmol, 1.0 eq.) dropwise at 0 °C, followed by allylbromide 5.0 mL, 56.8 mmol, 1.5 eq), the reaction mass was then stirred at room temperature overnight. The reaction mixture was cooled to 0 °C and quenched carefully by the dropwise addition of water. The aqueous phase was extracted with ethyl acetate, combined organic extracts were washed with brine, dried over anhydrous MgSO₄, filtered and concentrated under reduced pressure. The crude product was purified by silica gel column chromatography (0 - 30 % EA in CH) to give the title compound as a colorless oil (6.14 g, 94 %). **¹H NMR** (300 MHz, CDCl₃): δ 5.89 (ddt, *J* = 17.2, 10.4, 5.7 Hz, 1H), 5.27 (dq, *J* = 17.3, 1.7 Hz, 1H), 5.21 - 5.15 (m, 1H), 4.33 - 4.22 (m, 1H), 4.09 - 3.98 (m, 3H), 3.73 (dd, *J* = 8.2, 6.4 Hz, 1H), 3.52 (dd, *J* = 9.8, 5.8 Hz, 1H), 3.44 (dd, *J* = 9.8, 5.4 Hz, 1H), 1.42 (s, 3H), 1.35 (s, 3H) ppm.

### (S)-4-((allyloxy)methyl)-2,2-dimethyl-1,3-dioxolane (S50)

Synthesis as shown for **S49** with (S)-solketal as substrate. **Yield:** 7.65 g, 84 %, colorless oil. **¹H NMR** (300 MHz, CDCl₃): δ 5.97 - 5.80 (m, 1H), 5.31 - 5.12 (m, 2H), 4.34 - 4.20 (m, 1H), 4.15 - 3.93 (m, 3H), 3.78 - 3.65 (m, 1H), 3.57 - 3.38 (m, 2H), 1.41 (s, 3H), 1.35 (s, 3H) ppm.

### (R)-3-(allyloxy)propane-1,2-diol (S51)

**S49** (5.83 g, 33.9 mmol, 1.0 eq.) was dissolved in 85 mL of a mixture of acetic acid and water (4:1) and stirred at 60 °C. Volatiles were removed under reduced pressure. The title compound was obtained as a colorless oil (4.14 g, 93 %) without further **purification. ¹H NMR** (300 MHz, CDCl₃): δ 5.98 - 5.80 (m, 1H), 5.34 - 5.15 (m, 2H), 4.07 - 3.97 (m, 2H), 3.93 - 3.80 (m, 1H), 3.75 - 3.59 (m, 2H), 3.58 - 3.46 (m, 2H), 2.74 (s, 2H) ppm.

### (S)-3-(allyloxy)propane-1,2-diol (S52)

Synthesis as shown for **S51** with **S50** as substrate. **Yield:** 5.15 g, 88 %, colorless oil. **¹H NMR** (300 MHz, CDCl₃): δ 5.99 - 5.81 (m, 1H), 5.34 - 5.12 (m, 2H), 4.01 (dt, *J* = 5.7, 1.4 Hz, 2H), 3.91 - 3.83 (m, 1H), 3.75 - 3.56 (m, 2H), 3.55 - 3.42 (m, 2H), 2.96 (s, 2H) ppm.

### (R)-3-(allyloxy)-2-hydroxypropyl 4-methylbenzenesulfonate (S53)

Dibutyltin oxide (381 mg, 1.51 mmol, 0.1 eq.) was added to **S51** (2.00 g, 15.1 mmol, 1.0 eq.) in DCM (27 mL). Tosyl chloride (2.91 g, 15.1 mmol, 1.0 eq.) and triethylamine (2.13 mL, 15.1 mmol, 1.0 eq.) were added, and the mixture was stirred at room temperature overnight. Water was added, and the layers were separated. The aqueous layer was extracted twice with DCM, and the combined organic layers were washed with water and brine, dried with MgSO₄, filtered and concentrated under reduced pressure. The crude product was purified by silica gel column chromatography (0 - 60 % EA in CH) to give the title compound as a blue oil (3.61 g, 83 **%).¹H NMR** (300 MHz, CDCl₃): δ 7.83 - 7.76 (m, 2H), 7.40 - 7.29 (m, 2H), 5.91 - 5.76 (m, 1H), 5.35 - 5.12 (m, 2H), 4.14 - 4.02 (m, 2H), 4.01 - 3.92 (m, 3H), 3.52 - 3.40 (m, 2H), 2.45 (s, 3H), 2.24 (s, 1H) ppm. **MS** (m/z): (ESI⁺) calculated for: C₁₃H₁₉O₅S [M+H]⁺: 287.1, found: 287.0

### (S)-3-(allyloxy)-2-hydroxypropyl 4-methylbenzenesulfonate (S54)

Synthesis as shown for S53 with **S52** as substrate. **Yield:** 5.01 g, 83 %. **¹H NMR** (300 MHz, CDCl₃): δ 7.83 - 7.76 (m, 2H), 7.39 - 7.31 (m, 2H), 5.90 - 5.75 (m, 1H), 5.33 - 5.09 (m, 2H), 4.16 - 4.03 (m, 2H), 4.02 - 3.91 (m, 3H), 3.52 - 3.41 (m, 2H), 2.44 (s, 3H), 2.27 (s, 1H) ppm. **MS** (m/z): (ESI⁺) calculated for: C₁₃H₁₉O₅S [M+H]⁺: 287.1, found: 287.0.

### (R)-3-(allyloxy)-2-((tert-butyldimethylsilyl)oxy)propyl 4-methylbenzenesulfonate (B15)

To a solution of alcohol **S53** (1.0 g, 3.49 mmol, 1.0 eq.) and 2,6-lutidine (1.23 mL, 10.5 mmol, 3.0 eq.) in dichloromethane (12 mL) at 0 °C was added TBSOTf (1.1 g, 0.97 mL, 1.40 mmol, 1.2 eq.). The reaction mixture allowed to reach room temperature and stirred for 5h. The reaction was quenched by the addition of saturated aqueous NH₄Cl solution. The aqueous layer was extracted with DCM and combined organic layers were dried over MgSO4. After evaporation of the solvent under reduced pressure, the crude product was purified by silica gel column chromatography (0 - 20 % EA in CH) to give the title compound as a colorless oil (1.19 g, 85 **%).¹H NMR** (300 MHz, CDCl₃): δ 5.89 - 5.73 (m, 1H), 5.26 - 5.10 (m, 2H), 4.10 - 4.04 (m, 1H), 4.03 - 3.85 (m, 4H), 3.36 (d, *J* = 5.5 Hz, 2H), 2.44 (s, 3H), 0.85 - 0.81 (m, 9H), 0.06 - 0.01 (m, 5H) ppm. **MS** (m/z): (ESI⁺) calculated for: C₁₉H₃₃O₅SSi [M+H]⁺: 401.2, found: 401.2

### (S)-3-(allyloxy)-2-((tert-butyldimethylsilyl)oxy)propyl 4-methylbenzenesulfonate (B16)

Synthesis as shown for **B15** with **S54** as substrate. **Yield:** 1.27 g, 91 %, colorless oil. **¹H NMR** (300 MHz, CDCl₃): δ 7.83 - 7.77 (m, 2H), 7.36 (d, *J* = 8.0 Hz, 2H), 5.84 (ddt, *J* = 17.3, 10.8, 5.5 Hz, 1H), 5.39 - 5.10 (m, 2H), 4.10 (dd, *J* = 9.0, 3.4 Hz, 1H), 4.05 - 3.88 (m, 4H), 3.38 (d, *J* = 5.3 Hz, 2H), 2.46 (s, 3H), 0.85 (s, 9H), 0.06 (s, 3H), 0.05 (s, 3H) ppm. **MS** (m/z): (ESI⁺) calculated for: C1₉H₃₃O₅SSi [M+H]⁺: 401.2, found: 401.2

### (R)-ethyl 3-(allyloxy)butanoate (S57)

Trifluoromethanesulfonic acid (91 µL, 1.03 mmol, 0.07 eq.) was added dropwise to a solution of ethyl (R)-3-hydroxybutyrate (2.00 g, 15.1 mmol, 1.0 eq.) and allyl 2,2,2-trichloroacetimidate (6.19 g, 4.64 mL, 30.3 mmol, 2.0 eq.) in dry dichloromethane (17 mL) and n-hexane (34 mL). The mixture was stirred at room temperature overnight. The reaction mixture was filtered through a pad of celite and the filtrate was concentrated under reduced pressure. Flash column chromatography (0 -25 % pentane in Et₂O) afforded the title compound as a colorless oil (1.40 g, 54 **%). ¹H NMR** (300 MHz, CDCl₃): δ 5.96 - 5.81 (m, 1H), 5.30 - 5.10 (m, 2H), 4.14 (q, *J=* 7.1 Hz, 2H), 4.07 - 3.86 (m, 2H), 2.65 - 2.53 (m, 1H), 2.37 (dd, *J* = 15.0, 5.9 Hz, 1H), 1.30 - 1.19 (m, 6H) ppm. **MS** (m/z): (ESI⁺) calculated for: C₉H₁₇O₃ [M+H]⁺: 173.1, found: 173.0

### (S)-ethyl 3-(allyloxy)butanoate (S58)

Synthesis as shown for **S57** with (S)-3-hydroxybutyrate as substrate. **Yield:** 2.06 g, 63 %, colorless oil. **¹H NMR** (500 MHz, CDCl₃): δ 5.96 - 5.83 (m, 1H), 5.30 - 5.21 (m, 1H), 5.17 - 5.11 (m, 1H), 4.13 (q, *J* = 7.1 Hz, 2H), 4.06 - 4.00 (m, 1H), 3.98 - 3.88 (m, 2H), 2.59 (dd, *J* = 15.0, 7.2 Hz, 1H), 2.37 (dd, *J* = 15.0, 5.9 Hz, 1H), 1.25 (t, *J* = 7.1 Hz, 3H), 1.21 (d, *J =* 6.2 Hz, 3H) ppm. **MS** (m/z): (ESI⁺) calculated for: C₉H₁₇O₃ [M+H]⁺: 173.1, found: 173.0

### (R)-3-(allyloxy)butan-1-ol (S59)

A solution of LiAlH₄ (4.3 mL of 2 M solution in THF, 8.54 mmol, 1.05 eq.) was added to a solution of **S57** (1.40 g, 8.13 mmol, 1.0 eq.) in THF (15 mL) at 0 °C. The mixture was stirred at room temperature until completion. Excess LiAlH₄ was quenched by careful addition of 1 M NaOH at 0 °C. The biphasic mixture was stirred vigorously for 2 h and the aqueous layer extracted with diethyl ether. The combined organic extracts were dried (MgSO₄), filtered and the solvent was evaporated. Flash column chromatography (25 - 80 % pentane in Et₂O) afforded the title compound as a colorless oil (383 mg, 36 %). **¹H NMR** (300 MHz, CDCl₃): δ 5.91 (ddt, *J* = 17.1, 10.2, 5.6 Hz, 1H), 5.32 - 5.12 (m, 2H), 4.15 - 4.05 (m, 1H), 3.96 - 3.87 (m, 1H), 3.86 - 3.67 (m, 3H), 1.80 - 1.65 (m, 2H), 1.20 (d, *J* = 6.1 Hz, 3H) ppm.

### (S)-3-(allyloxy)butan-1-ol (S60)

Synthesis as shown for **S59** with **S58** as substrate. **Yield**:1.08 g, 69 %, colorless oil. **¹H NMR** (500 MHz, CDCl₃): δ 5.97 - 5.84 (m, 1H), 5.32 - 5.22 (m, 1H), 5.19 - 5.12 (m, 1H), 4.16 - 4.05 (m, 1H), 3.94 - 3.87 (m, 1H), 3.81 - 3.67 (m, 3H), 2.67 (s, 1H), 1.76 - 1.72 (m, 2H), 1.19 (d, *J* = 6.1 Hz, 3H) ppm.

### (R)-3-(allyloxy)butyl 4-methylbenzenesulfonate (B13)

General procedure N was applied to **S59. Yield:** 550 mg, 66 %, colorless oil. **¹H NMR** (500 MHz, CDCl₃): δ 7.86 - 7.75 (m, 2H), 7.41 - 7.30 (m, 2H), 5.78 (ddt, *J* = 17.2, 10.3, 5.6 Hz, 1H), 5.22 - 5.15 (m, 1H), 5.13 - 5.04 (m, 1H), 4.22 - 4.15 (m, 1H), 4.13 - 4.06 (m, 1H), 3.97 (ddt, *J* = 12.5, 5.6, 1.4 Hz, 1H), 3.75 (ddt, *J* = 12.5, 5.6, 1.5 Hz, 1H), 3.58 - 3.50 (m, 1H), 2.45 (s, 3H), 1.83 - 1.73 (m, 2H), 1.11 (d, *J =* 6.1 Hz, 3H) ppm. **MS** (m/z): (ESI⁺) calculated for: C₁₄H₂₁O₄S [M+H]⁺: 285.1, found: 285.2

### (S)-3-(allyloxy)butyl 4-methylbenzenesulfonate (B14)

General procedure N was applied to **S59. Yield:1.57** g, 70 %, colorless oil. **¹H NMR** (500 MHz, CDCl₃): δ 7.83 - 7.76 (m, 2H), 7.36 - 7.30 (m, 2H), 5.78 (ddt, *J* = 17.2, 10.4, 5.6 Hz, 1H), 5.21 - 5.15 (m, 1H), 5.14 - 5.07 (m, 1H), 4.28 - 4.14 (m, 1H), 4.09 (dt, *J* = 9.7, 5.5 Hz, 1H), 3.97 (ddt, *J* = 12.5, 5.7, 1.5 Hz, 1H), 3.74 (ddt, *J* = 12.5, 5.6, 1.5 Hz, 1H), 3.58 - 3.49 (m, 1H), 2.44 (s, 3H), 1.82 - 1.74 (m, 2H), 1.11 (d, *J* = 6.2 Hz, 3H) ppm. **MS** (m/z): (ESI⁺) calculated for: C₁₄H₂₁O₄S [M+H]⁺: 285.1, found: 285.2

### (2R)-ethyl 2-((tetrahydro-2H-pyran-2-yl)oxy)propanoate (S61)

Synthesized as shown for **S62** with (R)-ethyl 2-hydroxypropanoate as the substrate.

### (2S)-ethyl 2-((tetrahydro-2H-pyran-2-yl)oxy)propanoate (S62)

### Synthesized according to literature procedure.⁶

### (2R)-2-((tetrahydro-2H-pyran-2-yl)oxy)propan-1-ol (S63)

**S61** (12.6 g, 62.1 mmol, 1.0 eq.) was dissolved in anhydrous THF (230 mL) under an argon atmosphere. The solution was cooled to 0 °C. LiAlH₄ (2.0 M in THF, 34.2 mL, 68.3 mmol, 1.1 eq.) was added dropwise. The reaction mixture was stirred at this temperature for 30 minutes, allowed to reach ambient temperature and stirred for further 30 minutes. Water was added followed by 1M NaOH. The resulting mixture was stirred for 10 minutes at room temperature and then filtered through a pad of celite. The filtrate was dried with MgSO₄, filtered and concentrated under reduced pressure. The title compound was obtained as a colorless oil without further purification (9.34 g, 93 %). **¹H NMR** (500 MHz, CDCl₃) δ 4.98 - 4.53 (m, 1H), 4.28 - 3.26 (m, 5H), 2.66 (s, 1H), 1.91 - 1.46 (m, 6H), 1.38 - 1.10 (m, 3H) ppm.

### (2S)-2-((tetrahydro-2H-pyran-2-yl)oxy)propan-1-ol (S64)

Synthesized as shown for **S63** with **S62** as substrate. **Yield:** 5.18 g, 93 %, dr: 1:1.4, colorless oil.**¹H NMR** (500 MHz, CDCl₃) δ 4.77 - 4.48 (m, 1H), 4.03 - 3.90 (m, 1H), 3.90 - 3.77 (m, 1H), 3.70 - 3.54 (m, 1H), 3.53 - 3.42 (m, 2H), 1.91 - 1.68 (m, 2H), 1.63 - 1.48 (m, 4H), 1.23 - 1.09 (m, 3H) ppm.

### 2-(((R)-1-(allyloxy)propan-2-yl)oxy)tetrahydro-2H-pyran (S65)

To as solution of **S63** (2.20 g, 13.7 mmol, 1.0 eq.) in anhydrous THF (40 mL) was added NaH (60 wt% in mineral oil, 1098 mg, 27.5 mmol, 2.0 eq.) at 0°C. After stirring for 30 minutes at this temperature allylbromide (4.80 mL, 54.9 mmol, 4.0 eq.) was added and the reaction mixture was allowed to reach ambient temperature and stirred overnight. The reaction was quenched with water and diluted with diethyl ether. The organic phase was washed with brine and was dried with MgSO₄, filtered and concentrated under reduced pressure. The crude product was purified by flash column chromatography (0 - 25 % EA in CH). The title compound was obtained as a yellow oil (2.15 g, 78 %, dr: 1:1.5) **¹H NMR** (500 MHz, CDCl₃) δ 5.90 (dtt, *J =* 16.0, 10.5, 5.5 Hz, 1H), 5.31 - 5.22 (m, 1H), 5.17 - 5.12 (m, 1H), 4.72 (t, *J* = 3.5 Hz, 1H), 4.08 - 3.84 (m, 4H), 3.57 - 3.33 (m, 3H), 1.88 - 1.64 (m, 2H), 1.54 (tdd, *J* = 17.4, 7.5, 3.9 Hz, 4H), 1.28 - 1.12 (m, 3H), 0.85 (dt, *J* = 19.0, 6.4 Hz, 1H) ppm.

### 2-(((S)-1-(allyloxy)propan-2-yl)oxy)tetrahydro-2H-pyran (S66)

Synthesis as shown for **S65** with **S64** as substrate. **Yield:** 1.97 g, 63 %, dr: 1:1.6, yellow oil.¹H **NMR** (500 MHz, CDCl₃) δ 5.97 - 5.81 (m, 1H), 5.32 - 5.21 (m, 1H), 5.18 - 5.13 (m, 1H), 4.86 - 4.69 (m, 1H), 4.07 - 3.88 (m, 5H), 3.59 - 3.45 (m, 2H), 3.42 - 3.35 (m, 1H), 1.92 - 1.45 (m, 7H), 1.25 - 1.11 (m, 3H) ppm.

### (R)-1-(allyloxy)propan-2-ol (S67)

p-Toluenesulfonic acid monohydrate (413 mg, 2.15 mmol, 0.2 eq.) was added to a solution **of S65** (2.15 g, 10.7 mmol, 1.0 eq) in MeOH (30 mL). The reaction mixture was stirred at room temperature for 15 minutes. The reaction was quenched by the addition of saturated aqueous NaHCO₃ solution. The mixture was extracted with diethyl ether. Combined organic layers were washed with brine, dried with MgSO₄, filtered and concentrated under reduced pressure. The crude product was purified by flash column chromatography (0 - 40 % EA in CH) and gave the title compound as a yellow oil (518 mg, 42 %). **¹H NMR** (500 MHz, CDCl₃) δ 5.91 (dddd, *J* = 15.9, 11.1, 8.4, 5.5 Hz, 1H), 5.34 - 5.24 (m, 2H), 5.22 - 5.15 (m, 1H), 4.04 - 4.00 (m, 2H), 4.00 - 3.93 (m, 1H), 3.47 - 3.40 (m, 1H), 3.27 - 3.19 (m, 1H), 2.33 (s, 1H), 1.14 (d, *J=* 6.4 Hz, 3H) ppm.

### (S)-1-(allyloxy)propan-2-ol (S68)

Synthesis as shown for **S67** with **S66** as substrate. **Yield:** 858 mg, 81 %, yellow oil. **¹H NMR** (500 MHz, CDCl₃) δ 5.95 - 5.86 (m, 1H), 5.31 - 5.25 (m, 1H), 5.22 - 5.16 (m, 1H), 4.02 (dt, *J* = 5.8, 1.4 Hz, 2H), 4.00 - 3.94 (m, 1H), 3.45 - 3.41 (m, 1H), 3.27 - 3.20 (m, 1H), 2.21 (s, 1H), 1.15 (d, *J* = 6.4 Hz, 3H) ppm.

### (S)-1-(allyloxy)propan-2-yl 4-methylbenzenesulfonate (B9)

General procedure N was applied to **S68. Yield:** 1.16 g, 76 %, yellowish oil. **¹H NMR** (500 MHz, CDCl₃) δ 7.81 (d, *J =* 8.0 Hz, 2H), 7.32 (d, *J* = 7.9 Hz, 2H), 5.84 - 5.67 (m, 1H), 5.22 - 5.10 (m, 2H), 4.78 - 4.64 (m, 1H), 3.92 - 3.83 (m, 2H), 3.47 (dd, *J* = 10.8, 5.9 Hz, 1H), 3.41 (dd, *J* = 10.8, 4.6 Hz, 1H), 2.44 (s, 3H), 1.29 (d, *J* = 6.4 Hz, 3H) ppm. **MS** (m/z): (ESI⁺) calculated for: C₁₃H₁₉O₅S [M+H]⁺: 271.1, found: 271.0

### (R)-1-(allyloxy)propan-2-yl 4-methylbenzenesulfonate (B10)

General procedure N was applied to **S68. Yield:** 766 g, 74 %, yellowish oil. **¹H NMR** (500 MHz, CDCl₃) δ 7.80 (d, *J* = 7.9 Hz, 2H), 7.32 (d, *J* = 8.0 Hz, 2H), 5.76 (ddt, *J* = 16.4, 10.9, 5.6 Hz, 1H), 5.20 - 5.11 (m, 2H), 4.79 - 4.64 (m, 1H), 3.94 - 3.81 (m, 2H), 3.46 (dd, *J* = 10.8, 5.9 Hz, 1H), 3.40 (dd, *J* = 10.8, 4.6 Hz, 1H), 2.44 (s, 3H), 1.29 (d, *J* = 6.5 Hz, 3H) ppm. **MS** (m/z): (ESI⁺) calculated for: C₁₃H₁₉O₅S [M+H]⁺: 271.1, found: 271.0

### (3R)-methyl 3-((tetrahydro-2H-pyran-2-yl)oxy)butanoate (S71)

(*R*)-methyl 3-hydroxybutanoate (3.0 g, 25.4 mmol, 1.0 eq.) was dissolved in anhydrous diethyl ether (23 mL) and a catalytic amount of *p*-toluenesulfonic acid monohydrate (20 mg, 0.102 mmol, 0.36 mol%) was added under argon. The solution was cooled in an ice bath and 3,4-dihydro-2*H-*pyran (3.04 mL, 33.0 mmol, 1.3 eq.) was added slowly. The mixture was stirred at 0°C for 1 h and then at room temperature overnight. The reaction was quenched by adding saturated aqueous NaHCO₃ solution and stirred for 15 min. The organic phase was decanted and the aqueous phase extracted with diethyl ether. The combined organic layers were washed with brine, dried with MgSO₄ and concentrated under reduced pressure. The crude product was purified by flash column chromatography (0 -25 % EA in CH). The title compound was obtained as a colorless oil (5.28 g, quant., dr: 1:1.1). **¹H NMR** (300 MHz, CDCl₃) δ 4.74-4.70 (m, 1H), 4.29-4.11 (m, 2H), 3.94 - 3.74 (m, 1H), 3.66 (d, *J* = 3.4 Hz, 3H), 3.56 - 3.38 (m, 1H), 2.74 - 2.50 (m, 1H), 2.49 - 2.31 (m, 1H), 1.87 - 1.39 (m, 5H), 1.18 (d, *J* = 6.1 Hz, 3H) ppm. **MS** (m/z): (ESI⁺) calculated for: C₁₀H₁₈O₄Na [M+Na]⁺: 225.1, found: 225.2

### (3S)-methyl 3-((tetrahydro-2H-pyran-2-yl)oxy)butanoate (S72)

Synthesis as shown for S71 with (*S*)-methyl 3-hydroxybutanoate as the substrate. **Yield:** 4.90 g, 95 %, dr: 1:1.1, colorless oil **¹H NMR** (300 MHz, CDCl₃) δ 4.72 (t, *J* = 3.5 Hz, 1H), 4.33-4.10 (m, 1H), 3.96 - 3.78 (m, 1H), 3.69 - 3.65 (m, 3H), 3.54 - 3.42 (m, 2H), 2.73 - 2.49 (m, 1H), 2.47 - 2.28 (m, 1H), 1.90 - 1.43 (m, 5H), 1.22 - 1.16 (m, 3H) ppm. **MS** (m/z): (ESI⁺) calculated for: C₁₀H₁₈O₄Na [M+Na]⁺: 225.1, found: 225.2

### (3R)-3-((tetrahydro-2H-pyran-2-yl)oxy)butan-1-ol (S73)

**S71** (4.00 g, 20.7 mmol, 1.0 eq.) was dissolved in anhydrous THF (44 mL) under an argon atmosphere. The solution was cooled to 0 °C. LiAlH₄ (2.0 M in THF, 10.9 mL, 21.8 mmol, 1.1 eq.) was added dropwise. The reaction mixture was stirred at this temperature for 30 minutes and then allowed to reach ambient temperature. After stirring for 30 minutes at room temperature saturated aqueous Rochelle salt solution was added and the mixture was stirred at room temperature. The aqueous phase was extracted with diethyl ether. Combined organic layers were dried with MgSO₄, filtered and concentrated under reduced pressure. The product was obtained as a yellowish oil (3.44 g, quant.) without further purification. **¹H NMR** (300 MHz, CDCl₃) δ 4.74 - 4.49 (m, 1H), 4.18 - 3.56 (m, 4H), 3.56 - 3.39 (m, 1H), 2.70 (s, 1H), 1.97 - 1.37 (m, 8H), 1.36 - 1.08 (m, 3H) ppm. **MS** (m/z): (ESI⁺) calculated for: C₉H₁₈O₃Na [M+Na]⁺: 197.1, found: 197.2

### (3S)-3-((tetrahydro-2H-pyran-2-yl)oxy)butan-1-ol (S74)

Synthesis as shown for **S73** with **S72** as substrate. **Yield:** 3.37 g, 90 %, yellowish oil. |**¹H NMR** (300 MHz, CDCl₃) δ 4.72 - 4.45 (m, 1H), 4.12 - 3.58 (m, 4H), 3.57 - 3.41 (m, 1H), 2.59 (s, 1H), 1.88 - 1.35 (m, 8H), 1.31 - 1.08 (m, 3H) ppm. **MS** (m/z): (ESI⁺) calculated for: C₉H₁₈O₃Na [M+Na]⁺: 197.1, found: 197.2

### 2-(((S)-4-(allyloxy)butan-2-yl)oxy)tetrahydro-2H-pyran (S75)

To as solution of **S74** (3.37 g, 19.3 mmol, 1.0 eq.) in anhydrous THF (60 mL) was added NaH (60 wt% in mineral oil, 1.55 g, 38.7 mmol, 2.0 eq.) at 0°C. After stirring for 30 minutes at this temperature allylbromide (6.75 mL, 55.0 mmol, 4.0 eq.) was added and the reaction mixture was allowed to reach ambient temperature and stirred overnight. The reaction was quenched with water and diluted with diethyl ether. The organic phase was washed with brine and was dried with MgSO₄, filtered and concentrated under reduced pressure. The crude product was purified by flash column chromatography (0 - 20 % EA in CH) to give the title compound as a yellowish oil (2.98 g, 77 %, dr: 1:1.1). **¹H NMR** (500 MHz, CDCl₃) δ 5.96 - 5.81 (m, 1H), 5.29 - 5.22 (m, 1H), 5.18 - 5.11 (m, 1H), 4.71 - 4.56 (m, 1H), 4.03 - 3.80 (m, 4H), 3.65 - 3.40 (m, 3H), 1.87 - 1.44 (m, 8H), 1.25 - 1.11 (m, 3H) ppm. **MS** (m/z): (ESI⁺) calculated for: C₁₂H₂₂O₃Na [M+Na]⁺: 237.2, found: 237.2

### 2-(((R)-4-(allyloxy)butan-2-yl)oxy)tetrahydro-2H-pyran (S76)

Synthesis as shown for **S75** with **S73** as substrate. **Yield:** 1.57 g, 57 %, dr: 1:1, yellowish oil. **¹H NMR** (500 MHz, CDCl₃) δ 5.96 - 5.84 (m, 1H), 5.32 - 5.21 (m, 1H), 5.18 - 5.11 (m, 1H), 4.74 - 4.57 (m, 1H), 4.03 - 3.77 (m, 4H), 3.61 - 3.41 (m, 3H), 1.92 - 1.64 (m, 4H), 1.61 - 1.38 (m, 4H), 1.29 - 1.07 (m, 3H) ppm. **MS** (m/z): (ESI⁺) calculated for: C₁₂H₂₂O₃Na [M+Na]⁺: 237.2, found: 237.2

### (R)-4-(allyloxy)butan-2-ol (S77)

*p*-Toluenesulfonic acid monohydrate (282 mg, 1.47 mmol, 0.2 eq.) was added to a solution of **S76** (1.57 g, 7.33 mmol, 1.0 eq.) in MeOH (24 mL). The reaction mixture was stirred at room temperature. The reaction was quenched by the addition of saturated aqueous NaHCO₃ solution. The mixture was extracted with diethyl ether. Combined organic layers were washed with brine, dried with MgSO₄, filtered and concentrated under reduced pressure. The crude product was purified by flash column chromatography (0 - 60 % diethyl ether in pentane). The title compound was obtained as a colorless oil (730 mg, 77 %). **¹H NMR** (500 MHz, CDCl₃) δ 5.89 (ddt, *J* = 16.4, 10.8, 5.6 Hz, 1H), 5.28 - 5.21 (m, 1H), 5.21 -5.12 (m, 1H), 4.03 - 3.93 (m, 3H), 3.66 (dt, *J* = 10.1, 5.2 Hz, 1H), 3.59 (td, *J* = 8.8, 4.5 Hz, 1H), 2.70 (s, 1H), 1.79 - 1.63 (m, 2H), 1.19 (d, *J* = 6.3 Hz, 3H) ppm. **MS** (m/z): (ESI⁺) calculated for: C₇H₁₄O₂Na [M+Na]⁺: 153.1, found: 153.0

### (S)-4-(allyloxy)butan-2-ol (S78)

Synthesis as shown for **S77** with **S75** as substrate. **Yield:** 1.53 g, 85 %, colorless oil. **¹H NMR** (500 MHz, CDCl₃) δ 5.88 (ddt, *J* = 16.4, 10.8, 5.6 Hz, 1H), 5.27 - 5.22 (m, 1H), 5.16 (d, *J* = 10.4 Hz, 1H), 3.97 (d, *J* = 5.7 Hz, 3H), 3.65 (dt, *J* = 10.2, 5.3 Hz, 1H), 3.58 (td, *J* = 8.8, 4.6 Hz, 1H), 2.79 (s, 1H), 1.77 - 1.64 (m, 2H), 1.18 (d, *J=* 6.2 Hz, 3H) ppm. **MS** (m/z): (ESI⁺) calculated for: C₇H₁₄O₂Na [M+Na]⁺: 153.1, found: 153.0

### (R)-4-(allyloxy)butan-2-yl 4-methylbenzenesulfonate (B17)

General procedure N was applied to **S77. Yield:** 1.16 g, 74 %, yellowish oil. **¹H NMR** (500 MHz, CDCl₃) δ 7.79 (d, *J* = 7.9 Hz, 2H), 7.32 (d, *J* = 7.9 Hz, 2H), 5.81 (ddt, *J* = 16.4, 10.8, 5.6 Hz, 1H), 5.28 - 5.06 (m, 2H), 4.86 - 4.72 (m, 1H), 3.95 - 3.69 (m, 2H), 3.37 (dt, *J* = 10.7, 5.6 Hz, 1H), 3.27 (td, *J* = 8.7, 5.3 Hz, 1H), 2.43 (s, 3H), 1.87 (ddt, *J* = 13.6, 7.7, 5.5 Hz, 1H), 1.80 - 1.71 (m, 1H), 1.30 (d, *J =* 6.3 Hz, 3H) ppm. **MS** (m/z): (ESI⁺) calculated for: C₁₄H₂₁O₄S [M+H]⁺: 285.1, found: 285.0

### (S)-4-(allyloxy)butan-2-yl 4-methylbenzenesulfonate (B18)

General procedure N was applied to **S78. Yield:** 1.66 g, 76 %, yellowish oil. **¹H NMR** (500 MHz, CDCl₃) δ 7.79 (d, *J* = 7.9 Hz, 2H), 7.32 (d, *J* = 7.9 Hz, 2H), 5.80 (ddt, *J* = 16.4, 10.9, 5.6 Hz, 1H), 5.22 - 5.15 (m, 1H), 5.14 - 5.11 (m, 1H), 4.82 - 4.73 (m, 1H), 3.82 - 3.70 (m, 2H), 2.43 (s, 3H), 1.91-1.81 (m, 1H), 1.79-1.70 (m, 1H), 1.29 (d, *J* = 6.3 Hz, 3H) ppm. **MS** (m/z): (ESI⁺) calculated for: C₁₄H₂₀O₄SNa [M+Na]⁺: 307.1, found: 307.2

### Example 2e: Synthesis of Cyclization Precursors

### Synthesis of p2-Building Blocks

### (S)-allyl 1-((S)-2-(4-((tert-butyldiphenylsilyl)oxy)-3,5-dimethoxyphenyl)-2-cyclohexylacetyl)piperidine-2-carboxylate (p2(1))

General procedure A was applied to carboxylic acid **p1** and amine **A1. Yield:** 1.87 g, 97 %, colorless resin. **¹H NMR** (500 MHz, CDCl₃, mixture of rotamers, major rotamer reported): δ 7.75 - 7.62 (m, 4H), 7.32 (ttd, J = 6.0, 3.1, 1.7 Hz, 2H), 7.28 - 7.21 (m, 4H), 6.25 - 6.23 (m, 2H), 5.76 - 5.68 (m, 1H), 5.30 - 5.24 (m, 1H), 5.19 - 5.06 (m, 2H), 4.68 - 4.63 (m, 1H), 4.51 - 4.40 (m, 1H), 3.86 - 3.75 (m, 1H), 3.42 - 3.37 (m, 6H), 3.22 (d, J = 9.5 Hz, 1H), 2.88 - 2.76 (m, 1H), 2.27 - 2.15 (m, 1H), 2.10 - 1.79 (m, 3H), 1.71 - 1.50 (m, 7H), 1.36 - 1.24 (m, 2H), 1.20 - 1.00 (m, 11H), 0.92 - 0.80 (m, 1H), 0.69 - 0.54 (m, 1H) ppm. **MS** (m/z): (ESI⁺) calculated for: C₄₁H₅₄NO₆Si [M+H]⁺: 684.5, found: 684.2

### (S)-but-3-en-1-yl 1-((S)-2-(4-((tert-butyldiphenylsilyl)oxy)-3,5-dimethoxyphenyl)-2-cyclohexylacetyl)piperidine-2-carboxylate (p2(2))

General procedure A was applied to carboxylic acid **p1** and amine **A2. Yield:** 1.45 g, 75 %, colorless resin. **¹H NMR** (500 MHz, CDCl₃, mixture of rotamers, major rotamer reported): δ 7.67 (ddd, J = 6.5, 4.9, 2.4 Hz, 4H), 7.32 (dddd, J = 8.8, 5.7, 2.9, 1.4 Hz, 2H), 7.28 - 7.23 (m, 4H), 6.25 (s, 2H), 5.68 - 5.57 (m, 1H), 5.28 - 5.24 (m, 1H), 5.17 - 5.06 (m, 1H), 5.03 - 4.97 (m, 1H), 4.28 - 4.16 (m, 1H), 4.03 (ddt, J = 30.5, 10.8, 6.8 Hz, 1H), 3.40 (s, 6H), 3.22 (d, J = 9.5 Hz, 1H), 2.88 - 2.79 (m, 1H), 2.46 - 2.37 (m, 1H), 2.24 - 2.16 (m, 2H), 2.09 - 1.86 (m, 2H), 1.83 (d, J = 12.6 Hz, 1H), 1.70 - 1.48 (m, 6H), 1.40 - 1.21 (m, 3H), 1.10 (s, 12H), 0.91 - 0.70 (m, 1H), 0.68 - 0.54 (m, 1H) ppm.

### (S)-2-(allyloxy)ethyl 1-((S)-2-(4-((tert-butyldiphenylsilyl)oxy)-3,5-dimethoxyphenyl)-2-cyclohexylacetyl)piperidine-2-carboxylate (p2(3))

General procedure A was applied to carboxylic acid **p1** and amine **A3. Yield:** 1.70 g, 89 %, colorless resin. **¹H NMR** (500 MHz, CDCl₃, mixture of rotamers, major rotamer reported): δ 7.73 - 7.61 (m, 4H), 7.39 - 7.19 (m, 6H), 6.24 (s, 2H), 5.98 - 5.73 (m, 1H), 5.27 - 5.09 (m, 3H), 4.20 (ddd, J = 11.9, 6.5, 3.9 Hz, 1H), 4.11 - 4.04 (m, 1H), 4.01 - 3.96 (m, 2H), 3.92 - 3.85 (m, 1H), 3.79 (d, J = 14.2 Hz, 1H), 3.49 (ddd, J = 11.1, 5.9, 3.9 Hz, 1H), 3.39 (s, 6H), 3.22 (d, J = 9.5 Hz, 1H), 2.91-2.82 (m, 1H), 2.22 (d, J = 14.0 Hz, 1H), 2.09-1.80 (m, 3H), 1.72 - 1.48 (m, 6H), 1.41 - 1.20 (m, 3H), 1.11 (s, 11H), 0.91 - 0.75 (m, 1H), 0.67 - 0.53 (m, 1H) ppm. **MS** (m/z): (ESI⁺) calculated for: C₄₃H₅₈NO₇Si [M+H]⁺: 728.4, found: 728.4

### (S)-3-(allyloxy)propyl 1-((S)-2-(4-((tert-butyldiphenylsilyl)oxy)-3,5-dimethoxyphenyl)-2-cyclohexylacetyl)piperidine-2-carboxylate (p2(4))

General procedure A was applied to carboxylic acid **p1** and amine **A4. Yield:** 1.48 g, 71 %, colorless resin. **¹H NMR** (500 MHz, CDCl₃, mixture of rotamers, major rotamer reported): δ 7.73 - 7.61 (m, 4H), 7.43 - 7.20 (m, 6H), 6.24 (s, 2H), 5.96 - 5.81 (m, 1H), 5.35 - 5.06 (m, 3H), 4.30 - 4.24 (m, 1H), 4.15 - 4.02 (m, 1H), 3.99 - 3.76 (m, 2H), 3.49 (t, J = 6.1 Hz, 1H), 3.39 (s, 6H), 3.35 - 3.24 (m, 1H), 3.22 (d, J = 9.4 Hz, 1H), 3.00 (d, J = 9.6 Hz, 1H), 2.86 - 2.78 (m, 1H), 2.26 - 2.16 (m, 1H), 2.08 - 1.85 (m, 3H), 1.78 - 1.49 (m, 7H), 1.41 - 1.21 (m, 3H), 1.21 - 0.99 (m, 12H), 0.93 - 0.73 (m, 1H), 0.70 - 0.56 (m, 1H). ppm. **MS** (m/z): (ESI⁺) calculated for: C₄₃H₅₈NO₇Si [M+H]⁺: 728.4, found: 728.4

### Synthesis of m2 Building Blocks

### (S)-allyl 1-((S)-2-(3-((tert-butyldiphenylsilyl)oxy)-4,5-dimethoxyphenyl)-2-cyclohexylacetyl)piperidine-2-carboxylate (m2(1))

General procedure A was applied to carboxylic acid **m1** and amine **A1. Yield:** 243 mg, 51 %, colorless resin. **¹H NMR** (500 MHz, CDCl₃, mixture of rotamers, major rotamer reported): δ 7.78 - 7.67 (m, 4H), 7.47 - 7.30 (m, 6H), 6.42 (s, 1H), 5.91 (s, 1H), 5.68 (ddt, J = 16.4, 10.8, 5.5 Hz, 1H), 5.14 - 5.08 (m, 1H), 4.51 - 4.40 (m, 2H), 3.87 (s, 3H), 3.77 (s, 3H), 3.32 (d, J = 13.5 Hz, 1H), 2.81 (d, J = 9.7 Hz, 1H), 2.51 -2.43 (m, 1H), 2.22 -2.17 (m, 1H), 1.93 - 1.80 (m, 1H), 1.76 - 1.66 (m, 1H), 1.64 - 1.44 (m, 6H), 1.39 - 1.15 (m, 4H), 1.11 (s, 9H), 1.07 - 0.90 (m, 3H), 0.83 (ddd, J = 29.5, 14.5, 7.7 Hz, 1H), 0.67 - 0.52 (m, 1H), 0.29 - 0.13 (m, 1H) ppm. **MS** (m/z): (ESI⁺) calculated for: C₄₁H₅₄NO₆Si [M+H]⁺: 684.4, found: 684.0

### (S)-but-3-en-1-yl 1-((S)-2-(3-((tert-butyldiphenylsilyl)oxy)-4,5-dimethoxyphenyl)-2-cyclohexylacetyl)piperidine-2-carboxylate (m2(2))

General procedure A was applied to carboxylic acid **m1** and amine **A2. Yield:** 411 mg, 85 %, colorless resin **¹H NMR** (500 MHz, CDCl₃, mixture of rotamers, major rotamer reported): δ 7.77 - 7.68 (m, 4H), 7.47 - 7.29 (m, 6H), 6.45 - 6.41 (m, 1H), 5.95 - 5.90 (m, 1H), 5.76 - 5.54 (m, 1H), 5.25 - 5.20 (m, 1H), 5.08 - 4.94 (m, 2H), 4.16 (t, J = 6.6 Hz, 1H), 4.07 (dt, J = 10.7, 6.8 Hz, 1H), 3.95 (dt, J = 10.8, 6.6 Hz, 1H), 3.85 (s, 3H), 3.79 (s, 3H), 3.34 (d, J = 13.8 Hz, 1H), 2.81 (d, J = 9.7 Hz, 1H), 2.56 - 2.49 (m, 1H), 2.25 - 2.08 (m, 2H), 1.88 - 1.79 (m, 1H), 1.78 - 1.53 (m, 5H), 1.52 - 1.14 (m, 4H), 1.11 (s, 10H), 1.08 - 0.74 (m, 3H), 0.69 - 0.50 (m, 1H), 0.25 - 0.13 (m, 1H) ppm. **MS** (m/z): (ESI⁺) calculated for: C₄₂H₅₆NO₆Si [M+H]⁺: 698.4, found: 698.0

### (S)-2-(allyloxy)ethyl 1-((S)-2-(3-((tert-butyldiphenylsilyl)oxy)-4,5-dimethoxyphenyl)-2-cyclohexylacetyl)piperidine-2-carboxylate (m2(3))

General procedure A was applied to carboxylic acid **m1** and amine **A3. Yield:** 201 mg, 77 %, colorless resin. **¹H NMR** (500 MHz, CDCl₃, mixture of rotamers, major rotamer reported): δ 7.78 - 7.69 (m, 4H), 7.45 - 7.31 (m, 6H), 6.45 - 6.39 (m, 1H), 5.95 - 5.91 (m, 1H), 5.89 - 5.79 (m, 1H), 5.29 - 5.11 (m, 3H), 4.28 - 4.19 (m, 1H), 4.07 - 4.01 (m, 1H), 3.93 - 3.87 (m, 2H), 3.87 - 3.83 (m, 3H), 3.81 - 3.77 (m, 3H), 3.51 - 3.46 (m, 1H), 3.41 - 3.29 (m, 1H), 2.82 (d, J = 9.6 Hz, 1H), 2.64 - 2.41 (m, 1H), 2.23 - 2.14 (m, 1H), 1.84 (dd, J = 19.4, 11.3 Hz, 1H), 1.78 - 1.44 (m, 8H), 1.40 - 1.14 (m, 3H), 1.12 (s, 9H), 1.09 - 0.72 (m, 3H), 0.68 - 0.53 (m, 1H), 0.25 - 0.15 (m, 1H) ppm. **MS** (m/z): (ESI⁺) calculated for: C₄₃H₅₈NO₇Si [M+H]⁺: 728.4, found: 728.0

### (S)-3-(allyloxy)propyl 1-((S)-2-(3-((tert-butyldiphenylsilyl)oxy)-4,5-dimethoxyphenyl)-2-cyclohexylacetyl)piperidine-2-carboxylate (m2(4))

General procedure A was applied to carboxylic acid **m1** and amine **A3. Yield:** 392 mg, 94 %, colorless resin. **¹H NMR** (500 MHz, CDCl₃, mixture of rotamers, major rotamer reported): δ 7.73 (dt, J = 10.3, 6.6 Hz, 4H), 7.44 - 7.31 (m, 6H), 6.45 - 6.40 (m, 2H), 5.93 (d, J = 2.3 Hz, 1H), 5.92 - 5.80 (m, 2H), 5.30 - 5.10 (m, 4H), 4.08 (ddt, J = 31.5, 11.2, 6.5 Hz, 2H), 3.97 - 3.88 (m, 2H), 3.85 (s, 3H), 3.79 (s, 3H), 3.44 (t, J = 6.1 Hz, 1H), 3.41 - 3.25 (m, 2H), 2.82 (d, J = 9.7 Hz, 1H), 2.59 - 2.49 (m, 1H), 2.16 (d, J = 14.0 Hz, 1H), 1.93 - 1.45 (m, 8H), 1.32 - 1.16 (m, 2H), 1.11 (s, 9H), 1.07 - 0.83 (m, 3H), 0.71 - 0.52 (m, 1H), 0.29 - 0.14 (m, 1H) ppm. **MS** (m/z): (ESI⁺) calculated for: C₄₄H₆₀NO₇Si [M+H]⁺: 742.4, found: 742.4

### (S)-(S)-pent-4-en-2-yl 1-((S)-2-(3-((tert-butyldiphenylsilyl)oxy)-4,5-dimethoxyphenyl)-2-cyclohexylacetyl)piperidine-2-carboxylate (m2(5))

General procedure A was applied to carboxylic acid **m1** and amine **A5. Yield:** 383 mg, 89 %, colorless resin. **¹H NMR** (300 MHz, CDCl₃, mixture of rotamers, major rotamer reported): δ 7.81 - 7.29 (m, 10H), 6.54 - 6.41 (m, 1H), 5.97 - 5.87 (m, 1H), 5.74 - 5.52 (m, 1H), 5.26 - 4.73 (m, 4H), 4.64 - 4.20 (m, 1H), 3.92 - 3.75 (m, 7H), 3.42 - 3.28 (m, 1H), 2.88 - 2.61 (m, 1H), 2.59 - 2.23 (m, 1H), 2.23-2.07 (m, 2H), 1.95-1.46 (m, 8H), 1.33-1.18 (m, 2H), 1.15-1.11 (m, 10H), 1.06 (d, J = 6.3 Hz, 3H), 1.03 - 0.82 (m, 3H), 0.71 - 0.10 (m, 2H) ppm.

### (S)-(S)-pent-4-en-2-yl 1-((S)-2-(3-((tert-butyldiphenylsilyl)oxy)-4,5-dimethoxyphenyl)-2-cyclohexylacetyl)piperidine-2-carboxylate (m2(6))

General procedure A was applied to carboxylic acid **m1** and amine **A6. Yield:** 623 mg, 93 %, colorless resin.. **¹H NMR** (500 MHz, CDCl₃, mixture of rotamers, major rotamer reported): δ 7.81 - 7.64 (m, 6H), 7.46 - 7.29 (m, 6H), 6.46 (d, J = 1.9 Hz, 1H), 5.92 (d, J = 1.9 Hz, 1H), 5.60 - 5.42 (m, 1H), 5.22 (d, J = 5.6 Hz, 1H), 5.08 - 4.92 (m, 3H), 4.85 (q, J = 6.3 Hz, 1H), 4.49 (d, J = 13.5 Hz, 1H), 4.23 (d, J = 5.5 Hz, 1H), 3.85 (s, 6H), 3.36 (d, J = 13.7 Hz, 1H), 2.80 (d, J = 9.8 Hz, 1H), 2.71 - 2.43 (m, 2H), 2.39 - 2.13 (m, 2H), 2.13 - 1.98 (m, 2H), 1.96 - 1.35 (m, 6H), 1.11 (s, 9H), 1.04 (d, J = 6.3 Hz, 3H), 1.00 - 0.79 (m, 3H), 0.59 (q, J = 14.8, 12.7 Hz, 1H), 0.27 - 0.09 (m, 1H) ppm.

### (S)-(R)-1-((4-methoxybenzyl)oxy)pent-4-en-2-yl 1-((S)-2-(3-((tert-butyldiphenylsilyl)oxy)-4,5-dimethoxyphenyl)-2-cyclohexylacetyl)piperidine-2-carboxylate (m2(7))

General procedure A was applied to carboxylic acid **m1** and amine **A7. Yield:** 251 mg, 79 %, colorless resin. **¹H NMR** (500 MHz, CDCl₃, mixture of rotamers, major rotamer reported): δ 7.75 - 7.69 (m, 4H), 7.44 - 7.27 (m, 6H), 7.23 - 7.18 (m, 2H), 6.88 - 6.83 (m, 2H), 6.46 (d, J = 1.8 Hz, 1H), 5.95 (d, J = 1.9 Hz, 1H), 5.67 - 5.55 (m, 1H), 5.28 - 5.27 (m, 1H), 5.18 - 4.87 (m, 3H), 4.43 - 4.29 (m, 2H), 3.83 (s, 3H), 3.79 - 3.78 (m, 3H), 3.75 (s, 3H), 3.45 - 3.30 (m, 2H), 2.83 (d, J = 9.8 Hz, 1H), 2.68 - 2.61 (m, 1H), 2.28 - 2.06 (m, 2H), 1.90 - 1.65 (m, 3H), 1.63 - 1.43 (m, 5H), 1.37 - 1.15 (m, 4H), 1.14 - 1.07 (m, 9H), 1.06 - 0.80 (m, 4H), 0.67 - 0.51 (m, 1H), 0.25 - 0.10 (m, 1H) ppm.

### (S)-(S)-1-((4-methoxybenzyl)oxy)pent-4-en-2-yl 1-((S)-2-(3-((tert-butyldiphenylsilyl)oxy)-4,5-dimethoxyphenyl)-2-cyclohexylacetyl)piperidine-2-carboxylate (m2(8))

General procedure A was applied to carboxylic acid **m1** and amine **A8. Yield:** 346 mg, 87 %, colorless resin. **¹H NMR** (500 MHz, CDCl₃, mixture of rotamers, major rotamer reported): δ 7.78 - 7.67 (m, 4H), 7.43 - 7.29 (m, 6H), 7.22 (d, J = 8.5 Hz, 2H), 6.89 - 6.84 (m, 2H), 6.45 - 6.42 (m, 1H), 5.94 - 5.90 (m, 1H), 5.67 - 5.50 (m, 1H), 5.27 - 5.25 (m, 1H), 5.06 - 4.85 (m, 3H), 4.51 - 4.35 (m, 3H), 3.85 (s, 2H), 3.80 - 3.79 (m, 4H), 3.50 - 3.28 (m, 3H), 2.80 (d, J = 9.8 Hz, 1H), 2.63 - 2.48 (m, 2H), 2.37 - 2.25 (m, 1H), 2.22 - 2.14 (m, 1H), 2.13 - 2.04 (m, 1H), 1.89 - 1.80 (m, 1H), 1.77 - 1.66 (m, 1H), 1.61 - 1.44 (m, 5H), 1.40 - 1.16 (m, 4H), 1.12 (s, 9H), 1.02 - 0.81 (m, 4H), 0.69 - 0.48 (m, 1H), 0.26 - 0.09 (m, 1H) ppm.

### (S)-(2R,3S)-3-methylpent-4-en-2-yl 1-((S)-2-(3-((tert-butyldiphenylsilyl)oxy)-4,5-dimethoxyphenyl)-2-cyclohexylacetyl)piperidine-2-carboxylate (m2(9))

General procedure A was applied to carboxylic acid **m1** and amine **A9. Yield:** 1.82 g, 95 %, colorless resin. **¹H NMR** (500 MHz, CDCl₃, mixture of rotamers, major rotamer reported): δ 7.80 - 7.68 (m, 4H), 7.44 - 7.30 (m, 6H), 6.48 (d, J = 2.0 Hz, 1H), 5.92 (d, J = 1.9 Hz, 1H), 5.48 - 5.40 (m, 1H), 5.26 - 5.22 (m, 1H), 5.01 - 4.83 (m, 3H), 4.82 - 4.76 (m, 1H), 3.85 (s, 3H), 3.78 (s, 3H), 3.39 - 3.34 (m, 1H), 2.79 (d, J = 9.9 Hz, 1H), 2.66 - 2.57 (m, 1H), 2.21 - 2.15 (m, 1H), 2.15 - 2.07 (m, 1H), 1.90 - 1.45 (m, 8H), 1.29 - 1.16 (m, 2H), 1.12 (s, 9H), 1.00 (d, J = 6.4 Hz, 3H), 0.99 - 0.90 (m, 2H), 0.90 - 0.85 (m, 1H), 0.84 (d, J = 7.0 Hz, 3H), 0.69 - 0.47 (m, 1H), 0.24 - 0.10 (m, 1H) ppm.

### (S)-(2R,3R)-3-methylpent-4-en-2-yl 1-((S)-2-(3-((tert-butyldiphenylsilyl)oxy)-4,5-dimethoxyphenyl)-2-cyclohexylacetyl)piperidine-2-carboxylate (m2(10))

General procedure A was applied to carboxylic acid **m1** and amine **A10. Yield** 398 mg, 73 %, colorless reisn. **¹H NMR** (500 MHz, CDCl₃, mixture of rotamers, major rotamer reported): δ 7.77 - 7.70 (m, 5H), 7.44 - 7.31 (m, 6H), 6.47 (d, J = 2.0 Hz, 1H), 5.94 - 5.89 (m, 1H), 5.54 (ddd, J = 16.5, 10.9, 7.6 Hz, 1H), 5.25 (d, J = 5.7 Hz, 1H), 5.04 - 4.95 (m, 2H), 4.75 - 4.68 (m, 1H), 3.86 (s, 3H), 3.79 (d, J = 3.6 Hz, 3H), 3.37 (d, J = 14.0 Hz, 1H), 2.79 (d, J = 9.9 Hz, 1H), 2.64 - 2.56 (m, 1H), 2.19 (d, J = 13.9 Hz, 1H), 2.12 (p, J = 6.9 Hz, 1H), 1.84 (q, J = 11.5 Hz, 1H), 1.77 - 1.44 (m, 5H), 1.33 - 1.16 (m, 4H), 1.11 (s, 9H), 1.02 (d, J = 6.4 Hz, 3H), 1.00 - 0.80 (m, 3H), 0.77 (d, J = 6.8 Hz, 3H), 0.68 - 0.48 (m, 1H), 0.24 - 0.10 (m, 1H) ppm.

### (2R,3S)-3-(methoxymethoxy)pent-4-en-2-yl (S)-1-((S)-2-(3-((tert-butyldiphenylsilyl)oxy)-4,5-dimethoxyphenyl)-2-cyclohexylacetyl)piperidine-2-carboxylate (m2(11))

General procedure A was applied to carboxylic acid **m1** amine **A11. Yield:** 2.00 g, 92 %, colorless resin. **¹H NMR** (500 MHz, CDCl₃, mixture of rotamers, major rotamer reported): δ 7.78 - 7.69 (m, 4H), 7.45 - 7.30 (m, 6H), 6.47 (d, J = 1.9 Hz, 1H), 5.93 (d, J = 1.9 Hz, 1H), 5.32 - 5.19 (m, 2H), 5.18 - 5.09 (m, 1H), 4.99 (td, J = 6.5, 4.2 Hz, 1H), 4.60 (d, J = 6.7 Hz, 1H), 4.54 - 4.48 (m, 1H), 3.89 - 3.82 (m, 4H), 3.78 (s, 3H), 3.37 - 3.33 (m, 1H), 3.33 (s, 3H), 2.79 (d, J = 9.8 Hz, 1H), 2.75 - 2.67 (m, 1H), 2.24 - 2.17 (m, 1H), 1.89 - 1.78 (m, 2H), 1.78 - 1.43 (m, 6H), 1.33 - 1.16 (m, 4H), 1.12 (s, 9H), 1.06 (d, J = 6.5 Hz, 3H), 1.03 - 0.76 (m, 3H), 0.65 - 0.50 (m, 1H), 0.25 - 0.12 (m, 1H ppm.

### Synthesis of p3 Building Blocks

### (S)-allyl 1-((S)-2-(4-((tert-butyldiphenylsilyl)oxy)-3,5-dimethoxyphenyl)-2-cyclohexylacetyl)piperidine-2-carboxylate (p3(1))

General procedure B was applied to TBDPS-protected phenol **p2(1). Yield:** 1.14 g, 92 %, beige solid. **¹H NMR** (500 MHz, CDCl₃, mixture of rotamers, major rotamer reported): δ 6.46 (s, 2H), 5.69 (ddt, J = 17.3, 10.7, 5.5 Hz, 1H), 5.38 - 5.34 (m, 1H), 5.20 - 5.04 (m, 2H), 4.68 (ddd, J = 5.4, 2.4, 1.2 Hz, 1H), 4.55 - 4.39 (m, 2H), 3.91 (dt, J = 13.7, 3.3 Hz, 1H), 3.86 (s, 6H), 3.34 (d, J = 9.6 Hz, 1H), 2.89 (td, J = 13.2, 3.1 Hz, 1H), 2.31 - 2.22 (m, 1H), 2.15 - 2.02 (m, 2H), 1.91 - 1.84 (m, 1H), 1.74 -1.51 (m, 6H), 1.36 - 1.00 (m, 5H), 0.90 (tdd, J = 12.6, 10.9, 3.5 Hz, 1H), 0.83 - 0.64 (m, 1H) ppm. **MS** (m/z): (ESI⁺) calculated for: C₂₅H₃₆NO₆ [M+H]⁺: 446.3, found: 446.2

### (S)-but-3-en-1-yl 1-((S)-2-cyclohexyl-2-(4-hydroxy-3,5-dimethoxyphenyl)acetyl)piperidine-2-carboxylate (p3(2))

General procedure B was applied to TBDPS-protected phenol **p2(2). Yield:** 892 mg, 98 %, beige solid. **¹H NMR** (500 MHz, CDCl₃, mixture of rotamers, major rotamer reported): δ 6.48 (s, 2H), 5.64 - 5.52 (m, 1H), 5.35 - 5.29 (m, 1H), 4.98 (dq, J = 4.5, 1.7 Hz, 1H), 4.34 - 4.20 (m, 1H), 4.11 - 3.94 (m, 1H), 3.88 (s, 0H), 3.86 (s, 4H), 3.33 (d, J = 9.7 Hz, 1H), 2.89 (td, J = 13.3, 3.0 Hz, 1H), 2.43 (qt, J = 6.6, 1.4 Hz, 1H), 2.26 - 1.99 (m, 4H), 1.90 - 1.83 (m, 1H), 1.72 - 1.50 (m, 8H), 1.48 - 1.37 (m, 1H), 1.36 - 1.22 (m, 3H), 1.21 - 0.99 (m, 4H), 0.96 - 0.59 (m, 2H) ppm. **MS** (m/z): (ESI⁺) calculated for: C₂₆H₃₈NO₆ [M+H]⁺: 460.3, found: 460.2

### (S)-2-(allyloxy)ethyl 1-((S)-2-cyclohexyl-2-(4-hydroxy-3,5-dimethoxyphenyl)acetyl)piperidine-2-carboxylate (p3(3))

General procedure B was applied to TBDPS-protected phenol **p2(3). Yield:** 948 mg, 98 %, beige solid. **¹H NMR** (500 MHz, CDCl₃, mixture of rotamers, major rotamer reported): δ 6.49 (d, J = 4.8 Hz, 2H), 5.83 (ddt, J = 17.4, 10.7, 5.5 Hz, 1H), 5.42 (s, 1H), 5.41 - 5.36 (m, 1H), 5.27 - 5.12 (m, 2H), 4.47 - 4.33 (m, 1H), 4.25 (ddd, J = 11.9, 6.8, 3.7 Hz, 1H), 4.19 - 3.99 (m, 1H), 3.89 (d, J = 2.1 Hz, 7H), 3.70 (t, J = 4.8 Hz, 1H), 3.51 (ddd, J = 11.1, 5.8, 3.7 Hz, 1H), 3.41 - 3.32 (m, 1H), 2.96 (td, J = 13.2, 3.0 Hz, 1H), 2.74 - 2.51 (m, 0H), 2.32 - 2.23 (m, 1H), 2.16 - 2.02 (m, 1H), 1.93 - 1.87 (m, 1H), 1.77 - 1.53 (m, 6H), 1.52 - 1.41 (m, 1H), 1.39-1.00 (m, 6H), 0.99-0.63 (m, 2H) ppm. **MS** (m/z): (ESI⁺) calculated for: C₂₇H₄₀NO₇ [M+H]⁺: 460.3, found: 490.4.

### (S)-3-(allyloxy)propyl 1-((S)-2-cyclohexyl-2-(4-hydroxy-3,5-dimethoxyphenyl)acetyl)piperidine-2-carboxylate (p3(4))

General procedure B was applied to TBDPS-protected phenol **p2(4). Yield:** 878 mg, 90 %, beige solid. **¹H NMR** (500 MHz, CDCl₃, mixture of rotamers, major rotamer reported): δ 6.48 (s, 2H), 5.97 - 5.77 (m, 1H), 5.40 (s, 1H), 5.35 - 5.32 (m, 1H), 5.27 - 5.09 (m, 2H), 4.36 - 4.26 (m, 1H), 4.12 (dt, J = 11.1, 6.5 Hz, 1H), 4.05 (dt, J = 11.0, 6.3 Hz, 1H), 3.97 (dt, J = 5.6, 1.4 Hz, 1H), 3.95 - 3.89 (m, 1H), 3.88 - 3.84 (m, 6H), 3.51 (t, J = 6.1 Hz, 1H), 3.34 (d, J = 9.7 Hz, 1H), 3.32 - 3.19 (m, 2H), 2.89 (td, J = 13.3, 3.0 Hz, 1H), 2.29 - 2.21 (m, 1H), 2.16 - 1.98 (m, 1H), 1.95 (q, J = 6.2 Hz, 1H), 1.87 (d, J = 12.7 Hz, 1H), 1.77 - 1.51 (m, 6H), 1.49 - 1.38 (m, 1H), 1.37 - 0.99 (m, 5H), 0.97 - 0.63 (m, 2H) ppm. **MS** (m/z): (ESI⁺) calculated for: C₂₈H₄₂NO₇ [M+H]⁺: 504.3, found: 504.4.

### Synthesis of m3 Building Blocks

### (S)-allyl 1-((S)-2-cyclohexyl-2-(3-hydroxy-4,5-dimethoxyphenyl)acetyl)piperidine-2-carboxylate (m3(1))

General procedure B was applied to TBDPS-protected phenol **m2(1). Yield:** 142 mg, 87 %, beige solid. **¹H NMR** (500 MHz, CDCl₃, mixture of rotamers, major rotamer reported): δ 6.46 (d, J = 1.9 Hz, 1H), 6.42 (d, J = 1.8 Hz, 1H), 5.75 - 5.72 (m, 1H), 5.72 - 5.65 (m, 1H), 5.37 (dd, J = 6.1, 1.9 Hz, 1H), 5.17 - 5.07 (m, 1H), 4.79 - 4.58 (m, 1H), 4.50 (ddt, J = 13.4, 5.6, 1.4 Hz, 1H), 4.45 (ddt, J = 13.5, 5.5, 1.4 Hz, 1H), 3.93 (dt, J = 14.0, 3.2 Hz, 1H), 3.87 (s, 3H), 3.82 (s, 3H), 3.33 (d, J = 9.7 Hz, 1H), 2.91 (td, J = 13.3, 3.0 Hz, 1H), 2.31 -2.22 (m, 1H), 2.16-2.04 (m, 1H), 1.87 (d, J = 13.4 Hz, 1H), 1.72 - 1.53 (m, 7H), 1.37 - 1.21 (m, 3H), 1.12 (tt, J = 15.3, 11.1 Hz, 2H), 0.97 - 0.82 (m, 1H), 0.74 (tdd, J = 23.8, 11.2, 3.2 Hz, 1H) ppm. **MS** (m/z): (ESI⁺) calculated for: C₂₅H₃₆NO₆ [M+H]⁺: 446.2, found: 446.0.

### (S)-but-3-en-1-yl 1-((S)-2-cyclohexyl-2-(3-hydroxy-4,5-dimethoxyphenyl)acetyl)piperidine-2-carboxylate (m3(2))

General procedure B was applied to TBDPS-protected phenol **m2(2). Yield:** 244 mg, 94 %, beige solid. **¹H NMR** (500 MHz, CDCl₃, mixture of rotamers, major rotamer reported): δ 6.48 - 6.46 (m, 1H), 6.44 - 6.43 (m, 1H), 5.77 - 5.75 (m, 1H), 5.62 - 5.52 (m, 1H), 5.34 (d, J = 5.8 Hz, 1H), 5.00 - 4.91 (m, 2H), 4.08 (dt, J = 10.8, 6.8 Hz, 1H), 3.98 (t, J = 6.6 Hz, 1H), 3.94 - 3.89 (m, 1H), 3.86 (s, 3H), 3.83 (s, 3H), 3.32 (d, J = 9.8 Hz, 1H), 2.94 - 2.86 (m, 1H), 2.27 - 2.03 (m, 3H), 1.89 (dd, J = 27.4, 12.5 Hz, 2H), 1.73 - 1.52 (m, 7H), 1.36 - 1.23 (m, 3H), 1.13 (td, J = 13.1, 9.8 Hz, 2H), 0.95 - 0.67 (m, 2H) ppm. **MS** (m/z): (ESI⁺) calculated for: C₂₆H₃₈NO₆ [M+H]⁺: 460.3, found: 460.0.

### (S)-2-(allyloxy)ethyl 1-((S)-2-cyclohexyl-2-(3-hydroxy-4,5-dimethoxyphenyl)acetyl)piperidine-2-carboxylate (m3(3))

General procedure B was applied to TBDPS-protected phenol **m2(3). Yield:** 115 mg, 85 %, colorless resin. **¹H NMR** (500 MHz, CDCl₃, mixture of rotamers, major rotamer reported): δ 6.48 - 6.45 (m, 1H), 6.42 - 6.40 (m, 1H), 5.98 - 5.89 (m, 1H), 5.89 - 5.78 (m, 1H), 5.39 - 5.35 (m, 1H), 5.33 - 5.13 (m, 2H), 4.26 - 4.19 (m, 1H), 4.13 - 4.08 (m, 1H), 3.92 - 3.85 (m, 6H), 3.84 - 3.82 (m, 3H), 3.55 - 3.49 (m, 1H), 3.43 - 3.36 (m, 1H), 3.33 (d, J = 9.5 Hz, 1H), 2.91 (td, J = 13.2, 3.0 Hz, 1H), 2.12 - 2.05 (m, 1H), 1.88 - 1.82 (m, 1H), 1.70 - 1.53 (m, 8H), 1.48 - 1.20 (m, 3H), 1.20 - 1.04 (m, 2H), 0.95 - 0.83 (m, 1H), 0.81 - 0.67 (m, 1H) ppm. **MS** (m/z): (ESI⁺) calculated for: C₂₇H₄₀NO₇ [M+H]⁺: 490.3, found: 490.0.

### (S)-3-(allyloxy)propyl 1-((S)-2-cyclohexyl-2-(3-hydroxy-4,5-dimethoxyphenyl)acetyl)piperidine-2-carboxylate (m3(4))

General procedure B was applied to TBDPS-protected phenol **m2(4). Yield:** 202 mg, 85 %, yellowish resin. **¹H NMR** (500 MHz, CDCl₃, mixture of rotamers, major rotamer reported): δ 6.45 (s, 1H), 6.42 (s, 1H), 5.97 - 5.77 (m, 2H), 5.37 - 5.33 (m, 1H), 5.26 - 5.20 (m, 1H), 5.17 - 5.12 (m, 1H), 4.30 (hept, J = 5.6, 4.8 Hz, 1H), 4.16 - 4.02 (m, 2H), 3.99 - 3.96 (m, 1H), 3.95 - 3.90 (m, 1H), 3.86 (s, 3H), 3.83 (s, 3H), 3.51 (t, J = 6.2 Hz, 1H), 3.39 - 3.27 (m, 2H), 2.92 - 2.83 (m, 1H), 2.24 (d, J = 13.7 Hz, 1H), 2.13-2.05 (m, 1H), 1.98-1.81 (m, 2H), 1.77 - 1.50 (m, 7H), 1.48 - 1.37 (m, 1H), 1.36 - 1.02 (m, 5H), 0.96 - 0.86 (m, 1H), 0.80 - 0.66 (m, 1H) ppm. **MS** (m/z): (ESI⁺) calculated for: C₂₈H₄₂NO₇ [M+H]⁺: 504.3, found: 504.2.

### (S)-(S)-pent-4-en-2-yl 1-((S)-2-cyclohexyl-2-(3-hydroxy-4,5-dimethoxyphenyl)acetyl)piperidine-2-carboxylate (m3(5))

General procedure B was applied to TBDPS-protected phenol **m2(5). Yield:** 222 mg, 88 %, beige solid. **¹H NMR** (500 MHz, CDCl₃, mixture of rotamers, major rotamer reported): δ 6.47 (d, J = 1.9 Hz, 1H), 6.46 (d, J = 1.8 Hz, 1H), 5.79 - 5.72 (m, 1H), 5.58 (ddt, J = 17.4, 10.5, 7.1 Hz, 1H), 5.31 (dd, J = 6.1, 2.1 Hz, 1H), 5.04 - 4.96 (m, 2H), 4.87 (h, J = 6.3 Hz, 1H), 3.98 - 3.90 (m, 1H), 3.85 (s, 3H), 3.83 (s, 3H), 3.33 (d, J = 9.8 Hz, 1H), 2.92 (td, J = 13.3, 2.8 Hz, 1H), 2.42 - 2.31 (m, 1H), 2.26 - 2.20 (m, 1H), 1.89 - 1.83 (m, 1H), 1.76 - 1.49 (m, 6H), 1.42 (qt, J = 12.9, 4.2 Hz, 1H), 1.37 - 1.20 (m, 4H), 1.18 - 1.08 (m, 2H), 1.04 (d, J = 6.3 Hz, 3H), 0.93 - 0.78 (m, 1H), 0.78 - 0.65 (m, 1H) ppm. **MS** (m/z): (ESI⁺) calculated for: C₂₇H₄₀NO₆ [M+H]⁺: 474.3, found: 474.2.

### (S)-(R)-pent-4-en-2-yl 1-((S)-2-cyclohexyl-2-(3-hydroxy-4,5-dimethoxyphenyl)acetyl)piperidine-2-carboxylate (m3(6))

General procedure B was applied to TBDPS-protected phenol **m2(6). Yield:** 194 mg, 83 %, beige solid. **¹H NMR** (500 MHz, CDCl₃, mixture of rotamers, major rotamer reported): δ 6.47 (d, J = 1.9 Hz, 1H), 6.46 (d, J = 1.8 Hz, 1H), 5.77 - 5.75 (m, 1H), 5.64 - 5.52 (m, 1H), 5.31 (dd, J = 6.1, 2.1 Hz, 1H), 5.04 - 4.96 (m, 2H), 4.87 (h, J = 6.3 Hz, 1H), 3.98 - 3.91 (m, 1H), 3.85 (s, 3H), 3.83 (s, 3H), 3.33 (d, J = 9.8 Hz, 1H), 2.92 (td, J = 13.3, 2.8 Hz, 1H), 2.35 (hept, J = 7.0 Hz, 1H), 2.23 (dt, J = 13.4, 2.6 Hz, 1H), 2.18 - 2.00 (m, 2H), 1.89 - 1.82 (m, 1H), 1.78 - 1.47 (m, 6H), 1.42 (tdt, J = 12.9, 7.8, 4.1 Hz, 1H), 1.37 - 1.22 (m, 3H), 1.20 - 1.08 (m, 2H), 1.04 (d, J = 6.3 Hz, 3H), 0.95 - 0.79 (m, 1H), 0.79 - 0.62 (m, 1H) ppm. **MS** (m/z): (ESI⁺) calculated for: C₂₇H₄₀NO₆ [M+H]⁺: 474.3, found: 474.2.

### (S)-(R)-1-((4-methoxybenzyl)oxy)pent-4-en-2-yl 1-((S)-2-cyclohexyl-2-(3-hydroxy-4,5-dimethoxyphenyl)acetyl)piperidine-2-carboxylate (m3(7))

General procedure B was applied to TBDPS-protected phenol **m2(7). Yield:** 149 mg, 86 %, beige solid. **¹H NMR** (500 MHz, CDCl₃, mixture of rotamers, major rotamer reported): δ 7.28 - 7.14 (m, 2H), 6.89 - 6.82 (m, 2H), 6.50 (d, J = 1.9 Hz, 1H), 6.45 (t, J = 2.3 Hz, 1H), 5.98 (s, 1H), 5.58 (ddt, J = 17.1, 10.1, 7.0 Hz, 1H), 5.40 - 5.37 (m, 1H), 5.04 - 4.97 (m, 3H), 4.31 (d, J = 11.4 Hz, 1H), 4.23 (d, J = 11.4 Hz, 1H), 3.88 - 3.78 (m, 9H), 3.52 - 3.46 (m, 1H), 3.32 (dd, J = 10.3, 3.9 Hz, 2H), 3.08 - 3.02 (m, 1H), 2.95 - 2.87 (m, 1H), 2.27 - 2.22 (m, 1H), 2.20 - 2.02 (m, 3H), 1.87 (d, J = 13.8 Hz, 1H), 1.70 - 1.44 (m, 6H), 1.41 - 1.21 (m, 4H), 1.18 - 1.06 (m, 2H), 0.92 - 0.60 (m, 2H) ppm. **MS** (m/z): (ESI⁺) calculated for: C₃₅H₄₈NO₈[M+H]⁺: 610.3, found: 610.2.

### (S)-(S)-1-((4-methoxybenzyl)oxy)pent-4-en-2-yl 1-((S)-2-cyclohexyl-2-(3-hydroxy-4,5-dimethoxyphenyl)acetyl)piperidine-2-carboxylate (m3(8))

General procedure B was applied to TBDPS-protected phenol **m2(8). Yield:** 218 mg, 88 %, beige solid. **¹H NMR** (500 MHz, CDCl₃, mixture of rotamers, major rotamer reported): δ 7.22 - 7.20 (m, 2H), 6.86 - 6.84 (m, 2H), 6.48 (d, J = 2.0 Hz, 1H), 6.42 (d, J = 2.1 Hz, 1H), 5.95 (s, 2H), 5.59 - 5.48 (m, 1H), 5.38 (dd, J = 5.9, 2.2 Hz, 1H), 5.07 - 4.99 (m, 1H), 4.97 - 4.86 (m, 2H), 4.41 (d, J = 11.5 Hz, 1H), 4.37 (d, J = 11.6 Hz, 1H), 3.92 - 3.85 (m, 1H), 3.84 (s, 3H), 3.79 (s, 6H), 3.51 (d, J = 5.5 Hz, 1H), 3.40 - 3.29 (m, 2H), 2.88 (td, J = 13.2, 2.8 Hz, 1H), 2.29 - 2.22 (m, 1H), 2.13 - 2.01 (m, 2H), 1.94 - 1.83 (m, 2H), 1.71 - 1.45 (m, 6H), 1.38 - 1.07 (m, 5H), 0.96 - 0.58 (m, 2H) ppm. **MS** (m/z): (ESI⁺) calculated for: C₃₅H₄₈NO₈[M+H]⁺: 610.3, found: 610.2.

### (S)-(2R,3S)-3-methylpent-4-en-2-yl 1-((S)-2-cyclohexyl-2-(3-hydroxy-4,5-dimethoxyphenyl)acetyl)piperidine-2-carboxylate (m3(9))

General procedure B was applied to TBDPS-protected phenol **m2(9). Yield:** 1.07 g, 89 %, beige solid. **¹H NMR** (500 MHz, CDCl₃, mixture of rotamers, major rotamer reported): δ 6.48 (d, J = 1.9 Hz, 1H), 6.47 (d, J = 1.9 Hz, 1H), 5.47 - 5.39 (m, 1H), 5.36 - 5.33 (m, 1H), 4.91 (ddd, J = 10.3, 1.9, 0.9 Hz, 1H), 4.84 (ddd, J = 17.2, 1.9, 1.1 Hz, 1H), 4.80 - 4.75 (m, 1H), 3.97 - 3.92 (m, 1H), 3.86 (s, 3H), 3.83 (s, 3H), 3.32 (d, J = 10.0 Hz, 1H), 2.96 (td, J = 13.3, 2.8 Hz, 1H), 2.29 - 2.21 (m, 1H), 2.15 - 2.02 (m, 3H), 1.86 (d, J = 12.6 Hz, 1H), 1.71 - 1.51 (m, 7H), 1.50-1.01 (m, 5H), 0.99 (d, J = 6.3 Hz, 3H), 0.93 - 0.84 (m, 1H), 0.82 (d, J = 6.9 Hz, 3H), 0.77 - 0.64 (m, 1H) ppm. **MS** (m/z): (ESI⁺) calculated for: C₂₈H₄₂NO₆[M+H]⁺: 488.3, found: 488.4.

### (S)-(2R,3R)-3-methylpent-4-en-2-yl 1-((S)-2-cyclohexyl-2-(3-hydroxy-4,5-dimethoxyphenyl)acetyl)piperidine-2-carboxylate (m3(10))

General procedure B was applied to TBDPS-protected phenol **m2(10). Yield:** 240 mg, 92 %. **¹H NMR** (500 MHz, CDCl₃, mixture of rotamers, major rotamer reported): δ 6.49 (d, J = 1.9 Hz, 1H), 6.46 (d, J = 1.9 Hz, 1H), 5.88 (s, 1H), 5.57 - 5.47 (m, 1H), 5.34 (dd, J = 5.9, 2.3 Hz, 1H), 4.97 - 4.91 (m, 2H), 4.75 - 4.69 (m, 1H), 3.98 - 3.93 (m, 1H), 3.85 (s, 3H), 3.83 - 3.82 (m, 3H), 3.31 (d, J = 10.0 Hz, 1H), 2.93 (td, J = 13.3, 2.8 Hz, 1H), 2.28 - 2.21 (m, 1H), 2.16 - 2.01 (m, 2H), 1.86 (d, J = 12.1 Hz, 1H), 1.72 - 1.51 (m, 6H), 1.47 - 1.24 (m, 4H), 1.19 - 1.07 (m, 2H), 1.00 (d, J = 6.3 Hz, 3H), 0.87 (qd, J = 13.0, 12.6, 3.7 Hz, 1H), 0.75 (d, J = 6.8 Hz, 3H), 0.72 - 0.62 (m, 1H) ppm. **MS** (m/z): (ESI⁺) calculated for: C₂₈H₄₂NO₆[M+H]⁺: 488.3, found: 488.2.

### (S)-(2R,3R)-3-methylpent-4-en-2-yl 1-((S)-2-cyclohexyl-2-(3-hydroxy-4,5-dimethoxyphenyl)acetyl)piperidine-2-carboxylate (m3(11))

General procedure B was applied to TBDPS-protected phenol **m2(11). Yield:** 1.38 g, 100 %. **¹H NMR** (500 MHz, CDCl₃, mixture of rotamers, major rotamer reported): δ 6.48 (d, J = 1.9 Hz, 1H), 6.47 (d, J = 2.0 Hz, 1H), 5.41 - 5.37 (m, 1H), 5.34 - 5.21 (m, 1H), 5.14 (dd, J = 10.4, 1.9 Hz, 1H), 5.10 - 5.02 (m, 1H), 4.99 - 4.92 (m, 1H), 4.55 (d, J = 6.7 Hz, 1H), 4.47 (d, J = 6.8 Hz, 1H), 3.95 - 3.89 (m, 1H), 3.86 (s, 3H), 3.82 (s, 3H), 3.78 (dd, J = 7.8, 4.7 Hz, 1H), 3.43 - 3.27 (m, 4H), 3.03 - 2.96 (m, 1H), 2.30 - 2.24 (m, 1H), 2.15 - 2.05 (m, 1H), 1.86 (d, J = 12.4 Hz, 1H), 1.70 - 1.52 (m, 6H), 1.48 - 1.37 (m, 1H), 1.36 - 1.07 (m, 6H), 1.05 (d, J = 6.5 Hz, 3H), 0.92 - 0.62 (m, 2H) ppm. **MS** (m/z): (ESI⁺) calculated for: C₂₉H₄₃NO₈Na[M+Na]⁺: 556.3, found: 556.4.

### Synthesis of p4 Building Blocks

### (S)-(2R,3S)-3-methylpent-4-en-2-yl 1-((S)-2-(3-((tert-butyldiphenylsilyl)oxy)-4,5-dimethoxyphenyl)-2-cyclohexylacetyl)piperidine-2-carboxylate (p4(1,1))

General procedure C was applied to phenol **p3(1**with allyl bromide **(B1),** 3.0 eq.), potassium carbonate, in acetonitrile **Yield:** 529 mg, 97 %, colorless resin. **¹H NMR** (500 MHz, CDCl₃, mixture of rotamers, major rotamer reported): δ 6.46 (s, 2H), 6.14 - 6.01 (m, 1H), 5.69 (ddt, J = 17.3, 10.7, 5.5 Hz, 1H), 5.38 - 5.34 (m, 1H), 5.34 - 5.23 (m, 2H), 5.18 - 5.08 (m, 2H), 4.68 (dd, J = 5.9, 1.5 Hz, 1H), 4.51 - 4.41 (m, 4H), 3.96 - 3.89 (m, 1H), 3.81 (s, 6H), 3.36 (d, J = 9.7 Hz, 1H), 2.97 - 2.88 (m, 1H), 2.32 - 2.21 (m, 1H), 2.16 - 2.01 (m, 1H), 1.91 - 1.83 (m, 1H), 1.72 - 1.51 (m, 6H), 1.38 - 1.27 (m, 3H), 1.22 - 1.03 (m, 2H), 1.02 - 0.67 (m, 2H ppm. **MS** (m/z): (ESI⁺) calculated for: C₂₈H₄₀NO₆ [M+H]⁺: 486.3, found: 486.2

### (S)-allyl 1-((S)-2-(4-(but-3-en-1-yloxy)-3,5-dimethoxyphenyl)-2-cyclohexylacetyl)piperidine-2-carboxylate (p4(1,2))

General procedure C was applied to phenol **p3(1)** with 4-bromo-1-butene **(B2),** 5.0 eq.), potassium carbonate in acetonitrile. **Yield:** 525 mg, 97 %, colorless resin. **¹H NMR** (500 MHz, CDCl₃, mixture of rotamers, major rotamer reported): δ 6.45 (s, 2H), 5.99 - 5.85 (m, 1H), 5.69 (ddd, J = 17.2, 10.7, 5.4 Hz, 1H), 5.38 - 5.34 (m, 1H), 5.19 - 5.00 (m, 3H), 4.70 - 4.66 (m, 1H), 4.47 (qdt, J = 13.4, 5.5, 1.4 Hz, 2H), 4.00 (td, J = 7.0, 4.3 Hz, 2H), 3.97 - 3.88 (m, 1H), 3.81 (s, 6H), 3.36 (d, J = 9.8 Hz, 1H), 2.97 - 2.87 (m, 1H), 2.51 (qt, J = 7.1, 1.4 Hz, 2H), 2.34 - 2.19 (m, 1H), 2.15 - 2.00 (m, 1H), 1.90 - 1.82 (m, 1H), 1.76 - 1.50 (m, 6H), 1.38 - 1.00 (m, 6H), 0.98 - 0.85 (m, 1H), 0.84 - 0.66 (m, 1H ppm. **MS** (m/z): (ESI⁺) calculated for: C₂₉H₄₂NO₆ [M+H]⁺: 500.3, found: 500.2.

### (S)-allyl 1-((S)-2-(4-(2-(allyloxy)ethoxy)-3,5-dimethoxyphenyl)-2-cyclohexylacetyl)piperidine-2-carboxylate (p4(1,3))

General procedure C was applied to phenol **p3(1)** with **B3** (4.0 eq.), potassium carbonate (4.0 eq.), in acetonitrile. **Yield:** 140 mg, 82 %, colorless resin. **¹H NMR** (500 MHz, CDCl₃, mixture of rotamers, major rotamer reported): δ 6.45 (s, 2H), 6.04 - 5.86 (m, 1H), 5.81 - 5.61 (m, 1H), 5.37 (dd, J = 5.5, 3.2 Hz, 1H), 5.32 - 5.23 (m, 1H), 5.20 - 5.06 (m, 3H), 4.70 - 4.65 (m, 1H), 4.52 - 4.39 (m, 2H), 4.17 - 4.11 (m, 2H), 4.10 - 4.05 (m, 2H), 3.92 (d, J = 14.1 Hz, 1H), 3.81 (s, 6H), 3.78 - 3.72 (m, 2H), 3.36 (d, J = 9.7 Hz, 1H), 2.92 (td, J = 13.4, 2.5 Hz, 1H), 2.30 - 2.22 (m, 1H), 2.17 - 1.99 (m, 2H), 1.88 (d, J = 12.7 Hz, 1H), 1.75 - 1.51 (m, 4H), 1.50 - 0.98 (m, 4H), 0.96 - 0.85 (m, 1H), 0.83 - 0.68 (m, 1H) ppm. **MS** (m/z): (ESI⁺) calculated for: C₃₀H₄₄NO₇ [M+H]⁺: 530.3, found: 530.2.

### (S)-allyl 1-((S)-2-(4-(3-(allyloxy)propoxy)-3,5-dimethoxyphenyl)-2-cyclohexylacetyl)piperidine-2-carboxylate (p4(1,4))

General procedure C was applied to phenol **p3(1)** with **B4** (4.0 eq.), potassium carbonate (4.0 eq.), in acetonitrile. **Yield:** 138 mg, 77 %, colorless resin. **¹H NMR** (500 MHz, CDCl₃, mixture of rotamers, major rotamer reported): δ 6.45 (d, J = 1.5 Hz, 2H), 6.00 - 5.87 (m, 2H), 5.77 - 5.58 (m, 1H), 5.37 (dd, J = 5.8, 3.4 Hz, 1H), 5.30 - 5.23 (m, 2H), 5.20 - 5.07 (m, 3H), 4.46 (qdd, J = 13.5, 5.5, 1.8 Hz, 2H), 4.15 - 4.02 (m, 2H), 4.00 - 3.97 (m, 2H), 3.96 - 3.89 (m, 1H), 3.80 (s, 6H), 3.66 (td, J = 6.5, 1.5 Hz, 2H), 3.35 (d, J = 9.7 Hz, 1H), 2.92 (td, J = 13.6, 2.5 Hz, 1H), 2.30 - 2.20 (m, 1H), 2.18 - 2.03 (m, 1H), 1.88 (d, J = 12.8 Hz, 1H), 1.75 - 1.50 (m, 5H), 1.37 - 0.97 (m, 6H), 0.96-0.63 (m, 3H) (m, 1H) ppm. **MS** (m/z): (ESI⁺) calculated for: C₃₁H₄₆NO₇ [M+H]⁺: 542.3, found: 542.2.

### (S)-but-3-en-1-yl 1-((S)-2-(4-(allyloxy)-3,5-dimethoxyphenyl)-2-cyclohexylacetyl)piperidine-2-carboxylate (p4(2,1))

General procedure C was applied to phenol **p3(2)** with allylbromide **(B1,** 3.0 eq.), potassium carbonate (3.0 eq.), in acetonitrile. **Yield:** 434 mg, 90 %, colorless resin. **¹H NMR** (500 MHz, CDCl₃, mixture of rotamers, major rotamer reported): δ 6.47 (s, 2H), 6.09 (ddq, J = 18.1, 10.3, 6.0 Hz, 1H), 5.58 (ddt, J = 17.1, 10.5, 6.7 Hz, 1H), 5.35 - 5.32 (m, 1H), 5.18 - 5.08 (m, 2H), 4.99-4.91 (m, 2H), 4.52 - 4.44 (m, 2H), 4.33 - 4.21 (m, 1H), 4.08 (dt, J = 10.7, 6.7 Hz, 1H), 4.00 - 3.94 (m, 1H), 3.90 (d, J = 3.4 Hz, 1H), 3.82 (s, 6H), 3.36 (d, J = 9.8 Hz, 1H), 2.93 (td, J = 13.2, 3.0 Hz, 1H), 2.43 (qt, J = 6.7, 1.4 Hz, 1H), 2.27 - 1.97 (m, 5H), 1.90 - 1.82 (m, 1H), 1.73 - 1.47 (m, 4H), 1.41 - 1.05 (m, 5H), 1.03 - 0.64 (m, 2H) ppm. **MS** (m/z): (ESI⁺) calculated for: C₂₉H₄₂NO₆ [M+H]⁺: 500.3, found: 500.2.

### (S)-but-3-en-1-yl 1-((S)-2-(4-(but-3-en-1-yloxy)-3,5-dimethoxyphenyl)-2-cyclohexylacetyl)piperidine-2-carboxylate (p4(2,2)

General procedure C was applied to phenol **p3(2)** with 4-bromo-1-butene **(B2,** 7.0 eq.), potassium carbonate in acetonitrile. **Yield:** 441 mg, 88 %, colorless resin. **¹H NMR** (500 MHz, CDCl₃, mixture of rotamers, major rotamer reported): δ 6.47 (s, 2H), 5.99 - 5.85 (m, 1H), 5.63 - 5.50 (m, 1H), 5.33 (dd, J = 6.1, 2.2 Hz, 1H), 5.17 - 5.08 (m, 1H), 5.04 (ddq, J = 10.2, 3.2, 1.6 Hz, 1H), 4.99 - 4.91 (m, 2H), 4.25 (qt, J = 10.8, 6.6 Hz, 1H), 4.04 - 3.94 (m, 3H), 3.90 (q, J = 2.8 Hz, 1H), 3.82 (s, 6H), 3.35 (d, J = 9.8 Hz, 1H), 2.93 (td, J = 13.2, 3.0 Hz, 1H), 2.51 (qt, J = 7.0, 1.4 Hz, 2H), 2.31 - 2.04 (m, 3H), 1.87 (dt, J = 12.5, 3.1 Hz, 1H), 1.76 - 1.47 (m, 7H), 1.38 - 0.96 (m, 6H), 0.93 - 0.65 (m, 2H) ppm. **MS** (m/z): (ESI⁺) calculated for: C₃₀H₄₄NO₆ [M+H]⁺: 514.3, found: 514.2.

### (R)-2-(allyloxy)ethyl 1-((S)-2-(4-(allyloxy)-3,5-dimethoxyphenyl)-2-cyclohexylacetyl)piperidine-2-carboxylate (p4(3,1)

General procedure C was applied to phenol **p3(3)** with allyl bromide **(B1,** 3.0 eq.), potassium carbonate in acetonitrile. **Yield:** 391 mg, 78 %, colorless resin. **¹H NMR** (500 MHz, CDCl₃, mixture of rotamers, major rotamer reported): δ 6.16 - 6.02 (m, 1H), 5.96 - 5.73 (m, 1H), 5.39 - 5.33 (m, 1H), 5.34 - 5.07 (m, 4H), 4.52 - 4.45 (m, 2H), 4.25 (ddd, J = 11.8, 6.8, 3.5 Hz, 1H), 4.07 - 3.98 (m, 1H), 3.93 - 3.82 (m, 2H), 3.82 (s, 6H), 3.67 (t, J = 4.8 Hz, 1H), 3.49 (ddd, J = 11.1, 5.8, 3.6 Hz, 1H), 3.39 (d, J = 9.1 Hz, 1H), 3.39 - 3.30 (m, 1H), 3.04 - 2.93 (m, 1H), 2.36 - 2.22 (m, 1H), 2.16 - 2.01 (m, 1H), 1.87 (d, J = 12.8 Hz, 1H), 1.79 - 1.50 (m, 6H), 1.48 -1.10 (m, 6H), 0.95 - 0.70 (m, 2H) ppm. **MS** (m/z): (ESI⁺) calculated for: C₃₀H₄₄NO₇ [M+H]⁺: 530.3, found: 530.4.

### (S)-2-(allyloxy)ethyl 1-((S)-2-(4-(but-3-en-1-yloxy)-3,5-dimethoxyphenyl)-2-cyclohexylacetyl)piperidine-2-carboxylate (p4(3,2))

General procedure C was applied to phenol **p3(3**with 4-bromo-1-butene **(B2,** 7.0 eq.), potassium carbonate in acetonitrile. **Yield:** 186 mg, 67 %, colorless **resin. ¹H NMR** (500 MHz, CDCl₃, mixture of rotamers, major rotamer reported): δ 6.47 - 6.45 (m, 2H), 5.99 - 5.86 (m, 2H), 5.81 (dddd, J = 17.6, 10.8, 6.2, 5.0 Hz, 1H), 5.39 - 5.32 (m, 1H), 5.33 - 5.00 (m, 4H), 4.41 - 4.30 (m, 1H), 4.25 (ddd, J = 10.8, 6.9, 3.7 Hz, 1H), 4.08 - 3.96 (m, 3H), 3.94 - 3.83 (m, 2H), 3.81 (s, 6H), 3.73 - 3.63 (m, 1H), 3.49 (ddd, J = 11.5, 5.6, 3.5 Hz, 1H), 3.39 - 3.33 (m, 2H), 3.07 - 2.91 (m, 1H), 2.51 (qd, J = 7.0, 3.6 Hz, 2H), 2.25 (d, J = 13.7 Hz, 1H), 2.08 (dt, J = 29.6, 14.0 Hz, 2H), 1.87 (d, J = 12.7 Hz, 1H), 1.71 - 1.50 (m, 5H), 1.39 - 0.97 (m, 4H), 0.97 - 0.64 (m, 2H) ppm. **MS** (m/z): (ESI⁺) calculated for: C₃₁H₄₆NO₇ [M+H]⁺: 543.3, found: 544.2.

### (S)-3-(allyloxy)propyl 1-((S)-2-(4-(allyloxy)-3,5-dimethoxyphenyl)-2-cyclohexylacetyl)piperidine-2-carboxylate (p4(4,1))

General procedure C was applied to phenol **p3(4)** with allyl bromide **(B1,** 7.0 eq.), potassium carbonate in acetonitrile. **Yield:** 322 mg, 77 %, colorless resin. **¹H NMR** (500 MHz, CDCl₃, mixture of rotamers, major rotamer reported): δ 6.47 (s, 2H), 6.16 - 6.02 (m, 1H), 5.96 - 5.79 (m, 1H), 5.36 - 5.33 (m, 1H), 5.32 - 5.24 (m, 2H), 5.25 - 5.10 (m, 3H), 4.54 - 4.46 (m, 3H), 4.34 - 4.22 (m, 1H), 4.12 (dt, J = 10.9, 6.6 Hz, 1H), 4.05 (dt, J = 10.9, 6.3 Hz, 1H), 3.98 - 3.95 (m, 1H), 3.96 - 3.90 (m, 1H), 3.89 - 3.85 (m, 1H), 3.82 (s, 6H), 3.51 (t, J = 6.1 Hz, 1H), 3.36 (d, J = 9.7 Hz, 1H), 3.34 - 3.21 (m, 1H), 2.93 (td, J = 13.3, 3.0 Hz, 1H), 2.32 - 2.18 (m, 1H), 2.15 - 1.98 (m, 2H), 1.95 (d, J = 6.2 Hz, 1H), 1.87 (d, J = 12.6 Hz, 1H), 1.76 - 1.67 (m, 2H), 1.66 - 1.60 (m, 2H), 1.55 (t, J = 13.0 Hz, 1H), 1.36 - 1.07 (m, 4H), 1.04 - 0.64 (m, 2H) ppm. **MS** (m/z): (ESI⁺) calculated for: C₃₁H₄₆NO₇ [M+H]⁺: 543.3 found: 542.4.

### Synthesis of m4 Building Blocks

### Allyl (S)-1-((S)-2-(3-(allyloxy)-4,5-dimethoxyphenyl)-2-cyclohexylacetyl)piperidine-2-carboxylate (m4(1,1))

**S79⁴** (1.57 g, 3.90 mmol, 1.0 eq.) was dissolved in acetonitrile (100 mL) and potassium carbonate (1.62 g, 11.6 mmol, 3.0 eq.) was added and the resulting mixture was heated to reflux and stirred overnight. The mixture was filtered over a pad of celite. Solvents were evaporated under reduced pressure. Flash column chromatography (0-40 % EA in CH) afforded the title compound as a colorless resin (1.66 g, 83 %). **¹H NMR** (500 MHz, CDCl₃, mixture of rotamers, major rotamer reported): δ 6.48 - 6.43 (m, 2H), 6.08 - 5.98 (m, 1H), 5.67 (ddt, J = 17.3, 10.7, 5.5 Hz, 1H), 5.45 - 5.31 (m, 3H), 5.27 - 5.18 (m, 1H), 5.15 - 5.05 (m, 1H), 4.55 (ddt, J = 6.8, 2.8, 1.5 Hz, 2H), 4.48 (ddt, J = 13.4, 5.5, 1.5 Hz, 1H), 4.43 (ddt, J = 13.5, 5.5, 1.4 Hz, 1H), 3.96 - 3.86 (m, 1H), 3.82 (s, 3H), 3.81 (s, 3H), 3.33 (d, J = 9.7 Hz, 1H), 2.97 - 2.87 (m, 1H), 2.29 - 2.20 (m, 1H), 2.16 - 1.99 (m, 1H), 1.88 - 1.82 (m, 1H), 1.74 - 1.52 (m, 5H), 1.48 - 1.37 (m, 1H), 1.37 - 1.24 (m, 3H), 1.23 - 1.01 (m, 3H), 0.98 - 0.83 (m, 1H), 0.82 - 0.63 (m, 1H) ppm. **MS** (m/z): (ESI⁺) calculated for: C₂₈H₄₀NO₆ [M+H]⁺: 486.3 found: 486.2.

### Allyl (S)-1-((S)-2-(3-(but-3-en-1-yloxy)-4,5-dimethoxyphenyl)-2-cyclohexylacetyl)piperidine-2-carboxylate (m4(1,2))

General procedure C was applied to phenol **m3(1)** 4-bromobut-1-ene **(B2,** 8.0 eq.), potassium carbonate, in acetonitrile. **Yield:** 75 mg, 99 %, colorless resin. **¹H NMR** (500 MHz, CDCl₃, mixture of rotamers, major rotamer reported): δ 6.46 (s, 2H), 6.04 - 5.84 (m, 2H), 5.68 (ddt, J = 16.4, 10.7, 5.5 Hz, 1H), 5.36 (dd, J = 6.3, 2.2 Hz, 1H), 5.21 - 5.00 (m, 4H), 4.55 - 4.46 (m, 1H), 4.46 - 4.41 (m, 1H), 4.03 (td, J = 6.8, 1.8 Hz, 2H), 3.82 (s, 6H), 3.34 (d, J = 9.7 Hz, 1H), 2.92 (td, J = 13.2, 3.0 Hz, 1H), 2.59 - 2.49 (m, 3H), 2.29 - 2.22 (m, 1H), 2.14 - 2.01 (m, 1H), 1.87 (d, J = 12.7 Hz, 1H), 1.78 - 1.52 (m, 5H), 1.50 - 1.38 (m, 1H), 1.37 - 1.03 (m, 5H), 0.99 - 0.60 (m, 2H) ppm. **MS** (m/z): (ESI⁺) calculated for: C₂₉H₄₂NO₆ [M+H]⁺: 500.3 found: 500.2.

### Allyl (S)-1-((S)-2-(3-(2-(allyloxy)ethoxy)-4,5-dimethoxyphenyl)-2-cyclohexylacetyl)piperidine-2-carboxylate (m4(1,3))

General procedure C was applied to phenol **m3(1)** with **B3** (4.0 eq.), potassium carbonate in acetonitrile. **Yield:** 73 mg, 96 %, colorless resin. **¹H NMR** (500 MHz, CDCl₃, mixture of rotamers, major rotamer reported): δ 6.49 - 6.44 (m, 2H), 5.97 - 5.87 (m, 1H), 5.68 (ddt, J = 16.3, 10.7, 5.5 Hz, 1H), 5.40 - 5.07 (m, 5H), 4.51 - 4.47 (m, 1H), 4.46 - 4.40 (m, 1H), 4.15 (t, J = 5.1 Hz, 2H), 4.09 (dd, J = 5.6, 1.6 Hz, 2H), 3.99 - 3.87 (m, 1H), 3.83 (s, 3H), 3.81 (s, 3H), 3.34 (d, J = 9.6 Hz, 1H), 2.90 (td, J = 13.2, 3.0 Hz, 1H), 2.31 - 2.21 (m, 1H), 2.15 - 2.04 (m, 1H), 1.90 - 1.84 (m, 1H), 1.79 - 1.53 (m, 6H), 1.49 - 1.37 (m, 1H), 1.37 - 1.01 (m, 7H), 0.95 - 0.65 (m, 2H) ppm. **MS** (m/z): (ESI⁺) calculated for: C₃₀H₄₅NO₇ [M+H]⁺: 530.3 found: 530.2.

### Allyl (S)-1-((S)-2-(3-(3-(allyloxy)propoxy)-4,5-dimethoxyphenyl)-2-cyclohexylacetyl)piperidine-2-carboxylate (m4(1,4))

General procedure C was applied to phenol **m3(1)** with **B4** (1.5 eq.), cesium carbonate (, in DMF. **Yield:** 64 mg, 75 %, colorless resin. **¹H NMR** (500 MHz, CDCl₃, mixture of rotamers, major rotamer reported): δ 6.49 - 6.42 (m, 2H), 5.90 (ddt, J = 16.7, 10.9, 5.6 Hz, 1H), 5.67 (ddt, J = 16.4, 10.8, 5.5 Hz, 1H), 5.37 - 5.34 (m, 1H), 5.30 - 5.23 (m, 2H), 5.17 - 5.06 (m, 2H), 4.51 - 4.39 (m, 2H), 4.07 (t, J = 6.3 Hz, 2H), 4.00 - 3.96 (m, 2H), 3.92 (d, J = 13.7 Hz, 1H), 3.83 - 3.79 (m, 6H), 3.62 (t, J = 6.3 Hz, 2H), 3.33 (d, J = 9.7 Hz, 1H), 2.96 - 2.86 (m, 1H), 2.24 (d, J = 13.6 Hz, 1H), 2.14 - 2.00 (m, 4H), 1.93 - 1.74 (m, 2H), 1.73 - 1.48 (m, 5H), 1.47 - 1.37 (m, 1H), 1.37 - 1.00 (m, 5H), 0.95 - 0.64 (m, 2H) ppm. **MS** (m/z): (ESI⁺) calculated for: C₃₁H₄₅NO₇ [M+H]+: 544.3, found: 544.4

### but-3-en-1-yl (S)-1-((S)-2-(3-(allyloxy)-4,5-dimethoxyphenyl)-2-cyclohexylacetyl)piperidine-2-carboxylate (m4(2,1))

General procedure C was applied to phenol ***m*3(2)** with allyl bromide **(B1,** 5.0 eq.), cesium carbonate in DMF. **Yield:** 98 mg, 82 %, colorless resin. **¹H NMR** (500 MHz, CDCl₃, mixture of rotamers, major rotamer reported): δ 6.53 - 6.40 (m, 2H), 6.09 - 5.98 (m, 1H), 5.84 - 5.52 (m, 1H), 5.46 - 5.31 (m, 2H), 5.28 - 5.21 (m, 1H), 5.18 - 4.92 (m, 2H), 4.73 - 4.51 (m, 2H), 4.31 - 4.03 (m, 2H), 4.01 - 3.93 (m, 1H), 3.93 - 3.87 (m, 1H), 3.87 - 3.80 (m, 6H), 3.39 - 3.04 (m, 1H), 2.99 - 2.55 (m, 1H), 2.48 - 2.36 (m, 1H), 2.27 - 1.99 (m, 3H), 1.95 - 1.82 (m, 1H), 1.71 - 1.51 (m, 6H), 1.37 - 0.99 (m, 5H), 0.94 - 0.64 (m, 2H) ppm. **MS** (m/z): (ESI⁺) calculated for: C₂₉H₄₂NO₆ [M+H]+: 500.3, found: 500.2

### but-3-en-1-yl (S)-1-((S)-2-(3-(but-3-en-1-yloxy)-4,5-dimethoxyphenyl)-2-cyclohexylacetyl)piperidine-2-carboxylate (m4(2,2))

General procedure C was applied to phenol **m3(2)** with 4-bromobut-1-ene **(B2,** 5.0 eq.), cesium carbonate in DMF. **Yield:** 106 mg, 89 %, colorless resin. **¹H NMR** (500 MHz, CDCl₃, mixture of rotamers, major rotamer reported): δ 6.47 (s, 2H), 5.95 - 5.85 (m, 1H), 5.62 - 5.51 (m, 1H), 5.36 - 5.31 (m, 1H), 5.20 - 5.06 (m, 2H), 5.00 - 4.90 (m, 2H), 4.32 - 4.21 (m, 1H), 4.10 - 3.95 (m, 4H), 3.93 - 3.87 (m, 1H), 3.83 (s, 3H), 3.81 (s, 3H), 3.34 (d, J = 9.7 Hz, 1H), 2.92 (td, J = 13.2, 2.9 Hz, 1H), 2.55 (qt, J = 6.8, 1.3 Hz, 2H), 2.27 - 1.99 (m, 4H), 1.86 (d, J = 12.2 Hz, 1H), 1.75 - 1.51 (m, 6H), 1.37 - 1.01 (m, 5H), 0.94 - 0.66 (m, 2H). ppm. **MS** (m/z): (ESI⁺) calculated for: C₃₀H₄₄NO₆ [M+H]+: 514.3, found: 514.2

### but-3-en-1-yl (S)-1-((S)-2-(3-(2-(allyloxy)ethoxy)-4,5-dimethoxyphenyl)-2-cyclohexylacetyl)piperidine-2-carboxylate (m4(2,3))

General procedure C was applied to phenol **m3(2)** with **B3** (2.0 eq.), cesium carbonate in DMF. **Yield:** 59 mg, 73 %, colorless resin. **¹H NMR** (500 MHz, CDCl₃, mixture of rotamers, major rotamer reported): δ 6.53 - 6.46 (m, 2H), 5.99 - 5.89 (m, 1H), 5.63 - 5.54 (m, 1H), 5.38 - 5.09 (m, 2H), 5.01 - 4.94 (m, 1H), 4.33 - 4.23 (m, 1H), 4.20 - 4.15 (m, 2H), 4.14 - 4.06 (m, 3H), 3.99 (dt, J = 10.7, 6.5 Hz, 1H), 3.91 (dd, J = 10.3, 7.2 Hz, 1H), 3.88 - 3.80 (m, 8H), 3.35 (d, J = 9.7 Hz, 1H), 2.93 (td, J = 13.3, 3.0 Hz, 1H), 2.45 (qt, J = 6.7, 1.4 Hz, 1H), 2.27 - 2.03 (m, 3H), 1.88 (d, J = 12.6 Hz, 1H), 1.80 - 1.54 (m, 6H), 1.45 (qt, J = 12.8, 3.9 Hz, 1H), 1.39 - 1.03 (m, 6H), 0.98 - 0.65 (m, 2H) ppm. **MS** (m/z): (ESI⁺) calculated for: C₃₁H₄₆NO₇ [M+H]+: 544.3, found: 544.2

### but-3-en-1-yl (S)-1-((S)-2-(3-(3-(allyloxy)propoxy)-4,5-dimethoxyphenyl)-2-cyclohexylacetyl)piperidine-2-carboxylate (m4(2,4))

General procedure C was applied to phenol **m3(2)** with **B4** (2.0 eq.), cesium carbonate in DMF **Yield:** 66 mg, 72 %, colorless resin. **¹H NMR** (500 MHz, CDCl₃, mixture of rotamers, major rotamer reported): δ 6.49 - 6.43 (m, 2H), 5.96 - 5.86 (m, 1H), 5.62 - 5.52 (m, 1H), 5.35 - 5.31 (m, 1H), 5.30 - 5.24 (m, 1H), 5.19 - 5.14 (m, 1H), 4.98 - 4.91 (m, 1H), 4.31 - 4.20 (m, 1H), 4.11 - 4.04 (m, 3H), 4.00 - 3.94 (m, 3H), 3.94 - 3.88 (m, 1H), 3.85 - 3.79 (m, 6H), 3.63 (t, J = 6.2 Hz, 2H), 3.34 (d, J = 9.7 Hz, 1H), 2.92 (td, J = 13.3, 2.9 Hz, 1H), 2.46-2.40 (m, 1H), 2.26-2.00 (m, 5H), 1.97 - 1.82 (m, 1H), 1.81 - 1.51 (m, 6H), 1.48 - 1.00 (m, 6H), 1.00 - 0.64 (m, 2H) ppm. **MS** (m/z): (ESI⁺) calculated for: C₃₂H₄₈NO₇ [M+H]+: 558.3, found: 558.2.

### 2-(allyloxy)ethyl (S)-1-((S)-2-(3-(allyloxy)-4,5-dimethoxyphenyl)-2-cyclohexylacetyl)piperidine-2-carboxylate (m4(3,1))

General procedure C was applied to phenol **m3(3)** with allyl bromide **B1,** 5.0 eq.), cesium carbonate in DMF. **Yield:** 102 mg, 79 %, colorless resin. **¹H NMR** (500 MHz, CDCl₃, mixture of rotamers, major rotamer reported): δ 6.47 (s, 2H), 6.15 - 5.98 (m, 1H), 5.85 - 5.74 (m, 1H), 5.44 - 5.08 (m, 5H), 4.66 - 4.51 (m, 2H), 4.42 - 4.29 (m, 1H), 4.25 (ddd, J = 11.9, 6.8, 3.5 Hz, 1H), 4.09 - 3.98 (m, 2H), 3.92 - 3.86 (m, 1H), 3.84 (s, 3H), 3.83 (s, 3H), 3.67 (t, J = 4.8 Hz, 1H), 3.49 (ddd, J = 11.1, 5.8, 3.5 Hz, 1H), 3.35 (d, J = 9.9 Hz, 1H), 2.97 (td, J = 13.2, 3.0 Hz, 1H), 2.31 - 2.22 (m, 1H), 2.16 - 2.02 (m, 1H), 1.87 (d, J = 12.7 Hz, 1H), 1.71 - 1.51 (m, 6H), 1.48 - 1.38 (m, 1H), 1.38 - 0.98 (m, 5H), 0.95 - 0.63 (m, 2H) ppm. **MS** (m/z): (ESI⁺) calculated for: C₃₀H₄₄NO₇ [M+H]+: 530.3, found: 530.2

### 3-(allyloxy)propyl (S)-1-((S)-2-(3-(allyloxy)-4,5-dimethoxyphenyl)-2-cyclohexylacetyl)piperidine-2-carboxylate (m4(4,1))

General procedure C was applied to phenol **m3(4)** with allyl bromide **(B1,** 3.0 eq.), cesium carbonate in DMF. **Yield:** 86 mg, 80 %, colorless resin. **¹H NMR** (500 MHz, CDCl₃, mixture of rotamers, major rotamer reported): δ δ 6.44 (s, 2H), 6.06 - 5.94 (m, 1H), 5.90 - 5.75 (m, 1H), 5.39 - 5.06 (m, 5H), 4.55 - 4.50 (m, 2H), 4.11 - 4.05 (m, 2H), 4.01 (dt, J = 11.6, 6.4 Hz, 1H), 3.96 - 3.74 (m, 8H), 3.47 (t, J = 6.2 Hz, 1H), 3.31 (d, J = 9.9 Hz, 1H), 3.28 - 3.17 (m, 1H), 2.89 - 2.84 (m, 1H), 2.19 (d, J = 13.7 Hz, 1H), 2.10 - 1.96 (m, 2H), 1.90 (dt, J = 11.9, 6.3 Hz, 1H), 1.82 (d, J = 12.6 Hz, 1H), 1.72 - 1.48 (m, 6H), 1.46 -1.35 (m, 1H), 1.32 - 0.95 (m, 5H), 0.91 - 0.61 (m, 2H) ppm. **MS** (m/z): (ESI⁺) calculated for: C₃₁H₄₆NO₇ [M+H]+: 544.3, found: 544.2.

### (S)-pent-4-en-2-yl (S)-1-((S)-2-(3-(2-(allyloxy)ethoxy)-4,5-dimethoxyphenyl)-2-cyclohexylacetyl)piperidine-2-carboxylate (m4(5,3))

General procedure C was applied to phenol **m3(5)** (70 mg, 0.148 mmol, 1.0 eq.) with **B3** (52 mg, 0.596 mmol, 3.0 eq.), cesium carbonate (195 mg, 0.591 mmol, 4.0 eq.), in DMF (3 mL). Flash column chromatography (0-5 % MeOH in DCM) afforded the title compound as a colorless resin (67 mg, 82 %). **¹H NMR** (500 MHz, CDCl₃, mixture of rotamers, major rotamer reported): δ 6.56 - 6.43 (m, 2H), 6.02 - 5.51 (m, 2H), 5.36 - 4.55 (m, 6H), 4.22 - 4.05 (m, 4H), 3.99 - 3.75 (m, 9H), 3.37 - 3.05 (m, 1H), 2.96 - 2.54 (m, 1H), 2.39 - 2.19 (m, 1H), 2.16 - 1.97 (m, 3H), 1.89 (dd, J = 33.3, 12.7 Hz, 1H), 1.70 - 1.49 (m, 6H), 1.48 - 1.20 (m, 5H), 1.19 - 1.07 (m, 2H), 1.04 (d, J = 6.3 Hz, 2H), 0.95 - 0.62 (m, 2H) ppm.

**MS** (m/z): (ESI⁺) calculated for: C₃₂H₄₈NO₇ [M+H]+: 558.3, found: 558.4.

### (S)-pent-4-en-2-yl (S)-1-((S)-2-(3-(3-(allyloxy)propoxy)-4,5-dimethoxyphenyl)-2-cyclohexylacetyl)piperidine-2-carboxylate (m4(5,4))

General procedure C was applied to phenol **m3(5)** with **B4** (3.0 eq.), cesium in DMF. **Yield:** 78 mg, 83 %, colorless resin. **¹H NMR** (500 MHz, CDCl₃, mixture of rotamers, major rotamer reported): δ 6.49 (d, J = 1.9 Hz, 1H), 6.47 (d, J = 1.8 Hz, 1H), 5.90 (ddt, J = 16.3, 10.7, 5.4 Hz, 1H), 5.58 (ddt, J = 17.4, 10.4, 7.1 Hz, 1H), 5.36 - 4.94 (m, 5H), 4.89 - 4.80 (m, 1H), 4.08 (td, J = 6.3, 3.4 Hz, 2H), 4.01 - 3.96 (m, 2H), 3.97 - 3.92 (m, 1H), 3.82 (s, 3H), 3.80 (s, 3H), 3.67 - 3.60 (m, 2H), 3.34 (d, J = 9.8 Hz, 1H), 3.02 - 2.87 (m, 1H), 2.22 (dt, J = 13.5, 2.6 Hz, 1H), 2.14 - 1.99 (m, 5H), 1.96 - 1.83 (m, 1H), 1.76 - 1.51 (m, 6H), 1.48 - 1.37 (m, 1H), 1.37 - 1.06 (m, 5H), 1.03 (d, J = 6.3 Hz, 3H), 0.94 - 0.57 (m, 2H) ppm. **MS** (m/z): (ESI⁺) calculated for: C₃₃H₅₀NO₇ [M+H]+: 572.4, found: 572.4.

### (R)-pent-4-en-2-yl (S)-1-((S)-2-(3-(2-(allyloxy)ethoxy)-4,5-dimethoxyphenyl)-2-cyclohexylacetyl)piperidine-2-carboxylate (m4(6,3))

General procedure C was applied to phenol **m3(6)** with **B3** (3.0 eq.), cesium carbonate in DMF. **Yield:** 62 mg, 81 %, colorless resin. **¹H NMR** (500 MHz, CDCl₃, mixture of rotamers, major rotamer reported): δ 6.52 (d, J = 1.8 Hz, 1H), 6.50 (d, J = 1.7 Hz, 1H), 6.08 - 5.87 (m, 1H), 5.55 - 5.46 (m, 1H), 5.35 - 4.81 (m, 5H), 4.20 - 4.13 (m, 2H), 4.12 - 4.06 (m, 2H), 3.96 - 3.89 (m, 1H), 3.83 - 3.81 (m, 6H), 3.81 - 3.79 (m, 1H), 3.33 (d, J = 9.9 Hz, 1H), 2.92 (td, J = 13.3, 2.8 Hz, 1H), 2.24 (d, J = 14.3 Hz, 1H), 2.11 - 1.98 (m, 2H), 1.87 (d, J = 12.6 Hz, 1H), 1.77 - 1.50 (m, 6H), 1.48 - 1.37 (m, 1H), 1.38 - 1.04 (m, 8H), 1.02 (d, J = 6.3 Hz, 3H), 0.92 - 0.63 (m, 2H) ppm. **MS** (m/z): (ESI⁺) calculated for: C₃₂H₄₈NO₇ [M+H]+: 558.3, found: 558.4.

### (R)-pent-4-en-2-yl (S)-1-((S)-2-(3-(3-(allyloxy)propoxy)-4,5-dimethoxyphenyl)-2-cyclohexylacetyl)piperidine-2-carboxylate (m4(6,4))

General procedure C was applied to phenol **m3(6) B4** (3.0 eq.), cesium carbonate in **Yield:** 54 mg, 82 %, colorless resin. **¹H NMR** (500 MHz, CDCl₃, mixture of rotamers, major rotamer reported): δ 6.51 (d, J = 1.9 Hz, 1H), 6.49 (d, J = 1.7 Hz, 1H), 5.96 - 5.85 (m, 1H), 5.60 - 5.46 (m, 1H), 5.37 - 5.30 (m, 1H), 5.28 - 5.06 (m, 2H), 4.99 - 4.79 (m, 2H), 4.10 - 4.05 (m, 2H), 4.00 - 3.96 (m, 2H), 3.94 (d, J = 13.2 Hz, 1H), 3.82 (s, 3H), 3.80 (s, 3H), 3.67 - 3.60 (m, 2H), 3.34 (d, J = 9.8 Hz, 1H), 2.92 (td, J = 13.3, 2.8 Hz, 1H), 2.27 - 2.20 (m, 1H), 2.14 - 1.96 (m, 5H), 1.88 (dd, J = 10.8, 7.4 Hz, 1H), 1.73 - 1.50 (m, 6H), 1.49 - 1.38 (m, 1H), 1.39 - 1.04 (m, 6H), 1.02 (d, J = 6.3 Hz, 3H), 0.96 - 0.66 (m, 2H) ppm. **MS** (m/z): (ESI⁺) calculated for: C₃₃H₅₀NO₇ [M+H]+: 572.4, found: 572.4.

### (R)-pent-4-en-2-yl (S)-1-((S)-2-(3-((S)-2-(allyloxy)propoxy)-4,5-dimethoxyphenyl)-2-cyclohexylacetyl)piperidine-2-carboxylate (m4(6,5))

General procedure C was applied to phenol **m3(6)** with **B5** (2.0 eq.), cesium carbonate in DMF. **Yield:** 29 mg, 60 %, colorless resin. **MS** (m/z): (ESI⁺) calculated for: C₃₃H₅₀NO₇ [M+H]+: 572.4, found: 572.5.

### (R)-pent-4-en-2-yl (S)-1-((S)-2-(3-((R)-2-(allyloxy)propoxy)-4,5-dimethoxyphenyl)-2-cyclo-hexylacetyl)piperidine-2-carboxylate (m4(6,6))

General procedure C was applied to phenol **m3(6)** with **B6** (3.0 eq.), cesium carbonate in DMF. **Yield:** 54 mg, 82 %, colorless resin. **MS** (m/z): (ESI⁺) calculated for: C₃₃H₅₀NO₇ [M+H]+: 572.4, found: 572.4.

### (R)-pent-4-en-2-yl (S)-1-((S)-2-(3-((R)-2-(allyloxy)-3-((tert-butyldimethylsilyl)oxy)propoxy)-4,5-dimethoxyphenyl)-2-cyclohexylacetyl)piperidine-2-carboxylate (m4(6,7))

General procedure C was applied to phenol **m3(6)** with **B7** (2.0 eq.), cesium carbonate in DMF **Yield:** 52 mg, 88 %, colorless resin. **¹H NMR** (500 MHz, CDCl₃, mixture of rotamers, major rotamer reported): δ 6.52 (d, J = 1.7 Hz, 1H), 6.47 (d, J = 1.9 Hz, 1H), 5.98 - 5.86 (m, 1H), 5.55 - 5.46 (m, 1H), 5.34 - 5.24 (m, 2H), 4.97 - 4.80 (m, 2H), 4.23 - 4.19 (m, 2H), 4.13 - 4.05 (m, 1H), 4.00 (dt, J = 10.3, 5.2 Hz, 1H), 3.94 - 3.88 (m, 1H), 3.85 - 3.72 (m, 8H), 3.32 (d, J = 9.8 Hz, 1H), 2.91 (td, J = 13.3, 2.9 Hz, 1H), 2.38 - 2.20 (m, 1H), 2.18 - 1.95 (m, 3H), 1.87 (d, J = 12.5 Hz, 1H), 1.69 - 1.49 (m, 5H), 1.48 - 1.04 (m, 10H), 1.02 (d, J = 6.3 Hz, 3H), 0.89 (s, 9H), 0.86 - 0.62 (m, 2H), 0.06 (s, 6H) ppm.

### (R)-pent-4-en-2-yl (S)-1-((S)-2-(3-((S)-2-(allyloxy)-3-((tert-butyldimethylsilyl)oxy)propoxy)-4,5-dimethoxyphenyl)-2-cyclohexylacetyl)piperidine-2-carboxylate (m4(6,8))

General procedure C was applied to phenol **m3(6)** with **B8** (2.0 eq.), cesium carbonate in DMF. **Yield:** 25 mg, 42 %, colorless resin. **¹H NMR** (500 MHz, CDCl₃, mixture of rotamers, major rotamer reported): δ 6.54 (d, J = 1.9 Hz, 1H), 6.47 (d, J = 2.2 Hz, 1H), 6.02 - 5.87 (m, 1H), 5.51 (ddt, J = 17.2, 10.3, 7.0 Hz, 1H), 5.34 - 5.24 (m, 1H), 5.20 - 4.99 (m, 2H), 4.97 - 4.80 (m, 2H), 4.26 - 4.13 (m, 2H), 4.08 (dt, J = 8.0, 3.8 Hz, 2H), 4.01 (dt, J = 10.5, 5.1 Hz, 2H), 3.97 - 3.90 (m, 1H), 3.86 - 3.71 (m, 8H), 3.70 - 3.47 (m, 1H), 3.33 (d, J = 9.8 Hz, 1H), 2.92 (td, J = 13.2, 2.9 Hz, 1H), 2.38-2.22 (m, 1H), 2.20 - 1.96 (m, 2H), 1.87 (d, J = 12.4 Hz, 1H), 1.71 -1.51 (m, 6H), 1.50 - 1.04 (m, 5H), 1.02 (d, J = 6.3 Hz, 3H), 0.89 (s, 9H), 0.87 - 0.77 (m, 2H), 0.76 - 0.63 (m, 1H), 0.06 (s, 6H) ppm.

### (R)-pent-4-en-2-yl (S)-1-((S)-2-(3-(((R)-1-(allyloxy)propan-2-yl)oxy)-4,5-dimethoxyphenyl)-2-cyclohexylacetyl)piperidine-2-carboxylate (m4(6,9))

General procedure C was applied to phenol **m3(6)** with **B9** (2.0 eq.), cesium carbonate in DMF. **Yield**:44 mg, 92 %, colorless resin. **¹H NMR** (500 MHz, CDCl₃, mixture of rotamers, major rotamer reported): δ 6.52 (s, 2H), 5.98 - 5.84 (m, 1H), 5.58 - 5.45 (m, 1H), 5.30 (d, J = 6.0 Hz, 1H), 5.29 - 5.24 (m, 1H), 5.23 - 5.02 (m, 2H), 4.98 - 4.86 (m, 2H), 4.83 (p, J = 6.1 Hz, 1H), 4.58 (d, J = 13.9 Hz, 1H), 4.55 - 4.48 (m, 1H), 4.05 - 4.02 (m, 2H), 3.83 - 3.78 (m, 6H), 3.68 - 3.60 (m, 1H), 3.57 - 3.49 (m, 1H), 3.31 (d, J = 9.9 Hz, 1H), 2.95 - 2.87 (m, 1H), 2.38 - 2.32 (m, 1H), 2.23 (d, J = 13.8 Hz, 1H), 2.15 - 1.97 (m, 3H), 1.87 (d, J = 12.6 Hz, 1H), 1.70 - 1.48 (m, 6H), 1.45 - 1.37 (m, 1H), 1.36 - 1.05 (m, 6H), 1.02 (d, J = 6.3 Hz, 3H), 0.93 - 0.59 (m, 2H) ppm. **MS** (m/z): (ESI⁺) calculated for: C₃₃H₅₀NO₇ [M+H]+: 572.4, found: 572.4.

### (R)-pent-4-en-2-yl (S)-1-((S)-2-(3-(((S)-1-(allyloxy)propan-2-yl)oxy)-4,5-dimethoxyphenyl)-2-cyclohexylacetyl)piperidine-2-carboxylate (m4(6,10))

General procedure C was applied to phenol **m3(6)** with **B10** (2.0 eq.), cesium carbonate in DMF. **Yield:** 39 mg, 81 %, colorless resin.**¹H NMR** (500 MHz, CDCl₃, mixture of rotamers, major rotamer reported): δ 6.55 (d, J = 1.8 Hz, 1H), 6.50 (d, J = 1.8 Hz, 1H), 5.95 - 5.85 (m, 1H), 5.51 (ddt, J = 17.1, 10.0, 7.0 Hz, 1H), 5.31 (s, 1H), 5.30 - 5.23 (m, 1H), 5.20 - 5.03 (m, 2H), 4.96 - 4.86 (m, 1H), 4.85 - 4.80 (m, 1H), 4.52 (h, J = 6.0 Hz, 1H), 4.04 (d, J = 5.5 Hz, 2H), 3.93 (d, J = 14.4 Hz, 1H), 3.86 - 3.77 (m, 6H), 3.69 - 3.61 (m, 1H), 3.54 (d, J = 4.9 Hz, 1H), 3.32 (d, J = 9.9 Hz, 1H), 2.98 - 2.85 (m, 1H), 2.38 - 2.32 (m, 1H), 2.24 (d, J = 13.8 Hz, 1H), 2.14 - 1.97 (m, 4H), 1.87 (d, J = 12.6 Hz, 1H), 1.70 - 1.49 (m, 6H), 1.48 - 1.38 (m, 1H), 1.35 - 0.99 (m, 9H), 0.95 - 0.62 (m, 2H) ppm.**MS** (m/z): (ESI⁺) calculated for: C₃₃H₅₀NO₇ [M+H]+: 572.4, found: 572.4.

### (R)-pent-4-en-2-yl (S)-1-((S)-2-(3-((S)-3-(allyloxy)-2-methylpropoxy)-4,5-dimethoxyphenyl)-2-cyclohexylacetyl)piperidine-2-carboxylate (m4(6,11))

General procedure C was applied to phenol **m3(6)** with **B11** (2.0 eq.), cesium carbonate in DMF. **Yield:** 40 mg, 82 %, colorless resin. **¹H NMR** (500 MHz, CDCl₃, mixture of rotamers, major rotamer reported): δ 6.50 (d, J = 1.9 Hz, 1H), 6.48 (d, J = 1.8 Hz, 1H), 6.02 - 5.84 (m, 1H), 5.49 (ddt, J = 17.1, 10.3, 7.0 Hz, 1H), 5.37 - 5.30 (m, 1H), 5.20 - 5.04 (m, 2H), 4.99 - 4.77 (m, 2H), 3.96 (ddt, J = 11.0, 9.1, 3.5 Hz, 5H), 3.82 (s, 3H), 3.79 (s, 3H), 3.56 - 3.49 (m, 1H), 3.46 - 3.40 (m, 1H), 3.33 (d, J = 9.9 Hz, 1H), 2.92 (td, J = 13.3, 2.8 Hz, 1H), 2.31 - 2.20 (m, 2H), 2.16 - 1.93 (m, 3H), 1.87 (d, J = 12.1 Hz, 1H), 1.73 - 1.50 (m, 6H), 1.49 - 1.37 (m, 1H), 1.36 - 1.10 (m, 6H), 1.08 (d, J = 6.9 Hz, 3H), 1.01 (d, J = 6.3 Hz, 3H), 0.94 - 0.63 (m, 2H ppm. **MS** (m/z): (ESI⁺) calculated for: C₃₄H₅₂NO₇ [M+H]+: 586.4, found: 586.2.

### (R)-pent-4-en-2-yl (S)-1-((S)-2-(3-((S)-3-(allyloxy)-2-methylpropoxy)-4,5-dimethoxyphenyl)-2-cyclohexylacetyl)piperidine-2-carboxylate (m4(6,12))

General procedure C was applied to phenol **m3(6)** with **B12** (2.0 eq.), cesium carbonate in DMF.**Yield**: 41 mg, 84%, colorless resin. **MS** (m/z): (ESI⁺) calculated for: C₃₄H₅₂NO₇ [M+H]+: 586.4, found: 586.4.

### (R)-pent-4-en-2-yl (S)-1-((S)-2-(3-((R)-3-(allyloxy)butoxy)-4,5-dimethoxyphenyl)-2-cyclohexylacetyl)piperidine-2-carboxylate (m4(6,13))

General procedure C was applied to phenol **m3(6)** with **B16** (2.0 eq.), cesium carbonate in DMF. **Yield:** 36 mg, 86 %, colorless **resin.¹H NMR** (500 MHz, CDCl₃, mixture of rotamers, major rotamer reported): δ 6.51 (d, J = 1.8 Hz, 1H), 6.48 (d, J = 1.9 Hz, 1H), 5.96 - 5.82 (m, 1H), 5.50 (ddt, J = 17.1, 10.2, 7.0 Hz, 1H), 5.34-5.28 (m, 1H), 5.24 (ddq, J = 17.2, 5.6, 1.7 Hz, 1H), 5.16-5.05 (m, 2H), 4.97 - 4.79 (m, 2H), 4.18 - 4.00 (m, 3H), 3.98 - 3.87 (m, 2H), 3.82 (s, 3H), 3.79 (s, 3H), 3.77 - 3.70 (m, 1H), 3.34 (d, J = 9.9 Hz, 1H), 2.92 (td, J = 13.3, 2.9 Hz, 1H), 2.38 - 2.31 (m, 1H), 2.29 - 2.19 (m, 1H), 2.16-1.81 (m, 6H), 1.79 - 1.49 (m, 7H), 1.48 - 1.37 (m, 1H), 1.21 (dd, J = 6.2, 2.2 Hz, 3H), 1.19 - 1.03 (m, 3H), 1.01 (d, J = 6.3 Hz, 3H), 0.93 - 0.78 (m, 1H), 0.77 - 0.64 (m, 1H) ppm. **MS** (m/z): (ESI⁺) calculated for: C₃₄H₅₂NO₇ [M+H]+: 586.4, found: 586.4.

### (R)-pent-4-en-2-yl (S)-1-((S)-2-(3-((S)-3-(allyloxy)butoxy)-4,5-dimethoxyphenyl)-2-cyclohexylacetyl)piperidine-2-carboxylate (m4(6,14))

General procedure C was applied to phenol **m3(6)** with **B15** (2.0 eq.), cesium in DMF (3 mL). **Yield:** 30 mg, 71 %, colorless resin. **¹H NMR** (500 MHz, CDCl₃, mixture of rotamers, major rotamer reported): δ 6.52 (d, J = 1.9 Hz, 1H), 6.51 (d, J = 1.9 Hz, 1H), 6.05 - 5.85 (m, 1H), 5.52 (ddt, J = 17.2, 10.2, 7.0 Hz, 1H), 5.37 - 5.32 (m, 1H), 5.19 - 5.07 (m, 2H), 5.00 - 4.80 (m, 2H), 4.19 - 4.04 (m, 3H), 4.03 - 3.91 (m, 2H), 3.84 (s, 3H), 3.81 (s, 3H), 3.77 (dddd, J = 7.8, 6.7, 5.1, 1.6 Hz, 1H), 3.36 (d, J = 9.9 Hz, 1H), 2.94 (td, J = 13.3, 2.9 Hz, 1H), 2.41 -2.34 (m, 1H), 2.30 - 2.23 (m, 1H), 2.15 - 1.91 (m, 4H), 1.91 - 1.86 (m, 1H), 1.78 - 1.53 (m, 5H), 1.48 - 1.41 (m, 1H), 1.38 - 1.25 (m, 4H), 1.25 - 1.23 (m, 3H), 1.22 - 1.06 (m, 3H), 1.04 (d, J = 6.3 Hz, 3H), 0.96 - 0.63 (m, 2H) ppm. **MS** (m/z): (ESI⁺) calculated for: C₃₄H₅₂NO₇ [M+H]+: 586.4, found: 586.4.

### (R)-pent-4-en-2-yl (S)-1-((S)-2-(3-((R)-3-(allyloxy)-2-((tert-butyldimethylsilyl)oxy)propoxy)-4,5-dimethoxyphenyl)-2-cyclohexylacetyl)piperidine-2-carboxylate (m4(6,15))

General procedure C was applied to phenol ***m*3(6)** with **B14** (2.0 eq.), cesium carbonate in DMF. **Yield:** 55 mg, quant., colorless resin. **¹H NMR** (500 MHz, CDCl₃, mixture of rotamers, major rotamer reported): δ 6.53 (d, J = 1.8 Hz, 1H), 6.47 - 6.35 (m, 1H), 5.93 - 5.84 (m, 1H), 5.49 (ddt, J = 17.1, 10.3, 7.0 Hz, 1H), 5.33 - 5.30 (m, 1H), 5.29 - 5.23 (m, 1H), 5.22 - 5.05 (m, 2H), 5.04 - 4.80 (m, 2H), 4.23 - 4.12 (m, 2H), 4.03 - 3.89 (m, 6H), 3.82 (s, 3H), 3.78 (s, 3H), 3.61 - 3.57 (m, 1H), 3.54 - 3.48 (m, 1H), 3.37 - 3.31 (m, 1H), 2.91 (td, J = 13.2, 2.8 Hz, 1H), 2.37 - 2.32 (m, 1H), 2.24 (d, J = 13.7 Hz, 1H), 2.15 - 1.84 (m, 3H), 1.81 - 1.50 (m, 6H), 1.24 (6, 5H), 1.00 (d, J = 6.2 Hz, 3H), 0.89 (d, J = 1.4 Hz, 9H), 0.73 (tdd, J = 19.0, 12.1, 3.1 Hz, 1H), 0.11 (d, J = 9.2 Hz, 6H) ppm.

### (R)-pent-4-en-2-yl (S)-1-((S)-2-(3-((S)-3-(allyloxy)-2-((tert-butyldimethylsilyl)oxy)propoxy)-4,5-dimethoxyphenyl)-2-cyclohexylacetyl)piperidine-2-carboxylate (m4(6,16))

General procedure C was applied to phenol **m3(6)** with **B16** (2.0 eq.), cesium carbonate in DMF. **Yield:** 49 mg, 89 %, colorless resin. **¹H NMR** (500 MHz, CDCl₃, mixture of rotamers, major rotamer reported): δ 6.53 (d, J = 1.8 Hz, 1H), 6.47 - 6.35 (m, 1H), 5.93 - 5.84 (m, 1H), 5.49 (ddt, J = 17.1, 10.3, 7.0 Hz, 1H), 5.33 - 5.30 (m, 1H), 5.29 - 5.23 (m, 1H), 5.22 - 5.05 (m, 2H), 5.04 - 4.80 (m, 2H), 4.23 - 4.12 (m, 2H), 4.03 - 3.89 (m, 6H), 3.82 (s, 3H), 3.78 (s, 3H), 3.61 - 3.57 (m, 1H), 3.54 - 3.48 (m, 1H), 3.37 - 3.31 (m, 1H), 2.91 (td, J = 13.2, 2.8 Hz, 1H), 2.37 - 2.32 (m, 1H), 2.24 (d, J = 13.7 Hz, 1H), 2.15 - 1.84 (m, 3H), 1.81 - 1.50 (m, 6H), 1.24 (6, 5H), 1.00 (d, J = 6.2 Hz, 3H), 0.89 (d, J = 1.4 Hz, 9H), 0.73 (tdd, J = 19.0, 12.1, 3.1 Hz, 1H), 0.11 (d, J = 9.2 Hz, 6H) ppm.

### (R)-pent-4-en-2-yl (S)-1-((S)-2-(3-((S)-3-(allyloxy)-2-hydroxypropoxy)-4,5-dimethoxyphenyl)-2-cyclohexylacetyl)piperidine-2-carboxylate (m4(6,16)-OH)

General procedure C was applied to phenol **m3(8)** with **B16** (1.5 eq.), cesium carbonate in DMF, then general procedure H without prior purification. **Yield:** 15 mg, 26 % over 2 steps, colorless resin.**¹H NMR** (500 MHz, CDCl₃, mixture of rotamers, major rotamer reported): δ 6.54 (s, 1H), 6.50 (s, 1H), 6.00 - 5.85 (m, 1H), 5.55 - 5.46 (m, 1H), 5.34 - 5.31 (m, 1H), 5.30 - 5.25 (m, 1H), 5.22 - 5.16 (m, 1H), 5.17 - 5.07 (m, 1H), 4.99 - 4.90 (m, 1H), 4.84 (q, J = 6.4 Hz, 1H), 4.21 - 4.13 (m, 1H), 4.14 - 3.99 (m, 4H), 3.94 (d, J = 13.9 Hz, 1H), 3.83 (s, 3H), 3.81 (s, 3H), 3.68 - 3.54 (m, 2H), 3.36 (d, J = 9.8 Hz, 1H), 2.93 (t, J = 13.2 Hz, 1H), 2.36 (t, J = 6.8 Hz, 1H), 2.26 (d, J = 13.8 Hz, 1H), 2.14 -1.95 (m, 3H), 1.85 (d, J = 12.6 Hz, 1H), 1.72 - 1.52 (m, 5H), 1.50 - 1.38 (m, 1H), 1.39 - 1.04 (m, 6H), 1.02 (d, J = 6.2 Hz, 3H), 0.94 - 0.62 (m, 2H).ppm. **HR-MS** (m/z): (ESI⁺) calculated for: C₃₃H₄₉NO₈ [M+H]+: 588.35309, found: 588.35369.

### (R)-pent-4-en-2-yl (S)-1-((S)-2-(3-(((R)-4-(allyloxy)butan-2-yl)oxy)-4,5-dimethoxyphenyl)-2-cyclohexylacetyl)piperidine-2-carboxylate (m4(6,17))

General procedure C was applied to phenol **m3(6)** with **B17** (1.5 eq.), cesium carbonate in DMF. **Yield:** 29 mg, 78%, colorless resin. **¹H NMR** (500 MHz, CDCl₃, mixture of rotamers, major rotamer reported): δ 6.55 (s, 1H), 6.48 (s, 1H), 5.95 - 5.84 (m, 1H), 5.56 - 5.45 (m, 1H), 5.31 (d, J = 5.6 Hz, 1H), 5.24 (t, J = 8.7 Hz, 1H), 5.18 - 5.04 (m, 2H), 4.96 - 4.85 (m, 2H), 4.56 - 4.48 (m, 1H), 3.99 - 3.91 (m, 3H), 3.82 (s, 3H), 3.78 (s, 3H), 3.64 - 3.55 (m, 2H), 3.32 (d, J = 9.9 Hz, 1H), 2.97 - 2.88 (m, 1H), 2.35 (t, J = 6.8 Hz, 1H), 2.24 (d, J = 13.8 Hz, 1H), 2.16 - 1.82 (m, 6H), 1.74 - 1.50 (m, 6H), 1.48 - 1.37 (m, 1H), 1.36 - 1.21 (m, 4H), 1.13 (h, J = 12.6 Hz, 3H), 1.01 (d, J = 6.2 Hz, 3H), 0.93 - 0.62 (m, 2H) ppm. **MS** (m/z): (ESI⁺) calculated for: C₃₄H₅₂NO₇ [M+H]+: 586.4, found: 586.4.

### (R)-pent-4-en-2-yl (S)-1-((S)-2-(3-(((S)-4-(allyloxy)butan-2-yl)oxy)-4,5-dimethoxyphenyl)-2-cyclohexylacetyl)piperidine-2-carboxylate (m4(6,18))

General procedure C was applied to phenol **m3(6)** with **B18** (1.5 eq.), cesium carbonate DMF. **Yield:** 34 mg, 92%, colorless resin. **¹H NMR** (500 MHz, CDCl₃, mixture of rotamers, major rotamer reported): δ 6.53 - 6.50 (m, 2H), 5.94 - 5.84 (m, 1H), 5.56 - 5.45 (m, 1H), 5.32 - 5.29 (m, 1H), 5.28 - 5.21 (m, 2H), 5.17 - 4.89 (m, 3H), 4.88 - 4.80 (m, 1H), 4.63 - 4.48 (m, 1H), 4.03 - 3.89 (m, 3H), 3.85 - 3.76 (m, 6H), 3.64 - 3.54 (m, 2H), 3.32 (d, J = 9.9 Hz, 1H), 2.92 (td, J = 13.4, 3.0 Hz, 1H), 2.37 - 1.97 (m, 5H), 1.98 - 1.80 (m, 2H), 1.72 - 1.49 (m, 6H), 1.46 - 1.37 (m, 1H), 1.35 - 0.99 (m, 10H), 0.94 - 0.65 (m, 2H) ppm. **MS** (m/z): (ESI⁺) calculated for: C₃₄H₅₂NO₇ [M+H]+: 586.4, found: 586.4.

### (R)-1-((4-methoxybenzyl)oxy)pent-4-en-2-yl (S)-1-((S)-2-(3-(2-(allyloxy)ethoxy)-4,5-dimethoxyphenyl)-2-cyclohexylacetyl)piperidine-2-carboxylate (m4(7,3))

General procedure C was applied to phenol **m3(7)** with **B3** (2.0 eq.), cesium carbonate in DMF **Yield:** 69 mg, quant., colorless resin. **¹H NMR** (500 MHz, CDCl₃, mixture of rotamers, major rotamer reported): δ 7.25 - 7.13 (m, 2H), 6.91 - 6.80 (m, 3H), 6.52 - 6.49 (m, 2H), 5.95 - 5.85 (m, 1H), 5.61 - 5.52 (m, 1H), 5.39 - 5.34 (m, 1H), 5.32 - 5.04 (m, 2H), 5.03 - 4.95 (m, 2H), 4.31 (d, J = 11.4 Hz, 1H), 4.23 (d, J = 11.5 Hz, 1H), 4.17 - 4.10 (m, 2H), 4.09 -4.04 (m, 2H), 3.81 (s, 3H), 3.79 (s, 3H), 3.77 (s, 3H), 3.37 - 3.29 (m, 2H), 2.96 - 2.90 (m, 1H), 2.27 - 2.21 (m, 1H), 2.20 - 1.98 (m, 3H), 1.86 (d, J = 12.8 Hz, 1H), 1.72 - 1.43 (m, 6H), 1.43 - 1.26 (m, 4H), 1.22 - 0.94 (m, 4H), 0.92 - 0.60 (m, 4H) ppm. **MS** (m/z): (ESI⁺) calculated for: C₄₀H₅₆NO₉ [M+H]+: 694.4, found: 694.0.

### (R)-1-((4-methoxybenzyl)oxy)pent-4-en-2-yl (S)-1-((S)-2-(3-(2-(allyloxy)ethoxy)-4,5-dimethoxyphenyl)-2-cyclohexylacetyl)piperidine-2-carboxylate (m4(7,4))

General procedure C was applied to phenol ***m*3(7)** with **B4** (2.0 eq.), cesium carbonate in DMF (3 mL). **Yield:** 58 mg, 83 %, colorless resin. **¹H NMR** (500 MHz, CDCl₃, mixture of rotamers, major rotamer reported): δ 7.26 - 7.14 (m, 2H), 6.89 - 6.81 (m, 2H), 6.53 - 6.46 (m, 1H), 5.94 - 5.83 (m, 1H), 5.56 (ddt, J = 17.2, 10.2, 7.1 Hz, 1H), 5.40 - 5.35 (m, 1H), 5.28 - 5.21 (m, 1H), 5.17 - 5.07 (m, 2H), 5.02 - 4.95 (m, 2H), 4.31 (d, J = 11.4 Hz, 1H), 4.23 (d, J = 11.4 Hz, 1H), 4.08 - 4.03 (m, 2H), 3.99 - 3.93 (m, 2H), 3.90 (d, J = 13.8 Hz, 1H), 3.80 (s, 3H), 3.79 (s, 3H), 3.78 (s, 3H), 3.61 (dtd, J = 10.0, 6.3, 1.6 Hz, 2H), 3.37 - 3.28 (m, 2H), 2.94 (td, J = 13.2, 3.0 Hz, 1H), 2.28 - 2.22 (m, 1H), 2.17 - 1.98 (m, 4H), 1.96 - 1.83 (m, 2H), 1.71 - 1.45 (m, 6H), 1.44 - 0.98 (m, 8H), 0.96 - 0.59 (m, 2H) ppm. **MS** (m/z): (ESI⁺) calculated for: C₄₁H₅₈NO₉Na [M+Na]+: 730.4, found: 730.4.

### (S)-1-((4-methoxybenzyl)oxy)pent-4-en-2-yl (S)-1-((S)-2-(3-(2-(allyloxy)ethoxy)-4,5-dimethoxyphenyl)-2-cyclohexylacetyl)piperidine-2-carboxylate (m4(8,3))

General procedure C was applied to phenol **m3(8)** with **B3** (2.0 eq.), cesium carbonate in DMF. **Yield:** 100 mg, 81 %, colorless resin. **¹H NMR** (500 MHz, CDCl₃, mixture of rotamers, major rotamer reported): δ 7.22 - 7.18 (m, 2H), 6.90 - 6.82 (m, 2H), 6.50 (d, J = 1.9 Hz, 1H), 6.49 (d, J = 1.9 Hz, 1H), 5.98- 5.86 (m, 1H), 5.55 (ddt, J = 17.1, 10.2, 7.0 Hz, 1H), 5.36 (d, J = 5.1 Hz, 1H), 5.34 - 5.30 (m, 1H), 5.29 - 5.27 (m, 1H), 5.23 - 5.09 (m, 2H), 5.05 - 4.90 (m, 2H), 4.39 (d, J = 11.5 Hz, 1H), 4.35 (d, J = 11.6 Hz, 1H), 4.14 (t, J = 5.0 Hz, 2H), 4.11 - 4.06 (m, 2H), 3.91 - 3.86 (m, 1H), 3.85 - 3.80 (m, 4H), 3.79 (s, 3H), 3.79 (s, 3H), 3.79 - 3.76 (m, 1H), 3.40 - 3.27 (m, 2H), 2.95 - 2.87 (m, 1H), 2.31 - 2.19 (m, 1H), 2.13 - 2.00 (m, 2H), 1.87 (d, J = 14.4 Hz, 1H), 1.71 - 1.47 (m, 6H), 1.47 - 1.28 (m, 4H), 1.22 - 0.99 (m, 3H), 0.97 - 0.58 (m, 2H) ppm. **MS** (m/z): (ESI⁺) calculated for: C₄₀H₅₆NO₉ [M+H]+: 694.4, found: 694.4.

### (S)-1-hydroxypent-4-en-2-yl (S)-1-((S)-2-(3-(((S)-1-(allyloxy)propan-2-yl)oxy)-4,5-dimethoxyphenyl)-2-cyclohexylacetyl)piperidine-2-carboxylate (m4(8,3)-OH)

General procedure C was applied to phenol **m3(8)** with **B3** (1.5 eq.), cesium carbonate in DMF Then general procedure I was applied without prior purification with a reaction time of 5 h. **Yield:** 30 mg, 54 % over 2 steps, colorless resin. **¹H NMR** (500 MHz, CD₃CN, mixture of rotamers, major rotamer reported): δ 6.48 (d, J = 1.8 Hz, 1H), 6.47 - 6.46 (m, 1H), 5.79 (ddt, J = 16.3, 10.7, 5.4 Hz, 1H), 5.44 - 5.32 (m, 1H), 5.20 - 5.11 (m, 1H), 5.05 - 4.99 (m, 2H), 4.82 - 4.71 (m, 2H), 4.68 - 4.62 (m, 1H), 4.05 - 3.92 (m, 3H), 3.92 - 3.88 (m, 2H), 3.65 (s, 3H), 3.63 - 3.58 (m, 3H), 3.56 (s, 3H), 3.50 - 3.39 (m, 1H), 3.22 - 3.13 (m, 1H), 2.88 (dd, J = 24.3, 13.8 Hz, 2H), 2.77 - 2.68 (m, 1H), 2.05 (d, J = 13.7 Hz, 1H), 2.02 - 1.85 (m, 2H), 1.84 - 1.59 (m, 2H), 1.58 - 1.25 (m, 6H), 1.20 - 0.90 (m, 5H), 0.86 - 0.57 (m, 2H). ppm. **HR-MS** (m/z): (ESI⁺) calculated for: C₃₂H₄₈NO₈ [M+H]+: 574.3374, found: 574.3374.

### (S)-1-((4-methoxybenzyl)oxy)pent-4-en-2-yl (S)-1-((S)-2-(3-(2-(allyloxy)ethoxy)-4,5-dimethoxyphenyl)-2-cyclohexylacetyl)piperidine-2-carboxylate (m4(8,4))

General procedure C was applied to phenol **m3(8)** with **B4** (2.0 eq.), cesium carbonate in DMF **Yield:** 99 mg, 78 %, colorless resin. **¹H NMR** (500 MHz, CDCl₃, mixture of rotamers, major rotamer reported): δ 7.20 (d, J = 8.3 Hz, 2H), 6.85 (d, J = 8.5 Hz, 2H), 6.49 (d, J = 1.9 Hz, 1H), 6.47 (d, J = 1.8 Hz, 1H), 5.97 - 5.85 (m, 1H), 5.62 - 5.49 (m, 1H), 5.38 - 5.34 (m, 1H), 5.30 - 5.22 (m, 2H), 5.19 - 5.10 (m, 2H), 5.08 - 4.88 (m, 2H), 4.39 (d, J = 11.6 Hz, 1H), 4.34 (d, J = 11.5 Hz, 1H), 4.06 (td, J = 6.3, 1.7 Hz, 2H), 4.00 - 3.96 (m, 2H), 3.93 - 3.87 (m, 1H), 3.80 - 3.78 (m, 9H), 3.66 - 3.59 (m, 3H), 3.32 (tq, J = 10.4, 4.6 Hz, 3H), 2.96 - 2.87 (m, 1H), 2.26 (td, J = 15.3, 14.6, 8.4 Hz, 1H), 2.11 - 2.04 (m, 4H), 1.87 (d, J = 14.2 Hz, 1H), 1.67 - 1.47 (m, 5H), 1.45 - 1.27 (m, 3H), 1.21 - 1.00 (m, 3H), 0.97 - 0.69 (m, 2H) ppm. **MS** (m/z): (ESI⁺) calculated for: C₄₁H₅₇NO₉Na [M+Na]+: 730.4, found: 730.4.

### (S)-1-hydroxypent-4-en-2-yl (S)-1-((S)-2-(3-(3-(allyloxy)propoxy)-4,5-dimethoxyphenyl)-2-cyclohexylacetyl)piperidine-2-carboxylate (m4(8,4)-OH)

General procedure C was applied to phenol **m3(8)** with **B4** (1.5 eq.), cesium carbonate in DMF. Then general procedure I was applied without prior with a reaction time of 5 h. **Yield:** 39 mg, 67 % over 2 steps, colorless resin. **¹H NMR** (500 MHz, CD₃CN, mixture of rotamers, major rotamer reported): δ 6.35 (s, 2H), 5.69 - 5.61 (m, 1H), 5.32 - 5.19 (m, 1H), 5.04 - 4.96 (m, 1H), 4.94 - 4.83 (m, 2H), 4.71 - 4.59 (m, 2H), 4.54 (dp, J = 7.5, 4.4 Hz, 1H), 3.90 (d, J = 13.9 Hz, 1H), 3.84 - 3.74 (m, 2H), 3.72 - 3.67 (m, 2H), 3.53 (s, 3H), 3.43 (s, 2H), 3.37 - 3.27 (m, 3H), 3.09 - 3.02 (m, 1H), 2.91 - 2.73 (m, 2H), 2.67 -2.57 (m, 1H), 1.93 (d, J = 13.7 Hz, 1H), 1.89 - 1.65 (m, 5H), 1.63 - 1.47 (m, 2H), 1.47 - 1.25 (m, 6H), 1.25 - 1.13 (m, 1H), 1.12 - 0.77 (m, 6H), 0.76 - 0.48 (m, 2H) ppm. **HR-MS** (m/z): (ESI⁺) calculated for: C₃₃H₅₀NO₈ [M+H]+: 588.3458, found: 588.3531.

### (S)-1-((4-methoxybenzyl)oxy)pent-4-en-2-yl (S)-1-((S)-2-(3-((S)-2-(allyloxy)propoxy)-4,5-dimethoxyphenyl)-2-cyclohexylacetyl)piperidine-2-carboxylate (m4(8,5))

General procedure C was applied to phenol **m3(8)** with **B5** (2.0 eq.), cesium carbonate in DMF. **Yield:** 60 mg, 86 %, yellowish resin. **¹H NMR** (500 MHz, CDCl₃, mixture of rotamers, major rotamer reported): δ 7.25 - 7.14 (m, 2H), 6.90 - 6.81 (m, 2H), 6.55 - 6.37 (m, 2H), 6.00 - 5.83 (m, 1H), 5.83 - 5.44 (m, 1H), 5.38 - 5.19 (m, 2H), 5.18 - 4.87 (m, 4H), 4.36 (d, J = 4.0 Hz, 1H), 4.17 - 4.10 (m, 2H), 4.09 - 3.84 (m, 4H), 3.83 - 3.74 (m, 9H), 3.50 (t, J = 4.7 Hz, 1H), 3.32 (dd, J = 6.4, 2.3 Hz, 2H), 2.49 - 2.18 (m, 2H), 2.17 - 1.81 (m, 3H), 1.75 - 1.44 (m, 6H), 1.43 - 0.99 (m, 11H), 0.96 - 0.57 (m, 2H) ppm. **MS** (m/z): (ESI⁺) calculated for: C₄₁H₅₈NO₉[M+H]+: 708.4, found: 708.4.

### (S)-1-hydroxypent-4-en-2-yl (S)-1-((S)-2-(3-((S)-2-(allyloxy)propoxy)-4,5-dimethoxyphenyl)-2-cyclohexylacetyl)piperidine-2-carboxylate (m4(8,5)-OH)

General procedure C was applied to phenol **m3(8)** with **B5** (1.5 eq.), cesium carbonate in DMF. Then general procedure I was applied without prior purification with a reaction time of 5 h. **Yield:** 15 mg, 26 % over 2 steps, colorless resin. **¹H NMR** (500 MHz, CD₃CN, mixture of rotamers, major rotamer reported): δ 6.41 (s, 1H), 6.39 (s, 1H), 5.79 - 5.65 (m, 1H), 5.36 - 5.23 (m, 1H), 5.13 - 5.02 (m, 1H), 4.98 - 4.93 (m, 1H), 4.93 - 4.87 (m, 1H), 4.75 - 4.63 (m, 2H), 4.63 - 4.54 (m, 1H), 3.98 - 3.91 (m, 1H), 3.90 - 3.85 (m, 2H), 3.77 - 3.68 (m, 2H), 3.65 - 3.59 (m, 1H), 3.57 (s, 3H), 3.48 (s, 3H), 3.43 - 3.32 (m, 1H), 3.15 - 3.06 (m, 1H), 2.89 - 2.77 (m, 2H), 2.72 - 2.59 (m, 1H), 1.98 (d, J = 13.8 Hz, 1H), 1.94 -1.75 (m, 2H), 1.69 - 1.59 (m, 1H), 1.56 (d, J = 13.0 Hz, 1H), 1.50 - 1.30 (m, 6H), 1.28 - 1.17 (m, 1H), 1.15 - 0.84 (m, 8H), 0.84 - 0.50 (m, 2H) ppm. **HR-MS** (m/z): (ESI⁺) calculated for: C₃₃H₅₀NO₈ [M+H]+: 588.3458, found: 588.3531.

### (S)-1-((4-methoxybenzyl)oxy)pent-4-en-2-yl (S)-1-((S)-2-(3-((R)-2-(allyloxy)propoxy)-4,5-dimethoxyphenyl)-2-cyclohexylacetyl)piperidine-2-carboxylate (m4(8,6))

General procedure C was applied to phenol ***m*3(8)** with **B6** (2.0 eq.), cesium carbonate in DMF **Yield:** 58 mg, 83 %, colorless resin. **¹H NMR** (500 MHz, CDCl₃, mixture of rotamers, major rotamer reported): δ 7.28 - 7.13 (m, 2H), 6.92 - 6.80 (m, 2H), 6.53 - 6.36 (m, 2H), 6.01 - 5.83 (m, 1H), 5.82 - 5.42 (m, 1H), 5.39 - 4.83 (m, 5H), 4.49 - 4.30 (m, 2H), 4.13 (ddt, J = 5.7, 4.8, 1.4 Hz, 2H), 4.09 - 3.84 (m, 4H), 3.83 - 3.75 (m, 9H), 3.56 - 3.25 (m, 3H), 2.97 - 2.85 (m, 1H), 2.50 - 1.98 (m, 4H), 1.95-1.81 (m, 2H), 1.61 (t, J = 10.8 Hz, 5H), 1.43 - 0.96 (m, 10H), 0.95-0.53 (m, 2H) ppm. **MS** (m/z): (ESI⁺) calculated for: C₄₁H₅₈NO₉ [M+H]+: 708.4, found: 708.4.

**(*S*)-1-((4-methoxybenzyl)oxy)pent-4-en-2-yl (*S*)-1-((*S*)-2-(3-((*R*)-2-(allyloxy)-3-((*tert*butyldimethylsilyl)oxy)propoxy)-4,5-dimethoxyphenyl)-2-cyclohexylacetyl)piperidine-2-carboxylate (m4(8,7))**

General procedure C was applied to phenol **m3(8)** with **B7** (2.0 eq.), cesium in DMF. **Yield:** 96 mg, 82 %, yellow resin. **¹H NMR** (500 MHz, CDCl₃, mixture of rotamers, major rotamer reported): δ 7.20 - 7.16 (m, 2H), 6.85 - 6.81 (m, 2H), 6.51 (d, J = 1.8 Hz, 1H), 6.44 (d, J = 1.8 Hz, 1H), 5.97 - 5.85 (m, 1H), 5.60 - 5.49 (m, 1H), 5.37 - 5.33 (m, 1H), 5.32 - 5.19 (m, 2H), 5.18 - 4.87 (m, 3H), 4.38 (d, J = 11.6 Hz, 1H), 4.34 (d, J = 11.6 Hz, 1H), 4.24 - 4.14 (m, 2H), 4.12 - 4.04 (m, 1H), 4.03 - 3.95 (m, 1H), 3.94 - 3.55 (m, 13H), 3.37 - 3.26 (m, 2H), 2.91 (td, J = 13.2, 2.9 Hz, 1H), 2.29 - 2.19 (m, 2H), 2.12 - 2.02 (m, 2H), 1.96 - 1.82 (m, 1H), 1.70 - 1.46 (m, 6H), 1.44 - 0.97 (m, 8H), 0.87 (d, J = 4.1 Hz, 11H), 0.08 - 0.03 (m, 6H) ppm. **MS** (m/z): (ESI⁺) calculated for: C₄₇H₇₂NO₁₀Si[M+H]+: 838.5, found: 838.5.

### (S)-1-((4-methoxybenzyl)oxy)pent-4-en-2-yl (S)-1-((S)-2-(3-((S)-2-(allyloxy)-3-((tertbutyldimethylsilyl)oxy)propoxy)-4,5-dimethoxyphenyl)-2-cyclohexylacetyl)piperidine-2-carboxylate (m4(8,8))

General procedure C was applied to phenol **m3(8)** with **B8** (2.0 eq.), cesium in DMF. **Yield:** 96 mg, 82 %, colorless resin. **¹H NMR** (500 MHz, CDCl₃, mixture of rotamers, major rotamer reported): δ 7.21 - 7.17 (m, 2H), 6.89 - 6.82 (m, 2H), 6.51 (d, J = 1.8 Hz, 1H), 6.44 (d, J = 1.9 Hz, 1H), 5.93 (dddt, J = 17.1, 10.4, 7.9, 5.6 Hz, 1H), 5.60 - 5.50 (m, 1H), 5.37 - 5.35 (m, 1H), 5.33-5.21 (m, 1H), 5.19-4.93 (m, 4H), 4.39 (d, J = 11.6 Hz, 1H), 4.34 (d, J = 11.5 Hz, 1H), 4.27 - 4.14 (m, 2H), 4.14 - 4.04 (m, 1H), 4.03 - 3.97 (m, 1H), 3.96 - 3.69 (m, 13H), 3.55 - 3.45 (m, 1H), 3.39-3.28 (m, 2H), 2.91 (td, J = 13.2, 2.9 Hz, 1H), 2.31 -2.04 (m, 4H), 1.94-1.83 (m, 1H), 1.72 -1.27 (m, 9H), 1.23 - 0.99 (m, 3H), 0.97 - 0.59 (m, 11H), 0.06 (s, 6H) ppm. **MS** (m/z): (ESI⁺) calculated for: C₄₇H₇₂NO₁₀Si[M+H]+: 838.5, found: 838.5.

### (S)-1-((4-methoxybenzyl)oxy)pent-4-en-2-yl (S)-1-((S)-2-(3-(((R)-1-(allyloxy)propan-2-yl)oxy)-4,5-dimethoxyphenyl)-2-cyclohexylacetyl)piperidine-2-carboxylate (m4(8,9))

General procedure C was applied to phenol **m3(8)** with **B9** (2.0 eq.), cesium carbonate in DMF **Yield:** 46 mg, 79 %, colorless resin. **¹H NMR** (500 MHz, CDCl₃, mixture of rotamers, major rotamer reported): δ 7.19 (d, J = 8.2 Hz, 2H), 6.84 (d, J = 8.3 Hz, 2H), 6.51 - 6.50 (m, 2H), 5.95 - 5.84 (m, 1H), 5.60 - 5.50 (m, 1H), 5.36 - 5.32 (m, 1H), 5.30 - 5.24 (m, 1H), 5.17 - 5.13 (m, 1H), 5.05 - 4.89 (m, 3H), 4.54 - 4.47 (m, 1H), 4.39 (d, J = 11.4 Hz, 1H), 4.35 (d, J = 11.5 Hz, 0H), 4.05 - 4.01 (m, 2H), 3.84 - 3.75 (m, 9H), 3.62 (d, J = 5.6 Hz, 1H), 3.54 - 3.49 (m, 2H), 3.39 - 3.28 (m, 2H), 2.91 (td, J = 13.3, 3.0 Hz, 1H), 2.30 - 2.18 (m, 2H), 2.14 - 1.99 (m, 2H), 1.87 (d, J = 13.7 Hz, 1H), 1.71 - 1.47 (m, 6H), 1.44 - 0.97 (m, 11H), 0.96 - 0.58 (m, 2H) ppm. **MS** (m/z): (ESI⁺) calculated for: C₄₁H₅₈NO₉[M+H]+: 708.4, found: 708.4.

### (S)-1-((4-methoxybenzyl)oxy)pent-4-en-2-yl (S)-1-((S)-2-(3-(((S)-1-(allyloxy)propan-2-yl)oxy)-4,5-dimethoxyphenyl)-2-cyclohexylacetyl)piperidine-2-carboxylate (m4(8,10))

General procedure C was applied to phenol **m3(8)** with **B10** (2.0 eq.), cesium carbonate in DMF. **Yield:** 55 mg, 95 %, colorless resin. **¹H NMR** (500 MHz, CDCl₃, mixture of rotamers, major rotamer reported): δ 7.20 (d, J = 8.2 Hz, 2H), 6.84 (d, J = 8.6 Hz, 2H), 6.53 (d, J = 1.7 Hz, 1H), 6.49 (d, J = 1.7 Hz, 1H), 5.94 - 5.84 (m, 2H), 5.55 (ddt, J = 17.1, 10.1, 7.1 Hz, 1H), 5.36 (d, J = 5.1 Hz, 1H), 5.30 - 5.24 (m, 1H), 5.18 - 5.13 (m, 1H), 4.98 - 4.88 (m, 2H), 4.53 - 4.47 (m, 1H), 4.39 (d, J = 11.5 Hz, 1H), 4.35 (d, J = 11.6 Hz, 1H), 4.07 - 4.01 (m, 2H), 3.89 (d, J = 13.8 Hz, 1H), 3.85 - 3.74 (m, 9H), 3.62 (d, J = 5.7 Hz, 1H), 3.55 - 3.49 (m, 2H), 3.37 - 3.29 (m, 2H), 2.96 - 2.87 (m, 1H), 2.38 - 2.19 (m, 2H), 2.14 - 1.98 (m, 2H), 1.89 - 1.84 (m, 1H), 1.81 - 1.45 (m, 6H), 1.43 - 0.98 (m, 9H), 0.96 - 0.58 (m, 2H) ppm. **MS** (m/z): (ESI⁺) calculated for: C₄₁H₅₈NO₉[M+H]+: 708.4, found: 708.4.

### (S)-1-hydroxypent-4-en-2-yl (S)-1-((S)-2-(3-(((S)-1-(allyloxy)propan-2-yl)oxy)-4,5-dimethoxyphenyl)-2-cyclohexylacetyl)piperidine-2-carboxylate (m4(8,10)-OH)

General procedure C was applied to phenol **m3(8)** with **B10** (1.5 eq.), cesium in DMF. Then general procedure I was applied without prior with a reaction time of 10 h. **Yield:** 8 mg, 14 % over 2 steps, colorless resin. **¹H NMR** (500 MHz, CD₃CN, mixture of rotamers, major rotamer reported): δ 6.49 (s, 1H), 6.46 (s, 1H), 5.80 - 5.71 (m, 1H), 5.39 (ddt, J = 15.9, 12.1, 7.1 Hz, 1H), 5.19 - 5.08 (m, 2H), 5.04 - 4.94 (m, 2H), 4.77 (dd, J = 20.5, 13.7 Hz, 1H), 4.69 - 4.62 (m, 1H), 4.43 - 4.34 (m, 1H), 4.00 (d, J = 13.6 Hz, 1H), 3.93 - 3.78 (m, 2H), 3.63 (s, 3H), 3.54 (s, 3H), 3.49 - 3.35 (m, 3H), 3.26 - 3.13 (m, 1H), 2.92 (t, J = 8.9 Hz, 1H), 2.77 - 2.70 (m, 1H), 2.06 - 1.60 (m, 6H), 1.60 - 1.24 (m, 7H), 1.24 - 0.92 (m, 8H), 0.90 - 0.60 (m, 2H) ppm. **HR-MS** (m/z): (ESI⁺) calculated for: C₃₃H₅₀NO₈ [M+H]+: 588.35309, found: 588.35375.

### (S)-1-((4-methoxybenzyl)oxy)pent-4-en-2-yl (S)-1-((S)-2-(3-((S)-3-(allyloxy)-2-methylpropoxy)-4,5-dimethoxyphenyl)-2-cyclohexylacetyl)piperidine-2-carboxylate (m4(8,11))

General procedure C was applied to phenol **m3(8)** with **B11** (2.0 eq.), cesium carbonate in DMF. **Yield:** 67 mg, 94 %, colorless resin. **¹H NMR** (500 MHz, CDCl₃, mixture of rotamers, major rotamer reported): δ 7.22 - 7.17 (m, 2H), 6.86 - 6.83 (m, 2H), 6.53 - 6.39 (m, 2H), 5.94 - 5.85 (m, 1H), 5.59 - 5.50 (m, 1H), 5.37 (d, J = 5.3 Hz, 1H), 5.30 - 5.22 (m, 2H), 5.18 - 4.88 (m, 2H), 4.39 (d, J = 11.6 Hz, 1H), 4.35 (d, J = 11.6 Hz, 1H), 4.00 - 3.76 (m, 14H), 3.55 - 3.50 (m, 2H), 3.37 - 3.27 (m, 3H), 2.95 - 2.89 (m, 1H), 2.30 - 2.15 (m, 3H), 2.13 - 2.01 (m, 2H), 1.97 - 1.83 (m, 1H), 1.76 - 1.46 (m, 7H), 1.43 - 1.01 (m, 9H), 0.97 - 0.59 (m, 2H) ppm. **MS** (m/z): (ESI⁺) calculated for: C₄₂H₅₉NO₉Na [M+Na]+: 744.4, found: 744.4.

### (S)-1-((4-methoxybenzyl)oxy)pent-4-en-2-yl (S)-1-((S)-2-(3-((R)-3-(allyloxy)-2-methylpropoxy)-4,5-dimethoxyphenyl)-2-cyclohexylacetyl)piperidine-2-carboxylate (m4(8,12))

General procedure C was applied to phenol **m3(8)** (60 mg, 0.098 mmol, 1.0 eq.) with **B12** (2.0 eq.), cesium carbonate in DMF. **Yield:** 66 mg, 93 %, colorless resin. **¹H NMR** (500 MHz, CDCl₃, mixture of rotamers, major rotamer reported): δ 7.21 - 7.15 (m, 2H), 6.90 - 6.81 (m, 2H), 6.51 - 6.45 (m, 2H), 5.94 - 5.84 (m, 1H), 5.54 (ddt, J = 17.1, 10.2, 7.0 Hz, 1H), 5.37 - 5.35 (m, 1H), 5.29-5.21 (m, 2H), 5.18-4.86 (m, 3H), 4.38 (d, J = 11.6 Hz, 1H), 4.34 (d, J = 11.5 Hz, 1H), 4.00 - 3.73 (m, 14H), 3.53 - 3.48 (m, 2H), 3.45 - 3.41 (m, 1H), 3.35 - 3.28 (m, 2H), 2.92 (td, J = 13.2, 2.8 Hz, 1H), 2.30 - 2.21 (m, 2H), 2.13 - 1.98 (m, 2H), 1.97 - 1.47 (m, 8H), 1.45 - 1.00 (m, 9H), 0.94 - 0.63 (m, 2H) ppm. **MS** (m/z): (ESI⁺) calculated for: C₄₂H₅₉NO₉Na [M+Na]+: 744.4, found: 744.4.

### (S)-1-((4-methoxybenzyl)oxy)pent-4-en-2-yl (S)-1-((S)-2-(3-((R)-3-(allyloxy)butoxy)-4,5-dimethoxyphenyl)-2-cyclohexylacetyl)piperidine-2-carboxylate (m4(8,13))

General procedure C was applied to phenol **m3(8)** with **B13** (2.0 eq.), cesium in DMF. **Yield:** 56 mg, 79 %, colorless resin. **¹H NMR** (500 MHz, CDCl₃, mixture of rotamers, major rotamer reported): δ 7.21 - 7.16 (m, 2H), 6.85 - 6.81 (m, 2H), 5.94 - 5.83 (m, 2H), 5.59 - 5.49 (m, 1H), 5.38 - 5.34 (m, 1H), 5.24 (ddq, J = 16.5, 6.7, 1.7 Hz, 1H), 5.15 - 5.08 (m, 2H), 5.03 - 4.98 (m, 1H), 4.97 - 4.89 (m, 1H), 4.38 (d, J = 11.6 Hz, 1H), 4.34 (d, J = 11.5 Hz, 1H), 4.13 - 4.00 (m, 3H), 3.96 - 3.87 (m, 2H), 3.81 - 3.77 (m, 9H), 3.76 - 3.70 (m, 1H), 3.37 - 3.26 (m, 3H), 2.92 (td, J = 13.0, 2.8 Hz, 1H), 2.30 - 2.19 (m, 2H), 2.14 - 1.83 (m, 5H), 1.71 - 1.36 (m, 7H), 1.35 - 0.98 (m, 9H), 0.97 - 0.60 (m, 2H) ppm. **MS** (m/z): (ESI⁺) calculated for: C₄₂H₅₉NO₉Na [M+Na]+: 744.4, found: 744.4.

### (S)-1-((4-methoxybenzyl)oxy)pent-4-en-2-yl (S)-1-((S)-2-(3-((S)-3-(allyloxy)butoxy)-4,5-dimethoxyphenyl)-2-cyclohexylacetyl)piperidine-2-carboxylate (m4(8,14))

General procedure C was applied to phenol **m3(8)** with **B13** (2.0 eq.), cesium carbonate in DMF. **Yield:** 71 mg, quant., colorless resin. **¹H NMR** (500 MHz, CDCl₃, mixture of rotamers, major rotamer reported): δ 7.20 - 7.14 (m, 2H), 6.84 - 6.82 (m, 2H), 6.48 (d, J = 1.9 Hz, 1H), 6.41 (s, 1H), 5.95 - 5.82 (m, 2H), 5.58 - 5.48 (m, 1H), 5.28 - 5.19 (m, 1H), 5.16 - 5.04 (m, 2H), 5.02 - 4.86 (m, 2H), 4.38 (d, J = 11.8 Hz, 1H), 4.33 (d, J = 11.6 Hz, 1H), 4.13 - 3.99 (m, 3H), 3.97 - 3.85 (m, 2H), 3.83 - 3.69 (m, 10H), 3.35 - 3.27 (m, 2H), 2.91 (td, J = 13.1, 2.8 Hz, 1H), 2.29 - 2.18 (m, 2H), 2.12 - 1.81 (m, 6H), 1.71 - 1.46 (m, 6H), 1.43 - 0.96 (m, 9H), 0.95 - 0.59 (m, 2H) ppm. **MS** (m/z): (ESI⁺) calculated for: C₄₂H₅₉NO₉Na [M+Na]+: 744.4, found: 744.4.

### (S)-1-((4-methoxybenzyl)oxy)pent-4-en-2-yl (S)-1-((S)-2-(3-((R)-3-(allyloxy)-2-((tertbutyldimethylsilyl)oxy)propoxy)-4,5-dimethoxyphenyl)-2-cyclohexylacetyl)piperidine-2-carboxylate (m4(8,15))

General procedure C was applied to phenol **m3(8)** with **B15** (2.0 eq.), cesium carbonate (in DMF. **Yield:** 82 mg, 70 %, colorless resin. **¹H NMR** (500 MHz, CDCl₃, mixture of rotamers, major rotamer reported): δ 7.21 - 7.17 (m, 2H), 6.84 (d, J = 8.6 Hz, 2H), 6.52 (d, J = 1.9 Hz, 1H), 6.43 (d, J = 1.8 Hz, 1H), 5.94 - 5.84 (m, 1H), 5.58 - 5.50 (m, 1H), 5.37 - 5.35 (m, 1H), 5.29 - 5.22 (m, 2H), 5.18-4.86 (m, 3H), 4.38 (d, J = 11.7 Hz, 1H), 4.34 (d, J = 11.6 Hz, 1H), 4.21 -4.15 (m, 1H), 4.04 - 3.97 (m, 3H), 3.95 - 3.87 (m, 2H), 3.82 - 3.75 (m, 9H), 3.60 - 3.56 (m, 1H), 3.54 - 3.49 (m, 2H), 3.35 - 3.28 (m, 2H), 2.92 (td, J = 13.2, 2.8 Hz, 1H), 2.30 - 2.16 (m, 2H), 2.13 - 1.97 (m, 2H), 1.93 - 1.84 (m, 1H), 1.76 - 0.97 (m, 12H), 0.88 (d, J = 2.7 Hz, 11H), 0.12 (s, 6H) ppm. **MS** (m/z): (ESI⁺) calculated for: C₄₇H₇₂NO₁₀Si [M+H]+: 838.5, found: 838.5.

### (S)-1-((4-methoxybenzyl)oxy)pent-4-en-2-yl (S)-1-((S)-2-(3-((S)-3-(allyloxy)-2-((tertbutyldimethylsilyl)oxy)propoxy)-4,5-dimethoxyphenyl)-2-cyclohexylacetyl)piperidine-2-carboxylate (m4(8,16))

General procedure C was applied to phenol **m3(8)** with **B16** (2.0 eq.), cesium carbonate in DMF. **Yield:** 96 mg, 82 %, colorless resin. **¹H NMR** (500 MHz, CDCl₃, mixture of rotamers, major rotamer reported): δ 7.21 - 7.18 (m, 2H), 6.84 (d, J = 8.6 Hz, 2H), 6.52 (d, J = 1.9 Hz, 1H), 6.43 (d, J = 1.8 Hz, 1H), 5.89 (ddt, J = 17.3, 10.8, 5.5 Hz, 1H), 5.54 (ddt, J = 17.1, 10.2, 7.0 Hz, 1H), 5.36 (d, J = 5.1 Hz, 1H), 5.30 - 5.21 (m, 2H), 5.18 - 4.88 (m, 3H), 4.38 (d, J = 11.6 Hz, 1H), 4.34 (d, J = 11.6 Hz, 1H), 4.19 (p, J = 5.4 Hz, 2H), 4.07 - 3.88 (m, 5H), 3.85 - 3.75 (m, 9H), 3.61 - 3.57 (m, 1H), 3.55 - 3.46 (m, 2H), 3.37 - 3.27 (m, 2H), 2.92 (td, J = 13.2, 2.8 Hz, 1H), 2.50 - 2.16 (m, 2H), 2.14 - 1.82 (m, 3H), 1.81 - 1.45 (m, 7H), 1.45 - 0.98 (m, 7H), 0.89 (s, 11H), 0.14 - 0.09 (m, 6H) ppm. **MS** (m/z): (ESI⁺) calculated for: C₄₇H₇₂NO₁₀Si [M+H]+: 838.5, found: 838.5.

### (S)-1-((4-methoxybenzyl)oxy)pent-4-en-2-yl (S)-1-((S)-2-(3-(((R)-4-(allyloxy)butan-2-yl)oxy)-4,5-dimethoxyphenyl)-2-cyclohexylacetyl)piperidine-2-carboxylate (m4(8,17))

General procedure C was applied to phenol **m3(8)** with **B17** (2.0 eq.), cesium carbonate (in DMF. **Yield:** 51 mg, 86%, colorless resin. **¹H NMR** (500 MHz, CDCl₃, mixture of rotamers, major rotamer reported): δ 7.24 - 7.16 (m, 2H), 6.86 (ddd, J = 8.7, 5.8, 2.4 Hz, 2H), 6.52 (d, J = 1.7 Hz, 1H), 6.46 (d, J = 1.9 Hz, 1H), 5.97 - 5.79 (m, 1H), 5.54 (ddt, J = 17.2, 10.3, 6.9 Hz, 1H), 5.39 - 5.34 (m, 1H), 5.30 - 4.84 (m, 6H), 4.63 - 4.29 (m, 4H), 4.03 - 3.90 (m, 2H), 3.82 - 3.75 (m, 9H), 3.68 - 3.45 (m, 3H), 3.39 - 3.28 (m, 2H), 2.97 - 2.85 (m, 1H), 2.51 - 2.17 (m, 2H), 2.15 - 1.96 (m, 3H), 1.95 - 1.78 (m, 2H), 1.61 (t, J = 11.3 Hz, 5H), 1.42 - 0.99 (m, 9H), 0.97 - 0.55 (m, 2H) ppm. **MS** (m/z): (ESI⁺) calculated for: C₄₂H₅₉NO₉Na [M+Na]+: 744.4, found: 744.4.

### (S)-1-((4-methoxybenzyl)oxy)pent-4-en-2-yl (S)-1-((S)-2-(3-(((S)-4-(allyloxy)butan-2-yl)oxy)-4,5-dimethoxyphenyl)-2-cyclohexylacetyl)piperidine-2-carboxylate (m4(8,18))

General procedure C was applied to phenol **m3(8)** with **B18** (2.0 eq.), cesium carbonate in DMF. **Yield:** 51 mg, 86 %, colorless resin.**¹H NMR** (500 MHz, CDCl₃, mixture of rotamers, major rotamer reported): δ 7.19 (d, J = 8.2 Hz, 2H), 6.84 (d, J = 8.4 Hz, 2H), 6.50 - 6.47 (m, 2H), 5.61 - 5.47 (m, 1H), 5.38 - 5.32 (m, 1H), 5.29 - 5.20 (m, 2H), 5.17 - 5.08 (m, 2H ), 4.99 - 4.87 (m, 2H), 4.57 - 4.49 (m, 1H), 4.40 - 4.32 (m, 2H), 4.02 - 3.92 (m, 2H), 3.89 (d, J = 14.1 Hz, 1H), 3.85 - 3.73 (m, 9H), 3.64 - 3.54 (m, 2H), 3.43 - 3.26 (m, 2H), 2.97 - 2.85 (m, 1H), 2.48 - 2.16 (m, 2H), 2.15 - 1.97 (m, 3H), 1.96 - 1.81 (m, 2H), 1.81 - 1.45 (m, 7H), 1.44 - 1.00 (m, 9H), 0.98 - 0.59 (m, 2H) ppm. **MS** (m/z): (ESI⁺) calculated for: C₄₂H₅₉NO₉Na [M+Na]+: 744.4, found: 744.4.

### (2R,3S)-3-methylpent-4-en-2-yl (S)-1-((S)-2-(3-(2-(allyloxy)ethoxy)-4,5-dimethoxyphenyl)-2-cyclohexylacetyl)piperidine-2-carboxylate (m4(9,3))

General procedure C was applied to phenol **m3(9)** with **B3** (2.0 eq.) and cesium carbonate in DMF. **Yield:** 38 mg, 81 %, colorless resin.**¹H NMR** (5 00 MHz, CDCl₃, mixture of rotamers, major rotamer reported): δ 6.52 (d, J = 1.9 Hz, 1H), 6.50 (d, J = 1.9 Hz, 1H), 5.99 - 5.85 (m, 1H), 5.49 - 5.30 (m, 2H), 5.29 - 5.05 (m, 2H), 5.00 - 4.71 (m, 2H), 4.18 - 4.12 (m, 2H), 4.10 - 4.05 (m, 2H), 3.98 - 3.90 (m, 1H), 3.87 - 3.75 (m, 8H), 3.33 (d, J = 9.9 Hz, 1H), 3.00 - 2.88 (m, 1H), 2.28 - 2.20 (m, 1H), 2.16 - 1.99 (m, 2H), 1.97 - 1.75 (m, 2H), 1.62 (d, J = 13.0 Hz, 6H), 1.47 - 0.99 (m, 8H), 0.99 - 0.95 (m, 3H), 0.93 - 0.59 (m, 5H) ppm. **MS** (m/z): (ESI⁺) calculated for: C₃₃H₅₀NO₇[M+H]+: 572.4, found: 572.4.

### (2R,3S)-3-methylpent-4-en-2-yl (S)-1-((S)-2-(3-(3-(allyloxy)propoxy)-4,5-dimethoxyphenyl)-2-cyclohexylacetyl)piperidine-2-carboxylate (m4(9,4))

General procedure C was applied to phenol **m3(9)** with **B4** (2.0 eq.), and cesium carbonate in DMF. **Yield:** 44 mg, 92 %, colorless resin. **¹H NMR** (500 MHz, CDCl₃, mixture of rotamers, major rotamer reported): δ 6.51 (d, J = 1.9 Hz, 1H), 6.49 (d, J = 1.9 Hz, 1H), 5.94 - 5.85 (m, 1H), 5.45 - 5.37 (m, 1H), 5.35 - 5.32 (m, 1H), 5.28 - 5.23 (m, 1H), 5.17 - 5.13 (m, 1H), 4.92 - 4.88 (m, 1H), 4.85-4.80 (m, 1H), 4.76 (dd, J = 6.5, 5.5 Hz, 1H), 4.10 - 4.04 (m, 2H), 3.99 - 3.96 (m, 2H), 3.96 - 3.92 (m, 1H), 3.81 (s, 3H), 3.79 (s, 3H), 3.65 - 3.60 (m, 2H), 3.33 (d, J = 9.9 Hz, 1H), 2.98 - 2.91 (m, 1H), 2.23 (d, J = 13.2 Hz, 1H), 2.13 - 1.99 (m, 4H), 1.87 - 1.82 (m, 1H), 1.81 - 1.50 (m, 6H), 1.42 (tdt, J = 12.8, 7.7, 4.1 Hz, 1H), 1.37 - 0.99 (m, 6H), 0.96 (d, J = 6.4 Hz, 3H), 0.92 - 0.82 (m, 1H), 0.80 (d, J = 6.9 Hz, 3H), 0.78 - 0.64 (m, 1H) ppm. **MS** (m/z): (ESI⁺) calculated for: C₃₄H₅₂NO₇[M+H]+: 586.4, found: 586.4.

### (2R,3S)-3-methylpent-4-en-2-yl (S)-1-((S)-2-(3-((S)-2-(allyloxy)propoxy)-4,5-dimethoxyphenyl)-2-cyclohexylacetyl)piperidine-2-carboxylate (m4(9,5))

General procedure C was applied to phenol **m3(9)** with **B5** (2.0 eq.), cesium carbonate (in DMF. **Yield:** 47 mg, 78%, colorless resin. **¹H NMR** (500 MHz, CDCl₃, mixture of rotamers, major rotamer reported): δ 6.52 - 6.45 (m, 1H), 5.99 - 5.84 (m, 1H), 5.49 - 5.28 (m, 1H), 5.27 - 5.02 (m, 2H), 5.01 - 4.52 (m, 3H), 4.15 - 4.11 (m, 2H), 4.07 - 3.83 (m, 4H), 3.82 - 3.78 (m, 6H), 3.32 (d, J = 9.8 Hz, 0H), 2.93 (td, J = 13.1, 2.9 Hz, 0H), 2.25 - 2.18 (m, 1H), 2.16 - 1.97 (m, 2H), 1.95 - 1.79 (m, 2H), 1.73 - 1.47 (m, 6H), 1.45 - 0.89 (m, 15H), 0.90 - 0.57 (m, 5H) ppm. **MS** (m/z): (ESI⁺) calculated for: C₃₄H₅₂NO₇[M+H]+: 586.4, found: 586.4.

### (2R,3S)-3-methylpent-4-en-2-yl (S)-1-((S)-2-(3-((R)-2-(allyloxy)propoxy)-4,5-dimethoxyphenyl)-2-cyclohexylacetyl)piperidine-2-carboxylate (m4(9,6))

General procedure C was applied to phenol **m3(9)** with **B6** (2.0 eq.), cesium carbonate in DMF. **Yield:** 54 mg, 90 %, colorless resin. **¹H NMR** (300 MHz, CDCl₃, mixture of rotamers, major rotamer reported): δ 6.51 (d, J = 1.8 Hz, 1H), 6.48 - 6.46 (m, 1H), 5.98 - 5.84 (m, 1H), 5.48 - 5.28 (m, 2H), 5.27 - 5.01 (m, 2H), 4.99 - 4.50 (m, 3H), 4.15 - 4.10 (m, 2H), 4.08 - 3.73 (m, 10H), 3.32 (d, J = 9.9 Hz, 1H), 2.22 (d, J = 13.6 Hz, 1H), 2.14 - 1.79 (m, 3H), 1.72 - 1.47 (m, 6H), 1.46 - 0.98 (m, 10H), 0.98 - 0.94 (m, 3H), 0.93 - 0.56 (m, 5H) ppm. **MS** (m/z): (ESI⁺) calculated for: C₃₄H₅₂NO₇[M+H]+: 586.4, found: 586.4.

### (2R,3S)-3-methylpent-4-en-2-yl (S)-1-((S)-2-(3-((R)-2-(allyloxy)-3-((tertbutyldimethylsilyl)oxy)propoxy)-4,5-dimethoxyphenyl)-2-cyclohexylacetyl)piperidine-2-carboxylate (m4(9,7))

General procedure C was applied to phenol **m3(9)** with **B7** (2.0 eq.), cesium carbonate in DMF. **Yield:** 62 mg, 85 %, colorless resin. **¹H NMR** (500 MHz, CDCl₃, mixture of rotamers, major rotamer reported): δ 6.54 (d, J = 1.8 Hz, 1H), 6.47 (d, J = 1.8 Hz, 1H), 5.97 - 5.88 (m, 1H), 5.46 - 5.38 (m, 1H), 5.35 - 5.32 (m, 1H), 5.32 - 5.26 (m, 2H), 5.18 - 5.12 (m, 1H), 4.93 - 4.89 (m, 1H), 4.86 -4.80 (m, 1H), 4.79 - 4.73 (m, 1H), 4.23 -4.19 (m, 2H), 4.08 (dd, J = 9.8, 4.3 Hz, 1H), 4.02 - 3.97 (m, 1H), 3.97 - 3.91 (m, 1H), 3.83 - 3.76 (m, 9H), 3.32 (d, J = 9.9 Hz, 1H), 2.94 (td, J = 13.3, 2.8 Hz, 1H), 2.28 - 2.20 (m, 1H), 2.12 - 2.04 (m, 2H), 1.86 (d, J = 12.5 Hz, 1H), 1.77 - 1.52 (m, 5H), 1.48 - 1.37 (m, 1H), 1.36 - 0.98 (m, 8H), 0.97 (d, J = 6.4 Hz, 3H), 0.88 (s, 9H), 0.81 (d, J = 6.9 Hz, 3H), 0.77 - 0.63 (m, 1H), 0.06 (s, 3H) ppm.

### (2R,3S)-3-methylpent-4-en-2-yl (S)-1-((S)-2-(3-((S)-2-(allyloxy)-3-((tertbutyldimethylsilyl)oxy)propoxy)-4,5-dimethoxyphenyl)-2-cyclohexylacetyl)piperidine-2-carboxylate (m4(9,8))

General procedure C was applied to phenol **m3(9)** with **B8** (2.0 eq.), cesium carbonate in DMF. **Yield:** 49 mg, 67 %, colorless resin. **¹H NMR** (500 MHz, CDCl₃, mixture of rotamers, major rotamer reported): δ 6.53 (d, J = 1.8 Hz, 1H), 6.47 (d, J = 1.9 Hz, 1H), 5.97 - 5.87 (m, 1H), 5.42 (ddd, J = 17.1, 10.4, 7.8 Hz, 1H), 5.34 - 5.32 (m, 1H), 5.31 - 5.25 (m, 1H), 5.17 - 5.12 (m, 1H), 5.10-5.04 (m, 1H), 4.93 - 4.80 (m, 2H), 4.79 - 4.73 (m, 1H), 4.25 - 4.16 (m, 2H), 4.08 (td, J = 9.4, 8.9, 3.9 Hz, 1H), 3.99 (dt, J = 9.8, 5.5 Hz, 1H), 3.96 - 3.89 (m, 1H), 3.84 - 3.72 (m, 9H), 3.32 (d, J = 9.9 Hz, 1H), 2.94 (td, J = 13.3, 2.8 Hz, 1H), 2.23 (d, J = 14.4 Hz, 1H), 2.12 - 1.99 (m, 3H), 1.85 (d, J = 12.6 Hz, 1H), 1.70 - 1.51 (m, 5H), 1.42 (tdt, J = 12.8, 7.6, 3.9 Hz, 1H), 1.36 - 0.98 (m, 5H), 0.96 (d, J = 6.4 Hz, 3H), 0.88 (s, 9H), 0.86 - 0.61 (m, 5H), 0.05 (s, 6H) ppm.

### (2R,3S)-3-methylpent-4-en-2-yl (S)-1-((S)-2-(3-(((R)-1-(allyloxy)propan-2-yl)oxy)-4,5-dimethoxyphenyl)-2-cyclohexylacetyl)piperidine-2-carboxylate (m4(9,9))

General procedure C was applied to phenol **m3(9)** with **B9** (2.0 eq.), cesium carbonate DMF. **Yield:** 46 mg, 96 %, colorless resin. **¹H NMR** (500 MHz, CDCl₃, mixture of rotamers, major rotamer reported): δ 6.55 (d, J = 1.8 Hz, 1H), 6.50 (d, J = 1.8 Hz, 1H), 5.95 - 5.84 (m, 1H), 5.50 - 5.36 (m, 1H), 5.33 (d, J = 5.8 Hz, 1H), 5.29 - 5.23 (m, 1H), 5.18 - 5.13 (m, 1H), 4.94 - 4.89 (m, 1H), 4.86 - 4.81 (m, 1H), 4.79 - 4.72 (m, 1H), 4.55 - 4.47 (m, 1H), 4.04 (d, J = 5.5 Hz, 2H), 3.98 - 3.91 (m, 1H), 3.84 - 3.79 (m, 6H), 3.68 - 3.60 (m, 1H), 3.53 (d, J = 5.0 Hz, 1H), 3.32 (d, J = 9.9 Hz, 1H), 2.95 (td, J = 13.2, 2.7 Hz, 1H), 2.23 (d, J = 13.8 Hz, 1H), 2.13 - 1.98 (m, 2H), 1.86 (d, J = 12.6 Hz, 1H), 1.78 - 1.50 (m, 6H), 1.42 (tdt, J = 12.8, 7.7, 3.9 Hz, 1H), 1.35 - 0.99 (m, 8H), 0.97 (d, J = 6.4 Hz, 3H), 0.92 - 0.62 (m, 5H) ppm. **MS** (m/z): (ESI⁺) calculated for: C₃₄H₅₂NO₇[M+H]+: 586.4, found: 586.4.

### (2R,3S)-3-methylpent-4-en-2-yl (S)-1-((S)-2-(3-(((S)-1-(allyloxy)propan-2-yl)oxy)-4,5-dimethoxyphenyl)-2-cyclohexylacetyl)piperidine-2-carboxylate (m4(9,10))

General procedure C was applied to phenol **m3(9)** with **B10** (2.0 eq.), cesium in DMF. **Yield:** 44 mg, 92 %, colorless resin. **¹H NMR** (500 MHz, CDCl₃, mixture of rotamers, major rotamer reported): δ 6.53 (s, 2H), 5.96 - 5.83 (m, 1H), 5.50 - 5.37 (m, 1H), 5.35 - 5.31 (m, 1H), 5.31 - 5.23 (m, 1H), 5.20 - 5.12 (m, 1H), 4.93 - 4.89 (m, 1H), 4.87 - 4.82 (m, 1H), 4.79 - 4.73 (m, 1H), 4.54 - 4.46 (m, 1H), 4.06 - 4.00 (m, 2H), 3.96 - 3.90 (m, 1H), 3.84 - 3.78 (m, 6H), 3.67 - 3.60 (m, 1H), 3.54 - 3.52 (m, 1H), 3.31 (d, J = 9.8 Hz, 1H), 2.95 (td, J = 13.3, 2.7 Hz, 1H), 2.23 (d, J = 13.8 Hz, 1H), 2.12 - 1.97 (m, 2H), 1.86 (d, J = 12.6 Hz, 1H), 1.72 - 1.49 (m, 6H), 1.48 - 1.37 (m, 1H), 1.35 - 0.99 (m, 8H), 0.97 (d, J = 6.4 Hz, 3H), 0.93 - 0.83 (m, 1H), 0.81 (d, J = 7.0 Hz, 3H), 0.78 - 0.60 (m, 1H) ppm. **MS** (m/z): (ESI⁺) calculated for: C₃₄H₅₂NO₇[M+H]+: 586.4, found: 586.4.

### (2R,3S)-3-methylpent-4-en-2-yl (S)-1-((S)-2-(3-((S)-3-(allyloxy)-2-methylpropoxy)-4,5-dimethoxyphenyl)-2-cyclohexylacetyl)piperidine-2-carboxylate (m4(9,11))

General procedure C was applied to phenol **m3(9)** with **B11** (2.0 eq.), cesium carbonate (in DMF. **Yield:** 48 mg, 98 %, colorless resin. **¹H NMR** (500 MHz, CDCl₃, mixture of rotamers, major rotamer reported): δ 6.50 (d, J = 1.8 Hz, 1H), 6.47 (d, J = 1.8 Hz, 1H), 5.94 - 5.82 (m, 1H), 5.45 - 5.36 (m, 1H), 5.34 - 5.32 (m, 1H), 5.27 - 5.21 (m, 1H), 5.13 (dq, J = 10.4, 1.4 Hz, 1H), 4.89 (ddd, J = 10.5, 1.9, 0.9 Hz, 1H), 4.82 (dt, J = 17.2, 1.4 Hz, 1H), 4.78 - 4.72 (m, 1H), 3.99 - 3.91 (m, 4H), 3.86 - 3.82 (m, 1H), 3.80 (s, 6H), 3.55 - 3.48 (m, 1H), 3.45 - 3.39 (m, 2H), 2.94 (td, J = 13.3, 2.8 Hz, 1H), 2.30 - 2.19 (m, 2H), 2.12 - 2.00 (m, 3H), 1.85 (d, J = 12.6 Hz, 1H), 1.70 - 1.52 (m, 6H), 1.42 (tdt, J = 12.9, 7.5, 4.1 Hz, 1H), 1.35 - 0.98 (m, 7H), 0.95 (d, J = 6.3 Hz, 3H), 0.92 - 0.81 (m, 1H), 0.79 (d, J = 6.9 Hz, 3H), 0.76 - 0.61 (m, 1H) ppm. **MS** (m/z): (ESI⁺) calculated for: C₃₅H₅₄NO₇[M+H]⁺: 600.4, found: 600.4.

### (2R,3S)-3-methylpent-4-en-2-yl (S)-1-((S)-2-(3-((S)-3-(allyloxy)-2-methylpropoxy)-4,5-dimethoxyphenyl)-2-cyclohexylacetyl)piperidine-2-carboxylate (m4(9,12))

General procedure C was applied to phenol **m3(9)** with **B12** (2.0 eq.), cesium carbonate in DMF. **Yield:** 45 mg, 92 %m colorless resin. **¹H NMR** (500 MHz, CDCl₃, mixture of rotamers, major rotamer reported): δ 6.50 (d, J = 1.7 Hz, 1H), 6.48 (d, J = 1.9 Hz, 1H), 5.95 - 5.84 (m, 1H), 5.45 - 5.37 (m, 1H), 5.35 - 5.32 (m, 1H), 5.28 - 5.23 (m, 1H), 5.14 (dt, J = 10.3, 1.5 Hz, 1H), 4.90 (dd, J = 10.7, 1.8 Hz, 1H), 4.85 - 4.79 (m, 1H), 4.76 (p, J = 6.2 Hz, 1H), 4.00 - 3.91 (m, 4H), 3.88 - 3.76 (m, 8H), 3.54 - 3.48 (m, 1H), 3.46-3.40 (m, 1H), 3.33 (d, J = 9.9 Hz, 1H), 2.95 (td, J = 13.3, 2.8 Hz, 1H), 2.26 (td, J = 17.5, 15.1, 9.1 Hz, 2H), 2.14 - 2.00 (m, 3H), 1.86 (d, J = 12.7 Hz, 1H), 1.72 - 1.51 (m, 7H), 1.48 - 1.37 (m, 1H), 1.36 - 1.03 (m, 5H), 0.96 (d, J = 6.4 Hz, 3H), 0.92 - 0.82 (m, 1H), 0.80 (d, J = 6.8 Hz, 3H), 0.78 - 0.62 (m, 1H) ppm. **MS** (m/z): (ESI⁺) calculated for: C₃₅H₅₄NO₇[M+H]⁺: 600.4, found: 600.4.

### (2R,3S)-3-methylpent-4-en-2-yl (S)-1-((S)-2-(3-((R)-3-(allyloxy)butoxy)-4,5-dimethoxyphenyl)-2-cyclohexylacetyl)piperidine-2-carboxylate (m4(9,13))

General procedure C was applied to phenol **m3(9)** with **B13** (2.0 eq.), cesium carbonate in DMF. **Yield:** 48 mg, 98%, colorless resin. **¹H NMR** (500 MHz, CDCl₃, mixture of rotamers, major rotamer reported): δ 6.52 (d, J = 1.8 Hz, 1H), 6.48 (d, J = 1.9 Hz, 1H), 5.95 - 5.83 (m, 1H), 5.46 - 5.38 (m, 1H), 5.35 - 5.32 (m, 1H), 5.14 - 5.05 (m, 2H), 4.92 - 4.88 (m, 1H), 4.86 - 4.81 (m, 1H), 4.79-4.73 (m, 1H), 4.13-4.01 (m, 3H), 3.99 - 3.87 (m, 3H), 3.81 (s, 3H), 3.79 (s, 3H), 3.76 - 3.69 (m, 1H), 3.33 (d, J = 9.9 Hz, 1H), 2.95 (td, J = 13.3, 2.8 Hz, 1H), 2.23 (d, J = 13.7 Hz, 1H), 2.12 - 1.82 (m, 4H), 1.70 - 1.49 (m, 5H), 1.48 - 1.37 (m, 1H), 1.37 - 0.97 (m, 9H), 0.96 (d, J = 6.4 Hz, 3H), 0.92 - 0.82 (m, 1H), 0.80 (d, J = 6.9 Hz, 3H), 0.79 - 0.63 (m, 1H) ppm. **MS** (m/z): (ESI⁺) calculated for: C₃₅H₅₄NO₇[M+H]⁺: 600.4, found: 600.4.

### (2R,3S)-3-methylpent-4-en-2-yl (S)-1-((S)-2-(3-((S)-3-(allyloxy)butoxy)-4,5-dimethoxyphenyl)-2-cyclohexylacetyl)piperidine-2-carboxylate (m4(9,14))

General procedure C was applied to phenol **m3(9)** with **B14** (2.0 eq.), cesium carbonate in DMF. **Yield:** 48 mg, 98 %, colorless resin. **¹H NMR** (500 MHz, CDCl₃, mixture of rotamers, major rotamer reported): δ 6.51 (d, J = 1.9 Hz, 1H), 6.49 (d, J = 1.9 Hz, 1H), 5.94 - 5.84 (m, 1H), 5.42 (ddd, J = 17.1, 10.3, 7.8 Hz, 1H), 5.34 (d, J = 4.7 Hz, 1H), 5.25 (dp, J = 17.2, 1.7 Hz, 1H), 5.14 - 5.05 (m, 2H), 4.79 - 4.73 (m, 1H), 4.15 - 4.01 (m, 3H), 3.97 - 3.89 (m, 2H), 3.82 (s, 3H), 3.80 (s, 3H), 3.78-3.71 (m, 1H), 3.33 (d, J = 9.9 Hz, 1H), 2.95 (td, J = 13.3, 2.8 Hz, 1H), 2.24 (d, J = 14.3 Hz, 1H), 2.15 - 1.89 (m, 4H), 1.86 (d, J = 12.7 Hz, 1H), 1.81 - 1.51 (m, 5H), 1.43 (dddd, J = 16.8, 13.0, 8.3, 4.0 Hz, 1H), 1.38 - 1.23 (m, 4H), 1.22 (d, J = 6.5 Hz, 7H), 1.20 - 0.99 (m, 3H), 0.97 (d, J = 6.4 Hz, 3H), 0.86 (td, J = 13.0, 12.1, 3.8 Hz, 1H), 0.81 (d, J = 6.9 Hz, 3H), 0.77 - 0.64 (m, 1H) ppm. **MS** (m/z): (ESI⁺) calculated for: C₃₅H₅₄NO₇[M+H]⁺: 600.4, found: 600.4.

### (2R,3S)-3-methylpent-4-en-2-yl (S)-1-((S)-2-(3-((R)-3-(allyloxy)-2-((tert-butyldimethylsilyl)oxy)propoxy)-4,5-dimethoxyphenyl)-2-cyclohexylacetyl)piperidine-2-carboxylate (m4(9,15))

General procedure C was applied to phenol **m3(9)** with **B15** (1.3 eq.), cesium carbonate in DMF. **Yield:** 58 mg, 79%, colorless resin. **¹H NMR** (500 MHz, CDCl₃, mixture of rotamers, major rotamer reported): δ 6.53 (d, J = 1.8 Hz, 1H), 6.46 (d, J = 1.9 Hz, 1H), 5.97 - 5.84 (m, 1H), 5.79 - 5.64 (m, 1H), 5.35 - 5.32 (m, 1H), 5.29 - 5.24 (m, 1H), 5.18 - 5.14 (m, 1H), 4.91 - 4.80 (m, 2H), 4.76 (p, J = 6.2 Hz, 1H), 4.04 - 3.89 (m, 5H), 3.88 - 3.77 (m, 6H), 3.58 - 3.56 (m, 1H), 3.54 - 3.51 (m, 1H), 3.33 (d, J = 9.8 Hz, 3H), 2.99 - 2.91 (m, 1H), 2.24 (d, J = 13.7 Hz, 1H), 2.18 - 1.89 (m, 3H), 1.86 (d, J = 12.5 Hz, 1H), 1.75 - 1.51 (m, 6H), 1.49 - 1.37 (m, 1H), 1.36 - 0.97 (m, 4H), 0.96 (d, J = 6.3 Hz, 3H), 0.88 (d, J = 2.1 Hz, 9H), 0.80 (d, J = 7.0 Hz, 3H), 0.76 - 0.66 (m, 1H), 0.12 (s, 6H) ppm.

### (2R,3R)-3-methylpent-4-en-2-yl (S)-1-((S)-2-(3-((R)-3-(allyloxy)-2-hydroxypropoxy)-4,5-dimethoxyphenyl)-2-cyclohexylacetyl)piperidine-2-carboxylate (m4(9,15)-OH)

General procedure C was applied to phenol **m3(9)** with **B15** (1.5 eq.), cesium carbonate in DMF. Then general procedure H was applied without prior purification. **Yield:** 8 mg, 42 % over 2 steps, colorless resin. **¹H NMR** (500 MHz, CDCl₃, mixture of rotamers, major rotamer reported): δ 6.55 (d, J = 1.9 Hz, 1H), 6.51 (d, J = 1.8 Hz, 1H), 5.96 - 5.86 (m, 1H), 5.67 - 5.48 (m, 1H), 5.35 (d, J = 5.2 Hz, 1H), 5.29 - 5.24 (m, 1H), 5.21 - 5.17 (m, 1H), 5.01 - 4.91 (m, 2H), 4.77 - 4.67 (m, 1H), 4.21 - 4.12 (m, 1H), 4.11 - 3.98 (m, 4H), 3.97 - 3.91 (m, 1H), 3.83 (s, 3H), 3.81 (s, 3H), 3.67 - 3.57 (m, 2H), 3.34 (d, J = 9.8 Hz, 1H), 2.98 - 2.88 (m, 1H), 2.25 (d, J = 13.8 Hz, 1H), 2.17 - 1.96 (m, 4H), 1.87 (d, J = 12.6 Hz, 1H), 1.75 - 1.51 (m, 5H), 1.44 (q, J = 13.1 Hz, 1H), 1.37 - 1.03 (m, 6H), 1.00 (d, J = 6.3 Hz, 3H), 0.93 - 0.80 (m, 1H), 0.77 (d, J = 6.9 Hz, 3H), 0.75 - 0.63 (m, 1H) ppm. **HR-MS** (m/z): (ESI⁺) calculated for: C₃₄H₅₂NO₈ [M+H]+: 602.36874, found: 602.36961.

### (2R,3S)-3-methylpent-4-en-2-yl (S)-1-((S)-2-(3-((S)-3-(allyloxy)-2-((tertbutyldimethylsilyl)oxy)propoxy)-4,5-dimethoxyphenyl)-2-cyclohexylacetyl)piperidine-2-carboxylate (m4(9,16))

General procedure C was applied to phenol **m3(9)** with **B16** (2.0 eq.), cesium carbonate DMF. **Yield:** 53 mg, 73%, colorless resin. **¹H NMR** (500 MHz, CDCl₃, mixture of rotamers, major rotamer reported): δ 6.54 (d, J = 1.8 Hz, 1H), 6.45 (d, J = 1.7 Hz, 1H), 5.94 - 5.84 (m, 2H), 5.45 - 5.37 (m, 1H), 5.35 - 5.33 (m, 1H), 5.30 - 5.24 (m, 1H), 5.18 - 5.14 (m, 1H), 4.92 - 4.88 (m, 1H), 4.85 - 4.80 (m, 1H), 4.79 - 4.73 (m, 1H), 4.22 - 4.16 (m, 2H), 4.04 - 3.89 (m, 5H), 3.81 (s, 3H), 3.79 (s, 3H), 3.62 - 3.56 (m, 1H), 3.55 - 3.48 (m, 1H), 3.33 (d, J = 9.9 Hz, 1H), 3.00 - 2.91 (m, 1H), 2.24 (d, J = 13.7 Hz, 1H), 2.15 - 1.99 (m, 2H), 1.86 (d, J = 12.6 Hz, 1H), 1.72 - 1.51 (m, 6H), 1.43 (tdt, J = 12.7, 7.7, 4.1 Hz, 1H), 1.37 - 0.98 (m, 3H), 0.96 (d, J = 6.4 Hz, 3H), 0.89 (s, 9H), 0.87 - 0.82 (m, 1H), 0.80 (d, J = 6.9 Hz, 3H), 0.77 - 0.64 (m, 1H), 0.13 (s, 4H), 0.11 (s, 3H) ppm.

### (2R,3S)-3-methylpent-4-en-2-yl (S)-1-((S)-2-(3-(((R)-4-(allyloxy)butan-2-yl)oxy)-4,5-dimethoxyphenyl)-2-cyclohexylacetyl)piperidine-2-carboxylate (m4(9,17))

General procedure C was applied to phenol **m3(9)** with **B17** (1.5 eq.) and cesium carbonate, in DMF. **Yield:** 34 mg, 92 %, colorless resin. **¹H NMR** (500 MHz, CDCl₃, mixture of rotamers, major rotamer reported): δ 6.55 (d, J = 1.7 Hz, 1H), 6.48 (d, J = 1.9 Hz, 1H), 5.96 - 5.80 (m, 1H), 5.42 (ddd, J = 17.8, 10.4, 7.8 Hz, 1H), 5.36 - 5.31 (m, 1H), 5.29 - 5.19 (m, 1H), 5.18 - 4.84 (m, 3H), 4.79 - 4.71 (m, 1H), 4.57 - 4.46 (m, 1H), 4.04 - 3.90 (m, 3H), 3.81 (s, 3H), 3.79 (s, 3H), 3.66 - 3.53 (m, 2H), 3.32 (d, J = 10.0 Hz, 1H), 2.96 (td, J = 13.2, 2.8 Hz, 1H), 2.30 - 2.18 (m, 1H), 2.19 - 1.79 (m, 5H), 1.73 - 1.40 (m, 7H), 1.39 - 0.93 (m, 11H), 0.91 - 0.61 (m, 5H) ppm. **MS** (m/z): (ESI⁺) calculated for: C₃₅H₅₄NO₇[M+H]⁺: 600.4, found: 600.4.

### (2R,3S)-3-methylpent-4-en-2-yl (S)-1-((S)-2-(3-(((S)-4-(allyloxy)butan-2-yl)oxy)-4,5-dimethoxyphenyl)-2-cyclohexylacetyl)piperidine-2-carboxylate (m4(9,18))

General procedure C was applied to phenol **m3(9)** with **B18** (1.5 eq.) and cesium carbonate in DMF (3 mL). **Yield:** 36 mg, 98 %, colorless resin. **¹H NMR** (500 MHz, CDCl₃, mixture of rotamers, major rotamer reported): δ 6.52 (s, 1H), 6.51 (s, 1H), 5.97 - 5.83 (m, 1H), 5.43 (ddd, J = 17.7, 10.3, 7.9 Hz, 1H), 5.37 - 5.31 (m, 1H), 5.29 - 5.22 (m, 1H), 5.18 - 5.10 (m, 1H), 4.94 - 4.88 (m, 1H), 4.87 - 4.80 (m, 1H), 4.76 (p, J = 6.2 Hz, 1H), 4.57 - 4.48 (m, 1H), 4.02 - 3.90 (m, 3H), 3.86 - 3.73 (m, 6H), 3.65 - 3.54 (m, 2H), 3.31 (d, J = 9.8 Hz, 1H), 3.00 - 2.90 (m, 1H), 2.23 (d, J = 13.8 Hz, 1H), 2.14 - 1.98 (m, 3H), 1.90-1.81 (m, 2H), 1.78 - 1.49 (m, 5H), 1.50 - 1.37 (m, 1H), 1.37 - 0.99 (m, 9H), 0.98 (d, J = 6.4 Hz, 3H), 0.92 - 0.62 (m, 5H) ppm. **MS** (m/z): (ESI⁺) calculated for: C₃₅H₅₄NO₇[M+H]⁺: 600.4, found: 600.4.

### (2R,3R)-3-methylpent-4-en-2-yl (S)-1-((S)-2-(3-(2-(allyloxy)ethoxy)-4,5-dimethoxyphenyl)-2-cyclohexylacetyl)piperidine-2-carboxylate (m4(10,3))

General procedure C was applied to phenol **m3(10)** with **B3** (2.0 eq.) and cesium carbonate in DMF. **Yield:** 39 mg, 83 %, colorless resin. **¹H NMR** (500 MHz, CDCl₃, mixture of rotamers, major rotamer reported): δ 6.52 (d, J = 1.9 Hz, 1H), 6.50 (d, J = 1.9 Hz, 1H), 5.98 - 5.86 (m, 1H), 5.57 - 5.48 (m, 1H), 5.35 - 5.33 (m, 1H), 5.32 - 5.27 (m, 1H), 5.21 - 5.16 (m, 1H), 4.97 - 4.91 (m, 2H), 4.18 - 4.13 (m, 2H), 4.10 - 4.06 (m, 2H), 3.98 - 3.91 (m, 1H), 3.82 (s, 6H), 3.81 - 3.78 (m, 2H), 3.33 (d, J = 9.8 Hz, 1H), 2.94 (td, J = 13.2, 2.7 Hz, 1H), 2.28-2.19 (m, 1H), 2.15 - 1.99 (m, 2H), 1.86 (d, J = 12.9 Hz, 1H), 1.73 - 1.51 (m, 6H), 1.48 - 1.37 (m, 1H), 1.36 -1.01 (m, 6H), 0.99 (d, J = 6.3 Hz, 3H), 0.95 - 0.79 (m, 1H), 0.76 (d, J = 6.8 Hz, 3H), 0.74 - 0.62 (m, 1H) ppm. **MS** (m/z): (ESI⁺) calculated for: C₃₃H₅₀NO₇[M+H]⁺: 572.4, found: 572.2.

### (2R,3R)-3-methylpent-4-en-2-yl (S)-1-((S)-2-(3-(3-(allyloxy)propoxy)-4,5-dimethoxyphenyl)-2-cyclohexylacetyl)piperidine-2-carboxylate (m4(10,4))

General procedure C was applied to phenol **m3(10)** with **B4** (2.0 eq.) and cesium carbonate in DMF. **Yield:** 42 mg, 88 %, colorless resin. **¹H NMR** (500 MHz, CDCl₃, mixture of rotamers, major rotamer reported): δ 6.51 (d, J = 1.9 Hz, 1H), 6.49 (d, J = 1.9 Hz, 1H), 5.94 - 5.85 (m, 1H), 5.54 - 5.47 (m, 1H), 5.37 - 5.32 (m, 1H), 5.29 - 5.23 (m, 1H), 5.17 - 5.13 (m, 1H), 4.98 - 4.90 (m, 2H), 4.71 (p, J = 6.2 Hz, 1H), 4.10 - 4.05 (m, 2H), 4.00 - 3.93 (m, 3H), 3.81 (d, J = 1.5 Hz, 6H), 3.67 - 3.60 (m, 2H), 3.33 (d, J = 9.9 Hz, 1H), 2.94 (td, J = 13.3, 2.8 Hz, 1H), 2.24 (dt, J = 14.0, 2.6 Hz, 1H), 2.14 - 2.00 (m, 3H), 1.88 - 1.84 (m, 1H), 1.81 - 1.50 (m, 6H), 1.43 (qt, J = 12.5, 4.1 Hz, 1H), 1.31 (s, 3H), 1.13 (tdd, J = 24.2, 13.5, 7.5 Hz, 3H), 0.98 (d, J = 6.4 Hz, 3H), 0.95 - 0.76 (m, 1H), 0.75 (d, J = 6.9 Hz, 3H), 0.71 - 0.62 (m, 1H) ppm. **MS** (m/z): (ESI⁺) calculated for: C₃₄H₅₂NO₇[M+H]+: 586.4, found: 586.2.

### (2R,3R)-3-methylpent-4-en-2-yl (S)-1-((S)-2-(3-((S)-2-(allyloxy)propoxy)-4,5-dimethoxyphenyl)-2-cyclohexylacetyl)piperidine-2-carboxylate (m4(10,5))

General procedure C was applied to phenol **m3(10)** with **B5** (2.0 eq.) and cesium carbonate in DMF. **Yield:** 5 mg, 94 %, colorless resin. **¹H NMR** (500 MHz, CDCl₃, mixture of rotamers, major rotamer reported): δ 6.49 (d, J = 1.9 Hz, 1H), 6.48 (d, J = 1.8 Hz, 1H), 5.93 (dddd, J = 16.1, 10.8, 6.7, 4.6 Hz, 1H), 5.57 - 5.46 (m, 1H), 5.36 - 5.25 (m, 2H), 5.17 - 5.03 (m, 1H), 4.97 - 4.90 (m, 2H), 4.74 - 4.66 (m, 1H), 4.16 - 4.12 (m, 3H), 4.02 - 3.85 (m, 3H), 3.81 (s, 3H), 3.80 (d, J = 1.3 Hz, 3H), 3.32 (d, J = 9.9 Hz, 1H), 2.93 (td, J = 13.3, 2.8 Hz, 1H), 2.28 - 2.20 (m, 1H), 2.16 - 2.00 (m, 3H), 1.86 (d, J = 11.9 Hz, 1H), 1.70 - 1.50 (m, 5H), 1.50 - 1.37 (m, 1H), 1.36 - 1.27 (m, 1H), 1.27 - 1.26 (m, 3H), 1.25 - 1.02 (m, 3H), 0.99 - 0.97 (m, 3H), 0.93 - 0.78 (m, 2H), 0.77 - 0.74 (m, 3H), 0.73 - 0.62 (m, 1H) ppm. **MS** (m/z): (ESI⁺) calculated for: C₃₄H₅₂NO₇[M+H]+: 586.4, found: 586.2.

### (2R,3R)-3-methylpent-4-en-2-yl (S)-1-((S)-2-(3-((R)-2-(allyloxy)propoxy)-4,5-dimethoxyphenyl)-2-cyclohexylacetyl)piperidine-2-carboxylate (m4(10,6))

General procedure C was applied to phenol **m3(10)** with **B6** (2.0 eq.) and cesium carbonate in DMF. **Yield:** 166 mg, 77 %, colorless resin. **¹H NMR** (500 MHz, CDCl₃, mixture of rotamers, major rotamer reported): δ 6.55 - 6.41 (m, 2H), 6.03 - 5.86 (m, 1H), 5.60 - 5.44 (m, 1H), 5.39 - 5.24 (m, 2H), 5.18 - 5.04 (m, 2H), 4.99-4.90 (m, 2H), 4.76 - 4.67 (m, 1H), 4.14 (dt, J = 5.6, 1.5 Hz, 2H), 4.11 - 3.84 (m, 4H), 3.86 - 3.78 (m, 7H), 3.34 (d, J = 9.9 Hz, 1H), 2.95 (t, J = 12.6 Hz, 1H), 2.32 - 1.81 (m, 4H), 1.77 - 1.50 (m, 6H), 1.48 - 0.96 (m, 11H), 0.95 - 0.63 (m, 5H) ppm. **MS** (m/z): (ESI⁺) calculated for: C₃₄H₅₂NO₇[M+H]+: 586.4, found: 586.4.

### (2R,3R)-3-methylpent-4-en-2-yl (S)-1-((S)-2-(3-((R)-2-(allyloxy)-3-((tertbutyldimethylsilyl)oxy)propoxy)-4,5-dimethoxyphenyl)-2-cyclohexylacetyl)piperidine-2-carboxylate (m4(10,7))

General procedure C was applied to phenol **m3(10)** with **B7** (2.0 eq.) and cesium carbonate in DMF. **Yield:** 65 mg, 87 %, colorless resin. **¹H NMR** (500 MHz, CDCl₃, mixture of rotamers, major rotamer reported): δ 6.53 (d, J = 1.8 Hz, 1H), 6.46 (d, J = 1.8 Hz, 1H), 5.97 - 5.86 (m, 1H), 5.57 - 5.46 (m, 1H), 5.35 - 5.24 (m, 2H), 5.18 - 5.05 (m, 2H), 4.97 - 4.91 (m, 2H), 4.74 - 4.67 (m, 1H), 4.25 - 4.15 (m, 2H), 4.08 (dd, J = 9.7, 4.1 Hz, 2H), 4.03 - 3.98 (m, 1H), 3.97 - 3.93 (m, 1H), 3.84 - 3.75 (m, 9H), 3.32 (d, J = 9.9 Hz, 1H), 2.93 (td, J = 13.0, 2.6 Hz, 1H), 2.27 - 2.20 (m, 1H), 2.14 - 1.98 (m, 1H), 1.88 - 1.83 (m, 1H), 1.82 - 1.73 (m, 1H), 1.71 - 1.51 (m, 5H), 1.49 - 1.36 (m, 1H), 1.37 - 1.17 (m, 4H), 1.11 (dt, J = 12.7, 6.2 Hz, 2H), 0.98 (d, J = 6.3 Hz, 3H), 0.88 (d, J = 2.8 Hz, 9H), 0.84 - 0.60 (m, 5H), 0.05 (s, 6H) ppm. **HR-MS** (m/z): (ESI⁺) calculated for: C₄₁H₆₈NO₈Si[M+H]⁺: 716.4479, found: 716.4552.

### (2R,3R)-3-methylpent-4-en-2-yl (S)-1-((S)-2-(3-((S)-2-(allyloxy)-3-hydroxypropoxy)-4,5-dimethoxyphenyl)-2-cyclohexylacetyl)piperidine-2-carboxylate (m4(10,7)-OH)

General procedure C was applied to phenol **m3(10)** with **B7** (1.5 eq.) and cesium carbonate in DMF. Then general procedure H was applied without prior **purification.Yield:** 46 mg, 58 % over 2 steps, colorless resin. **¹H NMR** (500 MHz, CDCl₃, mixture of rotamers, major rotamer reported): δ 6.51 (s, 1H), 6.47 (s, 1H), 6.02 - 5.88 (m, 1H), 5.58 - 5.47 (m, 1H), 5.38 - 5.28 (m, 2H), 5.24 - 5.17 (m, 1H), 5.02 - 4.90 (m, 2H), 4.75 - 4.67 (m, 1H), 4.29 - 4.22 (m, 1H), 4.20 - 4.02 (m, 3H), 3.95 (d, *J* = 13.9 Hz, 1H), 3.84 (q, *J* = 6.8, 4.4 Hz, 5H), 3.80 (s, 2H), 3.79 - 3.72 (m, 1H), 3.34 (d, *J =* 9.8 Hz, 1H), 2.94 (t, *J* = 13.7 Hz, 1H), 2.29 - 2.22 (m, 1H), 2.18 - 1.97 (m, 2H), 1.86 (d, *J* = 12.6 Hz, 1H), 1.73 - 1.52 (m, 6H), 1.50 - 1.38 (m, 1H), 1.37 - 1.04 (m, 7H), 1.00 (d, *J* = 6.3 Hz, 3H), 0.93 - 0.81 (m, 1H), 0.77 (d, *J* = 6.9 Hz, 3H), 0.75 - 0.62 (m, 1H) ppm. **HR-MS** (m/z): (ESI⁺) calculated for: C₃₄H₅₂NO₈ [M+H]+: 602.36874, found: 602.36840.

### (2R,3R)-3-methylpent-4-en-2-yl (S)-1-((S)-2-(3-((S)-2-(allyloxy)-3-((tertbutyldimethylsilyl)oxy)propoxy)-4,5-dimethoxyphenyl)-2-cyclohexylacetyl)piperidine-2-carboxylate (m4(10,8))

General procedure C was applied to phenol **m3(10)** with **B8** (2.0 eq.) and cesium carbonate in DMF. **Yield:** 60 mg, 80 %, colorless resin. **¹H NMR** (500 MHz, CDCl₃, mixture of rotamers, major rotamer reported): δ 6.52 (d, J = 1.8 Hz, 1H), 6.46 (d, J = 1.8 Hz, 1H), 5.98 - 5.86 (m, 1H), 5.55 - 5.46 (m, 1H), 5.35 - 5.33 (m, 1H), 5.31 - 5.25 (m, 1H), 5.19 - 5.06 (m, 2H), 4.97 - 4.90 (m, 2H), 4.74 - 4.66 (m, 1H), 4.11 - 4.06 (m, 1H), 3.99 (dt, J = 10.5, 5.2 Hz, 1H), 3.94 (d, J = 14.5 Hz, 1H), 3.84 - 3.71 (m, 8H), 3.32 (d, J = 9.8 Hz, 1H), 2.96 - 2.87 (m, 1H), 2.29 - 2.20 (m, 1H), 2.15 - 1.98 (m, 2H), 1.86 (d, J = 12.6 Hz, 1H), 1.71 - 1.50 (m, 5H), 1.48 - 1.38 (m, 1H), 1.37 - 1.01 (m, 5H), 0.98 (d, J = 6.3 Hz, 3H), 0.89 - 0.86 (m, 10H), 0.75 (d, J = 6.8 Hz, 3H), 0.72 - 0.62 (m, 1H), 0.06 (d, J = 1.6 Hz, 9H) ppm. **HR-MS** (m/z): (ESI⁺) calculated for: C₄₁H₆₈NO₈Si[M+H]⁺: 716.4479, found: 716.4557.

### (2R,3R)-3-methylpent-4-en-2-yl (S)-1-((S)-2-(3-(((S)-1-(allyloxy)propan-2-yl)oxy)-4,5-dimethoxyphenyl)-2-cyclohexylacetyl)piperidine-2-carboxylate (m4(10,9))

General procedure C was applied to phenol **m3(10)** with **B9** (2.0 eq.) and cesium carbonate in DMF. **Yield:** 33 mg, 92 %, colorless resin. **¹H NMR** (500 MHz, CDCl₃, mixture of rotamers, major rotamer reported): δ 6.55 (s, 1H), 6.50 (s, 1H), 5.95 - 5.84 (m, 1H), 5.57 - 5.48 (m, 1H), 5.35 - 5.32 (m, 1H), 5.31 -5.24 (m, 1H), 5.19 - 5.14 (m, 1H), 4.98-4.91 (m, 2H), 4.75-4.69 (m, 1H), 4.55 - 4.47 (m, 1H), 4.04 (d, J = 5.5 Hz, 2H), 3.98 - 3.92 (m, 1H), 3.84 - 3.79 (m, 6H), 3.69 - 3.62 (m, 1H), 3.53 (dd, J = 10.2, 4.9 Hz, 1H), 3.32 (d, J = 9.9 Hz, 1H), 3.00 - 2.90 (m, 1H), 2.24 (d, J = 13.8 Hz, 1H), 2.17 - 1.98 (m, 2H), 1.86 (d, J = 12.7 Hz, 1H), 1.78 - 1.51 (m, 6H), 1.50 - 1.37 (m, 1H), 1.36 - 0.96 (m, 11H), 0.93 - 0.79 (m, 1H), 0.78 - 0.64 (m, 4H) ppm. **MS** (m/z): (ESI⁺) calculated for: C₃₄H₅₂NO₇[M+H]+: 586.4, found: 586.4.

### (2R,3R)-3-methylpent-4-en-2-yl (S)-1-((S)-2-(3-(((R)-1-(allyloxy)propan-2-yl)oxy)-4,5-dimethoxyphenyl)-2-cyclohexylacetyl)piperidine-2-carboxylate (m4(10,10))

General procedure C was applied to phenol **m3(10)** with **B10** (2.0 eq.) and cesium carbonate in DMF. **Yield:** 43 mg, 90 %, colorless resin. **¹H NMR** (500 MHz, CDCl₃, mixture of rotamers, major rotamer reported): δ 6.52 (s, 1H), 5.98 - 5.84 (m, 1H), 5.57 - 5.48 (m, 1H), 5.33 (d, J = 5.7 Hz, 1H), 5.30 - 5.23 (m, 1H), 5.19 - 5.13 (m, 1H), 4.70 (dq, J = 10.9, 5.6, 5.0 Hz, 1H), 4.52 (p, J = 5.6 Hz, 1H), 4.07 - 4.02 (m, 2H), 3.94 (d, J = 13.8 Hz, 1H), 3.86 - 3.77 (m, 6H), 3.63 (dd, J = 10.3, 5.4 Hz, 1H), 3.52 (dd, J = 10.2, 4.6 Hz, 1H), 3.31 (d, J = 9.8 Hz, 1H), 2.93 (td, J = 13.5, 2.7 Hz, 1H), 2.23 (d, J = 13.8 Hz, 1H), 2.16 - 2.00 (m, 2H), 1.86 (d, J = 12.6 Hz, 1H), 1.81 - 1.49 (m, 6H), 1.42 (dtd, J = 17.1, 13.0, 6.6 Hz, 1H), 1.34 - 0.95 (m, 14H), 0.94 - 0.60 (m, 5H) ppm. **MS** (m/z): (ESI⁺) calculated for: C₃₄H₅₂NO₇[M+H]+: 586.4, found: 586.4.

### (2R,3R)-3-methylpent-4-en-2-yl (S)-1-((S)-2-(3-(((R)-1-(allyloxy)propan-2-yl)oxy)-4,5-dimethoxyphenyl)-2-cyclohexylacetyl)piperidine-2-carboxylate (m4(10,11))

General procedure C was applied to phenol **m3(10)** with **B11** (2.0 eq.) and cesium carbonate in DMF. **Yield:** 41 mg, 84 %, colorless resin. **¹H NMR** (500 MHz, CDCl₃, mixture of rotamers, major rotamer reported): δ 6.50 (d, J = 1.9 Hz, 1H), 6.48 (d, J = 1.9 Hz, 1H), 6.02 - 5.86 (m, 1H), 5.56 - 5.43 (m, 1H), 5.36 - 5.31 (m, 1H), 5.25 (dt, J = 17.3, 1.8 Hz, 1H), 5.14 (dd, J = 10.4, 1.7 Hz, 1H), 4.97 - 4.89 (m, 2H), 4.76 - 4.66 (m, 1H), 4.02 - 3.91 (m, 4H), 3.81 (s, 3H), 3.80 (s, 3H), 3.57 - 3.49 (m, 1H), 3.47 - 3.39 (m, 1H), 3.33 (d, J = 9.9 Hz, 1H), 2.94 (td, J = 13.3, 2.8 Hz, 1H), 2.32 - 2.21 (m, 2H), 2.15 - 2.00 (m, 2H), 1.86 (d, J = 12.0 Hz, 1H), 1.72 - 1.50 (m, 6H), 1.48 - 1.38 (m, 1H), 1.36 - 0.99 (m, 9H), 0.97 (d, J = 6.4 Hz, 3H), 0.90 - 0.82 (m, 1H), 0.74 (d, J = 6.9 Hz, 3H), 0.72 - 0.62 (m, 1H) ppm. **MS** (m/z): (ESI⁺) calculated for: C₃₅H₅₄NO₇[M+H]⁺: 600.4, found: 600.4.

### (2R,3R)-3-methylpent-4-en-2-yl (S)-1-((S)-2-(3-(((S)-1-(allyloxy)propan-2-l)oxy)-4,5-dimethoxyphenyl)-2-cyclohexylacetyl)piperidine-2-carboxylate (m4(10,12))

General procedure C was applied to phenol **m3(10)** with **B12** (2.0 eq.) and cesium carbonate in DMF. **Yield:** 40 mg, 82 %, colorless resin. **¹H NMR** (500 MHz, CDCl₃, mixture of rotamers, major rotamer reported): δ 6.50 (d, J = 1.7 Hz, 1H), 6.49 (d, J = 1.9 Hz, 1H), 5.95 - 5.85 (m, 1H), 5.55 - 5.47 (m, 1H), 5.35 (d, J = 5.8 Hz, 1H), 5.26 (dt, J = 17.3, 1.9 Hz, 2H), 4.99 - 4.91 (m, 2H), 4.75 - 4.69 (m, 1H), 4.00 - 3.93 (m, 4H), 3.86 - 3.77 (m, 8H), 3.54 - 3.49 (m, 1H), 3.47 - 3.42 (m, 1H), 3.33 (d, J = 9.9 Hz, 1H), 2.94 (td, J = 13.3, 2.8 Hz, 1H), 2.26 (td, J = 12.3, 11.5, 5.3 Hz, 2H), 2.17 - 2.00 (m, 2H), 1.87 (d, J = 12.6 Hz, 1H), 1.73 - 1.50 (m, 6H), 1.49 - 1.38 (m, 1H), 1.38 - 1.11 (m, 6H), 1.11 - 1.00 (m, 5H), 0.98 (d, J = 6.5 Hz, 3H), 0.93 - 0.78 (m, 1H), 0.75 (d, J = 6.8 Hz, 3H), 0.72 - 0.65 (m, 1H) ppm. **MS** (m/z): (ESI⁺) calculated for: C₃₅H₅₄NO₇[M+H]⁺: 600.4, found: 600.4.

### (2R,3R)-3-methylpent-4-en-2-yl (S)-1-((S)-2-(3-(((S)-1-(allyloxy)propan-2-l)oxy)-4,5-dimethoxyphenyl)-2-cyclohexylacetyl)piperidine-2-carboxylate (m4(10,13))

General procedure C was applied to phenol **m3(10)** with **B13** (2.0 eq.) and cesium carbonate in DMF. **Yield:** 35 mg, 81 %, colorless resin. **¹H NMR** (500 MHz, CDCl₃, mixture of rotamers, major rotamer reported): δ 6.52 (d, J = 1.8 Hz, 1H), 6.49 (d, J = 1.9 Hz, 1H), 5.98 - 5.85 (m, 1H), 5.58 - 5.46 (m, 1H), 5.41 - 5.33 (m, 1H), 5.25 (dtd, J = 17.2, 4.9, 4.2, 1.8 Hz, 2H), 5.15-5.07 (m, 2H), 4.98 - 4.91 (m, 2H), 4.75 - 4.68 (m, 2H), 4.15 - 4.02 (m, 3H), 4.00 - 3.89 (m, 2H), 3.82 (s, 3H), 3.80 (s, 3H), 3.77 - 3.71 (m, 1H), 3.34 (d, J = 9.9 Hz, 1H), 2.99 - 2.91 (m, 1H), 2.24 (dd, J = 13.9, 3.2 Hz, 1H), 2.16 - 1.83 (m, 5H), 1.74 - 1.53 (m, 7H), 1.44 (qt, J = 12.7, 4.2 Hz, 1H), 1.37 - 1.01 (m, 3H), 0.98 (d, J = 6.4 Hz, 3H), 0.96 - 0.77 (m, 2H), 0.78 - 0.65 (m, 4H) ppm. **MS** (m/z): (ESI⁺) calculated for: C₃₅H₅₄NO₇[M+H]⁺: 600.4, found: 600.4.

### (2R,3R)-3-methylpent-4-en-2-yl (S)-1-((S)-2-(3-(((R)-1-(allyloxy)propan-2-l)oxy)-4,5-dimethoxyphenyl)-2-cyclohexylacetyl)piperidine-2-carboxylate (m4(10,14))

General procedure C was applied to phenol **m3(10)** with **B14** (2.0 eq.) and cesium carbonate in DMF. **Yield:** 37 mg, 86 %, colorless resin. **¹H NMR** (500 MHz, CDCl₃, mixture of rotamers, major rotamer reported): δ 6.50 (d, J = 1.8 Hz, 1H), 6.48 (d, J = 1.9 Hz, 1H), 6.00 - 5.82 (m, 1H), 5.56 - 5.46 (m, 1H), 5.39 - 5.31 (m, 1H), 5.29 - 5.20 (m, 1H), 5.15 - 5.06 (m, 2H), 4.98 - 4.90 (m, 2H), 4.71 (t, J = 6.3 Hz, 1H), 4.17 - 4.01 (m, 3H), 4.00 - 3.87 (m, 2H), 3.82 (s, 3H), 3.79 (s, 3H), 3.75 (tq, J = 7.4, 2.0 Hz, 1H), 3.33 (d, J = 9.9 Hz, 1H), 2.93 (td, J = 13.3, 2.8 Hz, 1H), 2.28 - 2.21 (m, 1H), 2.16 - 2.02 (m, 2H), 2.02 - 1.88 (m, 2H), 1.88 - 1.83 (m, 1H), 1.71 - 1.51 (m, 5H), 1.51 - 1.37 (m, 1H), 1.37 - 1.07 (m, 7H), 0.98 (d, J = 6.3 Hz, 3H), 0.91 - 0.79 (m, 2H), 0.75 (d, J = 6.8 Hz, 3H), 0.72 - 0.63 (m, 1H) ppm. **MS** (m/z): (ESI⁺) calculated for: C₃₅H₅₄NO₇[M+H]⁺: 600.4, found: 600.4.

### (2R,3R)-3-methylpent-4-en-2-yl (S)-1-((S)-2-(3-((R)-3-(allyloxy)-2-((tertbutyldimethylsilyl)oxy)propoxy)-4,5-dimethoxyphenyl)-2-cyclohexylacetyl)piperidine-2-carboxylate (m4(10,15))

General procedure C was applied to phenol **m3(10)** with **B15** (2.0 eq.) and cesium carbonate in DMF. **Yield:** 23 mg, 39 %, colorless resin. **¹H NMR** (500 MHz, CDCl₃, mixture of rotamers, major rotamer reported): δ 6.54 (d, J = 1.8 Hz, 1H), 6.46 (d, J = 1.9 Hz, 1H), 5.94 - 5.86 (m, 1H), 5.56 - 5.47 (m, 1H), 5.35 (d, J = 5.7 Hz, 1H), 5.29 - 5.24 (m, 1H), 5.19 - 5.14 (m, 1H), 4.97 - 4.90 (m, 2H), 4.75 - 4.68 (m, 1H), 4.21 - 4.16 (m, 1H), 4.04 - 3.89 (m, 5H), 3.82 (d, J = 2.1 Hz, 3H), 3.79 (s, 3H), 3.59 (ddd, J = 10.1, 5.3, 2.0 Hz, 1H), 3.52 (ddd, J = 10.0, 5.4, 1.9 Hz, 1H), 3.33 (d, J = 9.9 Hz, 1H), 2.94 (td, J = 13.3, 2.8 Hz, 1H), 2.25 (d, J = 13.7 Hz, 1H), 2.14 - 2.03 (m, 2H), 1.86 (d, J = 12.5 Hz, 1H), 1.79 - 1.52 (m, 6H), 1.49 - 1.39 (m, 1H), 1.37 - 1.01 (m, 4H), 0.98 (d, J = 6.3 Hz, 3H), 0.89 (d, J = 2.2 Hz, 9H), 0.86 - 0.79 (m, 2H), 0.74 (d, J = 6.9 Hz, 3H), 0.71 - 0.64 (m, 1H), 0.13-0.10 (m, 6H) ppm.

### (2R,3R)-3-methylpent-4-en-2-yl (S)-1-((S)-2-(3-((S)-3-(allyloxy)-2-((tert-butyldimethylsilyl)oxy)propoxy)-4,5-dimethoxyphenyl)-2-cyclohexylacetyl)piperidine-2-carboxylate (m4(10,16))

General procedure C was applied to phenol **m3(10)** with **B16** (2.0 eq.) and cesium carbonate in DMF. **Yield:** 45 mg, 76 %, colorless resin. **¹H NMR** (500 MHz, CDCl₃, mixture of rotamers, major rotamer reported): δ 6.53 (d, J = 1.8 Hz, 1H), 6.45 (d, J = 1.9 Hz, 1H), 5.89 (ddt, J = 16.3, 10.7, 5.5 Hz, 1H), 5.59 - 5.46 (m, 1H), 5.34 (d, J = 5.0 Hz, 1H), 5.31 - 5.24 (m, 1H), 5.21 - 5.12 (m, 1H), 4.99 - 4.90 (m, 2H), 4.80 - 4.65 (m, 1H), 4.19 (p, J = 5.4 Hz, 1H), 4.06 - 3.95 (m, 3H), 3.92 (dt, J = 12.8, 5.5 Hz, 2H), 3.81 (s, 3H), 3.78 (s, 3H), 3.66 - 3.55 (m, 1H), 3.56 - 3.47 (m, 1H), 3.33 (d, J = 9.9 Hz, 1H), 2.94 (td, J = 13.3, 2.7 Hz, 1H), 2.24 (d, J = 13.8 Hz, 1H), 2.13 - 2.00 (m, 2H), 1.88 - 1.82 (m, 1H), 1.75 - 1.54 (m, 6H), 1.52 - 1.36 (m, 1H), 1.36 - 1.00 (m, 5H), 0.97 (d, J = 6.4 Hz, 3H), 0.89 (s, 9H), 0.84 - 0.66 (m, 5H), 0.12 (s, 3H), 0.11 (s, 3H) ppm.

### (2R,3R)-3-methylpent-4-en-2-yl (S)-1-((S)-2-(3-(((R)-4-(allyloxy)butan-2-yl)oxy)-4,5-dimethoxyphenyl)-2-cyclohexylacetyl)piperidine-2-carboxylate (m4(10,17))

General procedure C was applied to phenol **m3(10)** with **B17** (1.5 eq.) and cesium carbonate in DMF. **Yield:** 30 mg, 81 %, colorless resin. **¹H NMR** (500 MHz, CDCl₃, mixture of rotamers, major rotamer reported): δ 6.55 (s, 1H), 6.47 (s, 1H), 5.94 - 5.82 (m, 1H), 5.52 (ddd, J = 17.6, 9.9, 7.4 Hz, 1H), 5.35 - 5.32 (m, 1H), 5.17 - 5.06 (m, 2H), 4.99 - 4.90 (m, 2H), 4.70 (q, J = 6.4 Hz, 1H), 4.56 - 4.49 (m, 1H), 3.96 (d, J = 5.7 Hz, 3H), 3.81 (s, 3H), 3.79 (s, 3H), 3.65 - 3.55 (m, 2H), 3.32 (d, J = 9.9 Hz, 1H), 2.99 - 2.90 (m, 1H), 2.24 (d, J = 13.7 Hz, 1H), 2.15 - 2.03 (m, 2H), 1.98 - 1.80 (m, 2H), 1.74 - 1.49 (m, 6H), 1.43 (dddd, J = 16.4, 12.6, 8.3, 2.5 Hz, 1H), 1.37 - 0.94 (m, 12H), 0.94 - 0.77 (m, 1H), 0.76 - 0.63 (m, 4H) ppm. **HR-MS** (m/z): (ESI⁺) calculated for: C₃₅H₅₄NO₇[M+H]⁺: 600.3895, found: 600.3900.

### (2R,3R)-3-methylpent-4-en-2-yl (S)-1-((S)-2-(3-(((S)-4-(allyloxy)butan-2-yl)oxy)-4,5-dimethoxyphenyl)-2-cyclohexylacetyl)piperidine-2-carboxylate (m4(10,18))

General procedure C was applied to phenol **m3(10)** with **B18** (1.5 eq.) and cesium carbonate in DMF. **Yield:** 36 mg, 98 %, colorless resin. **¹H NMR** (500 MHz, CDCl₃, mixture of rotamers, major rotamer reported): δ 6.52 (s, 1H), 6.51 (s, 1H), 5.97 - 5.83 (m, 1H), 5.43 (ddd, J = 17.7, 10.3, 7.9 Hz, 1H), 5.37 - 5.31 (m, 1H), 5.29 - 5.22 (m, 1H), 5.18 - 5.10 (m, 1H), 4.94 - 4.88 (m, 1H), 4.87 - 4.80 (m, 1H), 4.76 (p, J = 6.2 Hz, 1H), 4.57 - 4.48 (m, 1H), 4.02 - 3.90 (m, 3H), 3.86 - 3.73 (m, 6H), 3.65 - 3.54 (m, 2H), 3.31 (d, J = 9.8 Hz, 1H), 3.00 - 2.90 (m, 1H), 2.23 (d, J = 13.8 Hz, 1H), 2.14 - 1.98 (m, 3H), 1.90 - 1.81 (m, 2H), 1.78 - 1.49 (m, 5H), 1.50 - 1.37 (m, 1H), 1.37 - 0.99 (m, 9H), 0.98 (d, J = 6.4 Hz, 3H), 0.92 - 0.62 (m, 5H) ppm. **HR-MS** (m/z): (ESI⁺) calculated for: C₃₅H₅₄NO₇[M+H]⁺: 600.4, found: 600.4.

### (2R,3S)-3-(methoxymethoxy)pent-4-en-2-yl (S)-1-((S)-2-(3-(3-(allyloxy)propoxy)-4,5-dimethoxyphenyl)-2-cyclohexylacetyl)piperidine-2-carboxylate (m4(11,4))

General procedure C was applied to phenol **m3(11)** with **B4** (2.0 eq.) and cesium carbonate DMF (3 mL). **Yield:** 44 mg, 75 %, colorless resin.

¹H **NMR** (500 MHz, CDCl₃, mixture of rotamers, major rotamer reported): δ 6.52 (d, J = 1.8 Hz, 1H), 6.49 (d, J = 1.9 Hz, 1H), 5.97 - 5.85 (m, 1H), 5.42 - 5.06 (m, 5H), 4.97 (qd, J = 6.5, 4.2 Hz, 1H), 4.53 (d, J = 6.7 Hz, 1H), 4.46 (d, J = 6.6 Hz, 1H), 4.10 - 4.05 (m, 2H), 4.01 - 3.96 (m, 2H), 3.92 (d, J = 13.9 Hz, 1H), 3.84 - 3.79 (m, 7H), 3.67 - 3.61 (m, 2H), 3.34 - 3.31 (m, 1H), 3.29 (s, 2H), 3.09 - 3.02 (m, 1H), 2.29 - 2.22 (m, 1H), 2.13 - 2.02 (m, 3H), 1.85 (d, J = 12.3 Hz, 1H), 1.71 - 1.49 (m, 6H), 1.48 - 1.04 (m, 8H), 1.03 (d, J = 6.5 Hz, 3H), 0.97 - 0.62 (m, 2H) ppm. **HR-MS** (m/z): (ESI⁺) calculated for: C₃₅H₅₃NO₉[M+H]⁺: 632.4, found: 632.4.

### (2R,3S)-3-(methoxymethoxy)pent-4-en-2-yl (S)-1-((S)-2-(3-((S)-2-(allyloxy)propoxy)-4,5-dimethoxyphenyl)-2-cyclohexylacetyl)piperidine-2-carboxylate (m4(11,5))

General procedure C was applied to phenol **m3(11)** with **B5** (2.0 eq.) and cesium carbonate in DMF. **Yield:** 66 mg, 79 %, colorless resin. **¹H NMR** (500 MHz, CDCl₃, mixture of rotamers, major rotamer reported): δ 6.50 (d, J = 1.6 Hz, 2H), 6.49 (d, J = 1.9 Hz, 1H), 6.06 - 5.86 (m, 1H), 5.40 - 5.07 (m, 5H), 4.97 (qd, J = 6.5, 4.1 Hz, 1H), 4.60 - 4.54 (m, 1H), 4.53 (d, J = 6.7 Hz, 1H), 4.45 (d, J = 6.6 Hz, 1H), 4.15 - 4.13 (m, 2H), 4.07 - 3.85 (m, 4H), 3.84 - 3.78 (m, 7H), 3.32 (d, J = 6.6 Hz, 1H), 3.29 (s, 3H), 3.04 - 2.98 (m, 1H), 2.29 - 2.22 (m, 1H), 2.13 - 2.01 (m, 1H), 1.87 - 1.82 (m, 1H), 1.72 - 1.50 (m, 5H), 1.49 - 1.03 (m, 9H), 1.02 (d, J = 6.5 Hz, 3H), 0.94 - 0.81 (m, 1H), 0.78 - 0.62 (m, 1H) ppm. **HR-MS** (m/z): (ESI⁺) calculated for: C₃₅H₅₃NO₉[M+H]⁺: 632.4, found: 632.4.

### (2R,3S)-3-(methoxymethoxy)pent-4-en-2-yl (S)-1-((S)-2-(3-((R)-2-(allyloxy)propoxy)-4,5-dimethoxyphenyl)-2-cyclohexylacetyl)piperidine-2-carboxylate (m4(11,6))

General procedure C was applied to phenol **m3(11)** with **B6** (2.0 eq.) and cesium carbonate , in DMF. **Yield:** 62 mg, 75 %, colorless resin. **¹H NMR** (500 MHz, CDCl₃, mixture of rotamers, major rotamer reported): δ 6.51 (d, J = 1.8 Hz, 1H), 6.47 (d, J = 1.9 Hz, 1H), 5.95 - 5.85 (m, 1H), 5.40 - 5.04 (m, 5H), 4.95 (qd, J = 6.5, 4.3 Hz, 1H), 4.51 (d, J = 6.7 Hz, 1H), 4.44 (d, J = 6.7 Hz, 1H), 4.15 - 4.09 (m, 2H), 4.08 - 3.94 (m, 2H), 3.93 - 3.83 (m, 2H), 3.83 - 3.78 (m, 7H), 3.34 - 3.29 (m, 1H), 3.28 (s, 3H), 3.05 - 3.01 (m, 1H), 2.28 - 2.21 (m, 1H), 2.10 - 2.01 (m, 2H), 1.83 (d, J = 12.3 Hz, 1H), 1.71 - 1.50 (m, 6H), 1.48 - 0.97 (m, 14H), 0.94 - 0.60 (m, 2H) ppm. **HR-MS** (m/z): (ESI⁺) calculated for: C₃₅H₅₃NO₉[M+H]⁺: 632.4, found: 632.4.

### (2R,3S)-3-(methoxymethoxy)pent-4-en-2-yl (S)-1-((S)-2-(3-((S)-2-(allyloxy)-3-((tertbutyldimethylsilyl)oxy)propoxy)-4,5-dimethoxyphenyl)-2-cyclohexylacetyl)piperidine-2-carboxylate (m4(11,8))

General procedure C was applied to phenol **m3(11)** with **B8** (2.0 eq.) and cesium carbonate in DMF. **Yield:** 86 mg, 86 %, colorless resin. **¹H NMR** (500 MHz, CDCl₃, mixture of rotamers, major rotamer reported): δ 6.53 (d, J = 1.8 Hz, 1H), 6.46 (d, J = 1.8 Hz, 1H), 5.91 (dddd, J = 15.9, 10.2, 7.5, 3.7 Hz, 2H), 5.41 - 5.05 (m, 5H), 4.96 (tt, J = 6.4, 3.3 Hz, 1H), 4.52 (d, J = 6.7 Hz, 1H), 4.45 (d, J = 6.7 Hz, 1H), 4.24 - 4.14 (m, 2H), 4.12 - 3.93 (m, 3H), 3.93 - 3.87 (m, 1H), 3.86 - 3.73 (m, 9H), 3.31 (d, J = 9.8 Hz, 1H), 3.28 (s, 3H), 3.04 - 2.97 (m, 1H), 2.28 - 2.21 (m, 1H), 2.13 - 2.02 (m, 2H), 1.86 - 1.82 (m, 1H), 1.73 - 1.49 (m, 5H), 1.48 - 0.98 (m, 8H), 0.97 - 0.79 (m, 10H), 0.78 - 0.61 (m, 2H), 0.06 - 0.03 (m, 6H) ppm.

### Example 2f: Synthesis of Macrocycles

### Experimental p5 und m5

### Macrocycle p5(1,1)-(E)

General procedure D was applied to **p4(1,1). Yield:** 529 mg, 97 % as a beige solid.

**HR-MS** (m/z): (ESI⁺) calculated for: C₂₆H₃₆NO₆ [M+H]⁺: 458.25371, found: 458.25391; **¹H NMR** (500 MHz, CDCl₃): δ 6.62 (d, J = 1.8 Hz, 1H), 6.30 (d, J = 1.8 Hz, 1H), 5.72 (ddd, J = 15.4, 8.9, 6.7 Hz, 1H), 5.47 (d, J = 5.0 Hz, 1H), 5.07 (ddd, J = 15.0, 8.7, 5.9 Hz, 1H), 4.62 (dd, J = 10.7, 6.7 Hz, 1H), 4.46 (ddtd, J = 26.7, 16.9, 11.9, 10.7, 8.9 Hz, 1H), 4.34 (dd, J = 11.7, 5.9 Hz, 1H), 4.12 -4.03 (m, 2H), 3.85 (d, J = 1.5 Hz, 3H), 3.79 (d, J = 1.5 Hz, 3H), 3.41 (d, J = 10.4 Hz, 1H), 2.71 (td, J = 13.2, 2.7 Hz, 1H), 2.33 - 2.23 (m, 2H), 2.02 - 1.91 (m, 1H), 1.73 - 1.61 (m, 6H), 1.59-1.10 (m, 6H), 0.91 - 0.77 (m, 2H) ppm.

### Macrocycle p5(1,1)-H₂

General procedure F was applied to macrocycle p5(1,1)-(E), reaction time 18 h. Yield: 11 mg, 42 %) as a beige solid. HR-MS (m/z): (ESI⁺) calculated for: C₂₆H₃₈NO₆ [M+H]⁺: 460.26964, found: 460.26936. ¹H NMR (500 MHz, CDCl₃): δ 6.62 (d, J = 1.8 Hz, 1H), 6.30 (d, J = 1.8 Hz, 1H), 5.72 (ddd, J = 15.4, 8.9, 6.7 Hz, 1H), 5.47 (d, J = 5.0 Hz, 1H), 5.07 (ddd, J = 15.0, 8.7, 5.9 Hz, 1H), 4.62 (dd, J = 10.7, 6.7 Hz, 1H), 4.46 (ddtd, J = 26.7, 16.9, 11.9, 10.7, 8.9 Hz, 1H), 4.34 (dd, J = 11.7, 5.9 Hz, 1H), 4.12 -4.03 (m, 2H), 3.85 (d, J = 1.5 Hz, 3H), 3.79 (d, J = 1.5 Hz, 3H), 3.41 (d, J = 10.4 Hz, 1H), 2.71 (td, J = 13.2, 2.7 Hz, 1H), 2.33 - 2.23 (m, 2H), 2.02 - 1.91 (m, 1H), 1.73 - 1.61 (m, 6H), 1.59 - 1.10 (m, 6H), 0.91 - 0.77 (m, 2H) ppm.

### Macrocycle p5(1,1)-Diol-I and p5(1,1)-Diol-II

General procedure E was applied to macrocycle p5(1,1)-(E). Yield p5(1,1)-Diol-I (32 mg, 49 %) and p5(1,1)-Diol-II (10 mg, 16 %) as white solids. p5(1,1)-Diol-I: HR-MS (m/z): (ESI⁺) calculated for: C₂₆H₃₇NO₈ [M+H]⁺: 492.25919, found: 492.25915; ¹H NMR (500 MHz, CDCl₃): δ 6.72 (d, J = 1.8 Hz, 1H), 6.37 (d, J = 1.9 Hz, 1H), 5.64 - 5.17 (m, 1H), 4.55 (dd, J = 13.4, 1.1 Hz, 1H), 4.26 (dd, J = 11.5, 7.1 Hz, 1H), 4.19 (dd, J = 13.2, 4.4 Hz, 1H), 3.91 (s, 4H), 3.84 (s, 3H), 3.44 (d, J = 10.4 Hz, 1H), 3.36 (ddd, J = 9.3, 4.5, 1.1 Hz, 1H), 3.19 (dd, J = 11.6, 1.4 Hz, 1H), 2.87 (ddd, J = 8.7, 7.1, 1.4 Hz, 1H), 2.77 (td, J = 12.8, 3.0 Hz, 1H), 2.38 (s, 2H), 2.35-2.28 (m, 1H), 2.18 (ddd, J = 14.5, 4.9, 2.8 Hz, 1H), 2.02 - 1.95 (m, 1H), 1.75 - 1.61 (m, 5H), 1.60 - 1.54 (m, 1H), 1.47 - 1.09 (m, 6H), 0.94 - 0.80 (m, 2H) ppm. p5(1,1)-Diol-II: HR-MS (m/z): (ESI⁺) calculated for: C₂₆H₃₈NO₈ [M+H]⁺: 492.25919, found: 492.25913; ¹H NMR (500 MHz, CDCl₃): δ 6.69 (d, J = 1.8 Hz, 1H), 6.38 (d, J = 1.9 Hz, 1H), 5.42 (d, J = 5.0 Hz, 1H), 4.37 (dd, J = 12.3, 3.1 Hz, 1H), 4.20 (dd, J = 12.3, 5.0 Hz, 1H), 4.03 (dd, J = 11.5, 4.4 Hz, 2H), 3.89 (s, 3H), 3.87 (s, 3H), 3.66 (dd, J = 11.4, 4.1 Hz, 1H), 3.49 (d, J = 10.5 Hz, 1H), 3.29 - 3.20 (m, 2H), 2.73 (td, J = 12.9, 2.6 Hz, 1H), 2.39 (s, 1H), 2.21 (dt, J = 13.0, 2.2 Hz, 1H), 1.93 (d, J = 12.7 Hz, 1H), 1.76 - 1.64 (m, 6H), 1.58 - 1.50 (m, 1H), 1.48 - 1.23 (m, 6H), 1.24 - 1.11 (m, 1H), 0.95 - 0.83 (m, 2H) ppm.

### Macrocycle p5(1,2)-(E)

General procedure D was applied to p4(1,2). Yield: (277 mg, 64 %, white solid). HR-MS (m/z): (ESI⁺) calculated for: C₂₇H₃₈NO₆ [M+H]⁺: 472.26936, found: 472.26948.

### Macrocycle p5(1,2)-H₂

General procedure F was applied to macrocycle p5(1,2)-(E), reaction time 18 h. Yield: 18 mg, 72%, white solid. HR-MS (m/z): (ESI⁺) calculated for: C₂₇H₄₀NO₆ [M+H]⁺: 474.28501, found: 474.28497; ¹H NMR (500 MHz, CDCl₃): δ 6.67 (d, J = 1.8 Hz, 1H), 6.34 (d, J = 1.9 Hz, 1H), 5.49 (d, J = 5.3 Hz, 1H), 4.41-4.29 (m, 1H), 4.18-4.11 (m, 1H), 4.10-4.00 (m, 1H), 3.92 -3.84 (m, 5H), 3.83 (s, 3H), 3.36 (d, J = 10.4 Hz, 1H), 2.71 -2.60 (m, 1H), 2.36 -2.22 (m, 2H), 1.99 - 1.89 (m, 1H), 1.76 - 1.58 (m, 5H), 1.60 - 1.26 (m, 5H), 1.27 - 1.22 (m, 4H), 1.23 - 1.04 (m, 2H), 0.93 - 0.78 (m, 2H), 0.78 - 0.70 (m, 1H), 0.63 - 0.41 (m, 1H) ppm.

### Macrocycles p5(1,2)-Diol- and p5(1,2)-Diol-II

General procedure E was applied to macrocycle p5(1,1)-(E). Yield: p5(1,2)-Diol-I (32 mg, 43 %) and p5(1,2)-Diol-II (5 mg, 7 %) as white solids. p5(1,2)-Diol-I: HR-MS (m/z): (ESI⁺) calculated for: C₂₇H₄₀NO₈ [M+H]⁺: 506.27484, found: 506.27508; ¹H NMR (500 MHz, CDCl₃): 6.64 (d, J = 1.8 Hz, 1H), 6.38 (d, J = 1.9 Hz, 1H), 5.50 (d, J = 4.9 Hz, 1H), 4.51 (ddd, J = 12.5, 8.6, 1.1 Hz, 1H), 4.35 - 4.22 (m, 2H), 3.89 (s, 3H), 3.81 (s, 3H), 3.72 (dd, J = 11.4, 3.1 Hz, 1H), 3.44 - 3.33 (m, 1H), 3.26-3.17 (m, 1H), 2.65 (td, J = 13.1, 2.6 Hz, 1H), 2.53-2.48 (m, 1H), 2.39-2.29 (m, 1H), 2.20 (qt, J = 10.9, 3.3 Hz, 1H), 2.06 - 1.97 (m, 1H), 1.97 - 1.90 (m, 1H), 1.77 - 1.73 (m, 0H), 1.74 - 1.65 (m, 6H), 1.55 (tdd, J = 13.2, 5.5, 3.6 Hz, 1H), 1.48 - 1.32 (m, 5H), 1.28 - 1.09 (m, 2H), 0.98 - 0.76 (m, 2H) ppm. **p5(1,2)-Diol-II: HR-MS** (m/z): (ESI⁺) calculated for: C₂₇H₄₀NO₈ [M+H]⁺: 506.27484, found: 506.27473; ¹H NMR (500 MHz, CDCl₃): δ 6.74 (d, J = 1.9 Hz, 1H), 6.35 (d, J = 2.0 Hz, 1H), 5.45 (d, J = 5.6 Hz, 1H), 4.61 - 4.52 (m, 1H), 4.33 - 4.23 (m, 1H), 4.22 - 4.10 (m, 1H), 4.06 (dd, J = 11.2, 5.0 Hz, 1H), 3.88 (s, 3H), 3.85 (s, 3H), 3.70 (dd, J = 11.2, 8.0 Hz, 1H), 3.49 (d, J = 10.5 Hz, 1H), 3.04 - 2.98 (m, 1H), 2.94 - 2.85 (m, 1H), 2.76 - 2.70 (m, 1H), 2.33 (s, 3H), 2.00 - 1.90 (m, 1H), 1.90 - 1.80 (m, 2H), 1.78 - 1.64 (m, 6H), 1.63 - 1.09 (m, 7H), 0.95 - 0.77 (m, 2H) ppm.

### Macrocycle p5(1,3)-(E)

General procedure D was applied to **p4(1,3). Yield:** 101 mg, 83 %, white solid. **HR-MS** (m/z): (ESI⁺) calculated for: C₂₈H₄₀NO₇ [M+H]⁺: 502.27993, found: 502.28005. ¹H NMR (500 MHz, CDCl₃): δ 6.73 (d, J = 1.8 Hz, 1H), 6.27 (d, J = 1.9 Hz, 1H), 5.53 - 5.49 (m, 1H), 5.37 - 5.30 (m, 1H), 5.29 - 5.22 (m, 1H), 4.49 - 4.39 (m, 2H), 4.37 - 4.31 (m, 2H), 4.15 - 4.06 (m, 1H), 3.84 (s, 3H), 3.81 (s, 4H), 3.80 - 3.74 (m, 2H), 3.74 - 3.69 (m, 1H), 3.34 (d, J = 10.1 Hz, 1H), 2.88 - 2.76 (m, 1H), 2.37 - 2.29 (m, 1H), 2.16 - 2.06 (m, 1H), 1.93 - 1.83 (m, 1H), 1.77 - 1.60 (m, 5H), 1.59 - 1.51 (m, 1H), 1.50 - 1.42 (m, 1H), 1.44 - 1.27 (m, 2H), 1.27 - 1.22 (m, 1H), 1.20 - 1.07 (m, 2H), 0.89 - 0.80 (m, 1H), 0.76 - 0.66 (m, 1H) ppm.

### Macrocycle p5(1,3)-H₂

General procedure G was applied to macrocycle **p5(1,2)-(E)** with 1 h reaction time. **Yield:** 7 mg, 49 %, white solid. HR-MS (m/z): (ESI⁺) calculated for: C₂₈H₄₂NO₇ [M+H]⁺: 504.29558, found: 504.29595. ¹H NMR (500 MHz, CDCl₃): δ 6.72 (d, J = 2.0 Hz, 1H), 6.27 (d, J = 2.1 Hz, 1H), 5.47 (d, J = 5.4 Hz, 1H), 4.41 - 4.26 (m, 2H), 4.13 (d, J = 14.1 Hz, 1H), 3.94 - 3.85 (m, 2H), 3.84 (s, 3H), 3.83 (s, 3H), 3.79 - 3.71 (m, 2H), 3.36 (d, J = 10.2 Hz, 1H), 3.27 - 3.20 (m, 1H), 3.20 - 3.12 (m, 1H), 2.84 - 2.76 (m, 1H), 2.35 (d, J = 13.6 Hz, 1H), 2.17 - 2.02 (m, 1H), 1.85 (d, J = 12.7 Hz, 1H), 1.76 - 1.38 (m, 8H), 1.37 - 1.06 (m, 8H), 0.89 - 0.80 (m, 1H), 0.79 - 0.63 (m, 1H) ppm.

### Macrocycles p₅(1,3)-Diol-I

General procedure E was applied to macrocycle **p5(1,3)-(E). Yield:** (32 mg, 43 %, mixture of diastereomeres, d.r: 1:1.4) as a white solid. HR-MS (m/z): (ESI⁺) calculated for: C₂₈H₄₂NO₉ [M+H]⁺: 536.28541, found: 536.29751. ¹H NMR (500 MHz, CDCl₃, mixture of diastereomers, major diastereomer reported): 6.86 (d, J = 2.1 Hz, 1H), 6.32 (d, J = 1.9 Hz, 1H), 5.51 - 5.45 (m, 1H), 4.35 - 4.27 (m, 2H), 4.17 - 4.13 (m, 1H), 4.09 (dd, J = 11.4, 9.1 Hz, 1H), 3.86 (s, 6H), 3.81 (dd, J = 11.3, 3.0 Hz, 1H), 3.77 - 3.71 (m, 1H), 3.48 - 3.36 (m, 2H), 3.34 - 3.21 (m, 3H), 3.04 - 2.92 (m, 1H), 2.37 - 2.31 (m, 1H), 2.14 - 2.07 (m, 1H), 1.96 - 1.44 (m, 11H), 1.43 - 1.24 (m, 3H), 1.19 - 1.09 (m, 2H), 0.98 - 0.79 (m, 1H), 0.77 - 0.64 (m, 1H) ppm.

### Macrocycle p5(1,4)-(E)

General procedure D was applied to **p4(1,4). Yield:** 86 mg, 71 %, white solid. **HR-MS** (m/z): (ESI⁺) calculated for: C₂₉H₄₂NO₇ [M+H]⁺: 516.29558, found: 516.29559. ¹H NMR (500 MHz, CDCl₃): δ 6.66 (d, J = 1.7 Hz, 1H), 6.28 (d, J = 1.7 Hz, 1H), 5.59 - 5.52 (m, 1H), 5.50 (d, J = 5.3 Hz, 1H), 5.38 - 5.28 (m, 1H), 4.51 (dd, J = 13.4, 5.9 Hz, 1H), 4.40 (ddt, J = 20.3, 13.3, 5.6 Hz, 2H), 4.11 - 4.00 (m, 2H), 3.94 - 3.87 (m, 1H), 3.83 (d, J = 1.5 Hz, 3H), 3.82 - 3.80 (m, 1H), 3.79 (s, 3H), 3.58 (dt, J = 9.5, 5.2 Hz, 1H), 3.32 (d, J = 10.0 Hz, 1H), 2.80 - 2.69 (m, 1H), 2.32 (d, J = 13.7 Hz, 1H), 2.17 - 2.08 (m, 1H), 2.02 - 1.93 (m, 1H), 1.93 - 1.81 (m, 2H), 1.68 (td, J = 19.9, 19.0, 11.0 Hz, 5H), 1.57 - 1.51 (m, 1H), 1.48 - 1.38 (m, 2H), 1.38 - 1.30 (m, 3H), 1.24 - 1.07 (m, 2H), 0.92 - 0.80 (m, 1H), 0.80 - 0.68 (m, 1H) ppm.

### Macrocycle p5(1,4)-H₂

General procedure F was applied to macrocycle p5(1,2)-(E). Yield: 5 mg, 7 %, white solid. HR-MS (m/z): (ESI⁺) calculated for: C₂₉H₄₄NO₇ [M+H]⁺: 518.31123, found: 518.31174.

¹H NMR (500 MHz, CDCl₃): δ 6.65 (d, J = 1.7 Hz, 1H), 6.30 (t, J = 1.7 Hz, 1H), 5.44 (d, J = 5.5 Hz, 1H), 4.33 - 4.22 (m, 2H), 4.22 - 4.15 (m, 1H), 4.03 - 3.95 (m, 2H), 3.84 (s, 3H), 3.83 (s, 4H), 3.39 - 3.27 (m, 3H), 3.27 - 3.23 (m, 1H), 3.21 - 3.14 (m, 1H), 2.80 - 2.70 (m, 1H), 2.33 (d, J = 13.7 Hz, 1H), 2.15 - 2.03 (m, 1H), 1.88 (d, J = 12.6 Hz, 1H), 1.76 - 1.62 (m, 5H), 1.60 - 1.50 (m, 2H), 1.50 - 1.44 (m, 1H), 1.44 - 1.30 (m, 5H), 1.30 - 1.23 (m, 2H), 1.21 - 1.06 (m, 2H), 0.95 - 0.83 (m, 1H), 0.76 (qd, J = 12.2, 3.2 Hz, 1H) ppm.

### Macrocycles p5(1,4)-Diol- and p5(1,4)-Diol-II

General procedure E was applied to macrocycle p5(1,1)-(E). Yield: p5(1,4)-Diol-I (9 mg, 35 %) and p5(1,4)-Diol-II (8 mg, 32 %) as white solids. p5(1,4)-Diol-I: HR-MS (m/z): (ESI⁺) calculated for: C₂₉H₄₄NO₉ [M+H]⁺: 532.30106, found: 550.30121. ¹H NMR (500 MHz, CDCl₃): 6.74 (s, 1H), 6.35 (s, 1H), 5.46 (d, J = 5.2 Hz, 1H), 4.39 - 4.31 (m, 1H), 4.18 - 4.10 (m, 2H), 4.01 - 3.89 (m, 2H), 3.88 - 3.79 (m, 7H), 3.63 (dt, J = 9.4, 5.2 Hz, 2H), 3.59 - 3.53 (m, 2H), 3.45 (d, J = 10.8 Hz, 2H), 3.42 (d, J = 6.5 Hz, 2H), 2.91 (t, J = 13.1 Hz, 1H), 2.35 (d, J = 13.8 Hz, 1H), 2.21 - 2.10 (m, 1H), 1.94 (ddd, J = 13.8, 9.2, 4.5 Hz, 1H), 1.89 - 1.83 (m, 2H), 1.76 - 1.63 (m, 5H), 1.61 - 1.52 (m, 1H), 1.51 - 1.44 (m, 0H), 1.44 - 1.29 (m, 3H), 1.25 - 1.08 (m, 2H), 0.89 (qd, J = 12.2, 3.0 Hz, 1H), 0.77 (qd, J = 12.2, 3.2 Hz, 1H) ppm. p5(1,4)-Diol-II: HR-MS (m/z): (ESI⁺) calculated for: C₂₉H₄₄NO₉ [M+H]⁺: 550.30106, found: 550.30116. ¹H NMR (500 MHz, CDCl₃): δ 6.78 (s, 1H), 6.33 (s, 1H), 5.37 - 5.31 (m, 1H), 4.53 (dd, J = 10.0, 4.8 Hz, 1H), 4.34 (t, J = 10.7 Hz, 1H), 4.16 (d, J = 14.1 Hz, 1H), 4.02 - 3.94 (m, 1H), 3.88 (s, 3H), 3.84 (d, J = 1.5 Hz, 4H), 3.72 - 3.64 (m, 1H), 3.54 (dt, J = 8.5, 3.9 Hz, 1H), 3.46-3.30 (m, 4H), 3.18 (d, J = 9.7 Hz, 1H), 2.84 (t, J = 13.1 Hz, 1H), 2.36 (d, J = 13.8 Hz, 1H), 2.20 - 2.08 (m, 1H), 1.98 (ddd, J = 15.1, 11.0, 3.9 Hz, 1H), 1.83 (d, J = 12.7 Hz, 1H), 1.79 - 1.57 (m, 7H), 1.52 (t, J = 13.2 Hz, 1H), 1.45 - 1.26 (m, 3H), 1.16 (dp, J = 25.1, 12.6 Hz, 2H), 0.89 (q, J = 11.9 Hz, 1H), 0.80-0.69 (m, 1H) ppm.

### Macrocycles p5(2,1)-(E)

General procedure D was applied to p4(2,1). Yield:(32 mg, 28 %) as a white solid.

HR-MS (m/z): (ESI⁺) calculated for: C₂₇H₃₈NO₆ [M+H]⁺: 486.26936, found: 472.26953. ¹H NMR (500 MHz, CDCl₃): 6.54 (d, J = 1.8 Hz, 1H), 6.35 (d, J = 1.8 Hz, 1H), 5.78 - 5.65 (m, 1H), 5.44 - 5.39 (m, 1H), 4.94 (dt, J = 15.5, 6.6 Hz, 1H), 4.55 (dd, J = 11.4, 6.6 Hz, 1H), 4.47 (ddd, J = 10.9, 5.3, 3.0 Hz, 1H), 4.30 (dd, J = 11.4, 9.0 Hz, 1H), 4.07 - 3.99 (m, 1H), 3.87 (s, 3H), 3.77 (s, 3H), 3.58 (ddd, J = 10.8, 9.5, 2.7 Hz, 1H), 3.35 (d, J = 10.3 Hz, 1H), 2.55 (td, J = 13.4, 2.8 Hz, 1H), 2.34 - 2.28 (m, 1H), 2.25 - 2.03 (m, 3H), 1.99 - 1.93 (m, 1H), 1.74 - 1.62 (m, 6H), 1.57 (tdd, J = 13.6, 5.7, 3.8 Hz, 1H), 1.39 - 1.33 (m, 1H), 1.32 - 1.27 (m, 1H), 1.27 - 1.20 (m, 1H), 1.20 - 1.08 (m, 2H), 0.92 - 0.78 (m, 2H) ppm.

### Macrocycles p5(2,1)-Diol-I and p5(2,1)-Diol-II

General procedure E was applied to macrocycle p5(2,1)-(E) (1.7 mg, 2 %) as white solids. p5(2,1)-Diol-I: MS (m/z): (ESI⁺) calculated for: C₂₇H₄₀NO₈ [M+H]⁺: 506.27484, found: 506.27462; ¹H NMR (500 MHz, CDCl₃): 6.80 (d, J = 1.8 Hz, 1H), 6.40 (d, J = 1.8 Hz, 1H), 5.51 (d, J = 4.7 Hz, 1H), 4.37 (d, J = 13.0 Hz, 1H), 4.31 - 4.21 (m, 3H), 3.93 (s, 3H), 3.91 (s, 3H), 3.89 - 3.84 (m, 2H), 3.56 (d, J = 10.6 Hz, 1H), 3.32 - 3.25 (m, 1H), 2.93 - 2.82 (m, 2H), 2.40 - 2.30 (m, 2H), 1.91 (d, J = 12.8 Hz, 1H), 1.79 - 1.66 (m, 6H), 1.65 - 1.59 (m, 1H), 1.60 - 1.47 (m, 1H), 1.46 - 1.39 (m, 2H), 1.34-1.12 (m, 3H), 0.95-0.81 (m, 2H) ppm. p5(2,1)-Diol-II: MS (m/z): (ESI⁺) calculated for: C₂₇H₄₀NO₈ [M+H]⁺: 506.27484, found: 506.27495.¹H NMR (500 MHz, CDCl₃): 6.68 (d, J = 1.7 Hz, 1H), 6.37 (d, J = 1.8 Hz, 1H), 5.58 - 5.46 (m, 1H), 4.61 (d, J = 13.0 Hz, 1H), 4.24 -4.11 (m, 2H), 3.97 - 3.90 (m, 2H), 3.90 (s, 3H), 3.84 (s, 3H), 3.32 (d, J = 10.3 Hz, 1H), 3.20 (dd, J = 9.0, 5.2 Hz, 1H), 2.58 (td, J = 13.3, 12.9, 2.5 Hz, 1H), 2.49 - 2.41 (m, 1H), 2.36 - 2.30 (m, 1H), 2.28 - 2.19 (m, 1H), 2.04 - 1.94 (m, 1H), 1.73 - 1.60 (m, 5H), 1.55 - 1.46 (m, 1H), 1.45 - 1.08 (m, 10H), 0.91 - 0.76 (m, 2H). ppm.

### Macrocycles p5(2,2)-(E) and p5(2,2)-(Z)

General procedure D was applied to p4(2,2). Yield p5(2,2)-(E) (60 mg, 21 %) and p5(2,2)-(Z) (77 mg, 20 %) as white solids. p5(2,2)-(E): HR-MS (m/z): (ESI⁺) calculated for: C₂₇H₃₈NO₆ [M+H]⁺: 486.2501, found: 486.28495. ¹H NMR (500 MHz, CDCl₃): 6.54 (d, J = 1.9 Hz, 1H), 6.31 (d, J = 1.9 Hz, 1H), 5.47 (d, J = 5.4 Hz, 1H), 5.35 (ddd, J = 15.0, 8.4, 5.2 Hz, 1H), 4.55 (dt, J = 10.9, 4.1 Hz, 1H), 4.38 (dt, J = 14.1, 6.6 Hz, 1H), 4.26 (td, J = 10.6, 3.2 Hz, 1H), 4.12 (ddd, J = 10.8, 7.5, 3.3 Hz, 1H), 4.03 (d, J = 14.1 Hz, 1H), 3.84 (s, 3H), 3.80 (t, J = 2.8 Hz, 0H), 3.78 (s, 3H), 3.31 (d, J = 10.0 Hz, 1H), 2.66 - 2.56 (m, 1H), 2.35 - 2.29 (m, 1H), 2.27 - 2.20 (m, 1H), 2.19 - 2.13 (m, 1H), 2.10 - 2.01 (m, 2H), 1.99 - 1.93 (m, 1H), 1.80 - 1.61 (m, 5H), 1.55 (tdd, J = 13.5, 5.6, 3.7 Hz, 1H), 1.51 - 1.42 (m, 1H), 1.39 - 1.21 (m, 5H), 1.21 - 1.07 (m, 2H), 0.92 - 0.74 (m, 2H) ppm. p5(2,2)-(Z): HR-MS (m/z): (ESI⁺) calculated for: C₂₇H₃₈NO₆ [M+H]⁺: 486.2501, found: 486.28485. ¹H NMR (500 MHz, CDCl₃): 6.70 (d, J = 1.9 Hz, 1H), 6.30 (d, J = 1.9 Hz, 1H), 5.45 (d, J = 5.5 Hz, 1H), 5.34 (qd, J = 8.5, 7.9, 3.8 Hz, 1H), 5.04 (dt, J = 11.0, 6.6 Hz, 1H), 4.37 (dt, J = 11.5, 7.2 Hz, 1H), 4.13 - 4.02 (m, 2H), 3.85 (s, 3H), 3.81 (s, 3H), 3.50 (td, J = 10.0, 4.5 Hz, 1H), 3.39 (d, J = 10.2 Hz, 1H), 2.70 (td, J = 13.3, 2.7 Hz, 1H), 2.39 - 2.23 (m, 3H), 2.23 - 2.15 (m, 1H), 1.93 (d, J = 12.5 Hz, 1H), 1.83 - 1.73 (m, 1H), 1.73 (s, 2H), 1.61 - 1.52 (m, 5H), 1.50 - 1.40 (m, 1H), 1.40 - 1.23 (m, 3H), 1.22 - 1.05 (m, 3H), 0.92 - 0.68 (m, 2H) ppm.

### Macrocycles p5(2,2)-H₂

General procedure F was applied to macrocycle p5(2,2)-(E). Yield: 6 mg, 37 %, white solid. MS (m/z): (ESI⁺) calculated for: C₂₉H₄₄NO₇ [M+H]⁺: 488.30066, found: 488.30058.¹H NMR (500 MHz, CDCl₃): δ 6.70 (d, J = 1.7 Hz, 1H), 6.31 (d, J = 1.7 Hz, 1H), 5.44 (d, J = 5.4 Hz, 1H), 4.42 (ddd, J = 10.6, 5.9, 4.3 Hz, 1H), 4.22 (ddd, J = 11.9, 8.3, 4.2 Hz, 1H), 4.08 (d, J = 13.9 Hz, 1H), 4.01 (dt, J = 10.6, 7.1 Hz, 1H), 3.84 (s, 3H), 3.83 (s, 3H), 3.64 (ddd, J = 10.6, 7.7, 5.7 Hz, 1H), 3.38 (d, J = 10.3 Hz, 1H), 2.73 - 2.68 (m, 1H), 2.32 (d, J = 13.7 Hz, 1H), 2.18 (qt, J = 10.8, 3.3 Hz, 1H), 1.92 (d, J = 12.5 Hz, 1H), 1.74 - 1.61 (m, 6H), 1.60 - 1.45 (m, 3H), 1.43 - 1.03 (m, 8H), 1.03 - 0.92 (m, 2H), 0.82 (dqd, J = 45.6, 12.2, 3.6 Hz, 3H) ppm.

### Macrocycles p5(2,2)-Diol-I and p5(2,2)-Diol-II

General procedure E was applied to macrocycle p5(2,2)-(E). Yield: p5(2,2)-Diol-I (3 mg, 8%) and p5(2,2)-Diol-II (18 mg, 56 %) as white solids. p5(2,2)-Diol-l: HR-MS (m/z): (ESI⁺) calculated for: C₂₈H₄₂NO₈ [M+H]⁺: 520.29049, found: 520.29051. ¹H NMR (500 MHz, CDCl₃): 6.70 (d, J = 1.8 Hz, 1H), 6.30 (d, J = 1.9 Hz, 1H), 5.45 (d, J = 5.3 Hz, 1H), 4.73 (dt, J = 11.0, 3.6 Hz, 1H), 4.37 (td, J = 11.6, 2.5 Hz, 1H), 4.08 - 3.91 (m, 3H), 3.83 (s, 3H), 3.82 (s, 3H), 3.63 - 3.56 (m, 1H), 3.35 (d, J = 10.3 Hz, 1H), 2.85 (ddd, J = 9.3, 5.8, 2.8 Hz, 1H), 2.70 - 2.61 (m, 1H), 2.45 - 2.29 (m, 1H), 2.13 (dt, J = 10.9, 3.2 Hz, 1H), 1.90 (d, J = 12.1 Hz, 1H), 1.76-1.61 (m, 6H), 1.60-1.51 (m, 2H), 1.50 - 1.39 (m, 2H), 1.39 - 1.20 (m, 3H), 1.20 - 1.07 (m, 2H), 1.02 (dtd, J = 10.7, 7.8, 2.7 Hz, 1H), 0.86 (qd, J = 12.3, 3.3 Hz, 1H), 0.81 - 0.73 (m, 1H) ppm. p5(2,2)-Diol-II: MS (m/z): (ESI⁺) calculated for: C₂₈H₄₂NO₈ [M+H]⁺: 520.2905, found: 520.2911. ¹H NMR (500 MHz, CDCl₃): 6.79 (d, J = 1.8 Hz, 1H), 6.30 (d, J = 1.8 Hz, 1H), 5.43 (d, J = 5.5 Hz, 1H), 4.69 (dt, J = 11.2, 4.1 Hz, 1H), 4.43 - 4.34 (m, 1H), 4.25 (dd, J = 12.1, 7.6 Hz, 1H), 4.17 (td, J = 10.3, 5.4 Hz, 1H), 3.86 (s, 3H), 3.84 (s, 3H), 3.76 (td, J = 10.3, 5.9 Hz, 1H), 3.55 (d, J = 10.5 Hz, 1H), 3.39 (tt, J = 7.1, 3.4 Hz, 1H), 3.04 (ddd, J = 10.1, 7.8, 2.0 Hz, 1H), 2.96 (td, J = 13.3, 2.8 Hz, 1H), 2.34 (d, J = 13.2 Hz, 1H), 2.28 - 2.15 (m, 1H), 1.84 (d, J = 12.8 Hz, 1H), 1.81 - 1.59 (m, 7H), 1.59 - 1.44 (m, 3H), 1.44 - 1.26 (m, 3H), 1.27 - 1.13 (m, 3H), 1.08 (dp, J = 15.3, 5.1 Hz, 1H), 0.95 - 0.85 (m, 1H), 0.82 - 0.65 (m, 2H) ppm.

### Macrocycles p5(2,2)-Diol-III and p₅(2,2)-Diol-IV

General procedure E was applied to macrocycle **p5(2,2)-(Z).Yield: p5(2,2)-Diol-III** (13 mg, 41 %) and **p₅(2,2)-Diol-IV** (3 mg, 9 %) as white solids**.p5(2,2)-Diol-III: HR-MS** (m/z): (ESI⁺) calculated for: C₂₈H₄₂NO₈ [M+H]⁺: 520.29049, found: 520.29068. **¹H NMR** (500 MHz, CDCl₃): 6.76 (d, J = 1.8 Hz, 1H), 6.32 (d, J = 1.9 Hz, 1H), 5.45 (d, J = 5.3 Hz, 1H), 4.66 (dt, J = 11.2, 4.1 Hz, 1H), 4.41 - 4.35 (m, 1H), 4.18 - 4.13 (m, 1H), 4.10 - 4.04 (m, 1H), 3.94 (dt, J = 10.7, 7.7 Hz, 1H), 3.85 (s, 6H), 3.54 (dt, J = 8.2, 2.6 Hz, 1H), 3.47 (d, J = 10.5 Hz, 1H), 3.15 (dt, J = 10.7, 2.9 Hz, 1H), 2.87 - 2.80 (m, 1H), 2.34 - 2.29 (m, 1H), 2.27 - 2.18 (m, 1H), 1.87 (d, J = 12.1 Hz, 1H), 1.76 - 1.62 (m, 7H), 1.56 (tdd, J = 13.2, 5.5, 3.5 Hz, 1H), 1.50 - 1.30 (m, 5H), 1.26 - 1.09 (m, 4H), 1.06 - 0.98 (m, 1H), 0.92 - 0.84 (m, 1H), 0.84 - 0.73 (m, 1H) ppm. **p5(2,2)-Diol-IV: HR-MS** (m/z): (ESI⁺) calculated for: C₂₈H₄₂NO₈ [M+H]⁺: 520.29049, found: 520.29057. **¹H NMR** (500 MHz, CDCl₃): 6.74 (d, J = 1.8 Hz, 1H), 6.30 (d, J = 1.8 Hz, 1H), 5.40 (d, J = 5.5 Hz, 1H), 4.80 - 4.73 (m, 1H), 4.30 (td, J = 11.3, 2.9 Hz, 1H), 4.23 - 4.16 (m, 1H), 4.13 - 4.06 (m, 1H), 3.83 (s, 3H), 3.83 (s, 3H), 3.80 - 3.77 (m, 1H), 3.74 - 3.67 (m, 1H), 3.44 - 3.37 (m, 2H), 2.78 (td, J = 13.8, 13.3, 2.4 Hz, 1H), 2.32 (d, J = 13.7 Hz, 1H), 2.19-2.08 (m, 1H), 1.91-1.85 (m, 1H), 1.76 - 1.69 (m, 3H), 1.69 - 1.60 (m, 4H), 1.59 - 1.52 (m, 1H), 1.51 - 1.41 (m, 2H), 1.40 - 1.21 (m, 4H), 1.20 - 1.11 (m, 2H), 1.09 - 0.99 (m, 1H), 0.92 - 0.80 (m, 2H), 0.80 - 0.70 (m, 1H) ppm.

### Macrocycle p5(3,1)-(E)

General procedure D was applied to **p4(3,1) Yield:** 265 mg, 74 %, white solid. **HR-MS** (m/z): (ESI⁺) calculated for: C₂₈H₄₀NO₇ [M+H]⁺: 502.2799, found: 502.2797; **¹H NMR** (500 MHz, CDCl₃): 6.66 (d, J = 1.7 Hz, 1H), 6.29 (d, J = 1.7 Hz, 1H), 5.85 (ddd, J = 14.7, 8.0, 6.2 Hz, 1H), 5.38 (dd, J = 15.3, 6.2 Hz, 2H), 4.93 (dd, J = 13.0, 9.8 Hz, 0H), 4.74 (dd, J = 12.3, 6.0 Hz, 1H), 4.63 (dd, J = 12.3, 8.2 Hz, 1H), 4.22 - 4.10 (m, 1H), 4.02 (d, J = 14.2 Hz, 1H), 3.98 - 3.86 (m, 2H), 3.86 (s, 3H), 3.78 (s, 3H), 3.78 - 3.73 (m, 1H), 3.33 (d, J = 10.0 Hz, 1H), 3.02 - 2.86 (m, 2H), 2.73 (td, J = 13.2, 2.7 Hz, 1H), 2.30 (d, J = 14.4 Hz, 1H), 2.11-2.03 (m, 1H), 1.88 (d, J = 12.5 Hz, 1H), 1.74 - 1.66 (m, 3H), 1.65 - 1.59 (m, 2H), 1.59 - 1.53 (m, 1H), 1.51 - 1.41 (m, 1H), 1.41 - 1.21 (m, 3H), 1.17 - 1.02 (m, 2H), 0.89 - 0.79 (m, 1H), 0.77 - 0.65 (m, 1H) ppm.

### Macrocycles p5(3,1)-H₂

General procedure F was applied to macrocycle **p5(3,1)-(E). Yield:** 20 mg, 80 %, white solid. **HR-MS** (m/z): (ESI⁺) calculated for: C₂₈H₄₂NO₇ [M+H]⁺: 504.29558, found: 504.29505. **¹H NMR** (500 MHz, CDCl₃): δ 6.66 (d, J = 1.7 Hz, 1H), 6.31 (d, J = 1.7 Hz, 1H), 5.46 (d, J = 5.4 Hz, 1H), 4.29 (t, J = 5.8 Hz, 2H), 4.24 (dt, J = 11.8, 4.8 Hz, 1H), 4.01 - 3.94 (m, 1H), 3.92 (dt, J = 11.2, 5.2 Hz, 1H), 3.85 (s, 3H), 3.83 (s, 3H), 3.31 (d, J = 10.1 Hz, 1H), 3.25 (dt, J = 10.0, 5.5 Hz, 4H), 2.72 (td, J = 13.4, 2.6 Hz, 1H), 2.30 (d, J = 13.7 Hz, 1H), 2.19 - 2.09 (m, 1H), 1.92 (d, J = 12.5 Hz, 1H), 1.74 - 1.30 (m, 14H), 1.29 - 1.06 (m, 2H), 0.87 (qt, J = 12.7, 5.4 Hz, 1H), 0.80 - 0.71 (m, 1H) ppm.

### Macrocycle p5(3,1)-Diol-I

General procedure E was applied to macrocycle **p5(3,1)-(E). Yield: p5(3,2)-Diol-I** 19 mg, 36 %, white solid. **HR-MS** (m/z): (ESI⁺) calculated for: C₂₈H₄₂NO₉ [M+H]⁺: 536.28541, found: 536.28549. **¹H NMR** (500 MHz, CDCl₃): 6.69 (d, J = 1.7 Hz, 1H), 6.33 (d, J = 1.8 Hz, 1H), 5.46 (d, J = 5.4 Hz, 1H), 4.43 (dd, J = 12.8, 2.8 Hz, 1H), 4.32 (ddd, J = 12.3, 6.1, 2.0 Hz, 1H), 4.27 - 4.16 (m, 1H), 3.93 - 3.87 (m, 5H), 3.85 (s, 3H), 3.50 - 3.39 (m, 2H), 3.35 - 3.25 (m, 3H), 3.23 - 3.12 (m, 2H), 2.68 (td, J = 13.5, 2.5 Hz, 1H), 2.31 (d, J = 13.5 Hz, 1H), 2.21 - 2.08 (m, 1H), 1.93 (d, J = 12.6 Hz, 1H), 1.74 - 1.60 (m, 4H), 1.58 - 1.50 (m, 1H), 1.49 - 1.21 (m, 6H), 1.21 - 1.04 (m, 2H), 0.92 - 0.70 (m, 3H) ppm.

### Macrocycle p5(3,2)-(E)

General procedure D was applied to **p4(3,2). Yield:** 93 mg, 54 %, white solid. **HR-MS** (m/z): (ESI⁺) calculated for: C₂₉H₄₁NO₇ [M+H]⁺: 516.29558, found: 516.29622. **¹H NMR** (500 MHz, CDCl₃): 6.65 (d, J = 2.0 Hz, 1H), 6.25 (d, J = 2.0 Hz, 1H), 5.71 (dt, J = 14.8, 7.2 Hz, 1H), 5.47 (d, J = 5.3 Hz, 1H), 5.30 (dt, J = 15.1, 6.3 Hz, 1H), 4.49-4.38 (m, 1H), 4.28-4.14 (m, 2H), 4.09-3.99 (m, 2H), 3.84 (s, 3H), 3.83 (s, 3H), 3.79 (dd, J = 12.3, 5.5 Hz, 1H), 3.68 (dd, J = 12.6, 6.8 Hz, 1H), 3.34 (d, J = 10.0 Hz, 1H), 3.29 - 3.19 (m, 2H), 2.83 (t, J = 12.4 Hz, 1H), 2.51 - 2.41 (m, 1H), 2.38 - 2.25 (m, 2H), 2.07 (q, J = 10.8 Hz, 1H), 1.85 (d, J = 12.8 Hz, 1H), 1.77 - 1.61 (m, 5H), 1.60 - 1.53 (m, 1H), 1.52 - 1.37 (m, 2H), 1.32 (d, J = 12.9 Hz, 2H), 1.22 - 1.05 (m, 2H), 0.97 - 0.84 (m, 1H), 0.78 - 0.67 (m, 1H) ppm.

### Macrocycle p5(3,2)-H₂

General procedure F was applied to macrocycle **p5(3,2)-(E). Yield:** 4 mg, 27 %, white solid. **HR-MS** (m/z): (ESI⁺) calculated for: C₂₉H₄₄NO₇ [M+H]⁺: 518.31123, found: 518.31174.

**¹H NMR** (500 MHz, CDCl₃): δ 6.65 (s, 1H), 6.30 (s, 1H), 5.44 (d, J = 5.5 Hz, 1H), 4.33 - 4.22 (m, 2H), 4.22 - 4.15 (m, 1H), 4.03 - 3.95 (m, 2H), 3.84 (s, 3H), 3.83 (s, 3H), 3.39 - 3.27 (m, 3H), 3.27 - 3.23 (m, 1H), 3.21 -3.14 (m, 1H), 2.80-2.70 (m, 1H), 2.33 (d, J = 13.7 Hz, 1H), 2.15 - 2.03 (m, 1H), 1.88 (d, J = 12.6 Hz, 1H), 1.76 - 1.62 (m, 6H), 1.60 - 1.50 (m, 2H), 1.50 - 1.44 (m, 1H), 1.44 - 1.30 (m, 5H), 1.30 - 1.23 (m, 2H), 1.21 - 1.06 (m, 2H), 0.95 - 0.83 (m, 1H), 0.82 - 0.70 (m, 1H) ppm.

### Macrocycles p5(3,2)-Diol-I and p5(3,2)-Diol-II

General procedure E was applied to macrocycle **p5(3,2)-(E). Yield: p₅(3,2)-Diol-I** (8 mg, 30 %) and **p5(3,2)-Diol-II** (11 mg, 39 %) as white solids. **p5(3,2)-Diol-l: HR-MS** (m/z): (ESI⁺) calculated for: C₂₉H₄₄NO₉ [M+H]⁺: 550.30106, found: **550.30154.¹H NMR** (500 MHz, CDCl₃): 6.68 (d, J = 1.7 Hz, 1H), 6.32 (d, J = 1.6 Hz, 1H), 5.45 (d, J = 5.5 Hz, 1H), 4.52 - 4.34 (m, 2H), 4.28 - 4.20 (m, 1H), 4.11 -4.03 (m, 1H), 4.01 (d, J = 13.8 Hz, 1H), 3.86 (s, 3H), 3.84 - 3.81 (m, 4H), 3.78 - 3.65 (m, 1H), 3.63 - 3.56 (m, 1H), 3.55 - 3.47 (m, 1H), 3.46 - 3.35 (m, 4H), 3.25 (dd, J = 10.2, 3.1 Hz, 1H), 2.85 (t, J = 12.7 Hz, 1H), 2.29 (d, J = 13.8 Hz, 1H), 2.12 (q, J = 11.0 Hz, 1H), 1.96-1.83 (m, 2H), 1.82 - 1.63 (m, 6H), 1.62 - 1.54 (m, 1H), 1.53 - 1.44 (m, 1H), 1.44 - 1.22 (m, 3H), 1.21 - 1.07 (m, 2H), 0.96 - 0.84 (m, 1H), 0.81 - 0.69 (m, 1H) ppm. **p5(3,2)-Diol-II: HR-MS** (m/z): (ESI⁺) calculated for: C₂₉H₄₄NO₉ [M+H]⁺: 550.30106, found: 550.30139. ¹H **NMR** (500 MHz, CDCl₃): 6.64 (s, 1H), 6.30 (s, 1H), 5.44 (d, J = 5.3 Hz, 1H), 4.49 - 4.25 (m, 5H), 4.23 - 4.16 (m, 1H), 4.16 - 4.10 (m, 1H), 3.94 (d, J = 14.3 Hz, 1H), 3.90 - 3.83 (m, 4H), 3.83 (s, 3H), 3.51 - 3.41 (m, 2H), 3.40 - 3.35 (m, 1H), 3.33 (d, J = 9.9 Hz, 1H), 3.31 - 3.23 (m, 2H), 2.67 (t, J = 13.2 Hz, 1H), 2.32 (d, J = 13.8 Hz, 1H), 2.08 (q, J = 11.1 Hz, 1H), 1.93 - 1.84 (m, 1H), 1.80 (dt, J = 17.8, 6.4 Hz, 1H), 1.75 - 1.61 (m, 5H), 1.61 - 1.54 (m, 1H), 1.52 - 1.40 (m, 1H), 1.39 - 1.24 (m, 3H), 1.24 - 1.07 (m, 2H), 0.96 - 0.82 (m, 1H), 0.82 - 0.67 (m, 1H) ppm.

### Macrocycle p5(4,1)-(E)

General procedure D was applied to **p4(4,1). Yield:** 142 mg, 52 %, white solid. **HR-MS** (m/z): (ESI⁺) calculated for: C₂₉H₄₂NO₇ [M+H]⁺: 516.29558, found: 516.29598.**¹H NMR** (500 MHz, CDCl₃): 6.66 (d, J = 1.8 Hz, 1H), 6.27 (d, J = 1.8 Hz, 1H), 5.83 (dt, J = 14.8, 7.3 Hz, 1H), 5.48 (dt, J = 15.4, 6.0 Hz, 1H), 5.42 (d, J = 5.7 Hz, 1H), 4.67 (d, J = 7.1 Hz, 2H), 4.24 (dt, J = 11.0, 5.4 Hz, 1H), 4.04 - 3.98 (m, 1H), 3.97 - 3.92 (m, 1H), 3.87 - 3.83 (m, 6H), 3.79 (s, 3H), 3.32 (d, J = 9.9 Hz, 1H), 3.03 - 2.96 (m, 2H), 2.79 (td, J = 13.3, 2.7 Hz, 1H), 2.30 (d, J = 13.7 Hz, 1H), 2.12 - 2.02 (m, 1H), 1.86 (d, J = 12.5 Hz, 1H), 1.76 - 1.65 (m, 4H), 1.64 - 1.58 (m, 2H), 1.58 - 1.52 (m, 0H), 1.47 (dt, J = 12.8, 3.8 Hz, 1H), 1.43-1.32 (m, 2H), 1.33-1.23 (m, 2H), 1.18-1.06 (m, 2H), 0.87 (qd, J = 12.5, 3.3 Hz, 1H), 0.72 (qd, J = 12.2, 3.5 Hz, 1H) ppm.

### Macrocycle p5(4,1)-H₂

General procedure F was applied to macrocycle **p5(4,1)-(E). Yield:** 34 mg, 71 %, beige solid. **HR-MS** (m/z): (ESI⁺) calculated for: C₂₉H₄₄NO₇ [M+H]⁺: 518.31123, found: 518.31094.**¹H NMR** (500 MHz, CDCl₃): δ 6.67 (d, J = 1.8 Hz, 1H), 6.30 (d, J = 1.8 Hz, 1H), 5.39 (d, J = 5.7 Hz, 1H), 4.29 - 4.21 (m, 1H), 4.19 - 4.07 (m, 2H), 4.02 (d, J = 13.9 Hz, 1H), 3.84 (s, 3H), 3.80 (s, 3H), 3.74 (dt, J = 10.5, 7.1 Hz, 1H), 3.35 (d, J = 10.1 Hz, 1H), 3.32 - 3.28 (m, 2H), 3.26 - 3.20 (m, 1H), 3.17 (t, J = 6.2 Hz, 2H), 2.76 (td, J = 13.3, 2.8 Hz, 1H), 2.28 (d, J = 13.7 Hz, 1H), 2.20-2.06 (m, 1H), 1.91 (d, J = 12.9 Hz, 1H), 1.78 - 1.58 (m, 8H), 1.57 - 1.51 (m, 1H), 1.51 -1.41 (m, 1H), 1.40 - 1.21 (m, 5H), 1.20 - 1.07 (m, 2H), 0.87 (qd, J = 12.4, 3.3 Hz, 1H), 0.79 - 0.64 (m, 1H) ppm.

### Macrocycle p5(4,1)-Diol-I and p5(4,1)-Diol-II

General procedure E was applied to macrocycle **p5(4,1)-(E). Yield: p5(4,1)-Diol-I** (6 mg, 19 %) and **p5(4,1)-Diol-II** (8 mg, 25 %) as white solids.**p5(4,1)-Diol-I: HR-MS** (m/z): (ESI⁺) calculated for: C₂₉H₄₃NO₉Na [M+Na]⁺: 536.28355, found: **572.28301.¹H NMR** (500 MHz, CDCl₃): 6.71 (d, J = 1.8 Hz, 1H), 6.32 (d, J = 1.9 Hz, 1H), 5.33 (d, J = 5.8 Hz, 1H), 4.16 (dd, J = 11.6, 2.1 Hz, 1H), 4.06 (td, J = 9.8, 6.2 Hz, 1H), 4.01 - 3.96 (m, 1H), 3.93 (t, J = 10.6 Hz, 1H), 3.88 (s, 3H), 3.85 (d, J = 8.8 Hz, 1H), 3.81 (s, 2H), 3.64 - 3.58 (m, 1H), 3.56 - 3.48 (m, 2H), 3.39 (d, J = 10.1 Hz, 2H), 3.37 - 3.31 (m, 2H), 3.25 - 3.18 (m, 2H), 3.11 (td, J = 9.0, 4.0 Hz, 1H), 2.78 (td, J = 13.1, 2.8 Hz, 1H), 2.26 (d, J = 13.7 Hz, 1H), 2.18-2.07 (m, 1H), 1.91 (d, J = 12.6 Hz, 1H), 1.78-1.57 (m, 7H), 1.57 - 1.41 (m, 2H), 1.40 - 1.24 (m, 2H), 1.24 - 1.16 (m, 1H), 1.16 - 1.06 (m, 2H), 0.92 - 0.81 (m, 1H), 0.79 - 0.69 (m, 1H) **ppm.p5(4,1)-Diol-II: HR-MS** (m/z): (ESI⁺) calculated for: C₂₉H₄₃NO₉Na [M+Na]⁺: 536.28355, found: 572.28311. **¹H NMR** (500 MHz, CDCl₃): 6.73 (d, J = 1.8 Hz, 1H), 6.34 (d, J = 1.8 Hz, 1H), 5.41 - 5.34 (m, 1H), 4.22 - 4.11 (m, 3H), 4.03 (d, J = 13.4 Hz, 1H), 3.86 (s, 3H), 3.84 (s, 3H), 3.81 (d, J = 4.0 Hz, 1H), 3.66 - 3.59 (m, 1H), 3.56 - 3.50 (m, 1H), 3.45 (dd, J = 9.6, 4.0 Hz, 1H), 3.42 - 3.32 (m, 3H), 3.25 - 3.16 (m, 1H), 2.82 (td, J = 13.3, 2.6 Hz, 1H), 2.28 (d, J = 13.7 Hz, 1H), 2.12 (q, J = 10.7 Hz, 1H), 1.89 (d, J = 12.4 Hz, 1H), 1.81 (q, J = 12.9, 10.5 Hz, 1H), 1.74 - 1.55 (m, 6H), 1.53 - 1.45 (m, 1H), 1.45 - 1.39 (m, 1H), 1.37 - 1.20 (m, 3H), 1.14 (q, J = 12.8, 12.3 Hz, 2H), 0.93 - 0.80 (m, 1H), 0.80 - 0.70 (m, 1H) ppm.

### Macrocycle m5(1,1)-(E)

General procedure D was applied to **m4(1,1). Yield:** 28 mg, 61 %, beige solid. **HR-MS** (m/z): (ESI⁺) calculated for: C₂₆H₃₆NO₆ [M+H]⁺: 458.3, found: 458.2. **¹H NMR** (500 MHz, CDCl₃): δ 6.62 (d, J = 1.8 Hz, 1H), 6.30 (d, J = 1.8 Hz, 1H), 5.72 (ddd, J = 15.4, 8.9, 6.7 Hz, 1H), 5.47 (d, J = 5.0 Hz, 1H), 5.07 (ddd, J = 15.0, 8.7, 5.9 Hz, 1H), 4.62 (dd, J = 10.7, 6.7 Hz, 1H), 4.46 (ddtd, J = 26.7, 16.9, 11.9, 10.7, 8.9 Hz, 1H), 4.34 (dd, J = 11.7, 5.9 Hz, 1H), 4.12 - 4.03 (m, 2H), 3.85 (d, J = 1.5 Hz, 3H), 3.79 (d, J = 1.5 Hz, 3H), 3.41 (d, J = 10.4 Hz, 1H), 2.71 (td, J = 13.2, 2.7 Hz, 1H), 2.33 - 2.23 (m, 2H), 2.02 - 1.91 (m, 1H), 1.73 - 1.61 (m, 6H), 1.59 - 1.10 (m, 6H), 0.91 - 0.77 (m, 2H) ppm.

### Macrocycle m5(1,1)-H₂

**m4(1,1)-(E)** (27 mg, 0.059 mmol, 1.0 eq.) was dissolved in MeOH (5 mL). The solution was degassed with argon for 10 minutes. Palladium on carbon (10 wt.%, 6.4 mg, 0.006 mmol, 0.1 eq.) was added and the resulting mixture was sparged with hydrogen gas for 15 minutes. The reaction mixture was stirred under a hydrogen atmosphere for 2.5 hours. The mixture was filtered through a pad of celite and the filtrate was concentrated under reduced pressure. Flash column chromatography (CH/EA 5:1) afforded the title compound as a colorless resin (12 mg, 44 %). **HR-MS** (m/z): (ESI⁺) calculated for: C₂₆H₃₈NO₆ [M+H]⁺: 460.26936, found: 460.26939. **¹H NMR** (500 MHz, CDCl₃): δ 6.47 (d, J = 1.9 Hz, 1H), 6.40 (d, J = 1.9 Hz, 1H), 5.43 (d, J = 5.5 Hz, 1H), 4.40 - 4.24 (m, 1H), 4.21 - 4.15 (m, 1H), 4.13 - 4.06 (m, 1H), 4.01 - 3.91 (m, 1H), 3.87 (s, 3H), 3.86 (s, 3H), 3.78 - 3.67 (m, 1H), 3.34 (d, J = 8.0 Hz, 1H), 2.49 (ddd, J = 14.0, 12.5, 3.0 Hz, 1H), 2.38 - 2.24 (m, 1H), 2.13 - 2.03 (m, 1H), 1.98 - 1.81 (m, 2H), 1.79 - 1.47 (m, 11H), 1.48 - 1.28 (m, 3H), 1.28 - 1.16 (m, 1H), 1.17 - 1.07 (m, 1H), 1.09-0.95 (m, 1H), 0.88 - 0.71 (m, 1H) ppm.

### Macrocycle m5(1,1)-Diol

General procedure E was applied to macrocycle **m5(1,1)-(E). Yield:** 12 mg, 40 %, white solid. **HR-MS** (m/z): (ESI⁺) calculated for: C₂₆H₃₈NO₈ [M+H]⁺: 492.25919, found: 492.25958.**¹H NMR** (500 MHz, CDCl₃): 6.45 (d, J = 1.8 Hz, 1H), 6.40 (d, J = 1.9 Hz, 1H), 5.43 - 5.40 (m, 1H), 5.30 - 5.29 (m, 1H), 4.31 (ddd, J = 12.3, 5.3, 2.1 Hz, 1H), 4.21 - 4.15 (m, 1H), 4.14 - 4.09 (m, 1H), 4.09 - 4.04 (m, 1H), 3.91 - 3.87 (m, 3H), 3.86 (s, 3H), 3.85 (s, 3H), 3.52 - 3.47 (m, 1H), 3.38 (d, J = 9.9 Hz, 1H), 2.63 (td, J = 13.7, 2.6 Hz, 1H), 2.31 (d, J = 13.8 Hz, 1H), 2.10 (d, J = 11.1 Hz, 1H), 1.90 (d, J = 12.4 Hz, 1H), 1.74 - 1.61 (m, 6H), 1.60 - 1.51 (m, 1H), 1.46 - 1.41 (m, 1H), 1.35 (tt, J = 13.2, 6.7 Hz, 2H), 1.28 -1.10 (m, 2H), 0.96 - 0.87 (m, 1H), 0.87 - 0.78 (m, 1H) ppm.

### Macrocycle m5(1,2)-(E)

General procedure D was applied to **m4(1,2). Yield:** 47 mg, 70 %, white solid. **HR-MS** (m/z): (ESI⁺) calculated for: C₂₇H₃₈NO₆ [M+H]⁺: 472.26936, found: 472.26943. **¹H NMR** (500 MHz, CDCl₃): δ 6.62 (d, J = 1.8 Hz, 1H), 6.30 (d, J = 1.8 Hz, 1H), 5.72 (ddd, J = 15.4, 8.9, 6.7 Hz, 1H), 5.47 (d, J = 5.0 Hz, 1H), 5.07 (ddd, J = 15.0, 8.7, 5.9 Hz, 1H), 4.62 (dd, J = 10.7, 6.7 Hz, 1H), 4.46 (ddtd, J = 26.7, 16.9, 11.9, 10.7, 8.9 Hz, 1H), 4.34 (dd, J = 11.7, 5.9 Hz, 1H), 4.12 - 4.03 (m, 2H), 3.85 (d, J = 1.5 Hz, 3H), 3.79 (d, J = 1.5 Hz, 3H), 3.41 (d, J = 10.4 Hz, 1H), 2.71 (td, J = 13.2, 2.7 Hz, 1H), 2.33 - 2.23 (m, 2H), 2.02 - 1.91 (m, 1H), 1.73 - 1.61 (m, 6H), 1.59 -1.10 (m, 6H), 0.91 - 0.77 (m, 2H) ppm.

### Macrocycle m5(1,2)-H₂

General procedure F was applied to macrocycle **m5(2,1)-(E). Yield:** 4 mg, 31 %, beige solid. **HR-MS** (m/z): (ESI⁺) calculated for: C₂₇H₄₀NO₆ [M+H]⁺: 474.2850, found: 474.2855. **¹H NMR** (500 MHz, CDCl₃): δ 6.64 (d, J = 1.9 Hz, 1H), 6.38 (d, J = 1.9 Hz, 1H), 5.57 - 5.43 (m, 1H), 4.27 (t, J = 5.8 Hz, 2H), 4.20 - 4.11 (m, 1H), 4.10 - 3.94 (m, 1H), 3.91 - 3.87 (m, 1H), 3.86 (s, 3H), 3.82 (s, 3H), 3.35 (d, J = 9.9 Hz, 1H), 2.83 - 2.76 (m, 1H), 2.29 (dt, J = 13.7, 2.4 Hz, 1H), 2.12 - 2.01 (m, 1H), 1.97 - 1.82 (m, 1H), 1.73 - 1.61 (m, 7H), 1.60 - 1.50 (m, 3H), 1.49 - 1.28 (m, 6H), 1.23 - 1.06 (m, 2H), 0.93 - 0.83 (m, 1H), 0.82 - 0.65 (m, 1H) ppm.

### Macrocycle m5(1,2)-Diol-I and m5(1,2)-Diol-II

General procedure E was applied to macrocycle **m5(1,2)-(E). Yield: m5(1,2)-Diol-I** (8 mg, 41 %) and **m5(1,2)-Diol-II** (3 mg, 12 %) as white solids. **m5(1,2)-Diol-I:_HR-MS** (m/z): (ESI⁺) calculated for: C₂₇H₄₀NO₈ [M+H]⁺: 506.27484, found: 506.27477. **¹H NMR** (500 MHz, CDCl₃): 6.64 (d, J = 1.9 Hz, 1H), 6.44 (d, J = 1.8 Hz, 1H), 5.46 (d, J = 5.5 Hz, 1H), 4.53 - 4.38 (m, 2H), 4.09 (d, J = 4.5 Hz, 2H), 4.01 (d, J = 13.6 Hz, 1H), 3.87 (s, 3H), 3.84 (s, 3H), 3.60 - 3.51 (m, 2H), 3.39 (d, J = 10.1 Hz, 1H), 2.98 - 2.84 (m, 1H), 2.29 - 2.22 (m, 1H), 2.20 - 2.02 (m, 1H), 2.02 - 1.94 (m, 1H), 1.85 (d, J = 12.5 Hz, 1H), 1.76 - 1.62 (m, 6H), 1.61 - 1.51 (m, 1H), 1.50 - 1.40 (m, 3H), 1.40 - 1.07 (m, 5H), 0.96 - 0.75 (m, 2H) ppm. **m5(1,2)-Diol-II:_HR-MS** (m/z): (ESI⁺) calculated for: C₂₈H₄₂NO₈ [M+H]⁺: 506.27484, found: 520.27477. **¹H NMR** (500 MHz, CDCl₃): 6.83 (d, J = 2.0 Hz, 1H), 6.29 (d, J = 1.8 Hz, 1H), 5.39 (d, J = 4.4 Hz, 1H), 4.62 - 4.53 (m, 1H), 4.44 - 4.32 (m, 2H), 3.99 - 3.91 (m, 2H), 3.86 (s, 3H), 3.83 (s, 3H), 3.76 (dt, J = 11.1, 3.4 Hz, 1H), 3.47 - 3.42 (m, 1H), 3.33 - 3.24 (m, 1H), 2.88 - 2.68 (m, 1H), 2.27 - 2.13 (m, 1H), 2.15 - 2.02 (m, 1H), 1.92 - 1.78 (m, 2H), 1.78 - 1.54 (m, 8H), 1.52 - 1.42 (m, 1H), 1.39 - 1.23 (m, 3H), 1.21 - 1.05 (m, 2H), 0.95 - 0.72 (m, 2H) ppm.

### Macrocycle m5(1,3)-(E)

General procedure D was applied to **m4(1,3). Yield:** 46 mg, 67 %, white solid. **HR-MS** (m/z): (ESI⁺) calculated for: C₂₈H₄₀NO₇ [M+H]⁺: 502.27993, found: 502.28037. **¹H NMR** (500 MHz, CDCl₃): δ 6.51 (d, J = 1.8 Hz, 1H), 6.49 (d, J = 1.8 Hz, 1H), 5.59 (dt, J = 15.5, 5.8 Hz, 1H), 5.44 - 5.35 (m, 2H), 4.45 - 4.34 (m, 3H), 4.32 - 4.25 (m, 1H), 4.16 - 4.07 (m, 2H), 3.96 (dd, J = 13.8, 6.1 Hz, 1H), 3.86 (s, 3H), 3.84 (s, 3H), 3.71 -3.60 (m, 2H), 3.41 (d, J = 10.3 Hz, 1H), 2.97 (td, J = 13.2, 2.8 Hz, 1H), 2.40 - 2.25 (m, 1H), 2.15 - 2.03 (m, 1H), 1.85 (d, J = 12.2 Hz, 1H), 1.77 - 1.57 (m, 6H), 1.49 (qt, J = 13.2, 4.1 Hz, 1H), 1.43-1.24 (m, 3H), 1.20-1.09 (m, 2H), 0.95-0.82 (m, 1H), 0.81 - 0.68 (m, 1H) ppm.

### Macrocycle m5(1,3)-H₂

General procedure G was applied to macrocycle **m5(3,1)-(E). Yield:** 7 mg, 70 %, white solid. **HR-MS** (m/z): (ESI⁺) calculated for: C₂₈H₄₂NO₇ [M+H]⁺: 504.29558, found: 504.29562.**¹H NMR** (500 MHz, CDCl₃): δ 6.63 (d, J = 1.8 Hz, 1H), 6.61 (d, J = 1.8 Hz, 1H), 5.43 (d, J = 5.6 Hz, 1H), 4.35 (dd, J = 5.6, 3.1 Hz, 2H), 4.15 - 4.07 (m, 1H), 4.04 (ddd, J = 11.0, 7.7, 3.4 Hz, 1H), 3.85 (s, 4H), 3.82 (s, 3H), 3.74 - 3.64 (m, 2H), 3.50 - 3.40 (m, 2H), 3.37 - 3.29 (m, 1H), 2.98 (td, J = 13.2, 2.7 Hz, 1H), 2.35-2.26 (m, 1H), 2.17-2.06 (m, 1H), 1.90-1.81 (m, 1H), 1.78 - 1.54 (m, 6H), 1.52 - 1.46 (m, 0H), 1.44 - 1.26 (m, 8H), 1.21 - 1.07 (m, 2H), 0.95 - 0.67 (m, 2H) ppm.

### Macrocycle m5(1,3)-Diol-I and m5(1,3)-Diol-II

General procedure E was applied to macrocycle **m5(1,2)-(E). Yield: m5(1,3)-Diol-I** (9 mg, 46 %, mixture of diasteromers) as a white solid. **HR-MS** (m/z): (ESI⁺) calculated for: C₂₇H₄₀NO₈ [M+H]⁺: 506.27484, found: 506.27477. **¹H NMR** (500 MHz, CDCl₃, mixture of diastereomers, major diastereomer reported): 6.71 (d, J = 1.8 Hz, 1H), 6.48 (d, J = 1.7 Hz, 1H), 5.33 - 5.28 (m, 1H), 4.50 - 4.38 (m, 1H), 4.33 - 4.20 (m, 1H), 4.15 (dd, J = 11.2, 6.5 Hz, 1H), 4.11 - 4.04 (m, 1H), 4.00 (dd, J = 11.1, 5.3 Hz, 1H), 3.86 (s, 3H), 3.84 (s, 3H), 3.74 (tt, J = 8.0, 4.4 Hz, 1H), 3.62 - 3.29 (m, 5H), 2.95 (td, J = 13.1, 2.9 Hz, 1H), 2.43 - 2.16 (m, 3H), 2.16-2.06 (m, 1H), 1.88-1.80 (m, 1H), 1.79 - 1.60 (m, 6H), 1.58 - 1.44 (m, 1H), 1.44 - 1.24 (m, 4H), 1.20 - 1.09 (m, 2H), 0.97 - 0.85 (m, 1H), 0.78 - 0.67 (m, 1H) ppm.

### Macrocycle m5(1,4)-(E)

General procedure D was applied to **m4(1,4). Yield:** 41 mg, 67 %, white solid. **HR-MS** (m/z): (ESI⁺) calculated for: C₂₈H₄₀NO₇ [M+H]⁺: 516.29558, found: 516.29593. **¹H NMR** (500 MHz, CDCl₃): δ 6.56 - 6.49 (m, 2H), 5.57 (dt, J = 15.7, 5.0 Hz, 1H), 5.48 - 5.39 (m, 2H), 4.41 (dd, J = 13.2, 5.7 Hz, 1H), 4.25 (dd, J = 13.0, 5.8 Hz, 1H), 4.20 - 4.14 (m, 1H), 4.14 - 4.06 (m, 2H), 3.92 - 3.88 (m, 2H), 3.85 (s, 3H), 3.83 (s, 3H), 3.71 (t, J = 5.6 Hz, 2H), 3.44 (d, J = 10.2 Hz, 1H), 3.01 - 2.89 (m, 1H), 2.34 - 2.26 (m, 1H), 2.21 - 2.08 (m, 1H), 2.08 - 1.94 (m, 2H), 1.85 (d, J = 12.6 Hz, 1H), 1.78 - 1.54 (m, 6H), 1.53 - 1.43 (m, 1H), 1.41 - 1.27 (m, 3H), 1.25 - 1.06 (m, 2H), 0.94 - 0.83 (m, 1H), 0.80 - 0.69 (m, 1H) ppm.

### Macrocycle m5(1,4)-H₂

General procedure G was applied to macrocycle **m5(1,4)-(E) Yield:** 5 mg, 25 %. white solid.

**HR-MS** (m/z): (ESI⁺) calculated for: C₂₉H₄₄NO₇ [M+H]⁺: 518.31123, found: 518.31179.¹H NMR (500 MHz, CDCl₃): δ 6.49 (s, 1H), 6.47 (s, 1H), 5.39 - 5.36 (m, 1H), 4.17 (dd, J = 9.7, 5.1 Hz, 1H), 4.09 - 4.02 (m, 2H), 3.97 (td, J = 9.7, 6.3 Hz, 1H), 3.86 (s, 3H), 3.84 - 3.77 (m, 4H), 3.65 (dtd, J = 14.6, 9.6, 5.2 Hz, 2H), 3.39 (t, J = 7.5 Hz, 3H), 2.86 (t, J = 12.9 Hz, 1H), 2.28 (d, J = 13.7 Hz, 1H), 2.21 - 2.06 (m, 1H), 2.06 - 1.98 (m, 2H), 1.91 - 1.83 (m, 1H), 1.77 - 1.53 (m, 6H), 1.51 - 1.26 (m, 8H), 1.15 (h, J = 12.6 Hz, 2H), 0.96 - 0.83 (m, 1H), 0.80 - 0.64 (m, 1H) ppm.

### Macrocycle m5(1,4)-Diol-I and m5(1,4)-Diol-II

General procedure E was applied to macrocycle **m5(1,4)-(E)** (20 mg, 39 µmol, 1.0 eq.) with NMO (7 mg, 58 µmol, 1.5 eq.), osmium tetroxide solution (2.5 wt% in t-BuOH, 24 µL, 2 µmol, 0.06 eq.), 2,6-lutidine (9 µL, 76 µmol, 2.0 eq.) in acetone (1 mL) and water (1 mL). Preparative HPLC (35-65 % solvent B) afforded **m5(1,4)-Diol-I** (7 mg, 32 %) and **m5(1,4)-Diol-II** (6 mg, 28 %) as white solids. **m5(1,4)-Diol-I: HR-MS** (m/z): (ESI⁺) calculated for: C₂₉H₄₄NO₉ [M+H]⁺: 550.30106, found: 550.30193. **¹H NMR** (500 MHz, CDCl₃): 6.62 (s, 1H), 6.43 (s, 1H), 5.45 - 5.39 (m, 1H), 4.18 - 4.02 (m, 4H), 4.00 - 3.95 (m, 1H), 3.91 (s, 2H), 3.86 (s, 3H), 3.85 (s, 3H), 3.80 - 3.70 (m, 2H), 3.60 (d, J = 9.0 Hz, 1H), 3.57 - 3.50 (m, 2H), 3.47 - 3.33 (m, 2H), 2.96 - 2.88 (m, 1H), 2.34 (d, J = 13.8 Hz, 1H), 2.15 - 1.93 (m, 3H), 1.86 (d, J = 12.7 Hz, 1H), 1.79 - 1.56 (m, 6H), 1.55 - 1.23 (m, 4H), 1.23-1.05 (m, 2H), 0.94 - 0.83 (m, 1H), 0.79 - 0.67 (m, 1H). ppm. **m5(1,4)-Diol-II: HR-MS** (m/z): (ESI⁺) calculated for: C₂₉H₄₄NO₉ [M+H]⁺: 550.30106, found: 550.30186. **¹H NMR** (500 MHz, CDCl₃): 6.50 (s, 1H), 6.42 (s, 1H), 5.25 - 5.22 (m, 1H), 4.18 - 4.04 (m, 3H), 4.03 - 3.94 (m, 2H), 3.89 - 3.87 (m, 1H), 3.86 (s, 3H), 3.84 (s, 3H), 3.79 - 3.72 (m, 1H), 3.68 - 3.60 (m, 1H), 3.55 - 3.42 (m, 3H), 3.39 (d, J = 10.0 Hz, 1H), 2.94 (t, J = 13.4 Hz, 1H), 2.31 - 2.20 (m, 1H), 2.13 - 1.98 (m, 3H), 1.89 - 1.80 (m, 1H), 1.77 - 1.61 (m, 6H), 1.58 - 1.43 (m, 1H), 1.40 - 1.25 (m, 3H), 1.23 - 1.05 (m, 2H), 0.98 - 0.84 (m, 1H), 0.83 - 0.69 (m, 1H) ppm.

### Macrocycle m5(2,1)-(E)

General procedure D was applied to **m4(2,1) Yield:** 82 mg, 90 %), white solid. **HR-MS** (m/z): (ESI⁺) calculated for: C₂₇H₃₈NO₆ [M+H]⁺: 472.2694, found: 472.2696. **¹H NMR** (500 MHz, CDCl₃): δ 6.48 (d, J = 1.9 Hz, 1H), 6.32 (d, J = 1.7 Hz, 1H), 5.53 (dt, J = 15.8, 4.2 Hz, 1H), 5.50 - 5.45 (m, 1H), 5.45 - 5.42 (m, 1H), 4.79 - 4.63 (m, 2H), 4.50 (dt, J = 10.8, 4.2 Hz, 1H), 4.03 - 3.96 (m, 1H), 3.86 (s, 6H), 3.71 (td, J = 10.6, 2.8 Hz, 1H), 3.34 (d, J = 10.1 Hz, 1H), 2.81 (td, J = 13.1, 2.9 Hz, 1H), 2.40 - 2.32 (m, 1H), 2.32 - 2.26 (m, 1H), 2.22 - 2.16 (m, 1H), 2.08 - 1.96 (m, 1H), 1.89 - 1.81 (m, 1H), 1.74 - 1.61 (m, 5H), 1.60 - 1.53 (m, 1H), 1.52 - 1.38 (m, 2H), 1.36 - 1.24 (m, 2H), 1.22 - 1.05 (m, 2H), 0.92 - 0.81 (m, 1H), 0.81 - 0.70 (m, 1H) ppm.

### Macrocycle m5(2,1)-Diol-I and m5(2,1)-Diol-II

General procedure E was applied to macrocycle **m5(2,1)-(E). Yield: m5(2,1)-Diol-I** (8 mg, 39 %) and **m5(2,1)-Diol-II** (12 mg, 64 %) as white solids. **m5(2,1)-Diol-I: HR-MS** (m/z): (ESI⁺) calculated for: C₂₇H₄₀NO₈ [M+H]⁺: 506.27484, found: 506.27520. **¹H NMR** (500 MHz, CDCl₃): δ 6.69 (d, J = 1.9 Hz, 1H), 6.48 (d, J = 1.8 Hz, 1H), 5.49 - 5.43 (m, 1H), 4.41 - 4.34 (m, 2H), 4.32 (dt, J = 11.4, 3.4 Hz, 1H), 4.10 - 4.02 (m, 2H), 3.89 (s, 3H), 3.88 (s, 3H), 3.61 - 3.57 (m, 1H), 3.56 - 3.51 (m, 1H), 3.42 (d, J = 10.2 Hz, 1H), 2.92 (t, J = 12.3 Hz, 1H), 2.36 - 2.26 (m, 1H), 2.20 - 2.09 (m, 1H), 1.95 - 1.84 (m, 1H), 1.81 - 1.56 (m, 9H), 1.54 - 1.27 (m, 5H), 1.26 - 1.11 (m, 2H), 0.97 - 0.86 (m, 1H), 0.85 - 0.75 (m, 1H) ppm. **m5(2,1)-Diol-II:**_**HR-MS** (m/z): (ESI⁺) calculated for: C₂₇H₄₀NO₈ [M+H]⁺: 506.2748, found: 506.2751. **¹H NMR** (500 MHz, CDCl₃): δ 6.69 (d, J = 1.9 Hz, 1H), 6.38 (d, J = 1.8 Hz, 1H), 5.42 (d, J = 5.3 Hz, 1H), 4.49 (dd, J = 12.8, 5.2 Hz, 1H), 4.46 - 4.41 (m, 1H), 4.26 - 4.19 (m, 1H), 4.16 (s, 2H), 3.95 (d, J = 13.9 Hz, 1H), 3.91 - 3.85 (m, 5H), 3.84 (s, 3H), 3.61 - 3.53 (m, 1H), 3.32 (d, J = 9.9 Hz, 1H), 2.75 - 2.68 (m, 1H), 2.41 - 2.27 (m, 1H), 2.11 - 1.99 (m, 1H), 1.85 (d, J = 12.6 Hz, 1H), 1.76 - 1.61 (m, 6H), 1.60 - 1.50 (m, 2H), 1.49 - 1.40 (m, 2H), 1.38 - 1.26 (m, 2H), 1.15 (tdd, J = 15.3, 12.7, 6.8 Hz, 2H), 0.92 - 0.83 (m, 1H), 0.83 - 0.72 (m, 1H) ppm.

### Macrocycle m5(2,2)-(E) and m5(2,2)-(Z)

General procedure D was applied to **m4(2,2). Yield: m5(2,2)-(E)** (38 mg, 38 %) and **m5(2,2)-(Z)** (9 mg, 9 %) as white solids. **m5(2,2)-(E): HR-MS** (m/z): (ESI⁺) calculated for: C₂₈H₄₀NO₆ [M+H]⁺: 486.2850, found: 486.2852. **¹H NMR** (500 MHz, CDCl₃): δ 6.68 (d, J = 1.8 Hz, 1H), 6.43 (d, J = 2.0 Hz, 1H), 5.55 - 5.46 (m, 1H), 5.38 - 5.27 (m, 2H), 4.55 - 4.46 (m, 1H), 4.28 - 4.21 (m, 1H), 4.16 -4.08 (m, 1H), 4.06 - 4.00 (m, 1H), 4.00 - 3.95 (m, 1H), 3.87 (s, 3H), 3.84 (s, 3H), 3.40 (d, J = 10.1 Hz, 1H), 2.97 - 2.88 (m, 1H), 2.51 - 2.43 (m, 1H), 2.42 - 2.33 (m, 2H), 2.26 - 2.00 (m, 3H), 1.94 - 1.81 (m, 1H), 1.80 - 1.64 (m, 5H), 1.63 - 1.43 (m, 3H), 1.43 - 1.26 (m, 2H), 1.25 - 1.06 (m, 2H), 0.97 - 0.86 (m, 1H), 0.82 - 0.72 (m, 1H) ppm. **m5(2,2)-(Z): HR-MS** (m/z): (ESI⁺) calculated for: C₂₈H₄₀NO₆ [M+H]⁺: 486.2850, found: 486.2855. **¹H NMR** (500 MHz, CDCl₃): δ 6.58 (d, J = 2.0 Hz, 1H), 6.45 (d, J = 1.9 Hz, 1H), 5.54 - 5.45 (m, 1H), 5.41 - 5.33 (m, 1H), 5.32 - 5.26 (m, 1H), 4.38 - 4.29 (m, 1H), 4.26 - 4.15 (m, 1H), 4.07 - 3.99 (m, 2H), 4.01 - 3.92 (m, 1H), 3.86 (s, 3H), 3.82 (s, 3H), 3.39 (d, J = 10.1 Hz, 1H), 3.05 - 2.91 (m, 1H), 2.52 - 2.31 (m, 2H), 2.27 - 2.18 (m, 1H), 2.19 - 1.98 (m, 3H), 1.82 (d, J = 3.8 Hz, 1H), 1.74 - 1.57 (m, 6H), 1.53 - 1.22 (m, 4H), 1.21 - 1.05 (m, 2H), 0.90 - 0.82 (m, 1H), 0.78 - 0.67 (m, 1H) ppm.

### Macrocycle m5(2,2)-H₂

General procedure F was applied to macrocycle **m5(2,2)-(E) Yield:** 6 mg, 53 %, beige solid. **HR-MS** (m/z): (ESI⁺) calculated for: C₂₉H₄₄NO₇ [M+H]⁺: 488.3007, found: 488.3015. **¹H NMR** (500 MHz, CDCl₃): δ 6.70 (d, J = 1.8 Hz, 1H), 6.41 (d, J = 1.7 Hz, 1H), 5.46 (d, J = 5.3 Hz, 1H), 4.39 - 4.31 (m, 1H), 4.15 (dt, J = 11.1, 5.3 Hz, 1H), 4.08 - 4.03 (m, 1H), 4.02 - 3.98 (m, 1H), 3.96 - 3.91 (m, 1H), 3.86 (s, 3H), 3.84 (s, 3H), 3.35 (d, J = 10.1 Hz, 1H), 2.93 - 2.83 (m, 1H), 2.34 - 2.28 (m, 1H), 2.17 - 2.07 (m, 1H), 1.90 - 1.75 (m, 2H), 1.75 - 1.50 (m, 6H), 1.45 (ddq, J = 12.2, 7.3, 3.6 Hz, 2H), 1.38 - 1.27 (m, 8H), 1.19 - 1.08 (m, 3H), 0.94 - 0.80 (m, 1H), 0.78 - 0.70 (m, 1H) ppm.

### Macrocycle m5(2,2)-Diol-I and m5(2,2)-Diol-II

General procedure E was applied to macrocycle **m5(2,2)-(E) Yield: m5(2,2)-Diol-I** (9 mg, 46 %) and **m5(2,2)-Diol-II** (8 mg, 40 %) as white solids. **m5(2,2)-Diol-I:_HR-MS** (m/z): (ESI⁺) calculated for: C₂₈H₄₂NO₈ [M+H]⁺: 520.29049, found: 520.29088. **¹H NMR** (500 MHz, CDCl₃): 6.72 (d, J = 1.7 Hz, 1H), 6.33 (d, J = 1.6 Hz, 1H), 5.38 (d, J = 5.2 Hz, 1H), 4.52 - 4.38 (m, 1H), 4.32 - 4.20 (m, 2H), 4.03 (d, J = 14.0 Hz, 1H), 3.97 (dt, J = 11.3, 4.1 Hz, 1H), 3.85 (s, 3H), 3.83 (s, 3H), 3.61 - 3.51 (m, 4H), 3.35 (d, J = 10.1 Hz, 1H), 2.86 (td, J = 13.0, 2.8 Hz, 1H), 2.32 (d, J = 13.5 Hz, 1H), 2.11 - 1.96 (m, 3H), 1.82 (d, J = 12.6 Hz, 1H), 1.77 - 1.62 (m, 6H), 1.60 - 1.51 (m, 1H), 1.50 - 1.37 (m, 3H), 1.35 - 1.26 (m, 2H), 1.22 - 1.06 (m, 2H), 0.92 - 0.83 (m, 1H), 0.80 - 0.70 (m, 1H) ppm.**m5(2,2)-Diol-II: HR-MS** (m/z): (ESI⁺) calculated for: C₂₈H₄₂NO₈ [M+H]⁺: 520.29049, found: 520.29069. **¹H NMR** (500 MHz, CDCl₃): 6.82 (d, J = 1.7 Hz, 1H), 6.35 (d, J = 1.7 Hz, 1H), 5.50-5.39 (m, 1H), 4.53 - 4.45 (m, 2H), 4.32 - 4.25 (m, 1H), 3.99 - 3.93 (m, 1H), 3.86 (s, 3H), 3.84 (s, 3H), 3.76 (t, J = 10.7 Hz, 1H), 3.57 - 3.51 (m, 1H), 3.34 - 3.26 (m, 2H), 2.82 - 2.70 (m, 1H), 2.38 - 2.31 (m, 1H), 2.20 - 1.97 (m, 2H), 1.87 - 1.83 (m, 1H), 1.82 - 1.41 (m, 11H), 1.39 - 1.26 (m, 2H), 1.24 - 1.07 (m, 2H), 0.92 - 0.84 (m, 1H), 0.83 - 0.72 (m, 1H) ppm.

### Macrocycles m5(2,3)-(E) and m5(2,3)-(Z)

General procedure D was applied to **m4(2,3). Yield: m5(2,3)-(E)** (58 mg, 54 %) and **m5(2,3)-(Z)** (5 mg, 5 %) as white solids. **m5(2,3)-(E): HR-MS** (m/z): (ESI⁺) calculated for: C₂₉H₄₂NO₇ [M+H]⁺: 516.29558, found: 516.29557. **¹H NMR** (500 MHz, CDCl₃): δ 6.57 (s, 1H), 6.54 (s, 1H), 5.45 - 5.42 (m, 1H), 5.22 (dt, J = 15.8, 4.7 Hz, 1H), 5.12 (dt, J = 15.1, 6.6 Hz, 1H), 4.46 - 4.32 (m, 2H), 4.20 - 4.14 (m, 1H), 4.11 - 4.04 (m, 1H), 3.90 - 3.86 (m, 2H), 3.86 - 3.80 (m, 6H), 3.80 - 3.69 (m, 2H), 3.36 (d, J = 10.2 Hz, 1H), 2.87 (td, J = 13.4, 2.5 Hz, 1H), 2.34 (d, J = 13.8 Hz, 1H), 2.18 - 2.00 (m, 3H), 1.85 (d, J = 12.7 Hz, 1H), 1.80 - 1.42 (m, 7H), 1.42 - 1.21 (m, 4H), 1.13 (q, J = 11.7, 10.4 Hz, 2H), 0.91 - 0.80 (m, 1H), 0.77 - 0.65 (m, 1H) ppm. **m5(2,3)-(Z): HR-MS** (m/z): (ESI⁺) calculated for: C₂₉H₄₂NO₇ [M+H]⁺: 516.29558, found: 516.29578. **¹H NMR** (500 MHz, CDCl₃): δ 6.55 (s, 1H), 6.47(s, 1H), 5.67 - 5.58 (m, 1H), 5.49 - 5.41 (m, 1H), 5.37 - 5.32 (m, 1H), 4.41 - 4.34 (m, 1H), 4.34 - 4.18 (m, 1H), 4.17 - 3.99 (m, 3H), 3.94 (d, J = 13.7 Hz, 1H), 3.85 (s, 3H), 3.84 (s, 3H), 3.82 - 3.79 (m, 1H), 3.79 - 3.74 (m, 1H), 3.37 (d, J = 9.8 Hz, 1H), 2.87 - 2.80 (m, 1H), 2.30 - 2.17 (m, 3H), 2.13 - 2.03 (m, 1H), 1.91 - 1.83 (m, 2H), 1.73 - 1.55 (m, 6H), 1.54 - 1.40 (m, 1H), 1.40 - 1.23 (m, 3H), 1.21 - 1.05 (m, 2H), 0.97 - 0.84 (m, 1H), 0.83 - 0.69 (m, 1H) ppm.

### Macrocycle m5(2,3)-H₂

General procedure G was applied to macrocycle **m5(2,3)-(E). Yield:** 3 mg, 60 %, white solid. **HR-MS** (m/z): (ESI⁺) calculated for: C₂₉H₄₄NO₇ [M+H]⁺: 518.31123, found: 518.31085.**¹H NMR** (500 MHz, CDCl₃): δ 6.57 (d, J = 1.8 Hz, 1H), 6.54 (d, J = 1.8 Hz, 1H), 5.43 (d, J = 5.6 Hz, 1H), 4.35 (ddd, J = 12.2, 5.6, 2.5 Hz, 1H), 4.26 - 4.20 (m, 1H), 4.11 - 4.01 (m, 3H), 3.91 - 3.77 (m, 9H), 3.73 - 3.69 (m, 1H), 3.48 - 3.33 (m, 3H), 2.95 - 2.77 (m, 1H), 2.31 (d, J = 13.8 Hz, 1H), 2.15-2.04 (m, 2H), 1.88 (d, J = 12.3 Hz, 1H), 1.66 - 1.53 (m, 1H), 1.51 - 1.22 (m, 9H), 1.18 - 0.96 (m, 5H), 0.93 - 0.63 (m, 2H) ppm.

### Macrocycle m5(2,3)-Diol-I and m5(2,3)-Diol-II

General procedure E was applied to macrocycle **m5(2,3)-(E). Yield: m5(2,3)-Diol-I** (10 mg, 31 %) and **m5(2,3)-Diol-II** (6 mg, 19 %) as white solids. **m5(2,3)-Diol-I: HR-MS** (m/z): (ESI⁺) calculated for: C₂₉H₄₄NO₉ [M+H]⁺: 550.30106, found: 550.30121. **¹H NMR** (500 MHz, CDCl₃): 6.84 (s, 1H), 6.39 (s, 1H), 5.39 - 5.35 (m, 1H), 4.55 - 4.35 (m, 3H), 4.28 - 4.22 (m, 1H), 4.20 - 4.13 (m, 1H), 4.12 - 4.06 (m, 1H), 4.05 - 3.98 (m, 1H), 3.94 - 3.88 (m, 1H), 3.85 (s, 6H), 3.72 - 3.66 (m, 1H), 3.63 (dd, J = 9.9, 3.6 Hz, 1H), 3.54 (dd, J = 10.3, 3.1 Hz, 1H), 3.45 - 3.34 (m, 3H), 2.96 (t, J = 13.0 Hz, 1H), 2.32 (d, J = 13.6 Hz, 1H), 2.14 - 2.02 (m, 1H), 1.86 - 1.78 (m, 1H), 1.78 - 1.53 (m, 8H), 1.53 - 1.37 (m, 2H), 1.38 - 1.26 (m, 2H), 1.22 - 1.05 (m, 2H), 0.96 - 0.83 (m, 1H), 0.80 - 0.67 (m, 1H) ppm. **m5(2,3)-Diol-II: HR-MS** (m/z): (ESI⁺) calculated for: C₂₉H₄₄NO₉ [M+H]⁺: 550.30106, found: 550.30182. **¹H NMR** (500 MHz, CDCl₃): 6.62 (s, 1H), 6.53 (s, 1H), 5.40 (d, J = 5.4 Hz, 1H), 4.47 - 4.39 (m, 1H), 4.32 (s, 2H), 4.19 - 4.00 (m, 4H), 3.86 (s, 3H), 3.84 (s, 3H), 3.83 - 3.75 (m, 2H), 3.52 (dd, J = 9.6, 3.9 Hz, 1H), 3.47 (t, J = 8.1 Hz, 1H), 3.38 - 3.33 (m, 2H), 3.24 (dt, J = 8.0, 3.5 Hz, 1H), 2.79 - 2.68 (m, 1H), 2.33 (d, J = 13.8 Hz, 1H), 2.10 (q, J = 11.2 Hz, 1H), 1.87 (d, J = 12.6 Hz, 1H), 1.77 - 1.42 (m, 9H), 1.41 - 1.23 (m, 3H), 1.23 - 1.06 (m, 2H), 0.92 - 0.81 (m, 1H), 0.80 - 0.70 (m, 1H) ppm.

### Macrocycles m5(2,4)-(E)

General procedure D was applied to **m4(2,4). Yield:** 29 mg, 46 %, white solid. **HR-MS** (m/z): (ESI⁺) calculated for: C₃₀H₄₂NO₇ [M+H]⁺: 530.31123, found: 530.31127. **¹H NMR** (500 MHz, CDCl₃, 1.2:1 mixture of rotamers, major rotamer reported): δ 6.59 - 6.51 (m, 2H), 5.72 (q, J = 4.6, 4.1 Hz, 1H), 5.44 (d, J = 5.6 Hz, 1H), 5.39 - 5.27 (m, 1H), 4.30 - 4.16 (m, 3H), 4.14 - 4.07 (m, 1H), 3.99 - 3.86 (m, 2H), 3.84 (s, 3H), 3.80 (s, 3H), 3.79 - 3.76 (m, 1H), 3.71 - 3.64 (m, 1H), 3.63 - 3.57 (m, 1H), 3.39 (d, J = 10.2 Hz, 0H), 2.94 (td, J = 13.4, 2.7 Hz, 1H), 2.50 - 2.38 (m, 1H), 2.30 (d, J = 13.7 Hz, 1H), 2.16 - 1.82 (m, 6H), 1.75 - 1.42 (m, 6H), 1.40 - 1.04 (m, 6H), 0.91 - 0.79 (m, 1H), 0.78 - 0.69 (m, 1H). ppm.

### Macrocycles m5(2,4)-H₂

General procedure G was applied to macrocycle **m5(2,4)-(E). Yield:** 7 mg, 69 %, white solid. **HR-MS** (m/z): (ESI⁺) calculated for: C₃₀H₄₆NO₇ [M+H]⁺: 532.32688, found: 532.32711. **¹H NMR** (500 MHz, CDCl₃, 2.7:1 mixture of rotamers, major rotamer reported): δ 6.53 (d, J = 1.8 Hz, 1H), 6.46 (d, J = 1.8 Hz, 1H), 5.42 (d, J = 5.5 Hz, 1H), 4.24 - 4.00 (m, 4H), 3.85 (s, 3H), 3.84 (s, 3H), 3.76 (dt, J = 10.7, 6.3 Hz, 1H), 3.70 - 3.57 (m, 2H), 3.43 (t, J = 5.3 Hz, 2H), 3.37 (d, J = 10.1 Hz, 1H), 2.94 - 2.86 (m, 1H), 2.28 (d, J = 13.7 Hz, 1H), 2.18 - 1.99 (m, 3H), 1.88 (d, J = 12.6 Hz, 1H), 1.77 - 1.51 (m, 7H), 1.49 - 0.98 (m, 11H), 0.92 - 0.80 (m, 1H), 0.79 - 0.68 (m, 1H)ppm.

### Macrocycles m5(2,4)-Diol-I and m5(2,4)-Diol-II

General procedure E was applied to macrocycle **m5(2,4)-(E). Yield: m5(2,4)-Diol-I** (3 mg, 9 %) and **m5(2,3)-Diol-II** (14 mg, 41 %) as white solids. **m5(2,4)-Diol-l: HR-MS** (m/z): (ESI⁺) calculated for: C₃₀H₄₆NO₉ [M+H]⁺: 564.31671, found: 564.31684. **¹H NMR** (500 MHz, CDCl₃): 6.63 (s, 1H), 6.44 (s, 1H), 5.46 - 5.38 (m, 1H), 4.35 - 4.25 (m, 1H), 4.23 - 3.95 (m, 5H), 3.87 (s, 3H), 3.86 (s, 3H), 3.84 - 3.77 (m, 2H), 3.69 - 3.59 (m, 4H), 3.40 (d, J = 10.1 Hz, 1H), 3.01 - 2.93 (m, 1H), 2.29 (d, J = 13.5 Hz, 1H), 2.19 - 1.84 (m, 5H), 1.79 - 1.59 (m, 8H), 1.54 - 1.07 (m, 6H), 0.96 - 0.83 (m, 1H), 0.81 - 0.69 (m, 1H) ppm**.m5(2,4)-Diol-II: HR-MS** (m/z): (ESI⁺) calculated for: C₃₀H₄₆NO₉ [M+H]⁺: 564.31671, found: 564.31749. **¹H NMR** (500 MHz, CDCl₃, 1.3:1 mixture of rotamers, major rotamer reported): 6.69 - 6.58 (m, 1H), 6.43 - 6.38 (m, 1H), 5.45 - 5.30 (m, 1H), 4.31 - 3.94 (m, 6H), 3.87 - 3.81 (m, 6H), 3.79 - 3.41 (m, 7H), 3.41 - 3.32 (m, 1H), 3.02 - 2.79 (m, 1H), 2.32 -2.23 (m, 1H), 2.12 - 1.42 (m, 12H), 1.41 - 1.05 (m, 6H), 0.93 - 0.68 (m, 2H) ppm.

### Macrocycle m5(3,1)-(E)

General procedure D was applied to **m4(3,1). Yield:** 51 mg, 53 %, white solid. **HR-MS** (m/z): (ESI⁺) calculated for: C₂₈H₄₀NO₇ [M+H]⁺: 502.2799, found: 502.2804. **¹H NMR** (500 MHz, CDCl₃): δ 6.58 (d, J = 1.7 Hz, 1H), 6.52 (d, J = 1.8 Hz, 1H), 5.77 (dt, J = 15.6, 6.2 Hz, 1H), 5.66 - 5.59 (m, 1H), 5.46 (d, J = 5.6 Hz, 1H), 4.79 (dd, J = 13.8, 5.2 Hz, 1H), 4.65 (dd, J = 13.8, 7.1 Hz, 1H), 4.50 - 4.43 (m, 1H), 4.10 (d, J = 13.6 Hz, 1H), 4.05 (dd, J = 13.3, 5.4 Hz, 1H), 3.83 (s, 6H), 3.81 - 3.74 (m, 2H), 3.41 (d, J = 10.4 Hz, 1H), 3.24 - 3.05 (m, 3H), 2.30 (dt, J = 13.6, 2.8 Hz, 1H), 2.13 - 2.04 (m, 1H), 1.84 (d, J = 12.2 Hz, 1H), 1.79 - 1.55 (m, 6H), 1.55 - 1.38 (m, 2H), 1.36 - 1.22 (m, 2H), 1.19 - 1.07 (m, 2H), 0.91 - 0.83 (m, 1H), 0.77 - 0.68 (m, 1H) ppm.

### Macrocycle m5(3,1)-H₂

General procedure F was applied to macrocycle **m5(3,1)-(E). Yield:** 2 mg, 16 %. **HR-MS** (m/z): (ESI⁺) calculated for: C₂₈H₄₂NO₇ [M+H]⁺: 504.2956, found: 504.2961. **¹H NMR** (500 MHz, CDCl₃): δ 6.55 (d, J = 1.8 Hz, 1H), 6.49 (d, J = 1.7 Hz, 1H), 5.51 (d, J = 5.4 Hz, 1H), 4.27 (t, J = 5.4 Hz, 2H), 4.20 (ddd, J = 12.1, 7.2, 2.1 Hz, 1H), 4.11 (d, J = 14.0 Hz, 1H), 3.95 - 3.89 (m, 1H), 3.85 (s, 3H), 3.84 (s, 3H), 3.50 - 3.38 (m, 3H), 3.31 - 3.19 (m, 2H), 3.02 (td, J = 13.1, 2.5 Hz, 1H), 2.29 (d, J = 14.0 Hz, 1H), 2.20 - 2.11 (m, 1H), 2.01 - 1.93 (m, J = 5.9, 5.1 Hz, 1H), 1.87 (d, J = 12.5 Hz, 1H), 1.75 - 1.53 (m, 8H), 1.51 - 1.23 (m, 5H), 1.21 - 1.07 (m, 2H), 0.93 - 0.83 (m, 1H), 0.79 - 0.69 (m, 1H) ppm.

### Macrocycles m₅(3,1)-Diol-I and m5(3,1)-Diol-II

General procedure E was applied to macrocycle **m5(3,1)-(E). Yield: m5(3,1)-Diol-I** (6 mg, 27 %) and **m5(3,1)-Diol-II** (8 mg, 40 %) as white solids. **m5(3,1)-Diol-I: HR-MS** (m/z): (ESI⁺) calculated for: C₂₈H₄₂NO₉ [M+H]⁺: 536.2854, found: 536.2858. **¹H NMR** (500 MHz, CDCl₃): 6.80 (d, J = 1.7 Hz, 1H), 6.40 (d, J = 1.8 Hz, 1H), 5.48 - 5.38 (m, 1H), 4.44 - 4.30 (m, 2H), 4.23 - 4.13 (m, 1H), 4.10 - 4.03 (m, 1H), 4.04 - 3.96 (m, 1H), 3.91 (dd, J = 6.6, 3.3 Hz, 1H), 3.86 (s, 3H), 3.84 (s, 3H), 3.66 (q, J = 4.7 Hz, 1H), 3.57 (dd, J = 10.3, 5.3 Hz, 1H), 3.52 - 3.45 (m, 3H), 3.41 (d, J = 10.2 Hz, 1H), 3.22 - 3.02 (m, 1H), 2.29 - 2.20 (m, 1H), 2.16 - 2.08 (m, 1H), 1.82 (d, J = 12.5 Hz, 1H), 1.79 - 1.58 (m, 6H), 1.54 - 1.25 (m, 4H), 1.24 - 1.07 (m, 2H), 0.98 - 0.84 (m, 1H), 0.83 - 0.68 (m, 1H) ppm. **m5(3,1)-Diol-II: HR-MS** (m/z): (ESI⁺) calculated for: C₂₈H₄₂NO₉ [M+H]⁺: 536.2854, found: 536.2858. **¹H NMR** (500 MHz, CDCl₃): 6.71 (d, J = 1.7 Hz, 1H), 6.47 (d, J = 1.7 Hz, 1H), 5.50 (s, 1H), 4.50 (s, 2H), 4.27 - 4.24 (m, 2H), 4.15 - 4.05 (m, 3H), 4.00 (q, J = 5.5 Hz, 1H), 3.88 (s, 3H), 3.86 (s, 3H), 3.83 - 3.79 (m, 1H), 3.58 (dd, J = 9.8, 3.8 Hz, 1H), 3.52 (ddd, J = 11.6, 6.3, 2.0 Hz, 1H), 3.48 - 3.39 (m, 3H), 3.00 - 2.90 (m, 1H), 2.31 (d, J = 13.9 Hz, 1H), 2.16 - 2.05 (m, 1H), 1.86 (d, J = 12.6 Hz, 1H), 1.80 - 1.57 (m, 6H), 1.55 - 1.27 (m, 4H), 1.25 - 1.11 (m, 2H), 0.99 - 0.85 (m, 1H), 0.83 - 0.73 (m, 1H) ppm.

### Macrocycles m5(4,1)-(E) and m5(4,1)-(Z)

General procedure D was applied to **m4(4,1). Yield: m5(4,1)-(E)** (37 mg, 42 %) and **m5(4,1)-(Z)** (9 mg, 10%) as white solids.**Yield: HR-MS** (m/z): (ESI⁺) calculated for: C₂₉H₄₂NO₇ [M+H]⁺: 516.29558, found: 516.29551. **¹H NMR** (500 MHz, CDCl₃, 4:1 mixture of rotamers, major rotamer reported): δ 6.56 (s, 2H), 5.77 (dt, J = 15.5, 6.0 Hz, 1H), 5.57 (dt, J = 15.4, 6.2 Hz, 1H), 5.43 (d, J = 5.4 Hz, 1H), 4.80 (dd, J = 13.9, 4.9 Hz, 1H), 4.61 (dd, J = 13.7, 6.4 Hz, 1H), 4.27 - 4.19 (m, 1H), 4.08 (d, J = 14.1 Hz, 1H), 4.01 (dd, J = 13.6, 5.3 Hz, 1H), 3.95 - 3.86 (m, 2H), 3.85 (s, 3H), 3.82 (s, 3H), 3.34 (d, J = 10.3 Hz, 1H), 2.97 - 2.87 (m, 2H), 2.86 - 2.80 (m, 1H), 2.33 (d, J = 13.5 Hz, 1H), 2.10 - 1.37 (m, 12H), 1.28 (dt, J = 34.1, 13.2 Hz, 2H), 1.19 - 1.04 (m, 2H), 0.91 -0.79 (m, 1H), 0.77 - 0.65 (m, 1H) ppm. **m5(4,1)-(Z): HR-MS** (m/z): (ESI⁺) calculated for: C₂₉H₄₂NO₇ [M+H]⁺: 516.29558, found: 516.29601. **¹H NMR** (500 MHz, CDCl₃, 11:1 mixture of rotamers, major rotamer reported): δ 6.56 (s, 1H), 6.49 (s, 1H), 5.89 (dt, J = 12.7, 7.0 Hz, 1H), 5.66 - 5.52 (m, 1H), 5.38 (d, J = 5.6 Hz, 1H), 4.89 (dd, J = 13.2, 8.0 Hz, 1H), 4.58 (dd, J = 13.2, 5.7 Hz, 1H), 4.47 - 4.28 (m, 1H), 4.27 - 4.21 (m, 1H), 4.00 (dd, J = 13.3, 6.4 Hz, 2H), 3.91 (dd, J = 12.6, 6.0 Hz, 2H), 3.87 (s, 3H), 3.82 (s, 3H), 3.33 (d, J = 9.9 Hz, 1H), 2.95 - 2.88 (m, 1H), 2.80 - 2.66 (m, 2H), 2.30 (d, J = 13.8 Hz, 1H), 2.06 (q, J = 11.2 Hz, 1H), 1.87 (d, J = 12.7 Hz, 1H), 1.75 - 1.23 (m, 11H), 1.15 (p, J = 12.0 Hz, 2H), 0.95 - 0.83 (m, 1H), 0.80 - 0.68 (m, 1H) ppm.

### Macrocycle m5(4,1)-H₂

General procedure G was applied to macrocycle **m5(4,1)-(E) Yield:** 3 mg, 50 %), white solid. **HR-MS** (m/z): (ESI⁺) calculated for: C₂₉H₄₄NO₇ [M+H]⁺: 518.31123, found: 518.31187. **¹H NMR** (500 MHz): δ 6.53 (s, 1H), 6.37 (s, 1H), 5.37 - 5.31 (m, 1H), 4.35 (dt, J = 10.7, 6.7 Hz, 1H), 4.19 (dt, J = 9.7, 7.3 Hz, 1H), 4.12 - 4.04 (m, 1H), 3.97 - 3.89 (m, 2H), 3.85 (s, 3H), 3.84 (s, 3H), 3.54 - 3.49 (m, 1H), 3.45 - 3.27 (m, 4H), 2.96 - 2.84 (m, 1H), 2.28 - 2.20 (m, 1H), 2.13 - 2.01 (m, 1H), 2.00 - 1.54 (m, 12H), 1.52-1.41 (m, 1H), 1.39 - 1.05(m,6H), 1.00 - 0.84 (m, 1H), 0.82 - 0.70 (m, 1H) ppm.

### Macrocycles m5(4,1)-Diol-I and Macrocycle m5(4,1)-Diol-II

General procedure E was applied to macrocycle **m5(4,1)-(E). Yield: m5(4,1)-Diol-I** (9 mg, 50 %) and **m5(4,1)-Diol-II** (2 mg, 11 %) as white solids. **m5(4,1)-Diol-I: HR-MS** (m/z): (ESI⁺) calculated for: C₂₉H₄₄NO₉ [M+H]⁺: 550.30106, found: 550.30114.**¹H NMR** (500 MHz, CDCl₃, 1.7:1 mixture of rotamers, major rotamer reported): 6.60 (d, J = 1.8 Hz, 1H), 6.39 (s, 1H), 5.32 (d, J = 5.7 Hz, 1H), 4.51 - 4.42 (m, 1H), 4.35 - 4.17 (m, 1H), 4.12 - 4.04 (m, 1H), 3.98 - 3.87 (m, 3H), 3.85 (s, 6H), 3.59 (dd, J = 10.3, 3.9 Hz, 1H), 3.56 - 3.51 (m, 1H), 3.48 - 3.41 (m, 1H), 3.40 - 3.37 (m, 2H), 2.91 - 2.83 (m, 1H), 2.31 - 2.19 (m, 1H), 2.09 - 1.98 (m, 1H), 1.89 - 1.73 (m, 3H), 1.73 - 1.60 (m, 6H), 1.54 - 1.41 (m, 1H), 1.40 - 1.04 (m, 5H), 0.96 - 0.85 (m, 1H), 0.84 - 0.71 (m, 1H) ppm. **m5(4,1)-Diol-II**: **HR-MS** (m/z): (ESI⁺) calculated for: C₂₉H₄₄NO₉ [M+H]⁺: 550.30106, found: 550.30107. **¹H NMR** (500 MHz, CDCl₃, 5:1 mixture of rotamers, major rotamer reported): 6.55 (s, 1H), 6.45 (s, 1H), 5.31 -5.28 (m, 1H), 4.35 - 4.27 (m, 2H), 4.25 - 4.19 (m, 1H), 4.06 - 3.99 (m, 1H), 3.98 - 3.89 (m, 3H), 3.88 (s, 3H), 3.87 (s, 3H), 3.64 - 3.36 (m, 4H), 3.33 - 3.25 (m, 1H), 2.84 - 2.77 (m, 1H), 2.29 - 2.21 (m, 1H), 2.15 - 2.01 (m, 1H), 1.92 - 1.85 (m, 1H), 1.84 - 1.62 (m, 8H), 1.47 (dd, J = 14.8, 10.7 Hz, 1H), 1.41 - 1.08 (m, 6H), 1.02 - 0.87 (m, 1H), 0.85 - 0.72 (m, 1H) ppm.

### Macrocycle m5(5,3)-(E)

General procedure D was applied to **m4(5,3). Yield:** 26 mg, 44 %, white solid. **HR-MS** (m/z): (ESI⁺) calculated for: C₃₀H₄₄NO₇ [M+H]⁺: 530.31123, found: 530.31178.**¹H NMR** (500 MHz, CDCl₃, 1.9:1 mixture of rotamers, major rotamer reported): 7.17 - 6.71 (m, 1H), 6.51 - 6.39 (m, 1H), 5.78 - 5.43 (m, 1H), 5.31 - 5.23 (m, 1H), 5.13 - 5.05 (m, 1H), 4.85 - 4.64 (m, 1H), 4.60 - 4.42 (m, 1H), 4.35 - 4.25 (m, 1H), 4.19 - 4.13 (m, 1H), 4.05 - 3.91 (m, 1H), 3.88 - 3.66 (m, 9H), 3.36 (dd, J = 62.3, 9.9 Hz, 1H), 2.96 (td, J = 13.4, 2.4 Hz, 1H), 2.50 - 2.32 (m, 2H), 2.23 (dt, J = 14.4, 4.6 Hz, 1H), 2.17 - 1.92 (m, 1H), 1.86 - 1.43 (m, 8H), 1.41 - 1.23 (m, 4H), 1.23 - 1.03 (m, 2H), 0.95 (d, J = 6.3 Hz, 2H), 0.93 - 0.62 (m, 2H) ppm.

### Macrocycle m5(5,3)-H₂

General procedure G was applied to macrocycle **m5(5,3)-(E). Yield:** 5 mg, 37 %, white solid. **HR-MS** (m/z): (ESI⁺) calculated for: C₃₀H₄₆NO₇ [M+H]⁺: 532.32733, found: 532.32688.**¹H NMR** (500 MHz, CDCl₃, 8:1 mixture of rotamers, major rotamer reported): δ 7.06 - 6.79 (m, 1H), 6.48 - 6.30 (m, 1H), 5.40 (d, J = 5.2 Hz, 1H), 4.84 - 4.75 (m, 1H), 4.66 - 4.45 (m, 1H), 4.13 - 4.04 (m, 1H), 4.06 - 3.93 (m, 2H), 3.84 (d, J = 3.6 Hz, 6H), 3.72 - 3.60 (m, 1H), 3.54 - 3.42 (m, 2H), 3.40 (d, J = 10.1 Hz, 1H), 2.85 (td, J = 13.3, 2.7 Hz, 1H), 2.52 - 2.29 (m, 1H), 2.18 - 2.04 (m, 1H), 1.97 - 1.81 (m, 1H), 1.76 - 1.59 (m, 5H), 1.58 - 1.45 (m, 2H), 1.44 - 1.24 (m, 6H), 1.22 - 1.05 (m, 3H), 0.95 - 0.83 (m, 1H), 0.80 (d, J = 6.3 Hz, 3H), 0.78 - 0.67 (m, 1H) ppm.

### Macrocycle m5(5,4)-(E)/(Z)

General procedure D was applied to **m4(5,4). Yield: m5(5,4)-(E)/(Z)** (34 mg, 47 %, inseparable mixture of isomers, ratio: 1:2) as a white solid. **HR-MS** (m/z): (ESI⁺) calculated for: C₃₁H₄₆NO₇ [M+H]⁺: 544.32688, found: 544.32666. **¹H NMR** (500 MHz, CDCl₃, mixture of rotamers and isomers, major isomer reported): 6.76 (d, J = 1.8 Hz, 1H), 6.53 (d, J = 1.7 Hz, 1H), 5.72 - 5.67 (m, 2H), 5.28 - 5.21 (m, 1H), 4.62 (d, J = 14.2 Hz, 1H), 4.46 - 3.88 (m, 5H), 3.83 (s, 3H), 3.82 (s, 3H), 3.63 - 3.55 (m, 1H), 3.48 - 3.41 (m, 2H), 3.30 (d, J = 10.1 Hz, 1H), 2.48 - 2.25 (m, 2H), 2.25 - 1.83 (m, 5H), 1.78-1.42 (m, 7H), 1.41 - 1.29 (m, 2H), 1.28 (d, J = 6.4 Hz, 3H), 1.25-1.08 (m, 3H), 0.93 - 0.84 (m, 1H), 0.80 - 0.67 (m, 1H) ppm.

### Macrocycle m5(5,4)-H₂

General procedure G was applied to macrocycle **m5(5,4)-(E)/(Z),** 2 h reaction time. **Yield:** 5 mg, 40 %, white solid. **HR-MS** (m/z): (ESI⁺) calculated for: C₃₁H₄₈NO₇ [M+H]⁺: 546.34253, found: 546.34289. **¹H NMR** (500 MHz, CDCl₃,3:1 mixture of rotamers, major rotamer reported): 6.79 (d, J = 1.8 Hz, 1H), 6.31 (d, J = 1.7 Hz, 1H), 5.47 (d, J = 5.4 Hz, 1H), 4.75 (dt, J = 12.9, 6.3 Hz, 1H), 4.21 - 4.14 (m, 2H), 4.07 (d, J = 14.0 Hz, 1H), 4.00 (dt, J = 8.8, 4.4 Hz, 1H), 3.84 (s, 3H), 3.82 (s, 3H), 3.74 (td, J = 9.6, 3.4 Hz, 1H), 3.57 (dd, J = 9.7, 4.8 Hz, 1H), 3.49 (tt, J = 16.1, 5.7 Hz, 2H), 3.36 (d, J = 10.2 Hz, 1H), 3.33 - 3.25 (m, 1H), 2.82 - 2.73 (m, 1H), 2.34 - 2.29 (m, 1H), 2.21 - 1.94 (m, 3H), 1.91 - 1.84 (m, 1H), 1.74 - 1.59 (m, 5H), 1.59 - 1.08 (m, 11H), 0.86 (qd, J = 12.4, 3.4 Hz, 1H), 0.74 (d, J = 6.3 Hz, 4H) ppm.

### Macrocycle m5(6,3)-(E)

General procedure D was applied to **m4(6,3). Yield:** 45 mg, 72 %, white solid. **HR-MS** (m/z): (ESI⁺) calculated for: C₃₀H₄₄NO₇ [M+H]⁺: 530.31123, found: 530.31189.**¹H NMR** (500 MHz, CDCl₃, 3:1 mixture of rotamers, major rotamer reported): 6.67 - 6.57 (m, 1H), 6.56 - 6.51 (m, 1H), 5.78 -5.64 (m, 0H), 5.37 (d, J = 5.6 Hz, 1H), 5.30 (dt, J = 15.6, 4.5 Hz, 1H), 5.22 (dt, J = 15.5, 6.7 Hz, 1H), 5.08 - 4.85 (m, 1H), 4.43 - 4.20 (m, 2H), 4.09 - 3.94 (m, 1H), 3.90 (d, J = 4.4 Hz, 2H), 3.85 - 3.79 (m, 6H), 3.76 - 3.57 (m, 2H), 3.34 (d, J = 10.2 Hz, 1H), 2.96 - 2.69 (m, 1H), 2.52 - 2.20 (m, 2H), 2.16 - 2.00 (m, 1H), 1.99 - 1.92 (m, 1H), 1.87 (d, J = 12.8 Hz, 1H), 1.76 - 1.53 (m, 6H), 1.52 - 1.06 (m, 6H), 0.99 (d, J = 6.4 Hz, 3H), 0.91 - 0.79 (m, 1H), 0.77 - 0.64 (m, 1H) ppm.

### Macrocycle m5(6,3)-H₂

General procedure G was applied to macrocycle **m5(6,3)-(E),** reaction time 16 h. **Yield:** 5 mg, 40 %, white solid. **HR-MS** (m/z): (ESI⁺) calculated for: C₃₀H₄₆NO₇ [M+H]⁺: 532.32688, found: 532.32726.**¹H NMR** (500 MHz, CDCl₃, 3:1 mixture of rotamers, major rotamer reported): 6.80 - 6.50 (m, 2H), 5.33 - 5.30 (m, 1H), 5.06 - 4.84 (m, 1H), 4.37 - 4.19 (m, 2H), 4.00 (d, J = 13.5 Hz, 1H), 3.87 - 3.80 (m, 6H), 3.76 - 3.63 (m, 2H), 3.61 - 3.32 (m, 3H), 2.96 - 2.79 (m, 1H), 2.26 - 1.99 (m, 2H), 1.94 - 1.79 (m, 1H), 1.75 - 1.54 (m, 6H), 1.53 - 1.37 (m, 3H), 1.37 - 1.21 (m, 6H), 1.20 - 0.99 (m, 6H), 0.92 - 0.57 (m, 2H) ppm.

### Macrocycle m5(6,4)-(E)

General procedure D was applied to **m4(6,4). Yield:** 32 mg, 67 %, white solid. **HR-MS** (m/z): (ESI⁺) calculated for: C₃₁H₄₆NO₇ [M+H]⁺: 544.32688, found: 544.32745. **¹H NMR** (500 MHz, CDCl₃, 3:1 mixture of rotamers, major rotamer reported): 6.57 (d, J = 1.8 Hz, 1H), 6.48 (d, J = 1.8 Hz, 1H), 5.43 - 5.36 (m, 1H), 5.36 - 5.25 (m, 2H), 4.82 (ddt, J = 8.8, 6.3, 3.2 Hz, 1H), 4.30 - 4.22 (m, 1H), 4.15 (dt, J = 10.2, 5.3 Hz, 1H), 4.07 (d, J = 13.3 Hz, 1H), 4.00 - 3.92 (m, 1H), 3.84 (s, 3H), 3.80 (s, 3H), 3.62 - 3.54 (m, 2H), 3.37 (d, J = 10.1 Hz, 1H), 3.02 - 2.91 (m, 1H), 2.33 - 2.23 (m, 1H), 2.19 - 2.00 (m, 4H), 1.91 - 1.78 (m, 2H), 1.75 - 1.55 (m, 6H), 1.53 - 1.41 (m, 1H), 1.38 - 1.03 (m, 6H), 0.99 (d, J = 6.3 Hz, 3H), 0.92 - 0.80 (m, 1H), 0.78 - 0.66 (m, 1H) ppm.

### Macrocycle m5(6,4)-H₂

General procedure G was applied to macrocycle **m5(6,4)-(E),** 16 h reaction time. **Yield:** 14 mg, 93 %, white solid. **HR-MS** (m/z): (ESI⁺) calculated for: C₃₁H₄₈NO₇ [M+H]⁺: 546.34253, found: 546.34288.**¹H NMR** (500 MHz, CDCl₃, 2:1 mixture of rotamers, major rotamer reported6.53 (d, J = 1.8 Hz, 1H), 6.46 (d, J = 1.9 Hz, 1H), 5.36 - 5.30 (m, 1H), 4.88 - 4.80 (m, 1H), 4.12 - 4.04 (m, 3H), 3.84 (s, 3H), 3.83 (s, 3H), 3.70 - 3.56 (m, 2H), 3.53 - 3.46 (m, 1H), 3.40 (d, J = 10.2 Hz, 1H), 3.36 - 3.30 (m, 1H), 3.08 - 2.98 (m, 1H), 2.25 (d, J = 13.9 Hz, 1H), 2.16 - 2.01 (m, 3H), 1.88 (d, J = 12.6 Hz, 1H), 1.76 - 1.52 (m, 5H), 1.51 - 1.39 (m, 3H), 1.38 - 1.21 (m, 6H), 1.21 - 1.00 (m, 4H), 0.92 (d, J = 6.3 Hz, 3H), 0.89 - 0.79 (m, 1H), 0.78 - 0.64 (m, 1H) ppm.

### Macrocycle m5(6,5)-(E)

General procedure D was applied to **m4(6,5). Yield:** 14 mg, 52 %, white solid. **HR-MS** (m/z): (ESI⁺) calculated for: C₃₁H₄₆NO₇ [M+H]⁺: 544.32688, found: 544.32685. **¹H NMR** (500 MHz, CDCl₃, 1.8:1 mixture of rotamers, major rotamer reported): 6.63 (d, J = 1.8 Hz, 1H), 6.53 (d, J = 1.8 Hz, 1H), 5.42 - 5.38 (m, 1H), 5.21 (dt, J = 15.5, 4.8 Hz, 1H), 5.10 - 5.01 (m, 1H), 4.85 -4.78 (m, 1H), 4.20 (dd, J = 12.4, 7.3 Hz, 1H), 4.13 (dd, J = 12.4, 3.7 Hz, 1H), 4.10 - 4.04 (m, 1H), 3.98 (ddd, J = 13.6, 5.0, 1.6 Hz, 1H), 3.86 - 3.83 (m, 1H), 3.83 (s, 3H), 3.80 (s, 3H), 3.35 (d, J = 10.2 Hz, 1H), 2.89 - 2.81 (m, 1H), 2.33 - 2.27 (m, 1H), 2.23 - 2.16 (m, 1H), 2.13 - 2.03 (m, 1H), 1.98 - 1.84 (m, 2H), 1.73 - 1.61 (m, 5H), 1.60-1.23 (m, 6H), 1.17 (d, J = 6.3 Hz, 3H), 1.16-1.03 (m, 2H), 0.98 (d, J = 6.3 Hz, 3H), 0.92 - 0.81 (m, 1H), 0.78 - 0.68 (m, 1H) ppm.

### Macrocycle m5(6,6)-(E)

General procedure D was applied to **m4(6,6). Yield:** 18 mg, 56 %, white solid. **HR-MS** (m/z): (ESI⁺) calculated for: C₃₁H₄₆NO₇ [M+H]⁺: 544.32688, found: 544.32660.**¹H NMR** (500 MHz, CDCl₃, 6:1 mixture of rotamers, major rotamer reported): 6.49 (d, J = 1.8 Hz, 1H), 6.46 (d, J = 1.8 Hz, 1H), 5.45 - 5.32 (m, 2H), 5.31 - 5.26 (m, 1H), 4.92 (dtd, J = 13.1, 6.4, 3.1 Hz, 1H), 4.26 - 4.20 (m, 1H), 4.14 - 3.89 (m, 4H), 3.84 (s, 3H), 3.83 (s, 3H), 3.73 - 3.65 (m, 1H), 3.29 (d, J = 10.1 Hz, 1H), 2.93 (td, J = 13.3, 3.0 Hz, 1H), 2.27 - 2.16 (m, 2H), 2.15 - 2.03 (m, 2H), 2.01 - 1.93 (m, 1H), 1.91 - 1.86 (m, 1H), 1.74 - 1.56 (m, 5H), 1.51 - 1.41 (m, 1H), 1.37 - 1.23 (m, 5H), 1.21 (d, J = 6.3 Hz, 3H), 1.10 (d, J = 6.4 Hz, 3H), 0.96 - 0.82 (m, 1H), 0.79 - 0.67 (m, 1H) ppm.

### Macrocycle m5(6,7)-(E)-OH

General procedure D was applied to **m4(6,7),** then general procedure H without prior purification. **Yield:** 9 mg, 21 % over 2 steps, white solid. **HR-MS** (m/z): (ESI⁺) calculated for: C₃₁H₄₆NO₈ [M+H]⁺: 560.32179, found: 560.32224.**¹H NMR** (500 MHz, CDCl₃): 6.60 (d, J = 1.8 Hz, 1H), 6.57 (d, J = 1.9 Hz, 1H), 5.39 - 5.30 (m, 2H), 5.22 - 5.15 (m, 1H), 4.91 - 4.81 (m, 1H), 4.28 (d, J = 5.1 Hz, 2H), 4.06 - 3.97 (m, 3H), 3.84 (s, 3H), 3.80 (s, 3H), 3.80 - 3.75 (m, 1H), 3.74 - 3.67 (m, 2H), 3.35 (d, J = 10.0 Hz, 1H), 2.91 - 2.83 (m, 1H), 2.26 - 2.15 (m, 2H), 2.08 (qd, J = 11.0, 5.4 Hz, 1H), 2.01 - 1.80 (m, 3H), 1.78 - 1.53 (m, 5H), 1.50 - 1.10 (m, 6H), 1.06 (d, J = 6.3 Hz, 3H), 0.92 - 0.83 (m, 1H), 0.79 - 0.69 (m, 1H) ppm.

### Macrocycle m5(6,8)-(E)-OH

General procedure D was applied to **m4(6,8),** then general procedure H without prior purification. **Yield:** 2 mg, 10 % over 2 steps, , mixture of isomers, ratio: 6:1, white solid. **HR-MS** (m/z): (ESI⁺) calculated for: C₃₁H₄₆NO₈ [M+H]⁺: 560.32179, found: 560.32202.**¹H NMR** (500 MHz, CDCl₃, mixture of rotamers, major rotamer reported): 6.54 (d, J = 1.9 Hz, 1H), 6.47 (d, J = 1.8 Hz, 1H), 5.61 - 5.42 (m, 1H), 5.41 - 5.35 (m, 1H), 5.29 - 5.26 (m, 1H), 4.96 - 4.87 (m, 1H), 4.28 (dd, J = 12.3, 5.5 Hz, 1H), 4.19 (dd, J = 12.3, 6.4 Hz, 1H), 4.13 - 3.92 (m, 3H), 3.84 (s, 3H), 3.83 (s, 3H), 3.80 - 3.64 (m, 3H), 3.33 (d, J = 10.3 Hz, 1H), 2.94 (td, J = 13.3, 3.0 Hz, 1H), 2.27 - 2.16 (m, 2H), 2.11 - 1.95 (m, 2H), 1.87 (d, J = 12.5 Hz, 1H), 1.77 - 1.56 (m, 6H), 1.47 (qt, J = 12.8, 4.2 Hz, 1H), 1.39 - 1.22 (m, 3H), 1.13 (t, J = 7.4 Hz, 5H), 0.92 - 0.81 (m, 1H), 0.80 - 0.68 (m, 1H) ppm.

### Macrocycle m5(6,9)-(E)

General procedure D was applied to **m4(6,9). Yield:** 23 mg, 68 %, white solid. **HR-MS** (m/z): (ESI⁺) calculated for: C₃₁H₄₆NO₇ [M+H]⁺: 544.32688, found: 544.32791.**¹H NMR** (500 MHz, CDCl₃, 3:1 mixture of rotamers, major rotamer reported): 5.36 - 5.32 (m, 1H), 5.32 - 5.18 (m, 2H), 4.90 (td, J = 8.4, 4.2 Hz, 1H), 4.76 - 4.66 (m, 1H), 4.04 - 3.88 (m, 2H), 3.86 - 3.76 (m, 7H), 3.60 (dd, J = 11.1, 6.0 Hz, 1H), 3.37 (dd, J = 11.1, 5.4 Hz, 1H), 3.30 (d, J = 10.1 Hz, 1H), 3.00 - 2.87 (m, 1H), 2.30 - 2.19 (m, 2H), 2.09 - 1.92 (m, 2H), 1.85 (d, J = 12.7 Hz, 1H), 1.78 - 1.53 (m, 6H), 1.51 - 1.07 (m, 11H), 1.04 (d, J = 6.3 Hz, 3H), 0.94 - 0.60 (m, 2H) ppm.

### Macrocycle m5(6,10)-(E)

General procedure D was applied to **m4(6,10). Yield:** 26 mg, 62 %, white solid. **HR-MS** (m/z): (ESI⁺) calculated for: C₃₁H₄₆NO₇ [M+H]⁺: 544.32688, found: 544.32759. **¹H NMR** (500 MHz, CDCl₃, 3:1 mixture of rotamers, major rotamer reported): 6.65 (s, 1H), 6.50 (s, 1H), 5.42 (d, J = 5.4 Hz, 1H), 5.32 - 5.25 (m, 1H), 5.15 - 5.00 (m, 1H), 4.90 - 4.82 (m, 1H), 4.71 - 4.62 (m, 1H), 4.16 - 3.75 (m, 10H), 3.63 - 3.52 (m, 2H), 3.35 (d, J = 10.2 Hz, 1H), 2.94 - 2.73 (m, 1H), 2.38 - 2.23 (m, 2H), 2.20 - 1.81 (m, 3H), 1.78 - 1.51 (m, 6H), 1.50 - 1.03 (m, 8H), 0.95 (d, J = 6.4 Hz, 3H), 0.91 - 0.80 (m, 1H), 0.79 - 0.66 (m, 1H) ppm.

### Macrocycle m5(6,11)-(E)

General procedure D was applied to **m4(6,11). Yield:** 22 mg, 59 %, white solid. **HR-MS** (m/z): (ESI⁺) calculated for: C₃₂H₄₈NO₇ [M+H]⁺: 558.34253, found: 558.34285.**¹H NMR** (500 MHz, CDCl₃, 2:1 mixture of rotamers, major rotamer reported): 6.55 (d, J = 1.9 Hz, 1H), 6.47 (d, J = 1.8 Hz, 1H), 5.39 (d, J = 5.6 Hz, 1H), 5.35 - 5.24 (m, 2H), 4.86 - 4.79 (m, 1H), 4.12 (dd, J = 9.7, 4.9 Hz, 1H), 4.06 - 3.92 (m, 2H), 3.92 - 3.85 (m, 1H), 3.83 (s, 3H), 3.81 (s, 3H), 3.77 (dd, J = 13.2, 4.7 Hz, 1H), 3.47 - 3.30 (m, 3H), 2.97 - 2.83 (m, 1H), 2.36 - 1.87 (m, 5H), 1.84 (td, J = 9.1, 4.9 Hz, 1H), 1.75 - 1.39 (m, 6H), 1.36 - 1.09 (m, 6H), 1.07 (d, J = 7.0 Hz, 3H), 1.01 (d, J = 6.4 Hz, 3H), 0.92 - 0.64 (m, 2H) ppm.

### Macrocycle m5(6,12)-(E)

General procedure D was applied to **m4(6,12). Yield:** 19 mg, 49 %, white solid. **HR-MS** (m/z): (ESI⁺) calculated for: C₃₂H₄₈NO₇ [M+H]⁺: 558.34253, found: 558.32496. **¹H NMR** (500 MHz, CDCl₃, 1.6:1 mixture of rotamers, major rotamer reported): 6.58 (d, J = 1.8 Hz, 1H), 6.48 (d, J = 1.8 Hz, 1H), 5.42 - 5.16 (m, 2H), 4.85 - 4.80 (m, 1H), 4.04 (d, J = 13.6 Hz, 2H), 4.02 - 3.86 (m, 2H), 3.85 - 3.81 (m, 2H), 3.83 (s, 3H), 3.80 (s, 4H), 3.67 - 3.47 (m, 2H), 3.38 - 3.33 (m, 1H), 3.00 - 2.91 (m, 1H), 2.49 - 2.18 (m, 2H), 2.17 - 1.94 (m, 3H), 1.90 - 1.78 (m, 1H), 1.74 - 1.52 (m, 5H), 1.50 - 1.39 (m, 1H), 1.37 - 1.21 (m, 3H), 1.20 (d, J = 5.2 Hz, 3H), 1.17 - 1.01 (m, 2H), 0.99 (d, J = 6.4 Hz, 3H), 0.93 - 0.64 (m, 2H) ppm.

### Macrocycle m5(6,13)-(E)

General procedure D was applied to **m4(6,13). Yield:** 13 mg, 38 %, white solid. **HR-MS** (m/z): (ESI⁺) calculated for: C₃₂H₄₈NO₇ [M+H]⁺: 558.34253, found: 558.34314.**¹H NMR** (500 MHz, CDCl₃, 1.2:1 mixture of rotamers, major rotamer reported): 6.57 (d, J = 1.9 Hz, 1H), 6.51 (s, 1H), 5.41 (dt, J = 14.8, 7.1 Hz, 1H), 5.36 - 5.34 (m, 1H), 5.25 (dt, J = 15.4, 5.7 Hz, 1H), 4.86 (tt, J = 7.5, 5.6 Hz, 1H), 4.28 - 4.05 (m, 3H), 4.02 - 3.93 (m, 2H), 3.85 (s, 3H), 3.81 (s, 3H), 3.79 - 3.56 (m, 2H), 3.33 (d, J = 10.0 Hz, 1H), 3.07 - 2.97 (m, 1H), 2.44 (s, 1H), 2.31 (dt, J = 15.3, 7.8 Hz, 1H), 2.23 - 1.91 (m, 5H), 1.89 - 1.76 (m, 1H), 1.72 - 1.51 (m, 6H), 1.50 - 1.32 (m, 2H), 1.20 (d, J = 6.2 Hz, 3H), 1.18 -1.10 (m, 2H), 1.08 (d, J = 6.4 Hz, 3H), 0.91 - 0.78 (m, 1H), 0.77 - 0.63 (m, 1H) ppm.

### Macrocycle m5(6,14)-(E)

General procedure D was applied to **m4(6,14). Yield:** 15 mg, 52 %, mixture of isomers, ratio 5:1) as a white solid. **HR-MS** (m/z): (ESI⁺) calculated for: C₃₂H₄₈NO₇ [M+H]⁺: 558.34253, found: 558.34256.**¹H NMR** (5 00 MHz, CDCl₃, 4:1 mixture of rotamers, major rotamer reported): 6.60 (d, J = 1.8 Hz, 1H), 6.46 (d, J = 1.9 Hz, 1H), 5.38 - 5.23 (m, 3H), 4.90 - 4.77 (m, 1H), 4.27 - 4.21 (m, 1H), 4.14 - 4.03 (m, 2H), 4.03 - 3.98 (m, 1H), 3.84 (s, 3H), 3.80 (s, 2H), 3.80 - 3.70 (m, 2H), 3.38 (d, J = 10.3 Hz, 1H), 2.98 - 2.89 (m, 1H), 2.34 - 2.25 (m, 1H), 2.22 - 1.75 (m, 5H), 1.73 - 1.54 (m, 7H), 1.53 - 1.40 (m, 1H), 1.39 - 1.22 (m, 4H), 1.20 - 1.17 (m, 3H), 1.15 - 1.04 (m, 2H), 0.98 (d, J = 6.3 Hz, 3H), 0.93 - 0.80 (m, 1H), 0.79 - 0.70 (m, 1H) ppm.

### Macrocycle m5(6,15)-(E)-OH

General procedure D was applied to **m4(6,15),** then general procedure H without prior purification. **Yield:** 17 mg, 61 % over two steps, white solid. **HR-MS** (m/z): (ESI⁺) calculated for: C₃₁H₄₆NO₈ [M+H]⁺: 560.32179, found: 560.32146.**¹H NMR** (500 MHz, CDCl₃, 3:1 mixture of rotamers, major rotamer reported): 6.57 (d, J = 1.9 Hz, 2H), 6.53 (d, J = 1.8 Hz, 1H), 5.45 - 5.35 (m, 2H), 5.29 (dt, J = 15.4, 5.0 Hz, 1H), 4.90 - 4.82 (m, 1H), 4.21 - 3.94 (m, 5H), 3.87 (d, J = 5.6 Hz, 1H), 3.84 (s, 3H), 3.81 (s, 3H), 3.64 (dd, J = 10.0, 3.1 Hz, 1H), 3.57 (dd, J = 10.1, 3.9 Hz, 1H), 3.36 (d, J = 9.9 Hz, 1H), 3.00 - 2.92 (m, 1H), 2.33 - 2.05 (m, 2H), 1.99 - 1.84 (m, 4H), 1.73 - 1.53 (m, 5H), 1.50 - 1.39 (m, 1H), 1.36 - 1.07 (m, 4H), 1.03 (d, J = 6.3 Hz, 3H), 0.92 - 0.81 (m, 2H), 0.79 - 0.68 (m, 1H) ppm.

### Macrocycle m5(6,16)-(E)-OH

General procedure D was applied to **m4(6,16),** ), then general procedure H without prior purification. **Yield:**20 mg, 50 % over two steps, white solid. **HR-MS** (m/z): (ESI⁺) calculated for: C₃₁H₄₆NO₈ [M+H]⁺: 560.32179, found: 560.32195. **¹H NMR** (500 MHz, CDCl₃, 3:1 mixture of rotamers, major rotamer reported): 6.54 (s, 1H), 6.53 (d, J = 2.1 Hz, 1H), 5.47 - 5.40 (m, 1H), 5.38 - 5.34 (m, 1H), 5.33 - 5.26 (m, 1H), 4.91 - 4.79 (m, 1H), 4.16 (d, J = 4.7 Hz, 2H), 4.14 - 3.85 (m, 4H), 3.84 (d, J = 1.1 Hz, 3H), 3.82 (d, J = 3.0 Hz, 3H), 3.68 - 3.48 (m, 3H), 3.34 (d, J = 10.0 Hz, 1H), 3.00 - 2.85 (m, 1H), 2.38 - 2.13 (m, 3H), 2.13-2.04 (m, 1H), 2.04 - 1.93 (m, 1H), 1.87 (d, J = 12.6 Hz, 1H), 1.73 - 1.53 (m, 5H), 1.44 (ttd, J = 12.7, 8.6, 8.1, 4.0 Hz, 1H), 1.38 - 1.20 (m, 3H), 1.19-1.08 (m, 2H), 1.08 - 1.04 (m, 3H), 0.95 - 0.61 (m, 2H) (m, 1H) ppm.

### Macrocycle m5(6,17)-(E)

General procedure D was applied to **m4(6,17). Yield:** 19 mg, 74 %, white solid. **HR-MS** (m/z): (ESI⁺) calculated for: C₃₂H₄₈NO₇ [M+H]⁺: 558.34253, found: 558.34225.**¹H NMR** (500 MHz, CDCl₃, 2.2:1 mixture of rotamers, major rotamer reported): 6.63 - 6.62 (m, 1H), 6.49 (s, 1H), 5.78 (dt, J = 14.4, 6.9 Hz, 1H), 5.68 (dt, J = 15.7, 5.3 Hz, 1H), 5.11 - 5.00 (m, 1H), 4.68 (d, J = 5.7 Hz, 1H), 4.63 - 4.49 (m, 2H), 4.02 - 3.95 (m, 1H), 3.91 (dd, J = 12.1, 5.1 Hz, 1H), 3.83 - 3.80 (m, 6H), 3.70 - 3.64 (m, 1H), 3.57 - 3.44 (m, 2H), 2.90 (d, J = 10.0 Hz, 1H), 2.82 (td, J = 13.1, 3.3 Hz, 1H), 2.65 - 2.53 (m, 1H), 2.32 - 2.23 (m, 1H), 2.22 - 1.88 (m, 4H), 1.86 - 1.53 (m, 7H), 1.48 - 1.03 (m, 11H), 0.92 - 0.60 (m, 2H) ppm.

### Macrocycle m5(6,18)-(E)

General procedure D was applied to **m4(6,18) Yield:** 14 mg, 42 %, white solid. **HR-MS** (m/z): (ESI⁺) calculated for: C₃₂H₄₈NO₇ [M+H]⁺: 558.34253, found: 558.34291. **¹H NMR** (500 MHz, CDCl₃, 2:1 mixture of rotamers, major rotamer reported): 6.67 (s, 1H), 6.38 (s, 1H), 5.42 - 5.39 (m, 1H), 5.20 (dt, J = 15.1, 7.2 Hz, 1H), 5.05 (dt, J = 16.0, 4.9 Hz, 1H), 4.95-4.62 (m, 2H), 4.09 (d, J = 14.7 Hz, 1H), 4.04 - 3.97 (m, 1H), 3.84 (s, 3H), 3.79 (s, 3H), 3.78 - 3.67 (m, 2H), 3.46 - 3.30 (m, 3H), 2.89 - 2.79 (m, 1H), 2.33 (d, J = 13.8 Hz, 1H), 2.17 - 1.82 (m, 5H), 1.76 - 1.04 (m, 15H), 1.00 (d, J = 6.2 Hz, 3H), 0.91 - 0.81 (m, 1H), 0.79 - 0.67 (m, 1H) ppm.

### Macrocycle m5(7,3)-(E)-OH

General procedure D was applied to **m4(7,3),** reaction time 2 h. **Yield:** 10 mg, 59 %, white solid. **HR-MS** (m/z): (ESI⁺) calculated for: C₃₀H₄₄NO₈ [M+H]⁺: 546.30614, found: 546.30616.**¹H NMR** (500 MHz, CDCl₃, 4:1 mixture of rotamers, major rotamer reported): 6.78 (d, J = 1.9 Hz, 1H), 6.39 (d, J = 1.9 Hz, 1H), 5.57 - 5.54 (m, 1H), 5.38 - 5.31 (m, 1H), 5.25 - 5.14 (m, 1H), 4.73 - 4.68 (m, 1H), 4.42 (ddd, J = 12.5, 5.5, 2.8 Hz, 1H), 4.26 (ddd, J = 12.7, 6.6, 3.1 Hz, 1H), 4.20 - 3.88 (m, 2H), 3.84 (s, 3H), 3.82 (s, 3H), 3.81 - 3.66 (m, 3H), 3.43 - 3.34 (m, 1H), 3.17 (dd, J = 12.0, 8.3 Hz, 1H), 2.99 - 2.89 (m, 1H), 2.49 - 2.19 (m, 2H), 2.17 - 2.06 (m, 1H), 2.00 - 1.92 (m, 1H), 1.90 - 1.51 (m, 9H), 1.50 - 1.27 (m, 4H), 1.22 - 1.09 (m, 2H), 0.94 - 0.82 (m, 1H), 0.79 - 0.68 (m, 1H) ppm.

### Macrocycle m5(7,4)-(E)-OH

General procedure D was applied to **m4(7,4),** reaction time 2 h. **Yield:** 9 mg, 24 %, white solid. **HR-MS** (m/z): (ESI⁺) calculated for: C₃₁H₄₆NO₈ [M+H]⁺: 560.32179, found: 560.32170. **¹H NMR** (500 MHz, CDCl₃, mixture of rotamers and isomers, major isomer reported): 6.49 (d, J = 1.8 Hz, 0H), 6.47 (d, J = 1.9 Hz, 0H), 5.77 - 5.65 (m, 1H), 5.61 - 5.53 (m, 1H), 5.24 - 5.18 (m, 0H), 4.86 - 4.84 (m, 0H), 4.66 - 4.61 (m, 1H), 4.37 - 4.21 (m, 1H), 4.13 - 4.02 (m, 1H), 4.01 - 3.97 (m, 2H), 3.90 (dd, J = 10.8, 5.6 Hz, 1H), 3.86 - 3.76 (m, 7H), 3.75 - 3.48 (m, 3H), 3.48 - 3.26 (m, 2H), 2.96 - 2.83 (m, 1H), 2.48 - 2.23 (m, 2H), 2.22 - 1.99 (m, 3H), 1.98 - 1.83 (m, 2H), 1.77 - 1.27 (m, 11H), 1.23 - 1.07 (m, 3H), 0.98 - 0.66 (m, 2H) ppm.

### Macrocycle m5(8,3)-(E)-OH

General procedure D was applied to **m4(8,3),** then general procedure I without prior purification and 5 h reaction time. **Yield:** 19 mg, 33%, white solid. **HR-MS** (m/z): (ESI⁺) calculated for: C₃₀H₄₄NO₈ [M+H]⁺: 546.30614, found: 546.30699. **¹H NMR** (500 MHz, CDCl₃, 7:1 mixture of rotamers, major rotamer reported): 6.75 (d, J = 1.9 Hz, 1H), 6.49 (d, J = 1.9 Hz, 1H), 5.30 - 5.24 (m, 1H), 5.22 - 5.20 (m, 1H), 5.14 - 5.05 (m, 1H), 4.93 - 4.85 (m, 1H), 4.40 (qdd, J = 13.1, 5.8, 3.0 Hz, 2H), 4.20 - 4.15 (m, 1H), 3.91 (s, 3H), 3.88 - 3.83 (m, 2H), 3.82 (s, 3H), 3.79 - 3.67 (m, 2H), 3.40 (d, J = 10.4 Hz, 1H), 3.27 (dd, J = 12.2, 3.1 Hz, 1H), 2.92 (ddd, J = 13.7, 9.7, 3.8 Hz, 2H), 2.40 - 2.27 (m, 2H), 2.22 - 2.12 (m, 1H), 2.10 - 2.02 (m, 1H), 1.86 - 1.46 (m, 8H), 1.39 - 1.24 (m, 3H), 1.20 - 1.07 (m, 2H), 0.94 - 0.82 (m, 1H), 0.77 - 0.66 (m, 1H) ppm.

### Macrocycle m5(8,4)-(E)-OH

General procedure D was applied to **m4(8,4),** then general procedure I without prior purification with 2 h reaction time. **Yield:** 20 mg, 35 %, over 2 steps, white solid. **HR-MS** (m/z): (ESI⁺) calculated for: C₃₁H₄₆NO₈ [M+H]⁺: 560.32179, found: 560.32176. **¹H NMR** (500 MHz, CDCl₃, 8:1 mixture of rotamers and isomers, major isomer reported): 6.68 (d, J = 1.7 Hz, 1H), 6.41 (d, J = 1.9 Hz, 1H), 5.31 -5.21 (m, 3H), 4.79 - 4.74 (m, 1H), 4.30-4.20 (m, 2H), 4.15 (dt, J = 9.4, 4.6 Hz, 1H), 3.95 (dd, J = 14.4, 2.3 Hz, 1H), 3.89 (s, 3H), 3.79 (s, 3H), 3.77 - 3.68 (m, 2H), 3.56 (dq, J = 9.1, 4.3 Hz, 1H), 3.46 - 3.41 (m, 2H), 3.06 - 2.86 (m, 2H), 2.38 - 2.33 (m, 1H), 2.27 - 1.85 (m, 5H), 1.85 - 1.56 (m, 7H), 1.51 (ddd, J = 17.4, 8.7, 4.0 Hz, 1H), 1.42 - 1.21 (m, 3H), 1.14 (dddd, J = 15.6, 12.6, 8.2, 5.0 Hz, 2H), 0.92 - 0.83 (m, 1H), 0.73 (qd, J = 12.0, 3.6 Hz, 1H) ppm.

### Macrocycle m5(8,5)-(E)-OH

General procedure D was applied to **m4(8,5),** then general procedure I without prior purification with 7 h reaction time. **Yield:** 17 mg, 35 % over 2 steps, white solid. **HR-MS** (m/z): (ESI⁺) calculated for: C₃₁H₄₆NO₈ [M+H]⁺: 560.3488, found: 560.3211. **¹H NMR** (500 MHz, CDCl₃): 6.72 (d, J = 1.9 Hz, 1H), 6.53 (d, J = 1.9 Hz, 1H), 5.25 (d, J = 5.5 Hz, 1H), 5.21 (dt, J = 15.5, 4.3 Hz, 1H), 5.08 - 4.99 (m, 1H), 4.89 - 4.84 (m, 1H), 4.26 - 4.01 (m, 3H), 3.97 (ddd, J = 13.8, 4.1, 1.8 Hz, 1H), 3.89 (s, 3H), 3.86 - 3.77 (m, 5H), 3.41 (d, J = 10.4 Hz, 1H), 3.34 (dd, J = 12.3, 3.0 Hz, 1H), 2.97 - 2.87 (m, 2H), 2.40 - 2.33 (m, 1H), 2.32 - 2.26 (m, 1H), 2.14 (dtd, J = 14.2, 10.8, 9.2, 3.3 Hz, 1H), 2.09-2.01 (m, 1H), 1.82 (d, J = 12.6 Hz, 1H), 1.77 - 1.48 (m, 6H), 1.41 - 1.24 (m, 3H), 1.23 - 1.06 (m, 6H), 0.93 - 0.83 (m, 1H), 0.78 - 0.67 (m, 1H) ppm.

### Macrocycle m5(8,6)-(E)-OH

General procedure D was applied to **m4(8,6),** then general procedure I without prior purification with 5 h reaction time. **Yield:** 13 mg, 81 % over 2 steps, white solid. **HR-MS** (m/z): (ESI⁺) calculated for: C₃₁H₄₆NO₈ [M+H]⁺: 560.32179, found: 560.32187. **¹H NMR** (500 MHz, CDCl₃): 6.63 (d, J = 1.8 Hz, 1H), 6.36 (d, J = 1.8 Hz, 1H), 5.33 - 5.26 (m, 1H), 5.25 - 5.22 (m, 1H), 5.22 - 5.14 (m, 1H), 4.89 (tt, J = 7.0, 3.3 Hz, 1H), 4.22 (dd, J = 12.5, 6.1 Hz, 1H), 4.14 (dt, J = 13.2, 4.5 Hz, 2H), 4.09 - 4.04 (m, 1H), 3.89 (s, 3H), 3.85 (s, 3H), 3.85 - 3.81 (m, 1H), 3.80 - 3.74 (m, 1H), 3.48 (dd, J = 12.2, 3.1 Hz, 1H), 3.37 (d, J = 10.4 Hz, 1H), 3.14 (dd, J = 12.2, 7.4 Hz, 1H), 2.90 (td, J = 13.2, 2.9 Hz, 1H), 2.38 - 2.32 (m, 1H), 2.28 - 2.22 (m, 1H), 2.15 (qt, J = 11.0, 3.4 Hz, 1H), 2.09 - 2.01 (m, 1H), 1.82 (d, J = 12.6 Hz, 1H), 1.79 - 1.57 (m, 6H), 1.53 (tt, J = 13.0, 4.0 Hz, 1H), 1.40 - 1.22 (m, 4H), 1.21 (d, J = 6.4 Hz, 3H), 1.18 - 1.06 (m, 1H), 0.92 - 0.82 (m, 1H), 0.77 - 0.67 (m, 1H) ppm.

### Macrocycle m5(8,7)-(E)-OH

General procedure D was applied to **m4(8,8),** then general procedure I without prior purification, then general procedure H without prior purification. **Yield:** 23 mg, 35 % over 3 steps, white solid. **HR-MS** (m/z): (ESI⁺) calculated for: C₃₁H₄₆NO₉ [M+H]⁺: 576.31671, found: 576.31622. **¹H NMR** (500 MHz, CDCl₃): 6.66 (d, J = 1.8 Hz, 1H), 6.45 (d, J = 1.8 Hz, 1H), 5.36 (dt, J = 15.6, 5.3 Hz, 1H), 5.32 - 5.25 (m, 1H), 5.24 - 5.22 (m, 1H), 4.94 - 4.86 (m, 1H), 4.36 - 4.27 (m, 2H), 4.17 - 3.97 (m, 3H), 3.92 (s, 3H), 3.87 (s, 3H), 3.79 - 3.70 (m, 3H), 3.48 (dd, J = 12.2, 3.2 Hz, 1H), 3.40 (d, J = 10.4 Hz, 1H), 3.17 (dd, J = 12.2, 7.5 Hz, 1H), 2.91 (td, J = 13.2, 2.6 Hz, 1H), 2.37 - 2.32 (m, 1H), 2.30 - 2.24 (m, 1H), 2.23 - 2.14 (m, 1H), 2.14 - 2.08 (m, 2H), 1.87 - 1.83 (m, 1H), 1.78 - 1.60 (m, 6H), 1.59 - 1.48 (m, 1H), 1.42 - 1.26 (m, 4H), 1.24 - 1.10 (m, 2H), 0.96 - 0.85 (m, 1H), 0.80 - 0.69 (m, 1H) ppm.

### Macrocycle m5(8,8)-(E)-OH

General procedure D was applied to **m4(8,8),** then general procedure I without prior purification, then general procedure H without prior purification. **Yield**:16 mg, 24 % over 3 steps, white solid. **HR-MS** (m/z): (ESI⁺) calculated for: C₃₁H₄₆NO₉ [M+H]⁺: 576.31671, found: 576.31599. **¹H NMR** (500 MHz, CDCl₃): 6.73 (d, J = 1.8 Hz, 1H), 6.53 (d, J = 1.9 Hz, 1H), 5.31 - 5.21 (m, 2H), 5.11 (dt, J = 15.0, 7.1 Hz, 1H), 4.94-4.85 (m, 1H), 4.34 (d, J = 5.4 Hz, 2H), 4.17 (d, J = 13.8 Hz, 1H), 4.05 - 3.93 (m, 2H), 3.90 (s, 3H), 3.87 - 3.82 (m, 1H), 3.81 (s, 3H), 3.80 - 3.76 (m, 1H), 3.74 (p, J = 5.2 Hz, 1H), 3.67 (dd, J = 11.3, 4.8 Hz, 1H), 3.40 (d, J = 10.3 Hz, 1H), 3.34 (dd, J = 12.2, 3.1 Hz, 1H), 3.00 (dd, J = 12.2, 8.2 Hz, 1H), 2.94 - 2.87 (m, 1H), 2.38 - 2.33 (m, 1H), 2.32 - 2.26 (m, 0H), 2.20 - 2.05 (m, 2H), 1.77 - 1.48 (m, 8H), 1.46 - 1.29 (m, 3H), 1.23 - 1.08 (m, 2H), 0.88 (td, J = 12.5, 11.9, 6.3 Hz, 1H), 0.77 - 0.67 (m, 1H) ppm.

### Macrocycle m5(8,9)-(E)-OH

General procedure D was applied to **m4(8,9),** then general procedure I without prior purification with 7 h reaction time. **Yield:** 8 mg, 6 % over 2 steps, white solid **HR-MS** (m/z): (ESI⁺) calculated for: C₃₁H₄₆NO₈ [M+H]⁺: 560.32179, found: 560.32221. **¹H NMR** (500 MHz, CD₃CN): 6.61 (s, 1H), 6.60 (d, J = 1.9 Hz, 1H), 5.40 - 5.35 (m, 2H), 5.15 - 5.09 (m, 1H), 4.89 - 4.80 (m, 1H), 4.57 (h, J = 6.2 Hz, 1H), 4.10 (d, J = 13.7 Hz, 1H), 3.97 - 3.90 (m, 1H), 3.84 - 3.76 (m, 4H), 3.71 (d, J = 1.4 Hz, 3H), 3.59 - 3.52 (m, 1H), 3.49 (d, J = 10.2 Hz, 1H), 3.37 (dd, J = 11.8, 4.1 Hz, 1H), 3.33 - 3.23 (m, 2H), 2.99 - 2.91 (m, 1H), 2.27 - 2.20 (m, 1H), 2.19 - 2.13 (m, 1H), 2.12 - 2.05 (m, 1H), 2.01 (t, J = 10.8 Hz, 1H), 1.80 - 1.74 (m, 1H), 1.72 - 1.55 (m, 6H), 1.51 - 1.40 (m, 1H), 1.33 - 1.08 (m, 8H), 0.98 - 0.87 (m, 1H), 0.84 - 0.73 (m, 1H) ppm.

### Macrocycle m5(8,10)-(E)-OH

General procedure D was applied to **m4(8,10),** then general procedure I without prior purification with 7 h reaction time. **Yield:** 10 mg, 28 % over 2 steps, white solid. **HR-MS** (m/z): (ESI⁺) calculated for: C₃₁H₄₆NO₈ [M+H]⁺: 560.32179, found: 560.32230.**¹H NMR** (500 MHz, CD₃CN): 6.65 (s, 1H), 6.62 (d, J = 1.8 Hz, 1H), 5.27 (dt, J = 15.6, 4.6 Hz, 1H), 5.16 - 5.06 (m, 2H), 4.81 - 4.68 (m, 2H), 4.30 - 4.22 (m, 1H), 3.83 - 3.75 (m, 5H), 3.68 (s, 3H), 3.58 - 3.51 (m, 2H), 3.46 (dd, J = 11.2, 7.1 Hz, 1H), 3.18-3.12 (m, 1H), 2.94-2.78 (m, 2H), 2.28-2.17 (m, 2H), 2.15-2.00 (m, 2H), 1.80 - 1.74 (m, 1H), 1.74 - 1.53 (m, 7H), 1.53 - 1.43 (m, 1H), 1.37 - 1.10 (m, 8H), 1.00 - 0.85 (m, 1H), 0.83 - 0.71 (m, 1H) ppm.

### Macrocycle m5(8,11)-(E)-OH

General procedure D was applied to **m4(8,11),** then general procedure I without prior purification with 5 h reaction time. **Yield:** 14 mg, 26 % over 2 steps, white solid. **HR-MS** (m/z): (ESI⁺) calculated for: C₃₂H₄₈NO₈ [M+H]⁺: 574.33744, found: 574.33700. **¹H NMR** (500 MHz, CDCl₃, 7:1 mixture of rotamers and isomers, major isomer reported): 6.66 (d, J = 1.9 Hz, 1H), 6.40 (d, J = 1.9 Hz, 1H), 5.31 - 5.15 (m, 2H), 4.83 - 4.77 (m, 1H), 4.25 - 4.18 (m, 2H), 3.98 - 3.93 (m, 2H), 3.88 (s, 3H), 3.85 - 3.81 (m, 1H), 3.81 (s, 3H), 3.79 - 3.74 (m, 1H), 3.53 - 3.39 (m, 3H), 3.01 (dd, J = 12.4, 6.6 Hz, 1H), 2.92 (td, J = 13.2, 2.8 Hz, 1H), 2.44 - 1.97 (m, 4H), 1.97 - 1.88 (m, 1H), 1.88 - 1.42 (m, 8H), 1.41 - 1.10 (m, 6H), 1.09 (d, J = 7.0 Hz, 3H), 0.94 - 0.83 (m, 1H), 0.79 - 0.66 (m, 1H) ppm.

### Macrocycle m5(8,12)-(E)-OH

General procedure D was applied to **m4(8,12),** then general procedure I without prior purification with 5 h reaction time. **Yield**:14 mg, 26 % over 2 steps, white solid. **HR-MS** (m/z): (ESI⁺) calculated for: C₃₂H₄₈NO₈ [M+H]⁺: 574.33744, found: 574.33684. **¹H NMR** (500 MHz, CDCl₃, 5:1 mixture of rotamers and isomers, major isomer reported): 6.68 (d, J = 1.8 Hz, 1H), 6.42 (d, J = 1.9 Hz, 1H), 5.28 - 5.22 (m, 3H), 4.79 (ddt, J = 9.9, 6.4, 2.8 Hz, 1H), 4.23 (d, J = 13.2 Hz, 1H), 4.07 (t, J = 9.0 Hz, 1H), 4.03 - 3.68 (m, 9H), 3.44 (td, J = 12.3, 11.5, 3.9 Hz, 2H), 3.36 (dd, J = 9.1, 4.4 Hz, 1H), 3.00 - 2.90 (m, 2H), 2.38 - 2.34 (m, 1H), 2.29 - 2.13 (m, 3H), 1.95 - 1.88 (m, 1H), 1.85 - 1.47 (m, 7H), 1.45 - 1.06 (m, 6H), 1.05 (d, J = 7.0 Hz, 3H), 0.95 - 0.83 (m, 1H), 0.79 - 0.68 (m, 2H) ppm.

### Macrocycle m5(8,13)-(E)-OH

General procedure D was applied to **m4(8,13),** then general procedure I without prior purification with 5 h reaction time. **Yield:** 5 mg, 16 % over 2 steps, white solid. **HR-MS** (m/z): (ESI⁺) calculated for: C₃₂H₄₈NO₈ [M+H]⁺: 574.33744, found: 574.33818. **¹H NMR** (500 MHz, CDCl₃, 4:1 mixture of isomers, major isomer reported): 6.64 (d, J = 2.0 Hz, 1H), 6.48 (d, J = 1.9 Hz, 1H), 5.38 - 5.29 (m, 2H), 5.23 - 5.16 (m, 1H), 4.84 - 4.80 (m, 1H), 4.34 (dt, J = 9.5, 4.5 Hz, 1H), 4.20 - 4.05 (m, 3H), 4.05 - 3.98 (m, 1H), 3.88 (s, 3H), 3.82 (s, 3H), 3.78 - 3.69 (m, 2H), 3.59 (ddd, J = 12.7, 4.9, 1.5 Hz, 1H), 3.53 (dd, J = 12.2, 3.2 Hz, 1H), 3.37 (d, J = 10.3 Hz, 1H), 3.19 (dd, J = 12.2, 6.7 Hz, 1H), 3.00 - 2.92 (m, 2H), 2.32 - 2.28 (m, 1H), 2.23 - 2.18 (m, 1H), 2.14 - 2.05 (m, 3H), 2.01 - 1.93 (m, 2H), 1.86 - 1.57 (m, 9H), 1.55 - 1.47 (m, 1H), 1.41 - 1.27 (m, 4H), 1.21 (d, J = 6.3 Hz, 3H), 1.19 -1.07 (m, 3H), 0.96 - 0.83 (m, 1H), 0.76 - 0.64 (m, 1H) ppm.

### Macrocycle m5(8,14)-(E)-OH

General procedure D was applied to **m4(8,14),** then general procedure I without prior purification with 16 h reaction time. **Yield:** 20 mg, 48 % over 2 steps, white solid. **HR-MS** (m/z): (ESI⁺) calculated for: C₃₂H₄₈NO₈ [M+H]⁺: 574.33744, found: 574.33797. **¹H NMR** (500 MHz, CDCl₃): 6.68 (d, J = 1.8 Hz, 1H), 6.40 (d, J = 1.9 Hz, 1H), 5.28 - 5.21 (m, 3H), 4.80 - 4.72 (m, 1H), 4.29 - 4.18 (m, 2H), 4.12 - 3.99 (m, 2H), 3.88 (s, 3H), 3.87 - 3.80 (m, 2H), 3.79 (s, 3H), 3.52 - 3.45 (m, 2H), 2.98 (dd, J = 12.5, 6.0 Hz, 1H), 2.92 (td, J = 13.1, 2.6 Hz, 1H), 2.40 - 2.35 (m, 1H), 2.26 - 2.15 (m, 1H), 2.13 - 2.02 (m, 2H), 1.87 - 1.47 (m, 10H), 1.46 - 1.07 (m, 8H), 0.94 - 0.83 (m, 1H), 0.80 - 0.67 (m, 1H) ppm.

### Macrocycle m5(8,15)-(E)-OH

General procedure D was applied to **m4(8,15),** then general procedure I without prior purification with 12 h reaction time, then general procedure H without prior purification. **Yield** 26 mg, 40 % over 3 steps, white solid. **HR-MS** (m/z): (ESI⁺) calculated for: C₃₁H₄₆NO₉ [M+H]⁺: 576.31671, found: 576.31657. **¹H NMR** (500 MHz, CDCl₃): 6.71 (d, J = 1.8 Hz, 1H), 6.48 (d, J = 1.9 Hz, 1H), 5.34 - 5.22 (m, 3H), 4.83 - 4.79 (m, 1H), 4.22 - 4.12 (m, 4H), 4.03 - 3.99 (m, 1H), 3.92 (s, 3H), 3.87 - 3.84 (m, 1H), 3.82 (s, 3H), 3.75 (dd, J = 9.9, 2.5 Hz, 1H), 3.59 (dd, J = 9.9, 3.4 Hz, 1H), 3.48 (dd, J = 12.3, 3.0 Hz, 1H), 3.45 (d, J = 10.2 Hz, 1H), 3.07 (dd, J = 12.3, 6.3 Hz, 1H), 2.96 - 2.90 (m, 1H), 2.37 (d, J = 13.7 Hz, 1H), 2.22 - 2.16 (m, 1H), 2.02 - 1.95 (m, 1H), 1.85 (d, J = 13.0 Hz, 1H), 1.81 - 1.43 (m, 8H), 1.41 - 1.26 (m, 4H), 1.26 - 1.08 (m, 3H), 0.96 - 0.88 (m, 1H), 0.80 - 0.71 (m, 1H) ppm.

### Macrocycle m5(8,16)-(E)-OH

General procedure D was applied to **m4(8,16),** then general procedure I without prior purification with 18 h reaction time, then general procedure H without prior purification. **Yield:** 26 mg, 41% over 3 steps, white solid. **HR-MS** (m/z): (ESI⁺) calculated for: C₃₁H₄₆NO₉ [M+H]⁺: 576.31671, found: 576.31671. **¹H NMR** (500 MHz, CDCl₃): 6.66 (d, J = 1.8 Hz, 1H), 6.45 (d, J = 1.9 Hz, 1H), 5.33 - 5.24 (m, 2H), 5.23 - 5.17 (m, 1H), 4.82 - 4.76 (m, 1H), 4.24 (dd, J = 10.4, 3.9 Hz, 1H), 4.18 (dd, J = 10.4, 4.2 Hz, 1H), 4.12 (dd, J = 13.6, 4.6 Hz, 1H), 4.07 - 3.99 (m, 1H), 3.97 - 3.92 (m, 1H), 3.89 (s, 3H), 3.86 - 3.83 (m, 1H), 3.81 (s, 3H), 3.57 - 3.52 (m, 2H), 3.48 (dd, J = 12.3, 3.1 Hz, 1H), 3.40 (d, J = 10.2 Hz, 1H), 3.10 (dd, J = 12.3, 6.3 Hz, 1H), 2.90 (td, J = 13.3, 2.6 Hz, 1H), 2.37 -2.28 (m, 2H), 2.22 -2.10 (m, 2H), 2.06 - 1.98 (m, 1H), 1.83 (d, J = 12.6 Hz, 1H), 1.76 - 1.44 (m, 7H), 1.41 - 1.04 (m, 6H), 0.92 - 0.84 (m, 1H), 0.79 - 0.67 (m, 1H) ppm.

### Macrocycle m5(8,17)-(E)-OH

General procedure D was applied to **m4(8,17),** then general procedure I without prior purification with 18 h reaction time. **Yield:** 20 mg, 61 % over 2 steps, white solid. **HR-MS** (m/z): (ESI⁺) calculated for: C₃₂H₄₈NO₈ [M+H]⁺: 574.33744, found: 574.33787. **¹H NMR** (500 MHz, CDCl₃, 1.1:1 mixture of rotamers, major rotamer reported): 6.69 (s, 1H), 6.53 (s, 1H), 5.77 - 5.63 (m, 1H), 5.41 (dt, J = 14.6, 7.0 Hz, 1H), 5.31 - 5.18 (m, 2H), 4.81 (q, J = 4.6, 3.6 Hz, 1H), 4.46 - 4.37 (m, 2H), 4.12 - 3.99 (m, 2H), 3.77 (s, 3H), 3.67 (s, 3H), 3.59 (td, J = 13.6, 10.7, 7.4 Hz, 2H), 3.51 - 3.43 (m, 4H), 3.38 (tt, J = 12.1, 6.0 Hz, 2H), 2.56 - 2.26 (m, 2H), 2.21 - 2.08 (m, 2H), 1.90 - 1.50 (m, 7H), 1.44 - 1.02 (m, 9H), 0.99 - 0.64 (m, 2H) ppm.

### Macrocycle m5(8,18)-(E)

General procedure D was applied to **m4(8,18)** (51 mg, 0.071 mmol, 1.0 eq.) with Grubbs II catalyst (6 mg, 7 µmol, 0.1 eq.), 1,4-benzoquinone (1 mg, 7 µmol, 0.1 eq.) in DCM (14 mL). Preparative HPLC (65-85 % solvent B) afforded the title compound as a white solid (19 mg, 38 %).

**HPLC** (50 -100 % solvent B, 3.6 min): Rₜ = 1.513 min, purity (220 nm): 99 %
**¹H NMR** (500 MHz, CDCl₃, 3:1 mixture of rotamers, major rotamer reported): 7.21 (d, J = 8.2 Hz, 3H), 6.88 - 6.86 (m, 2H), 6.60 (d, J = 1.7 Hz, 1H), 6.40 (s, 1H), 5.48 - 5.44 (m, 1H), 5.24 (dt, J = 15.0, 7.3 Hz, 1H), 5.10 - 5.02 (m, 1H), 4.88 - 4.80 (m, 1H), 4.70 - 4.64 (m, 1H), 4.39 (s, 2H), 4.10 - 3.96 (m, 2H), 3.80 (s, 3H), 3.79 (s, 3H), 3.73 (s, 3H), 3.68 (td, J = 9.8, 3.7 Hz, 1H), 3.39 - 3.33 (m, 2H), 3.29 (dd, J = 10.1, 5.6 Hz, 1H), 3.19 (dd, J = 10.1, 5.8 Hz, 1H), 2.86 - 2.77 (m, 1H), 2.31 (d, J = 13.7 Hz, 1H), 2.20 - 1.83 (m, 5H), 1.80 - 1.07 (m, 16H), 0.93 - 0.83 (m, 1H), 0.78 - 0.69 (m, 1H) ppm.

**¹³C NMR** (126 MHz, CDCl₃, 3:1 mixture of rotamers, both rotamers reported): δ 173.0, 172.4, 170.7, 170.5, 159.4, 154.3, 154.0, 152.0, 151.3, 137.8, 137.4, 133.9, 133.4, 131.5, 130.4, 130.1, 129.9, 129.5, 129.4, 126.9, 126.7, 113.9, 109.9, 108.8, 103.7, 102.8, 73.1, 73.0, 72.9, 72.6, 72.0, 71.4, 71.0, 70.6, 70.5, 69.8, 66.8, 64.8, 60.9, 60.7, 56.4, 56.2, 56.1, 55.4, 55.4, 55.1, 52.4, 44.5, 41.1, 40.0, 37.1, 36.4, 33.8, 33.7, 33.2, 32.9, 30.6, 26.7, 26.4, 26.3, 26.3, 26.1, 26.0, 24.5, 21.1, 21.0, 20.9, 20.0 ppm.

**HR-MS** (m/z): (ESI⁺) calculated for: C₄₀H₅₆NO₉ [M+H]⁺: 694.39496, found: 694.39550.

### Macrocycle m5(8,18)-(E)-OH

General procedure D was applied to **m4(8,18),** then general procedure I without prior purification with 18 h reaction time. **Yield**:10 mg, 59 % over 2 steps, white solids. **HR-MS** (m/z): (ESI⁺) calculated for: C₃₂H₄₈NO₈ [M+H]⁺: 574.33744, found: 574.33796. **¹H NMR** (500 MHz, CD₃CN): 6.60 (s, 1H), 6.56 (d, J = 1.9 Hz, 1H), 5.24 - 5.13 (m, 2H), 5.12 - 4.99 (m, 1H), 4.66 - 4.54 (m, 2H), 4.27 - 4.19 (m, 1H), 3.91 (dd, J = 15.1, 2.9 Hz, 1H), 3.76 (s, 3H), 3.73 - 3.66 (m, 2H), 3.64 (s, 3H), 3.56 (td, J = 10.0, 4.4 Hz, 2H), 3.33 - 3.26 (m, 2H), 3.07 (dd, J = 11.9, 5.5 Hz, 1H), 2.79 - 2.70 (m, 1H), 2.20 - 2.00 (m, 4H), 1.88 - 1.80 (m, 1H), 1.81 - 1.73 (m, 2H), 1.70 - 1.57 (m, 6H), 1.54 - 1.38 (m, 0H), 1.32 - 1.08 (m, 9H), 0.94 - 0.72 (m, 2H) ppm.

### Macrocycle m5(9,3)-(E)

General procedure D was applied to **m4(9,3). Yield:** 31 mg, 84 %, white solid. **HR-MS** (m/z): (ESI⁺) calculated for: C₄₁H₄₆NO₇ [M+H]⁺: 544.32688, found: 544.32729. **¹H NMR** (500 MHz, CDCl₃, 3:1 mixture of rotamers, major rotamer reported): 6.61 (d, J = 1.8 Hz, 1H), 6.51 (d, J = 1.8 Hz, 1H), 5.35 (d, J = 5.7 Hz, 1H), 5.31 - 5.29 (m, 2H), 4.84 (qd, J = 6.5, 2.9 Hz, 1H), 4.41 (dt, J = 12.7, 5.5 Hz, 1H), 4.33 (dt, J = 12.7, 4.3 Hz, 1H), 4.04 (d, J = 14.2 Hz, 1H), 3.96 - 3.86 (m, 2H), 3.80 (d, J = 1.5 Hz, 6H), 3.74 - 3.69 (m, 2H), 3.32 (d, J = 10.2 Hz, 1H), 2.98 - 2.86 (m, 1H), 2.33 - 2.23 (m, 2H), 2.15 - 1.99 (m, 1H), 1.99 - 1.73 (m, 2H), 1.72 - 1.43 (m, 5H), 1.37 - 0.91 (m, 6H), 0.87 - 0.82 (m, 7H), 0.76 - 0.65 (m, 1H) ppm.

### Macrocycle m5(9,4)-(E)

General procedure D was applied to **m4(9,4)**. **Yield:** 20 mg, 47 %, white solid. **HR-MS** (m/z): (ESI⁺) calculated for: C₃₂H₄₈NO₇ [M+H]⁺: 558.34253, found: 558.34291.**¹H NMR** (500 MHz, CDCl₃, 1.3:1 mixture of rotamers, major rotamer reported): 6.57 (d, J = 1.9 Hz, 1H), 6.49 (d, J = 1.9 Hz, 1H), 5.47 (dd, J = 15.7, 6.9 Hz, 1H), 5.42 - 5.34 (m, 1H), 5.01 - 4.96 (m, 1H), 4.87 (qd, J = 6.6, 2.3 Hz, 1H), 4.31 - 4.22 (m, 3H), 3.83 (s, 3H), 3.81 (s, 3H), 3.64 - 3.56 (m, 2H), 3.36 (d, J = 10.0 Hz, 1H), 3.06 (td, J = 13.3, 2.8 Hz, 1H), 0.94 - 0.79 (m, 7H), 0.77 - 0.65 (m, 1H) ppm.

### Macrocycle m5(9,5)-(E)

General procedure D was applied to **m4(9,5). Yield:** 31 mg, 84 %, white solid. **HR-MS** (m/z): (ESI⁺) calculated for: C₃₂H₄₈NO₇ [M+H]⁺: 544.32688, found: 544.32729. **¹H NMR** (500 MHz, CDCl₃): 6.66 (d, J = 1.8 Hz, 1H), 6.52 (d, J = 1.8 Hz, 1H), 5.40 - 5.36 (m, 1H), 5.30 - 5.24 (m, 1H), 5.22 - 5.15 (m, 1H), 4.78 (qd, J = 6.5, 2.9 Hz, 1H), 4.23 (dd, J = 12.4, 7.4 Hz, 1H), 4.15 - 3.89 (m, 4H), 3.86 - 3.81 (m, 1H), 3.80 (s, 3H), 3.79 (s, 3H), 3.78 - 3.74 (m, 1H), 3.34 (d, J = 10.2 Hz, 1H), 2.94-2.85 (m, 1H), 2.29-2.22 (m, 1H), 2.18-2.06 (m, 1H), 1.87 (d, J = 12.9 Hz, 1H), 1.72 - 1.26 (m, 10H), 1.20 - 1.16 (m, 4H), 1.15 - 1.07 (m, 2H), 0.85 (d, J = 6.6 Hz, 3H), 0.79 (d, J = 7.0 Hz, 3H), 0.75 - 0.66 (m, 1H) ppm.

### Macrocycle m5(9,6)-(E)

General procedure D was applied to **m4(9,6). Yield:** 14 mg, 38 %. **HR-MS** (m/z): (ESI⁺) calculated for: C₃₂H₄₈NO₇ [M+H]⁺: 558.34253, found: 558.34296. **¹H NMR** (500 MHz, CDCl₃): 6.51 -6.47 (m, 2H), 5.47 - 5.40 (m, 1H), 5.33 (dt, J = 15.6, 5.3 Hz, 1H), 5.30 - 5.26 (m, 1H), 4.95 (qd, J = 6.6, 2.5 Hz, 1H), 4.22 (dd, J = 12.1, 5.2 Hz, 1H), 4.05 (dd, J = 12.1, 5.8 Hz, 1H), 4.02 - 3.85 (m, 3H), 3.84 (s, 3H), 3.81 (s, 3H), 3.74 (h, J = 6.1 Hz, 1H), 3.29 (d, J = 10.1 Hz, 1H), 2.95 (td, J = 13.3, 2.8 Hz, 1H), 2.33 - 2.06 (m, 3H), 1.88 (d, J = 12.4 Hz, 1H), 1.73 - 1.56 (m, 6H), 1.46 (tdt, J = 12.9, 8.3, 4.0 Hz, 1H), 1.39 - 1.28 (m, 3H), 1.26 - 1.18 (m, 3H), 1.13 (qd, J = 12.4, 6.4 Hz, 2H), 0.99 (d, J = 6.6 Hz, 3H), 0.89 (d, J = 7.0 Hz, 3H), 0.87 - 0.79 (m, 1H), 0.76 - 0.66 (m, 1H) ppm.

### Macrocycle m5(9,7)-(E)-OH

General procedure D was applied to **m4(9,7),** then general procedure H without prior purification. **Yield:** 20 mg, 38 % over 2 steps, white solid. **HR-MS** (m/z): (ESI⁺) calculated for: C₃₂H₄₈NO₈ [M+H]⁺: 574.33744, found: 574.33721. **¹H NMR** (500 MHz, CDCl₃): 6.53 (d, J = 1.8 Hz, 1H), 6.48 (d, J = 1.8 Hz, 1H), 5.50 (dd, J = 15.5, 8.1 Hz, 1H), 5.37 (ddd, J = 15.4, 7.2, 4.1 Hz, 1H), 5.29 - 5.24 (m, 1H), 4.97 (qd, J = 6.6, 2.3 Hz, 1H), 4.29-4.19 (m, 2H), 4.13 (ddd, J = 13.1, 4.1, 1.4 Hz, 1H), 4.00 - 3.92 (m, 2H), 3.83 (s, 6H), 3.75 - 3.66 (m, 3H), 3.31 (d, J = 10.1 Hz, 1H), 2.94 (td, J = 13.3, 2.9 Hz, 1H), 2.31-2.24 (m, 1H), 2.23 -2.17 (m, 1H), 2.10 (dt, J = 10.9, 3.3 Hz, 1H), 1.88 (d, J = 13.0 Hz, 2H), 1.72 - 1.53 (m, 6H), 1.45 (qt, J = 12.8, 4.3 Hz, 1H), 1.41 - 1.06 (m, 5H), 1.04 (d, J = 6.6 Hz, 3H), 0.91 (d, J = 7.0 Hz, 3H), 0.89 - 0.67 (m, 2H) ppm.

### Macrocycle m5(9,8)-(E)-OH

General procedure D was applied to **m4(9,7),** then general procedure H without prior purification. **Yield:** 21 mg, 44 % over 2 steps, white solid. **HR-MS** (m/z): (ESI⁺) calculated for: C₃₂H₄₈NO₈ [M+H]⁺: 574.33744, found: 574.33768. **¹H NMR** (500 MHz, CDCl₃): 6.66 (d, J = 1.8 Hz, 1H), 6.55 (d, J = 1.9 Hz, 1H), 5.37 (d, J = 5.5 Hz, 1H), 5.34 - 5.28 (m, 1H), 5.23 (dd, J = 15.7, 6.9 Hz, 1H), 4.86 - 4.79 (m, 1H), 4.29 (qd, J = 12.4, 5.4 Hz, 2H), 4.08 - 3.99 (m, 2H), 3.95 (dd, J = 12.8, 5.9 Hz, 1H), 3.81 (d, J = 10.2 Hz, 9H), 3.73 - 3.65 (m, 2H), 3.35 (d, J = 10.1 Hz, 1H), 2.94 - 2.85 (m, 1H), 2.24 (dq, J = 10.9, 3.8, 3.4 Hz, 2H), 2.11 (tdd, J = 10.9, 7.6, 3.5 Hz, 1H), 1.89-1.84 (m, 2H), 1.73 - 1.41 (m, 5H), 1.39 - 1.22 (m, 3H), 1.15 (dtd, J = 26.1, 13.4, 12.1, 3.8 Hz, 3H), 0.93 (d, J = 6.5 Hz, 3H), 0.90 - 0.84 (m, 1H), 0.82 (d, J = 7.0 Hz, 3H), 0.77 - 0.67 (m, 1H) ppm.

### Macrocycle m5(9,9)-(E)

General procedure D was applied to **m4(9,9). Yield:** 19 mg, 61 %, white solid. **HR-MS** (m/z): (ESI⁺) calculated for: C₃₂H₄₈NO₇ [M+H]⁺: 558.34253, found: 558.34305. **¹H NMR** (500 MHz, CDCl₃, 4:1 mixture of rotamers, major rotamer reported): 6.59 (d, J = 1.8 Hz, 1H), 6.49 (d, J = 1.9 Hz, 1H), 5.32 (d, J = 6.1 Hz, 1H), 5.24 (ddd, J = 19.9, 15.4, 5.9 Hz, 2H), 4.88 - 4.82 (m, 1H), 4.78 - 4.70 (m, 1H), 4.02 (d, J = 14.0 Hz, 1H), 3.91 (dd, J = 13.4, 4.5 Hz, 1H), 3.83 (s, 3H), 3.80 (s, 3H), 3.77 - 3.71 (m, 1H), 3.64 - 3.58 (m, 1H), 3.38 (dd, J = 10.7, 4.7 Hz, 1H), 3.29 (d, J = 10.2 Hz, 1H), 3.02 -2.89 (m, 1H), 2.39-2.30 (m, 1H), 2.27 (d, J = 14.1 Hz, 1H), 2.06 (q, J = 10.9 Hz, 1H), 1.85 (d, J = 12.7 Hz, 1H), 1.76 - 1.52 (m, 6H), 1.50 - 1.00 (m, 10H), 0.89 (d, J = 6.6 Hz, 3H), 0.87 - 0.79 (m, 4H), 0.75 - 0.61 (m, 1H) ppm.

### Macrocycle m5(9,10)-(E)

General procedure D was applied to **m4(9,10). Yield:** 22 mg, 54 %, white solid. **HR-MS** (m/z): (ESI⁺) calculated for: C₃₂H₄₈NO₇ [M+H]⁺: 558.34253, found: 558.34317. **¹H NMR** (500 MHz, CDCl₃, 4:1 mixture of rotamers, major rotamer reported): 6.69 (s, 1H), 6.53 (s, 1H), 5.77 - 5.63 (m, 1H), 5.41 (dt, J = 14.6, 7.0 Hz, 1H), 5.31 - 5.18 (m, 2H), 4.81 (q, J = 4.6, 3.6 Hz, 1H), 4.46 - 4.37 (m, 2H), 4.12 - 3.99 (m, 2H), 3.77 (s, 3H), 3.67 (s, 3H), 3.59 (td, J = 13.6, 10.7, 7.4 Hz, 2H), 3.51 - 3.43 (m, 4H), 3.38 (tt, J = 12.1, 6.0 Hz, 2H), 2.56 - 2.26 (m, 2H), 2.21 - 2.08 (m, 2H), 1.90 - 1.50 (m, 7H), 1.44 - 1.02 (m, 9H), 0.99 - 0.64 (m, 2H) ppm.

### Macrocycle m5(9,11)-(E)

General procedure D was applied to **m4(9,11). Yield:** 21 mg, 54 %, white solid. **HR-MS** (m/z): (ESI⁺) calculated for: C₃₃H₅₀NO₇ [M+H]⁺: 572.35818, found: 572.35888. **¹H NMR** (500 MHz, CDCl₃, 1.1:1 mixture of rotamers, major rotamer reported): 6.54 (d, J = 1.8 Hz, 1H), 6.48 (d, J = 1.9 Hz, 1H), 5.79 (ddt, J = 15.5, 7.6, 1.6 Hz, 1H), 5.67 - 5.58 (m, 1H), 5.39 (dt, J = 11.5, 4.5 Hz, 2H), 4.97 (qd, J = 6.6, 3.8 Hz, 1H), 4.05 - 3.99 (m, 4H), 3.93 - 3.86 (m, 3H), 3.84 (s, 3H), 3.81 (s, 3H), 3.80 - 3.74 (m, 1H), 3.51 -3.46 (m, 1H), 3.40 (dd, J = 9.5, 8.2 Hz, 1H), 3.33 (d, J = 10.0 Hz, 1H), 3.02 (td, J = 13.3, 2.8 Hz, 1H), 2.74 - 2.66 (m, 1H), 2.32 - 2.19 (m, 2H), 2.17 - 2.06 (m, 1H), 2.04 - 1.84 (m, 2H), 1.65 (d, J = 40.2 Hz, 3H), 1.51 - 1.22 (m, 3H), 1.21 (d, J = 6.6 Hz, 3H), 1.19 - 1.08 (m, 1H), 1.08 - 1.01 (m, 6H), 0.80 - 0.65 (m, 2H) ppm.

### Macrocycle m5(9,12)-(E)

General procedure D was applied to **m4(9,12). Yield:** 24 mg, 63 %, white solid. **HR-MS** (m/z): (ESI⁺) calculated for: C₃₃H₅₀NO₇ [M+H]⁺: 572.35818, found: 572.35899. **¹H NMR** (500 MHz, CDCl₃, 1.1:1 mixture of rotamers, major rotamer reported): 6.59 (d, J = 1.9 Hz, 1H), 6.47 (d, J = 1.9 Hz, 1H), 5.48 - 5.42 (m, 1H), 5.41 -5.32 (m, 2H), 4.89 - 4.82 (m, 1H), 4.13 - 3.88 (m, 3H), 3.88 - 3.74 (m, 8H), 3.58 - 3.49 (m, 2H), 3.35 (d, J = 10.1 Hz, 1H), 3.04 (td, J = 13.3, 2.7 Hz, 1H), 2.35 - 2.16 (m, 4H), 2.11 (q, J = 11.1 Hz, 2H), 1.60 - 1.01 (m, 15H), 0.96 - 0.78 (m, 5H), 0.77 - 0.64 (m, 2H) ppm.

### Macrocycle m5(9,13)-(E)

General procedure D was applied to **m4(9,13). Yield:** 17 mg, 44 %, white solid. **HR-MS** (m/z): (ESI⁺) calculated for: C₃₃H₅₀NO₇ [M+H]⁺: 572.35818, found: 572.35804. **¹H NMR** (500 MHz, CDCl₃, 1.4:1 mixture of rotamers, major rotamer reported): δ 6.57 (d, J = 1.9 Hz, 1H), 6.51 (d, J = 1.9 Hz, 1H), 5.46 - 5.40 (m, 1H), 5.37 (d, J = 5.5 Hz, 1H), 5.32 (dt, J = 15.5, 5.0 Hz, 1H), 4.84 - 4.79 (m, 1H), 4.32 (ddd, J = 9.5, 5.3, 3.7 Hz, 1H), 4.25 - 3.98 (m, 3H), 3.86 - 3.72 (m, 8H), 3.70 - 3.65 (m, 1H), 3.64 - 3.59 (m, 1H), 3.35 (d, J = 10.1 Hz, 1H), 3.10 - 3.01 (m, 1H), 2.28 - 2.20 (m, 2H), 2.13 - 1.92 (m, 3H), 1.88 - 1.77 (m, 1H), 1.75 - 1.54 (m, 6H), 1.43 (dddd, J = 26.4, 13.1, 8.1, 3.6 Hz, 1H), 1.36 - 0.96 (m, 6H), 0.91 (d, J = 6.6 Hz, 3H), 0.89 - 0.76 (m, 4H), 0.75 - 0.63 (m, 1H) ppm.

### Macrocycle m5(9,14)-(E)

General procedure D was applied to **m4(9,14). Yield:** 20 mg, 57 %, white solid . **HR-MS** (m/z): (ESI⁺) calculated for: C₃₃H₅₀NO₇ [M+H]⁺: 572.35818, found: 572.35861.**¹H NMR** (500 MHz, CDCl₃, 2:1 mixture of rotamers, major rotamer reported): δ 6.58 (d, *J =* 1.8 Hz, 1H), 6.46 (d, *J =* 1.9 Hz, 1H), 5.46 - 5.43 (m, 2H), 5.38 (d, *J =* 5.7 Hz, 1H), 4.95 - 4.89 (m, 1H), 4.31 - 3.91 (m, 6H), 3.83 (s, 3H), 3.81 (s, 3H), 3.77 - 3.65 (m, 2H), 3.38 (d, *J* = 10.2 Hz, 1H), 3.02 (td, J = 13.4, 2.6 Hz, 1H), 2.30 - 2.23 (m, 2H), 2.15 - 1.82 (m, 4H), 1.73 - 1.52 (m, 6H), 1.52 - 1.26 (m, 3H), 1.25 - 0.95 (m, 5H), 0.89 (d, *J* = 6.5 Hz, 3H), 0.87 - 0.80 (m, 4H), 0.80 - 0.68 (m, 1H) ppm.

### Macrocycle m5(9,15)-(E)-OH

General procedure D was applied to **m4(9,15),** then general procedure H without prior purification. **Yield:** 7 mg, 39 % over 2 steps, over 2 steps. **HR-MS** (m/z): (ESI⁺) calculated for: C₃₂H₅₈NO₈ [M+H]⁺: 574.33744, found: 574.33782. **¹H NMR** (500 MHz, CDCl₃, 2.6:1 mixture of rotamers, major rotamer reported): δ 6.58 (d, J = 1.8 Hz, 1H), 6.52 (d, J = 1.9 Hz, 1H), 5.49 (dd, J = 15.7, 7.3 Hz, 1H), 5.41 (t, J = 4.9 Hz, 1H), 5.39 - 5.37 (m, 1H), 4.90 (qd, J = 6.5, 2.2 Hz, 1H), 4.21 (dd, J = 10.1, 5.3 Hz, 1H), 4.17 - 4.08 (m, 2H), 4.05 - 3.87 (m, 3H), 3.84 (s, 3H), 3.81 (s, 3H), 3.60 (t, J = 4.5 Hz, 2H), 3.36 (d, J = 9.9 Hz, 1H), 3.04 (td, J = 13.3, 2.8 Hz, 1H), 2.34 - 2.29 (m, 1H), 2.24 (d, J = 13.9 Hz, 1H), 2.12 (d, J = 11.8 Hz, 1H), 1.86 (d, J = 12.8 Hz, 1H), 1.76 - 1.52 (m, 6H), 1.51 - 1.41 (m, 1H), 1.38 - 1.00 (m, 7H), 0.91 (d, J = 6.6 Hz, 3H), 0.88 (d, J = 7.0 Hz, 2H), 0.86 - 0.79 (m, 1H), 0.73 (dd, J = 12.3, 3.3 Hz, 1H) ppm.

### Macrocycle m5(9,16)-(E)-OH

General procedure D was applied to **m4(9,16),** then general procedure H without prior purification. **Yield**:14 mg, 39 % over 2 steps, white solid. **HR-MS** (m/z): (ESI⁺) calculated for: C₃₂H₅₈NO₈ [M+H]⁺: 574.33744, found: 574.33814. **¹H NMR** (500 MHz, CDCl₃, 1.5:1 mixture of rotamers, major rotamer reported): δ 6.55 - 6.52 (m, 2H), 5.54 - 5.48 (m, 1H), 5.43 - 5.39 (m, 1H), 5.38 - 5.36 (m, 1H), 4.92 (qd, J = 6.5, 2.1 Hz, 1H), 4.17 - 4.09 (m, 3H), 4.07 - 3.98 (m, 1H), 3.97 - 3.93 (m, 1H), 3.90 - 3.86 (m, 1H), 3.83 (s, 3H), 3.82 (s, 3H), 3.67 - 3.58 (m, 2H), 3.35 (d, J = 9.9 Hz, 1H), 3.04 (td, J = 13.4, 2.8 Hz, 1H), 2.30 (tt, J = 7.3, 4.1 Hz, 1H), 2.24 -2.19 (m, 1H), 2.13 - 2.05 (m, 1H), 2.02 - 1.93 (m, 1H), 1.86 (d, J = 12.5 Hz, 1H), 1.74 - 1.53 (m, 6H), 1.45 (tdt, J = 12.9, 7.9, 4.0 Hz, 1H), 1.38 - 1.28 (m, 2H), 1.22 - 1.06 (m, 3H), 0.95 (d, J = 6.6 Hz, 3H), 0.89 (d, J = 7.0 Hz, 3H), 0.88 - 0.62 (m, 2H) ppm.

### Macrocycle m5(9,17)-(E)

General procedure D was applied to **m4(9,17). Yield:** 21 mg, 77 %, white solid. **HR-MS** (m/z): (ESI⁺) calculated for: C₃₃H₅₀NO₇ [M+H]⁺: 572.35818, found: 572.35787. **¹H NMR** (500 MHz, CDCl₃, 3:1 mixture of rotamers, major rotamer reported): δ 6.61 (s, 1H), 6.55 (s, 1H), 5.77 (dd, J = 15.5, 7.9 Hz, 1H), 5.68 - 5.59 (m, 1H), 5.02 - 4.93 (m, 1H), 4.70 (d, J = 5.6 Hz, 1H), 4.62 - 4.44 (m, 3H), 4.08 - 3.96 (m, 1H), 3.91 (dd, J = 11.8, 6.7 Hz, 1H), 3.82 (s, 3H), 3.81 (s, 3H), 3.66 (dt, J = 9.1, 4.2 Hz, 1H), 3.58 - 3.45 (m, 2H), 2.96 - 2.82 (m, 2H), 2.78 - 2.71 (m, 1H), 2.22 - 2.14 (m, 1H), 2.08 (dd, J = 24.6, 12.1 Hz, 2H), 1.98 - 1.87 (m, 1H), 1.86 - 1.74 (m, 1H), 1.74 - 1.54 (m, 6H), 1.49 - 1.21 (m, 7H), 1.19 - 0.98 (m, 6H), 0.80 - 0.60 (m, 2H) ppm.

### Macrocycle m5(9,18)-(E)

General procedure D was applied to **m4(9,18). Yield:** 17 mg, 55 %, white solid. **HR-MS** (m/z): (ESI⁺) calculated for: C₃₃H₅₀NO₇ [M+H]⁺: 572.35818, found: 572.35818. **¹H NMR** (500 MHz, CDCl₃, 2.6:1 mixture of rotamers, major rotamer reported): δ 6.68 (d, J = 1.7 Hz, 1H), 6.39 (d, J = 1.8 Hz, 1H), 5.42 - 5.39 (m, 1H), 5.36 (dd, J = 16.0, 6.2 Hz, 1H), 5.29 (dt, J = 15.8, 4.7 Hz, 1H), 4.76 (qd, J = 6.5, 2.1 Hz, 1H), 4.68 - 4.53 (m, 2H), 4.17 - 4.12 (m, 1H), 4.04 - 3.98 (m, 1H), 3.82 (s, 3H), 3.80 (s, 3H), 3.79 - 3.69 (m, 2H), 3.48 - 3.42 (m, 1H), 3.39 (d, J = 10.3 Hz, 1H), 2.98 - 2.92 (m, 1H), 2.36-2.22 (m, 2H), 2.20 - 1.89 (m, 3H), 1.86 - 0.96 (m, 15H), 0.94 - 0.81 (m, 4H), 0.75 - 0.69 (m, 1H), 0.67 (d, J = 7.0 Hz, 3H) ppm.

### Macrocycle m5(10,3)-(E)

General procedure D was applied to **m4(10,3). Yield:** 22 mg, 61 %, white solid. **HR-MS** (m/z): (ESI⁺) calculated for: C₃₁H₄₆NO₇ [M+H]⁺: 544.32688, found: 544.32725. **¹H NMR** (500 MHz, CDCl₃, 3:1 mixture of rotamers, major rotamer reported): 6.63 (d, J = 1.9 Hz, 1H), 6.52 (d, J = 1.8 Hz, 1H), 5.38 (d, J = 5.7 Hz, 1H), 5.30 - 5.23 (m, 1H), 5.14 (ddt, J = 15.6, 7.8, 1.7 Hz, 1H), 4.80 -4.73 (m, 1H), 4.44 (dt, J = 12.8, 4.6 Hz, 1H), 4.34 (dt, J = 12.8, 4.8 Hz, 1H), 4.10 (d, J = 13.8 Hz, 1H), 3.96 - 3.86 (m, 2H), 3.80 (s, 3H), 3.80 (s, 4H), 3.75 - 3.72 (m, 2H), 3.37 (d, J = 10.2 Hz, 1H), 3.06 - 2.97 (m, 1H), 2.26 (dt, J = 13.7, 2.3 Hz, 1H), 2.13 - 1.90 (m, 3H), 1.84 (d, J = 12.6 Hz, 1H), 1.77 - 1.00 (m, 10H), 0.95 (d, J = 6.4 Hz, 3H), 0.91 - 0.79 (m, 4H), 0.76 - 0.64 (m, 1H) ppm.

### Macrocycle m5(10,4)-(E)

General procedure D was applied to **m4(10,4). Yield:** 16 mg, 38 %, white solid. **HR-MS** (m/z): (ESI⁺) calculated for: C₃₂H₄₈NO₇ [M+H]⁺: 558.34253, found: 558.34299. **¹H NMR** (500 MHz, CDCl₃, 1.3:1 mixture of rotamers, major rotamer reported): δ 6.60 (d, J = 1.9 Hz, 1H), 6.49 (d, J = 2.0 Hz, 1H), 5.72 - 5.58 (m, 1H), 5.41 (d, J = 5.5 Hz, 1H), 5.25 - 5.21 (m, 1H), 4.70 - 4.63 (m, 1H), 4.30 - 4.14 (m, 2H), 4.10 - 4.04 (m, 1H), 4.03 - 3.85 (m, 2H), 3.82 (s, 3H), 3.79 (s, 3H), 3.72 - 3.56 (m, 2H), 3.35 (d, J = 10.2 Hz, 1H), 3.01 - 2.92 (m, 1H), 2.24 (d, J = 13.8 Hz, 1H), 2.15 - 1.82 (m, 5H), 1.79 - 1.41 (m, 7H), 1.37 - 1.05 (m, 5H), 0.99 (d, J = 6.3 Hz, 3H), 0.90 - 0.62 (m, 5H) ppm.

### Macrocycle m5(10,5)-(E)

General procedure D was applied to **m4(10,5). Yield:** 20 mg, 47 %, white solid. **HR-MS** (m/z): (ESI⁺) calculated for: C₃₂H₄₈NO₇ [M+H]⁺: 558.34253, found: 558.34318. **¹H NMR** (500 MHz, CDCl₃, 3:1 mixture of rotamers, major rotamer reported): 6.53 (d, J = 1.8 Hz, 1H), 6.53 - 6.51 (m, 1H), 5.51 - 5.43 (m, 1H), 5.28 - 5.19 (m, 2H), 4.67 - 4.60 (m, 1H), 4.21 - 4.03 (m, 3H), 3.99 - 3.93 (m, 1H), 3.92 - 3.87 (m, 1H), 3.86 (s, 3H), 3.84 (s, 3H), 3.82 - 3.76 (m, 1H), 3.39 (d, J = 9.9 Hz, 1H), 2.97 (td, J = 13.3, 3.1 Hz, 1H), 2.37 - 2.28 (m, 1H), 2.26 - 2.20 (m, 1H), 2.11 - 2.03 (m, 1H), 1.88 (d, J = 12.4 Hz, 1H), 1.72 - 1.50 (m, 6H), 1.47 - 1.37 (m, 1H), 1.36 -1.05 (m, 8H), 1.00 (d, J = 6.4 Hz, 3H), 0.93 - 0.81 (m, 4H), 0.80 - 0.66 (m, 1H) ppm.

### Macrocycle m5(10,6)-(E)

General procedure D was applied to **m4(10,6). Yield:** 20 mg, 42 %, white solid. **HR-MS** (m/z): (ESI⁺) calculated for: C₃₂H₄₈NO₇ [M+H]⁺: 558.34253, found: 558.34325. **¹H NMR** (500 MHz, CDCl₃, 6.6:1 mixture of rotamers, major rotamer reported): 6.53 (d, J = 1.8 Hz, 1H), 6.53 - 6.51 (m, 1H), 5.51 - 5.43 (m, 1H), 5.28 - 5.19 (m, 2H), 4.67 - 4.60 (m, 1H), 4.21 - 4.03 (m, 3H), 3.99 - 3.93 (m, 1H), 3.92 - 3.87 (m, 1H), 3.86 (s, 3H), 3.84 (s, 3H), 3.82 - 3.76 (m, 1H), 3.39 (d, J = 9.9 Hz, 1H), 2.97 (td, J = 13.3, 3.1 Hz, 1H), 2.37 - 2.28 (m, 1H), 2.26 - 2.20 (m, 1H), 2.11 -2.03 (m, 1H), 1.88 (d, J = 12.4 Hz, 1H), 1.72 - 1.50 (m, 6H), 1.47 - 1.37 (m, 1H), 1.36 - 1.05 (m, 8H), 1.00 (d, J = 6.4 Hz, 3H), 0.93 - 0.81 (m, 4H), 0.80 - 0.66 (m, 1H) ppm.

### Macrocycle m5(10,7)-(E)-OH

General procedure D was applied to **m4(9,15),** then general procedure H without prior purification. **Yield:** 25 mg, 52 % over 2 steps, white solid. **HR-MS** (m/z): (ESI⁺) calculated for: C₃₂H₄₈NO₈ [M+H]⁺: 574.33744, found: 574.33809. **¹H NMR** (500 MHz, CDCl₃, 13:1 mixture of rotamers, major rotamer reported): 6.65 (d, J = 1.8 Hz, 1H), 6.43 (d, J = 2.0 Hz, 1H), 5.42 (ddt, J = 15.7, 6.9, 1.4 Hz, 1H), 5.35 - 5.28 (m, 2H), 4.65 (p, J = 6.5 Hz, 1H), 4.32 (dd, J = 12.4, 5.2 Hz, 1H), 4.20 (dd, J = 12.3, 6.0 Hz, 1H), 4.08 - 4.05 (m, 2H), 4.00 (d, J = 13.7 Hz, 1H), 3.82 (s, 3H), 3.81 (s, 3H), 3.77 (dd, J = 11.1, 3.5 Hz, 1H), 3.71 - 3.64 (m, 2H), 3.32 (d, J = 10.1 Hz, 1H), 2.96 (td, J = 13.2, 2.8 Hz, 1H), 2.26 - 2.19 (m, 1H), 2.16 - 1.93 (m, 3H), 1.89 - 1.84 (m, 1H), 1.76 - 1.55 (m, 6H), 1.52 - 1.28 (m, 4H), 1.18 - 1.09 (m, 2H), 1.08 (d, J = 6.3 Hz, 3H), 0.92 - 0.81 (m, 4H), 0.78 - 0.68 (m, 1H) ppm.

### Macrocycle m5(10,8)-(E)-OH

General procedure D was applied to **m4(9,15),** then general procedure H without prior purification. **Yield:** 16 mg, 41 % over 2 steps, white solid. **HR-MS** (m/z): (ESI⁺) calculated for: C₃₂H₄₈NO₈ [M+H]⁺: 574.33744, found: 574.33772. **¹H NMR** (500 MHz, CDCl₃, 11:1 mixture of rotamers, major rotamer reported): 6.65 (d, J = 1.8 Hz, 1H), 6.53 (d, J = 1.8 Hz, 1H), 5.40 - 5.37 (m, 1H), 5.31 (dt, J = 15.7, 5.7 Hz, 1H), 5.16 - 5.10 (m, 1H), 4.75 - 4.68 (m, 1H), 4.40 (dd, J = 12.5, 3.9 Hz, 1H), 4.30 - 4.19 (m, 1H), 4.07 (d, J = 14.1 Hz, 1H), 4.05 - 3.90 (m, 2H), 3.82 (s, 3H), 3.81 (s, 3H), 3.81 - 3.72 (m, 3H), 3.37 (d, J = 10.1 Hz, 1H), 3.02 - 2.94 (m, 1H), 2.31 - 2.22 (m, 1H), 2.10 - 1.98 (m, 2H), 1.83 (d, J = 12.5 Hz, 1H), 1.78 - 1.41 (m, 9H), 1.39 - 1.06 (m, 5H), 1.00 (d, J = 6.3 Hz, 3H), 0.95 - 0.66 (m, 5H) ppm.

### Macrocycle m5(10,9)-(E)

General procedure D was applied to **m4(10,9). Yield:** 26 mg, 56 %, white solid. **HR-MS** (m/z): (ESI⁺) calculated for: C₃₂H₄₈NO₇ [M+H]⁺: 558.34253, found: 558.34296. **¹H NMR** (500 MHz, CDCl₃, 2.4:1 mixture of rotamers, major rotamer reported): 6.73 (d, J = 1.9 Hz, 1H), 6.41 (s, 1H), 5.40 (d, J = 4.7 Hz, 1H), 5.22 (dt, J = 15.6, 4.4 Hz, 1H), 5.02 (dd, J = 15.7, 8.4 Hz, 1H), 4.80 (dt, J = 11.7, 6.0 Hz, 1H), 4.73 - 4.64 (m, 1H), 4.11 (d, J = 14.1 Hz, 1H), 3.96 - 3.91 (m, 1H), 3.87 - 3.77 (m, 7H), 3.56 (dd, J = 11.0, 6.7 Hz, 1H), 3.53 - 3.47 (m, 1H), 3.36 (d, J = 10.2 Hz, 1H), 2.25 (d, J = 11.8 Hz, 1H), 2.11 - 1.99 (m, 2H), 1.82 (d, J = 12.7 Hz, 1H), 1.77 - 1.44 (m, 7H), 1.38 (d, J = 6.4 Hz, 3H), 1.35 - 1.06 (m, 6H), 1.03 (d, J = 6.3 Hz, 3H), 0.94 - 0.81 (m, 1H), 0.77 (d, J = 6.9 Hz, 3H), 0.75 - 0.62 (m, 1H) ppm.

### Macrocycle m5(10,10)-(E)

General procedure D was applied to **m4(10,10). Yield:** 35 mg, 83 %, white solid. **HR-MS** (m/z): (ESI⁺) calculated for: C₃₂H₄₈NO₇ [M+H]⁺: 558.34253, found: 558.34349. **¹H NMR** (500 MHz, CDCl₃, 6.6:1 mixture of rotamers, major rotamer reported): 6.64 (s, 1H), 6.49 (s, 1H), 5.37 (s, 1H), 5.23 (dt, J = 16.3, 4.5 Hz, 1H), 5.15 (dd, J = 15.8, 7.4 Hz, 1H), 4.90 - 4.83 (m, 1H), 4.69 - 4.58 (m, 1H), 4.12 (d, J = 14.0 Hz, 1H), 4.00 (d, J = 5.6 Hz, 1H), 3.93 - 3.73 (m, 6H), 3.71 - 3.47 (m, 2H), 3.36 (d, J = 10.3 Hz, 1H), 3.06-2.99 (m, 1H), 2.41 -2.25 (m, 1H), 2.15 - 1.99 (m, 2H), 1.97 - 1.90 (m, 1H), 1.83 (d, J = 12.6 Hz, 1H), 1.78 - 1.43 (m, 7H), 1.42 - 0.99 (m, 8H), 0.96 - 0.62 (m, 8H) ppm.

### Macrocycle m5(10,11)-(E)

General procedure D was applied to **m4(10,11). Yield:** 18 mg, 40 %, white solid. **HR-MS** (m/z): (ESI⁺) calculated for: C₃₃H₅₀NO₇ [M+H]⁺: 572.35818, found: 572.35886. **¹H NMR** (500 MHz, CDCl₃, 1.3:1 mixture of rotamers, major rotamer reported): δ 6.56 (d, J = 1.8 Hz, 1H), 6.51 (d, J = 2.0 Hz, 1H), 5.41 (d, J = 5.5 Hz, 1H), 5.25 - 5.16 (m, 1H), 4.73 - 4.65 (m, 1H), 4.10 (dd, J = 9.6, 4.6 Hz, 1H), 4.08 - 4.04 (m, 1H), 4.04 - 3.98 (m, 1H), 3.98 - 3.84 (m, 2H), 3.82 (s, 3H), 3.80 (s, 3H), 3.54 (ddd, J = 17.2, 9.3, 4.2 Hz, 1H), 3.45 (dd, J = 9.4, 7.6 Hz, 1H), 3.39 - 3.28 (m, 1H), 3.05 - 2.94 (m, 1H), 2.40 - 2.17 (m, 2H), 2.15 - 2.04 (m, 1H), 2.04 - 1.82 (m, 2H), 1.74 - 1.53 (m, 5H), 1.52 - 1.41 (m, 1H), 1.41 - 1.09 (m, 5H), 1.08 - 1.06 (m, 5H), 0.98 (d, J = 6.3 Hz, 3H), 0.94 - 0.78 (m, 1H), 0.77 (d, J = 7.0 Hz, 3H), 0.74 - 0.62 (m, 1H) ppm.

### Macrocycle m5(10,12)-(E)

General procedure D was applied to **m4(10,12). Yield:** 21 mg, 46 %, white solid. **MS** (m/z): (ESI⁺) calculated for: C₃₃H₅₀NO₇ [M+H]⁺: 572.4, found: 572.4. **¹H NMR** (500 MHz, CDCl₃, 1.1:1 mixture of rotamers, major rotamer reported): δ 6.56 (d, J = 1.8 Hz, 1H), 6.34 (d, J = 1.9 Hz, 1H), 5.42 (dd, J = 4.7, 2.6 Hz, 1H), 5.19 - 5.15 (m, 2H), 4.84 (p, J = 6.3 Hz, 1H), 4.76 (d, J = 5.8 Hz, 1H), 4.58 - 4.51 (m, 1H), 4.14 - 3.99 (m, 1H), 3.82 (s, 3H), 3.79 (s, 3H), 3.56 (ddd, J = 11.2, 9.1, 4.3 Hz, 1H), 3.34 (d, J = 10.2 Hz, 1H), 3.27 (t, J = 9.6 Hz, 1H), 2.40 - 2.21 (m, 2H), 2.15 - 2.04 (m, 1H), 1.98 - 1.81 (m, 2H), 1.77 - 1.44 (m, 7H), 1.39 - 0.95 (m, 13H), 0.91 - 0.77 (m, 2H), 0.70 (d, J = 7.0 Hz, 4H) ppm.

### Macrocycle m5(10,13)-(E)

General procedure D was applied to **m4(10,13). Yield:** 16 mg, 35 %, white solid. **MS** (m/z): (ESI⁺) calculated for: C₃₃H₅₀NO₇ [M+H]⁺: 572.35818, found: 572.35940. **¹H NMR** (500 MHz, CDCl₃, 2.9:1 mixture of rotamers, major rotamer reported): δ 6.61 (d, J = 1.7 Hz, 1H), 6.48 (d, J = 1.9 Hz, 1H), 5.36 (d, J = 5.8 Hz, 1H), 5.27 - 5.17 (m, 2H), 4.73 - 4.65 (m, 1H), 4.28 (td, J = 9.9, 4.0 Hz, 1H), 4.22 - 4.06 (m, 2H), 4.01 (dd, J = 13.2, 3.2 Hz, 1H), 3.87 - 3.75 (m, 8H), 3.40 (d, J = 10.3 Hz, 1H), 3.06 (td, J = 13.4, 2.6 Hz, 1H), 2.32 -2.26 (m, 1H), 2.17 - 1.90 (m, 3H), 1.88 - 1.76 (m, 2H), 1.76 - 1.40 (m, 7H), 1.40 - 1.01 (m, 8H), 0.93 (d, J = 6.3 Hz, 3H), 0.93 - 0.81 (m, 4H), 0.81 - 0.68 (m, 1H) ppm.

### Macrocycle m5(10,14)-(E)

General procedure D was applied to **m4(10,14). Yield:** 31 mg, 67 %. **HR-MS** (m/z): (ESI⁺) calculated for: C₃₃H₅₀NO₇ [M+H]⁺: 572.35818, found: 572.35918. **¹H NMR** (500 MHz, CDCl₃, 1.3:1 mixture of rotamers, major rotamer reported): δ 6.73 (d, J = 1.9 Hz, 1H), 6.40 (d, J = 1.9 Hz, 1H), 5.46 (d, J = 5.1 Hz, 1H), 5.23 - 5.11 (m, 2H), 4.63 - 4.57 (m, 1H), 4.29 - 4.14 (m, 2H), 4.10-3.98 (m, 2H), 3.86 - 3.75 (m, 8H), 3.69 - 3.61 (m, 2H), 3.30 (d, J = 10.2 Hz, 1H), 2.84 (td, J = 13.3, 12.6, 2.2 Hz, 2H), 2.23 (d, J = 13.1 Hz, 1H), 2.14 - 1.90 (m, 4H), 1.89 - 1.77 (m, 1H), 1.75 - 1.42 (m, 7H), 1.38 - 1.00 (m, 6H), 0.92 - 0.61 (m, 4H), 0.57 (d, J = 6.9 Hz, 3H) ppm.

### Macrocycle m5(10,15)-(E)-OH

General procedure D was applied to **m4(9,15),** then general procedure H without prior purification. **Yield:** 25 mg, 54 % over 2 steps, white solid. **HR-MS** (m/z): (ESI⁺) calculated for: C₃₂H₄₈NO₈ [M+H]⁺: 574.33744, found: 574.33823. **¹H NMR** (500 MHz, CDCl₃, 1.6:1 mixture of rotamers, major rotamer reported): 6.61 (d, J = 1.8 Hz, 2H), 6.52 (d, J = 1.8 Hz, 1H), 5.40 (d, J = 5.6 Hz, 1H), 5.30 - 5.18 (m, 2H), 4.71 - 4.67 (m, 1H), 4.23 - 4.12 (m, 2H), 4.09 - 3.87 (m, 3H), 3.83 (s, 4H), 3.80 (s, 3H), 3.75 - 3.57 (m, 2H), 3.37 (d, J = 10.2 Hz, 1H), 3.05 - 2.96 (m, 1H), 2.28 - 2.22 (m, 1H), 2.13 - 1.91 (m, 2H), 1.88 - 1.81 (m, 1H), 1.78 - 1.38 (m, 7H), 1.35 - 1.04 (m, 5H), 1.00 (d, J = 6.3 Hz, 3H), 0.93 - 0.80 (m, 1H), 0.78 (d, J = 6.9 Hz, 3H), 0.76 - 0.59 (m, 1H) ppm.

### Macrocycle m5(10,16)-(E)-OH

General procedure D was applied to **m4(9,15),** then general procedure H without prior purification. **Yield:** 20 mg, 48 % over 2 steps, white solid. **HR-MS** (m/z): (ESI⁺) calculated for: C₃₂H₄₈NO₈ [M+H]⁺: 574.33744, found: 574.33799. **¹H NMR** (500 MHz, CDCl₃, 1.3:1 mixture of rotamers, major rotamer reported): 6.65 - 6.61 (m, 1H), 6.50 (d, J = 1.8 Hz, 1H), 5.40 (d, J = 5.6 Hz, 1H), 5.28 - 5.18 (m, 2H), 4.71 - 4.64 (m, 1H), 4.29 - 4.16 (m, 2H), 4.15 - 4.08 (m, 1H), 4.07 - 4.02 (m, 1H), 3.98 - 3.91 (m, 1H), 3.90 - 3.76 (m, 7H), 3.68 - 3.63 (m, 2H), 3.57 (dd, J = 9.8, 5.8 Hz, 1H), 3.37 (d, J = 10.3 Hz, 1H), 3.03 - 2.99 (m, 1H), 2.25 (d, J = 13.5 Hz, 1H), 2.11 - 1.97 (m, 2H), 1.86 - 1.81 (m, 1H), 1.77 - 1.39 (m, 7H), 1.36 - 1.04 (m, 7H), 1.02 (d, J = 6.3 Hz, 3H), 0.93 - 0.81 (m, 1H), 0.73 - 0.59 (m, 1H) ppm.

### Macrocycle m5(10,17)-(E)

General procedure D was applied to **m4(10,17). Yield:** 15 mg, 40 %, white solid. **HR-MS** (m/z): (ESI⁺) calculated for: C₃₃H₅₀NO₇ [M+H]⁺: 572.35818, found: 572.35852. **¹H NMR** (500 MHz, CDCl₃, 1.3:1 mixture of rotamers, major rotamer reported): δ 6.65 (s, 1H), 6.44 (s, 1H), 5.67 - 5.64 (m, 1H), 5.25 - 5.09 (m, 1H), 4.80 (p, J = 6.5 Hz, 1H), 4.75 - 4.71 (m, 1H), 4.58 (ddt, J = 30.0, 18.9, 5.9 Hz, 2H), 4.08 - 3.91 (m, 2H), 3.85 - 3.78 (m, 6H), 3.74 - 3.62 (m, 2H), 3.56 - 3.46 (m, 1H), 2.96 (t, J = 11.4 Hz, 1H), 2.82 (t, J = 11.8 Hz, 1H), 2.48 - 2.33 (m, 1H), 2.25 - 1.81 (m, 5H), 1.78 - 1.42 (m, 7H), 1.42 - 1.21 (m, 8H), 1.21 - 1.01 (m, 5H), 0.92 - 0.57 (m, 2H) ppm.

### Macrocycle m5(10,18)-(E)

General procedure D was applied to **m4(10,18). Yield:** 14 mg, 41 %, white solid. **HR-MS** (m/z): (ESI⁺) calculated for: C₃₃H₅₀NO₇ [M+H]⁺: 572.35818, found: 572.35921. **¹H NMR** (500 MHz, CDCl₃, 2:1 mixture of rotamers, major rotamer reported): δ 6.66 (d, J = 1.7 Hz, 1H), 6.41 (s, 1H), 5.37 (d, J = 5.6 Hz, 1H), 5.12 (dd, J = 15.8, 8.3 Hz, 1H), 4.95 (dt, J = 15.8, 5.0 Hz, 1H), 4.71 (dt, J = 12.2, 6.0 Hz, 1H), 4.65 - 4.53 (m, 1H), 4.13 - 4.06 (m, 1H), 4.04 - 3.94 (m, 1H), 3.83 (s, 3H), 3.80 (s, 3H), 3.74 - 3.68 (m, 1H), 3.48 - 3.39 (m, 1H), 3.36 (d, J = 10.2 Hz, 1H), 2.97 - 2.89 (m, 1H), 2.37 - 2.30 (m, 1H), 2.15 - 1.82 (m, 4H), 1.80 - 1.51 (m, 6H), 1.51 - 1.42 (m, 1H), 1.40 (d, J = 6.3 Hz, 3H), 1.37 - 1.04 (m, 7H), 0.97 (d, J = 6.2 Hz, 3H), 0.93 - 0.80 (m, 4H), 0.79 - 0.66 (m, 1H) ppm.

### Macrocycle m5(11,4)-(E)-OH

General procedure D was applied to **m4(11,4),** then general procedure J without prior purification. Yield: 9 mg, 23% over 2 steps, white solid. **HR-MS** (m/z): (ESI⁺) calculated for: C₃₁H₄₆NO₈ [M+H]⁺: 560.32179, found: 560.32172. **¹H NMR** (500 MHz, CD₃CN, 2.4:1 mixture of rotamers, major rotamer reported): 6.55 - 6.53 (m, 1H), 6.48 (d, J = 1.9 Hz, 1H), 5.72 - 5.63 (m, 1H), 5.48 - 5.39 (m, 2H), 5.17 - 5.13 (m, 1H), 4.77 - 4.70 (m, 1H), 4.48 - 4.31 (m, 2H), 4.27 - 4.13 (m, 1H), 4.09 - 3.92 (m, 3H), 3.90 - 3.84 (m, 1H), 3.79 (s, 3H), 3.70 (s, 3H), 3.66 - 3.47 (m, 3H), 3.17 - 3.13 (m, 1H), 2.77 (td, J = 13.1, 3.1 Hz, 1H), 2.15 - 1.88 (m, 2H), 1.84 - 1.49 (m, 5H), 1.48 - 1.06 (m, 8H), 1.05 (d, J = 6.5 Hz, 3H), 0.99 - 0.76 (m, 2H) ppm.

### Macrocycle m5(11,5)-(E)-OH

General procedure D was applied to **m4(11,5),** then general procedure J without prior purification. **Yield:** 18 mg, 31 % over 2 steps, white solid. **HR-MS** (m/z): (ESI⁺) calculated for: C₃₁H₄₆NO₈ [M+H]⁺: 560.32179, found: 560.32265.**¹H NMR** (500 MHz, CD₃CN, 10:1 mixture of rotamers, major rotamer reported): 6.68 (d, J = 1.8 Hz, 1H), 6.62 (d, J = 1.8 Hz, 1H), 5.54 - 5.47 (m, 1H), 5.24 - 5.15 (m, 2H), 4.84 (qd, J = 6.5, 5.0 Hz, 1H), 4.24 (dd, J = 12.4, 7.7 Hz, 1H), 4.16 (d, J = 13.5 Hz, 1H), 4.05 (dd, J = 12.4, 3.5 Hz, 1H), 3.98 (ddd, J = 14.2, 4.7, 1.8 Hz, 1H), 3.90 - 3.84 (m, 1H), 3.78 - 3.69 (m, 5H), 3.66 (s, 3H), 3.52 (d, J = 10.2 Hz, 1H), 3.01 (td, J = 13.1, 3.0 Hz, 1H), 2.94 (d, J = 4.4 Hz, 1H), 2.11 - 1.97 (m, 2H), 1.81 - 1.76 (m, 1H), 1.72 - 1.52 (m, 6H), 1.49 - 1.33 (m, 2H), 1.31 - 1.07 (m, 7H), 0.97 - 0.88 (m, 1H), 0.85 - 0.71 (m, 4H) ppm.

### Macrocycle m5(11,6)-(E)-OH

General procedure D was applied to **m4(11,6),** then general procedure J without prior purification. **Yield:** 24 mg, 44 % over 2 steps, white solid. **HR-MS** (m/z): (ESI⁺) calculated for: C₃₁H₄₆NO₈ [M+H]⁺: 560.32179, found: 560.32154. **¹H NMR** (500 MHz, CD₃CN, 7:1 mixture of rotamers, major rotamer reported): 6.60 (d, J = 1.8 Hz, 1H), 6.58 (d, J = 1.8 Hz, 1H), 5.43 (dtd, J = 15.6, 4.9, 1.1 Hz, 1H), 5.32 (ddt, J = 15.7, 6.2, 1.5 Hz, 1H), 5.18 - 5.16 (m, 1H), 4.76 - 4.69 (m, 1H), 4.15 - 4.05 (m, 2H), 4.01 - 3.97 (m, 1H), 3.89 (ddt, J = 14.0, 5.2, 1.2 Hz, 1H), 3.75 (s, 3H), 3.71 (s, 3H), 3.70 - 3.56 (m, 2H), 3.52 (d, J = 10.3 Hz, 1H), 2.92 (td, J = 13.1, 2.9 Hz, 1H), 2.18 - 2.02 (m, 2H), 1.83 - 1.52 (m, 9H), 1.50 - 1.09 (m, 9H), 0.98 - 0.73 (m, 5H) ppm.

### Macrocycle m5(11,8)-(E)-OH

General procedure D was applied to **m4(11,8),** then general procedure J without prior purification. **Yield:** 11 mg, 17 % over 2 steps, white solids. **HR-MS** (m/z): (ESI⁺) calculated for: C₃₁H₄₆NO₉ [M+H]⁺: 576.31671, found: 576.31685. **¹H NMR** (500 MHz, CD₃CN): 6.62 (d, J = 1.8 Hz, 1H), 6.60 (d, J = 1.8 Hz, 1H), 5.46 - 5.39 (m, 1H), 5.39 - 5.33 (m, 1H), 5.19 - 5.16 (m, 1H), 4.70 (p, J = 6.4 Hz, 1H), 4.34 (dd, J = 12.7, 4.1 Hz, 1H), 4.14 (dt, J = 13.6, 4.8 Hz, 2H), 4.04 - 3.99 (m, 1H), 3.98 - 3.92 (m, 1H), 3.75 (s, 3H), 3.71 (s, 3H), 3.68 - 3.55 (m, 4H), 3.50 (d, J = 10.3 Hz, 1H), 2.93 - 2.86 (m, 1H), 2.12 - 2.02 (m, 2H), 1.83 - 1.76 (m, 1H), 1.73 - 1.51 (m, 6H), 1.51 - 1.32 (m, 2H), 1.32 - 1.21 (m, 1H), 1.21 - 1.09 (m, 3H), 1.00 - 0.86 (m, 4H), 0.79 (qd, J = 12.1, 9.5 Hz, 1H) ppm.

### References

(1) Kozany, Christian, März, Andreas; Kress, C. Fluorescent Probes to Characterise FK506-Binding Proteins. *ChemBioChem* 2009, *10,* 1402-1410.
(2) Gnatzy, M. T.; Geiger, T. M.; Kuehn, A.; Gutfreund, N.; Walz, M.; Kolos, J. M.; Hausch, F. Development of NanoBRET-Binding Assays for FKBP-Ligand Profiling in Living Cells. ChemBioChem 2021, 22, 2257-2261.
(3) Markovič, M.; Koóš, P.; Sokoliová, S.; Boháčiková, N.; Vyskočil, T.; Moncol', J.; Gracza, T. A Universal Strategy for Synthesis of Agropyrenol Family. Total Synthesis of Agropyrenol, Sordarial, and Heterocornol A and B 2022, 87, 15947-15962.
(4) Bauder, M.; Meyners, C.; Purder, P. L.; Merz, S.; Sugiarto, W. O.; Voll, A. M.; Heymann, T.; Hausch, F. Structure-Based Design of High-Affinity Macrocyclic FKBP51 Inhibitors. Journal of Medicinal Chemistry 2021, 64, 3320-3349.
(5) van Maarseveen, J. H.-D. Syntheses and biological activity of some tetracyclic eudistomins and analogs : a study of intramolecular Pictet-Spengler condensations; Nijmegen : Universitair Publicatiebureau KUN, 2007.
(6) Buchwald, S.; Pliura, D. H.; Knowles, J. R. Stereochemistry and mechanism of an acid-catalyzed 1,2-phospho group migration: evidence for pseudorotation in the reaction of a phosphoric monoester. Journal of the American Chemical Society 1982, 104, 845-847.

### Example 3: Macrocycles with unsubstituted linkers

Preferred macrocyclic molecular frameworks were identified by exploring different exit vectors, linker lengths and back bone rigidities. Therefore, the first series of macrocycles was synthesized by incorporating unsubstituted linker building blocks (A1-4 and B1-4) in para- or meta-linked macrocycles. These were tested for their affinity to FKBP51 (KD) in a fluorescence polarization assay and compounds with KD < 500 nM were additionally tested in a nanoBRET assay (IC50) to assess their intracellular activity (Table 1,Table 2). Furthermore, for each macrocycle of these two series, the double bond, resulting from ring-closing metathesis ((E) or (Z)), was hydrogenated (H2) or dihydroxylated (diols) to cover linkers with higher flexibility or polarity. The (E) and (Z) isomers as well as the diol diastereomers were usually readily separable after macrocyclization or dihydroxylation, respectively, although usually only the major products could be isolated in useful amounts. Moreover, it could be observed that the NMR spectra after macrocyclization were substantially less complex. This could be attributed in part to the suppression of rotamers.

**Table 1 Summary of binding affinities of para-linked macrocycles for FKBP51, FKBP52, FKBP12 and FKBP12.6 (Kᵢ, determined by fluorescence polarization (FP)) and intracellular inhibitory activities on FKBP51 in a nanoBRET assay (IC₅₀). The curved bond represents one of the linker types shown in Figure 5B. LL= linker length, n.m. = not measured.**

| **Compound** | **LL** | **Linker Type** | **Linker Structure** | **K_{i FKBP51} [nM]** | **K_{i FKBP52} [nM]** | **K_{i FKBP12.6} [nM]** | **K_{i FKBP12} [nM]** | **IC_{50 *nano*BRET} [nM]** |
|---|---|---|---|---|---|---|---|---|
| **p5(1,1)-€** | 4 | (E) | 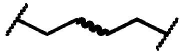 | 1035 | n.b. | n.b. | n.b. | n.m. |
| **p5(1,1)-H₂** | | H₂ | | 229 | n.b. | 4428 | 6643 | 4935 |
| **p5(1,1)-Diol-I^{a}** | | Diol | | 1798 | n.b. | 8848 | n.b. | n.m. |
| **p5(1,1)-Diol-II^{a}** | | Diol | | 544 | n.b. | 4736 | n.b. | n.m. |
| **p5(1,2)-€** | 5 | (E) | 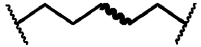 | 247 | n.b. | 2999 | n.b. | 5205 |
| **p5(1,2)-H₂** | | H₂ | | 268 | n.b. | 4787 | 2657 | 2721 |
| **p5(1,2)-Diol-I^{a}** | | Diol | | 772 | n.b. | 5129 | 3035 | n.m. |
| **p5(1,2)-Diol-II^{a}** | | Diol | | 1578 | n.b. | 8116 | n.b. | n.m. |
| **p5(2,1)-€** | | (E) | 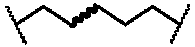 | 227 | n.b. | 1528 | 2036 | 1724 |
| **p5(2,1)-Diol-I^{a}** | | Diol | | 209 | n.b. | 3406 | 6896 | 5310 |
| **p5(2,1)-Diol-II^{a}** | | Diol | | 831 | n.b. | n.b. | n.b. | 11036 |
| **p5(2,2)-(E)** | 6 | (E) | 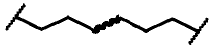 | 117 | n.b. | 2075 | 3507 | 1282 |
| **p5(2,2)-(Z)** | | (Z) | | 143 | n.b. | 2215 | 1364 | 968 |
| **p5(2,2)-H₂** | | H₂ | | 96 | n.b. | 996 | 731 | 1956 |
| **p5(2,2)-Diol-I^{a}** | | Diol | | 1038 | n.b. | n.b. | n.b. | n.m. |
| **p5(2,2) Diol-II^{a}** | | Diol | | 263 | n.b. | 5161 | n.b. | 4716 |
| **p5(2,2) Diol-III^{b}** | | Diol | | 1683 | n.b. | 2621 | n.b. | 6102 |
| **p5(2,2) Diol-IV^{b}** | | Diol | | 474 | n.b. | 6249 | n.b. | 4002 |
| **p5(1,3) -(E)** | 7 | (E) | 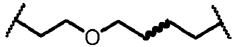 | 764 | n.b. | n.b. | n.b. | n.m. |
| **p5(1,3)-H₂** | | H₂ | | 505 | n.b. | 7851 | 7779 | 6362 |
| **p5(1,3)-Diol-I^{a,c}** | | Diol | | 1256 | n.b. | n.b. | n.b. | n.m. |
| **p5(3, 1) -(E)** | | (E) | 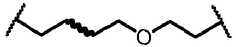 | 386 | n.b. | 2695 | 7393 | 2689 |
| **p5(3,1)-H₂** | | H₂ | | 424 | n.b. | 8676 | 9553 | 4085 |
| **p5(3,1)-Diol-I^{a}** | | Diol | | 386 | n.b. | 7062 | 9420 | 1151 |
| **p5(1,4) -(E)** | 8 | (E) | 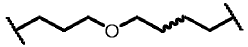 | 402 | n.b. | 5497 | 4810 | 6469 |
| **p5(1,4)-H₂** | | H₂ | | 249 | n.b. | 3795 | 8310 | 3170 |
| **p5(1,4)-Diol-I^{a}** | | Diol | | 878 | n.b. | n.b. | n.b. | n.d. |
| **p5(1,4)-Diol-II^{a}** | | Diol | | 697 | n.b. | 5728 | n.b. | 8558 |
| **p5(3,2) -(E)** | | (E) | 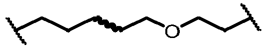 | 1170 | n.b. | n.b. | n.b. | n.d. |
| **p5(3,2)-H₂** | | H₂ | | 337 | n.b. | n.b. | 8993 | 2913 |
| **p5(3,2)-Diol-I^{a}** | | Diol | | 649 | n.b. | n.b. | n.b. | n.d. |
| **p5(3,2)-Diol-II^{a}** | | Diol | | 487 | n.b. | 9205 | n.b. | 4439 |
| **p5(4,1) -(E)** | 8 | (E) | 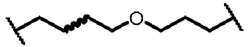 | 172 | n.b. | 1088 | 3427 | 1520 |
| **p5(4,1)-H₂** | | Hz | | 125 | n.b. | 444 | 7262 | 754 |
| **p5(4,1)-Diol-I^{a}** | | Diol | | 172 | n.b. | 2636 | 6043 | 1564 |
| **p5(4,1)-Diol-II^{a}** | | Diol | | 223 | n.b. | 6352 | 7057 | 2210 |

| | | | | | | | | |
|---|---|---|---|---|---|---|---|---|
| ^{a} prepared from (E)-isomer ^{b} prepared from (Z)-isomer; ^{c} mixture of diastereomers; n.b. = no binding (Kᵢ >10 µM); n.m. = not measured, LL= linker length | | | | | | | | |

**Table 2 Summary of binding affinities of meta-linked macrocycles for FKBP51, FKBP52, FKBP12 and FKBP12.6 (Kᵢ, determined by fluorescence polarization (FP)) and intracellular inhibitory activities on FKBP51 in a nanoBRET assay (IC₅₀). The curved bond represents one of the linker types shown in Figure 5B. LL= linker length, n.m. = not measured.**

| **Compound** | **LL** | **Linker-Type** | **Linker Structure** | **K_{i FKBP51}** | **K_{i FKBP52}** | **K_{i FKBP12.6}** | **K_{i FKBP12}** | **IC_{50 nanoBRET}** |
|---|---|---|---|---|---|---|---|---|
| | | | | **[nM]** | **[nM]** | **[nM]** | **[nM]** | **[nM]** |
| **m5(1,1)-(E)** | 4 | (E) | 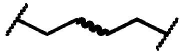 | 4500 | n.b. | 8700 | 3100 | n.m |
| **m5(1,1)-H₂** | | H₂ | | 8500 | n.b. | 3877 | 3500 | n.m |
| **m5(1,1)-Diol^{a}** | | Diol | | n.b. | n.b. | n.b. | n.b. | n.m |
| **m5(1,2)-(E)** | 5 | (E) | 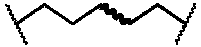 | n.b. | n.b. | n.b. | n.b. | n.m |
| **m5(1,2)-H₂** | | H₂ | | n.b. | n.b. | n.b. | n.b. | n.m |
| **m5(1,2)-Diol-I^{a}** | | Diol | | 3400 | n.b. | n.b. | n.b. | n.m |
| **m5(1,2)-Diol-II^{a}** | | Diol | | 6300 | n.b. | n.b. | n.b. | n.m |
| **m5(2,1)-(E)** | 5 | (E) | 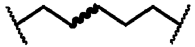 | 3000 | n.b. | n.b. | n.b. | n.m |
| **m5(2,1)-Diol-I^{a}** | | Diol | | n.b. | n.b. | n.b. | n.b. | n.m |
| **m5(2,1)-Diol-II^{a}** | | Diol | | n.b. | n.b. | n.b. | n.b. | n.m |
| **m5(2,2)-(E)** | 6 | (E) | 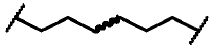 | 532 | n.b. | 4090 | 6501 | n.m |
| **m5(2,2)-(Z)** | | (Z) | | 2500 | n.b. | n.b. | n.b. | n.m |
| **m5(2,2)-H₂** | | H₂ | | 4467 | n.b. | n.b. | n.b. | n.m |
| **m5(2,2)-Diol-I^{a}** | | Diol | | 6744 | n.b. | n.b. | n.b. | n.m |
| **m5(2,2)-Diol-II^{a}** | | Diol | | 2590 | n.b. | n.b. | n.b. | n.m |
| **m5(3,1)-(E)** | 7 | (E) | 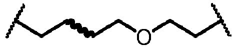 | 634 | n.b. | 7546 | 5585 | n.m |
| **m5(3,1)-H₂** | | H₂ | | 1531 | n.b. | n.b. | 7064 | n.m |
| **m5(3,1)-Diol-I^{a}** | | Diol | | 6182 | n.b. | n.b. | n.b. | 5334 |
| **m5(3,1)-Diol-II^{a}** | | Diol | | 3132 | n.b. | n.b. | n.b. | n.m |
| **m5(1,3)-(E)** | | (E) | 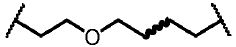 | 1218 | n.b. | 6487 | 5063 | n.m |
| **m5(1,3)-H₂** | | H₂ | | 216 | n.b. | 3856 | 2331 | 3127 |
| **m5(1,3)-Diol-I^{a,b}** | | Diol | | 850 | n.b. | n.b. | n.b. | 5334 |
| **m5(2,3)-(E)** | 8 | (E) | 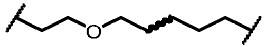 | 86 | n.b. | 454 | 398 | 806 |
| **m5(2,3)-(Z)** | | (Z) | | 198 | n.b. | 624 | 804 | 2110 |
| **m5(2,3)-H₂** | | H₂ | | 123 | n.b. | 649 | 1851 | 4000 |
| **m5(2,3)-Diol-I^{a}** | | Diol | | 1377 | n.b. | 4477 | 5819 | n.m |
| **m5(2,3)-Diol-II^{a}** | | Diol | | 2701 | n.b. | 4877 | 5400 | n.m |
| **m5(1,4)-(E)** | | (E) | 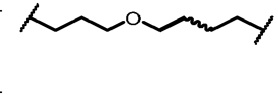 | 1421 | n.b. | 7307 | 7674 | n.m |
| **m5(1,4)-H₂** | | H₂ | | 452 | n.b. | 3248 | 3253 | 6429 |
| **m5(1,4)-Diol-I^{a}** | | Diol | | 979 | n.b. | 5631 | 9846 | n.m |
| **m5(1,4)-Diol-II^{a}** | | Diol | | 2763 | n.b. | n.b. | n.b. | n.m |
| **m5(4,1)-(E)** | | (E) | 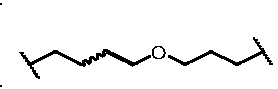 | 396 | n.b. | 2636 | 2258 | 3806 |
| **m5(4,1)-(Z)** | | (Z) | | 497 | n.b. | 1967 | 2508 | 6576 |
| **m5(4,1)-H₂** | | H₂ | | 607 | n.b. | 3889 | 4188 | n.m |
| **m5(4,1)-Diol-I^{a}** | | Diol | | 1377 | n.b. | 8534 | 8113 | n.m |
| **m5(4,1)-Diol-II^{a}** | | Diol | | 2701 | n.b. | n.b. | n.b. | n.m |
| **m5(2,4)-(E)** | 9 | (E) | 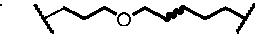 | 102 | n.b. | 625 | 630 | 1029 |
| **m5(2,4)-H₂** | | H₂ | | 84 | n.b. | 782 | 702 | 1700 |
| **m5(2,4)-Diol-I^{a}** | | Diol | | 607 | n.b. | 3165 | 4413 | n.m |
| **m5(2,4)-Diol-II^{a}** | | Diol | | 673 | n.b. | 3703 | 5199 | n.m |

| | | | | | | | | |
|---|---|---|---|---|---|---|---|---|
| ^{a}prepared from (E)-isomer; ^{b} mixture of diastereomers; n.b. = no binding (Kᵢ >10 µM); n.m. = not measured, LL= linker length | | | | | | | | |

### Example 3a: para-series

For the para-linked macrocycles, linker lengths between 4 and 8 atoms were. The 7- and 8-atom linkers contained an oxygen as a heteroatom at different positions in the linker. All para-linked macrocycles had better binding affinity compared to the linear starting point 6 with a 6-30-fold enhancement for the hydrogenated linkers. This did not depend on linker length and only marginally on the position of the oxygen (Table 1).

Most unsaturated linkers also bound rather well, with the exception of p5(3,2)-(E) and p5(1,1)-(E). Overall, for para-linked macrocycles a simple clamp between the pipecolate carbonyl and top of the phenyl ring seems to be sufficient to robustly provide an approximately ten-fold affinity increase with little additional effects.

### Example 3b: meta-series

To compare the influence of the exit vector, a similar set of meta-linked macrocycles was synthesized with linker lengths ranging between 4 and 9 atoms. Contrary to the para-series, the meta-series was strongly affected by the linker length (Table 2). Linkers with ≤ 6 atoms decreased affinity, likely because these shorter linkers compromise the active conformation. Similar to the para-series dihydroxylation decreased affinity while rigidification via a double bond usually improved affinity.

The most remarkable results within the meta-series were observed for linker lengths 8 and 9. The best macrocycle m5(2,3)-(E) has a Ki<100nM, corresponding to a 20-fold increased affinity compared to starting compound 6. This highly context-dependent since shifting the double bond and the ether oxygen by one position as in m5(1,4)-(E) reduced affinity. The precise positioning of the linker oxygen partially contributed to this, as revealed by the comparison of the hydrogenated analogs m5(2,3)-H2, m5(1,4)-H2,and m5(4,1)-H2, which only differ in the position of the oxygen. The beneficial effect of the m5(2,3)-(E)/H2-scaffold was tolerant to minor linker expansion as revealed by the larger m(2,4)-(E)/H2 analogs.

In summary, for the meta-series, there is a much stronger dependency of binding affinity on the length and composition of the linker. There is a clear dependence of binding affinity and linker length with a maximum in linker lengths 8 and 9. In addition, the precise position of the double bond and oxygen is equally important for optimal binding affinity (Table 2).

### Example 4: Advanced Linker Design

Co-crystal structures of several meta- and para-linked macrocycles revealed that for the para series the linker protrudes into the solvent while for the meta series it threads along the protein surface. Based on this observation, the linker in the meta-series was prioritized for subsequent modifications because it offered more possibilities for new protein interactions. Due to promising binding affinities of m5(2,3)-(E) and m5(2,4)-(E) were selected as new chemical templates for further optimization.

We first substituted position 1 of the linker with either a methyl or a hydroxymethyl functional group, each in both stereo configurations (Table 2: m5(5,3)-(E), m5(6,3)-(E), m5(5,4)-(E), m5(6,4)-(E), m5(7,3)-(E)-OH, m5(8,3)-(E)-OH, m5(7,4)-(E)-OH, m5(8,4)-(E)-OH).

While both residues were found to be compatible as R1' in both scaffolds, neither was viable as R1. This observations is consistent with the co-crystal structure of the unsubstituted derivative m5(2,3)-(E). While substitution in R1 was predicted to clash with the protein, R1' rather protrudes out of the binding pocket and places the CH₂OH group in an orientation that could allow the formation of a hydrogen bond to Tyr113.

To further elaborate the linker, we systematically synthesized a collection of 68 macrocycles featuring diverse linker derivatizations. This involved the inclusion of hydroxyl (OH) and methylene hydroxyl (CH₂OH) moieties as polar substituents and methyl groups as nonpolar residues (Table 3).

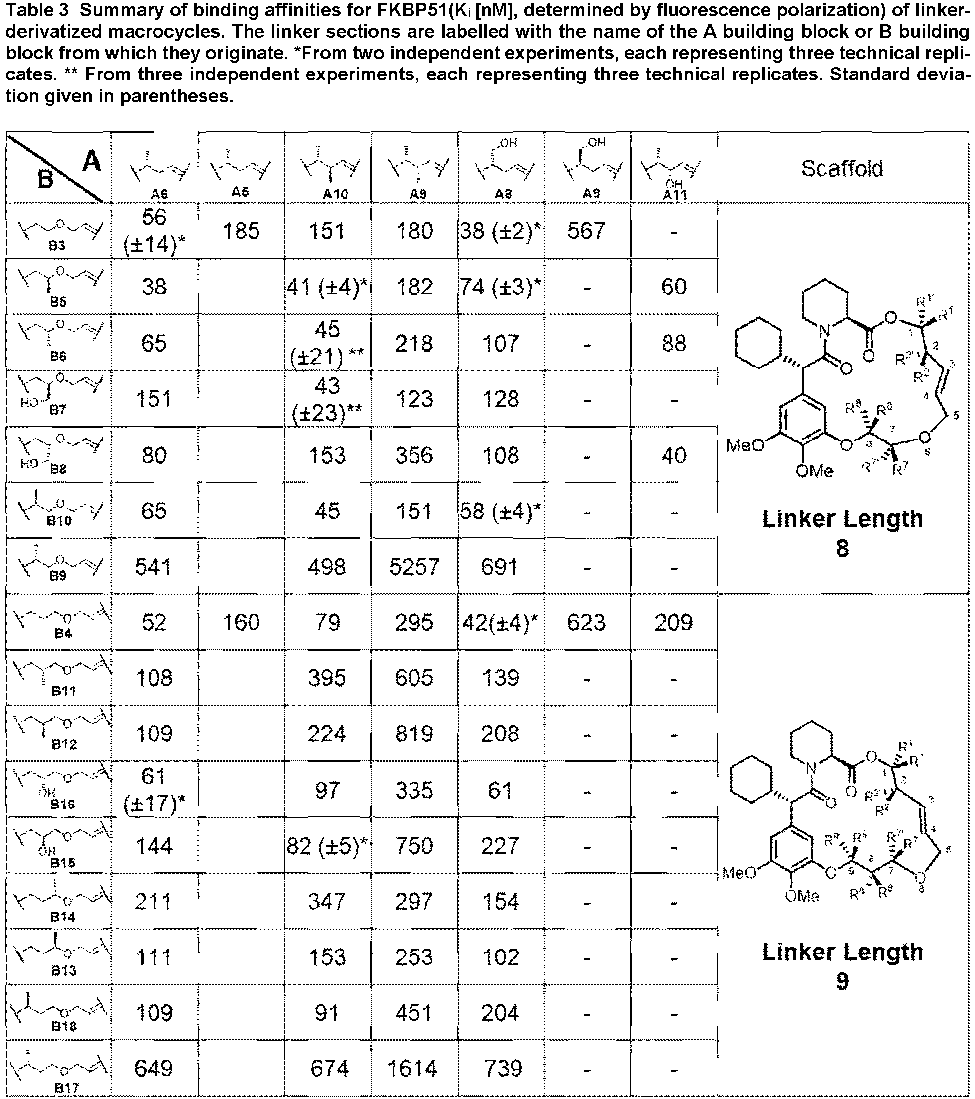

With this strategy we investigated the influence of linker substitution on several drug-relevant properties such as affinity, intracellular activity, physicochemical properties, and in vitro pharmacokinetic.

### Example 5: SAR analysis

The binding affinities of linker-derivatized macrocycles for FKBP51 and close homologs were analyzed. All macrocycles bound to FKBP51 discriminated against FKBP52 (Ki<50µM, data not shown).

A first general trend was observed for higher binding affinities for macrocycles with 8 compared to 9-atom linkers.

Starting from the R1'-Me substituted macrocycles m5(6,3)-(E) and m5(6,4)-(E), the effect of additional methyl groups of the linker at R2 or R2' was investigated. Independent from linker length and substituents in the B-part of the linker, a methyl group in R2' impaired binding affinity in almost all cases compared to the R2/R2' unsubstituted analogs. This observation was consistent with a series of co-crystal structures, which indicated a potential clash between R2'-Me and Val86 of FKBP51.

A methyl group in R2 was largely tolerated (0.3 - 3-fold change). The specific effects on affinity were dependent on the composition of the B-part. In the context of B3, B8, B4, B12 the addition of a methyl in R2 decreased affinity whereas for B7 and B15 affinity was increased.

In general, linker substitutions in the B-part were well tolerated and neither impaired nor improved affinity. A notable exception was the methylation in R8' (for 8-atom linkers) or R9' (for 9-atom linkers), which strongly impaired binding affinity. Co-crystal structures revealed that introduction of residues at this position might clash with the protein.

### Example 6: Comparison with Linear Precursors

The table below (Table 4) shows a comparison of selected diverse macrocycles with their linear precursors demonstrating the superiority of macrocycles regarding the affinity for FKBP51.

**Table 4 Comparison of binding affinities of a diverse set of macrocycles with the corresponding linear precursors.**

| Structure | Cpd | Ki FKBP 51 [nM] | Structure | Cpd | Ki FKBP 51 [nM] |
|---|---|---|---|---|---|
| 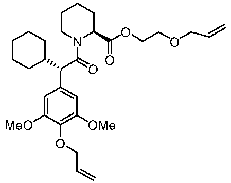 | p4(3,1) | 3159 | 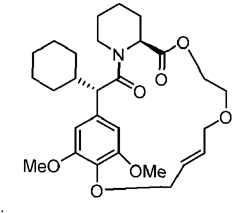 | p5(3,1)-(E) | 386 |
| 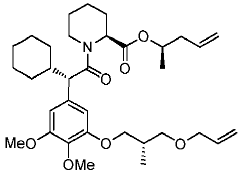 | m4(6, 11) | 1311 | 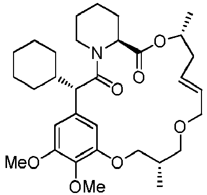 | m5(6,11)-(E) | 108 |
| 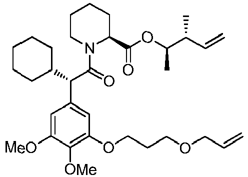 | m4(10,4) | 1650 | 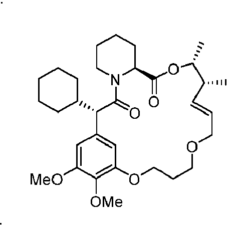 | m5(10,4)-(E) | 79 |
| 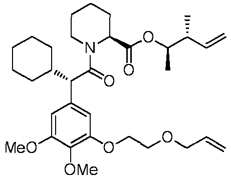 | m4(10,3) | 2143 | 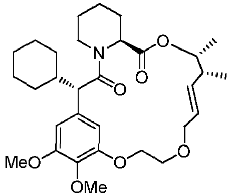 | m5(10,3)-(E) | 151 |
| 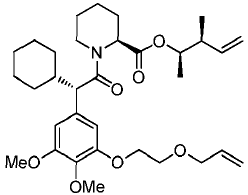 | m4(9,3) | 553 | 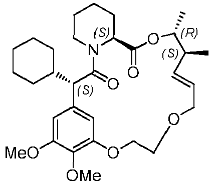 | m5(9,3)-(E) | 123 |
| 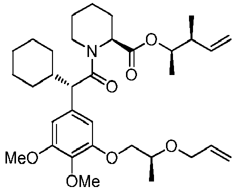 | m4(9,5) | 1581 | 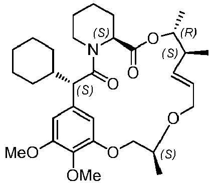 | m5(9,5)-(E) | 182 |
| 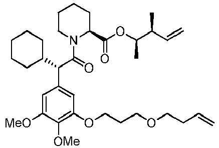 | m4(9,4) | 846 | 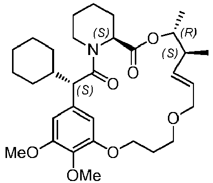 | m5(9,4)-(E) | 295 |
| 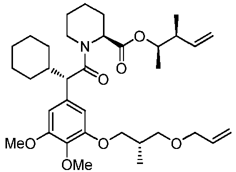 | m4(9,11) | 1331 | 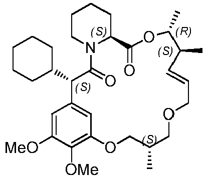 | m5(9,11)-(E) | 648 |
| 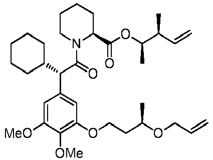 | m4(9,13) | 732 | 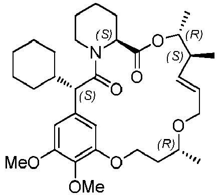 | m5(9,13)-(E) | 253 |
| 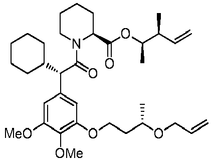 | m4(9,14) | 762 | 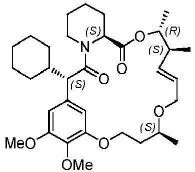 | m5(9,14)-(E) | 297 |
| 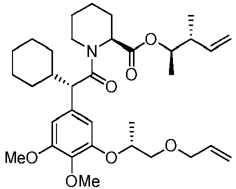 | m4(10,9) | 1770 | 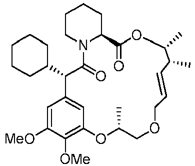 | m5(10,9)-(E) | 498 |
| 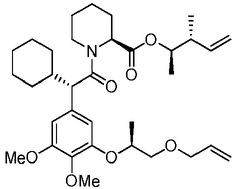 | m4(10,10) | 1200 | 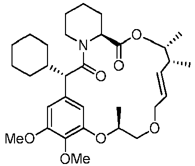 | m5(10,10)-(E) | 45 |
| 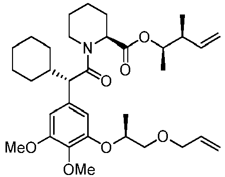 | m4(9,10) | 930 | 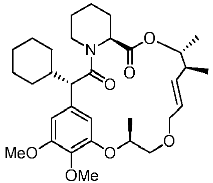 | m5(9,10)-(E) | 151 |
| 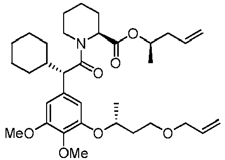 | m4(6,17) | 2107 | 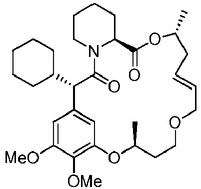 | m5(6,17)-(E) | 109 |
| 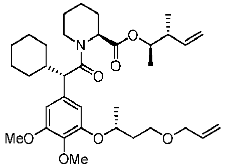 | m4(10,17) | 2454 | 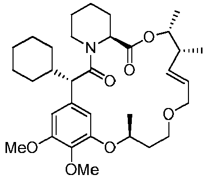 | m5(10,17)-(E) | 91 |
| 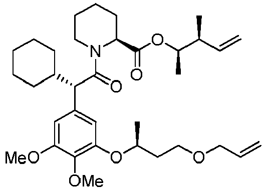 | m4(9,18) | 1573 | 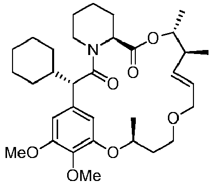 | m5(9,18)-(E) | 451 |
| 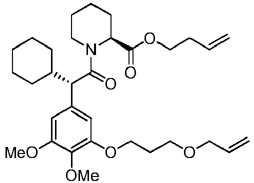 | m4(2,4) | 559 | 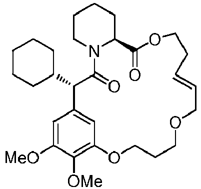 | m5(2,4)-(E) | 102 |
| 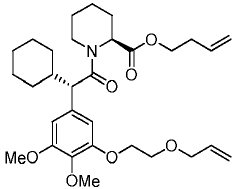 | m4(2,3) | 453 | 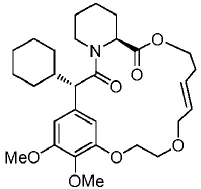 | m5(2,3)-(E) | 86 |
| 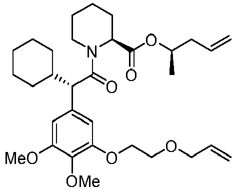 | m4(6,3) | 1013 | 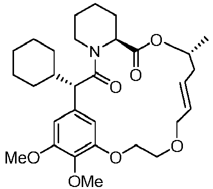 | m5(6,3)-(E) | 56 |
| 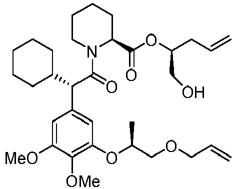 | m4(8,10)-OH | 1588 | 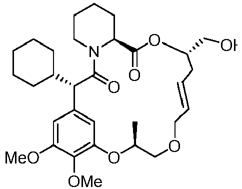 | m5(8,10)-(E)-OH | 58 |
| 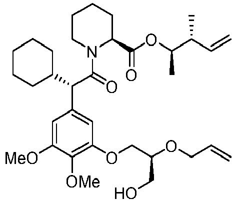 | m4(10,7)-OH | 2732 | 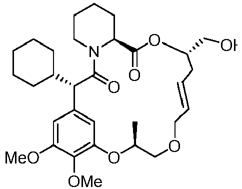 | m5(10,7)-(E)-OH | 43 |
| 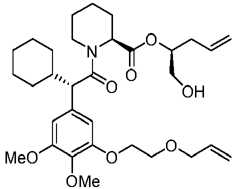 | m4(8,3)-OH | 1016 | 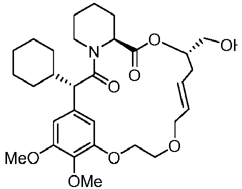 | m5(8,3)-(E)-OH | 38 |
| 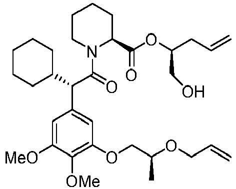 | m4(8,5)-OH | 1988 | 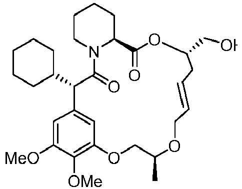 | m5(8,5)-(E)-OH | 74 |
| 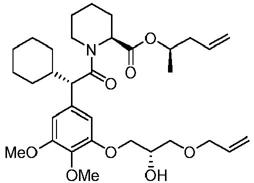 | m4(6,16)-OH | 926 | 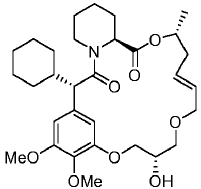 | m5(6,16)-(E)-OH | 61 |
| 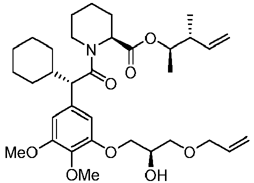 | m4(9,15)-OH | 1335 | 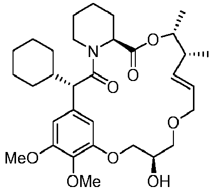 | m5(9,15)-(E) | 82 |
| 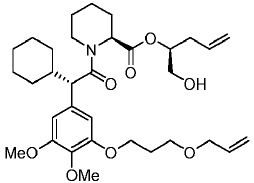 | m4(8,4)-OH | 1742 | 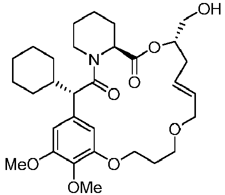 | m5(8,4)-(E)-OH | 42 |

All matched molecular pairs, the macrocycle showed a higher binding affinity compared to its linear precursor. This difference increased from approximately 5-fold in the case of unsubstituted linkers (e.g. m5(2,3)-(E)/m4(2,3) and m5(2,4)-(E)/m4(2,4))to up to 50-fold for macrocycles with functionalized linkers. This shows that the macrocyclic scaffold is crucial to affinity improvements by linker derivatization. A reason might be that only in the context of a macrocyclic scaffold the linker substituents can beneficially interact with the protein. More importantly seems to be the stabilization of higher-affinity conformations by the linker substituents that only work properly in the macrocyclic context. The success of macrocyclization is thus critically dependent on the flexibility and nature of the linker.

## Claims

1. A compound of the general formula 1: 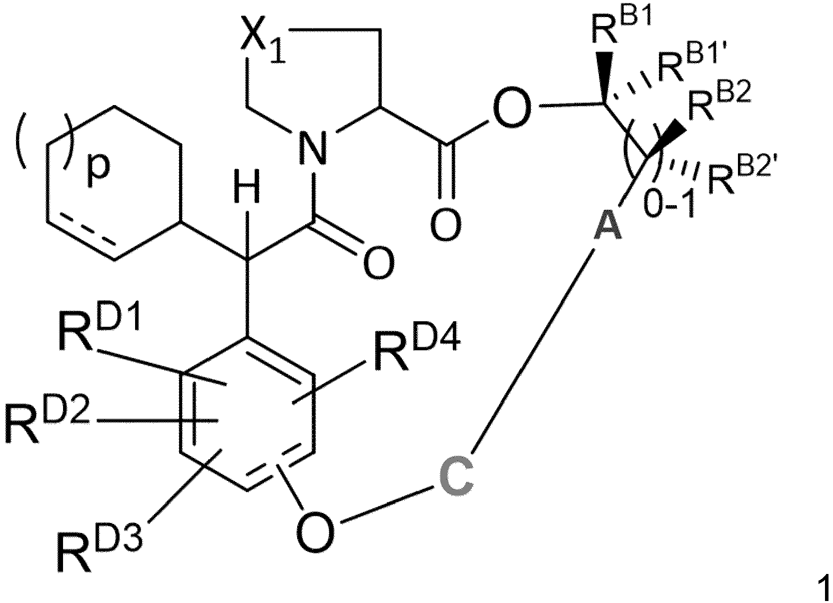 wherein
X1 represents -CH₂-, -CH₂CH₂-, -CH=CH-, -CH₂-S-, or -S-CH₂-;
p is an integer of 0 or 1;
" 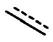 " represents a C=C bond or a C-C bond;
wherein C represents 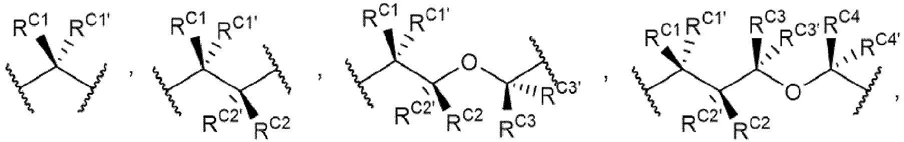 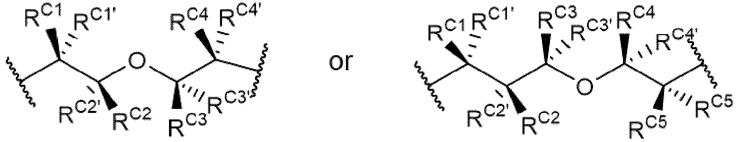
wherein R^{C1}, R^{C1'}, R^{C2}, R^{C2'}, R^{C3}, R^{C3'}, R^{C4}, R^{C4'}, R^{C5}, R^{C5'}, represent independently from each other: H, F, Me, C₂H₅, C₃H₇, CH₂CH=CH, CH=CH₂, C≡CH, cyclo-C₃H₅, OH, OMe, CH₂OH, CH₂OMe, NH₂, NH-Me, NH-Me₂, or CN;
wherein A represents 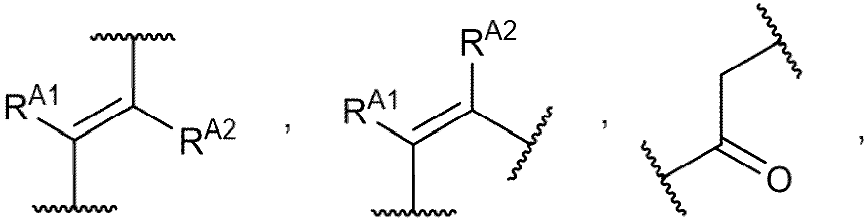 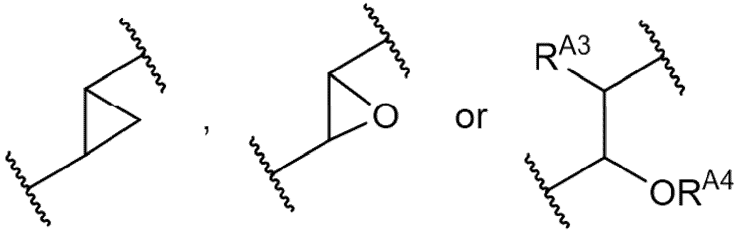
wherein R^{A1}, R^{A2} represent independently from each other: H, F, Me, or CH₂OH; and
wherein R^{A3} represents H, F, OH, O-Me, Me, CN, NH2, NH-Me, N-Me2, SH, or S-Me; and
wherein R^{A4} represents H, or Me.
wherein R^{B1}, R^{B1'}, R^{B2}, R^{B2'} represent independently from each other: H, F, Me, C₂H₅, C₃H₇, CH₂CH=CH₂, CH=CH₂, C≡CH, cyclo-C₃H₅, OH, O-Me, CH₂OH, CH₂O-Me, NH₂, NH-Me, NH-Me₂, or CN;
wherein R^{D1}, R^{D2}, R^{D3}, R^{D4} represent independently from each other: -H, -OH, -OCH₃, - OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OH, -C₂H₄-OH, -C₃H₆-OH, -CH(OH)-CH₂-OH, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅,-CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -Cl, -Br, -I,-P(O)(OH)₂, -P(O)(OCH₃)₂, -P(O)(OC₂H₅)₂, -P(O)(OCH(CH₃)₂)₂, -C(OH)[P(O)(OH)₂]₂,-Si(CH₃)₂(C(CH₃)₃), -Si(C₂H₅)₃, -Si(CH₃)₃, -N₃, -CN, -OCN, -NCO, -SCN, -NCS, -CHO,-COCH₃, -COC₂H₅, -COC₃H₇, -CO-cyclo-C₃H₅, -COCH(CH₃)₂, -COC(CH₃)₃, -COOH,-COCN, -COOCH₃, -COOC₂H₅, -COOC₃H₇, -COO-cyclo-C₃H₅, -COOCH(CH₃)₂,-COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CH₂-CONH₂, -CONHCH₃, -CONHC₂H₅, -CONHC₃H₇, -CONH-cyclo-C₃H₅,-CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂,-S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂] -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₅H₉, -cyclo-C₆H₁₁, -CH₂-cyclo-C₆H₁₁, -CH₂-CH₂-cyclo-C₆H₁₁, -cyclo-C₇H₁₃, -cyclo-C₈H₁₅, -Ph, -CH₂-Ph, -CH₂-CH₂-Ph, -CH=CH-Ph,-CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂,-CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂,-C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, -cyclo-C₃H₅, - cyclo-C₄H₇, -O-CHF₂, -O-CF₂-CH=CH₂, -O-CF₂-C₂H₅, -NH-COOCH₃, or -N(CH₃)-COCH_{3;}
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts.

2. The compound according to claim 1, wherein R^{B1}, R^{B1'}, R^{B2}, R^{B2'} are independently selected from -H, -CH₃, -C₂H₅, -OH, -OCH₃, -F, -Cl, or -NH₂.

3. The compound of any of the previous claims according to formula 2:

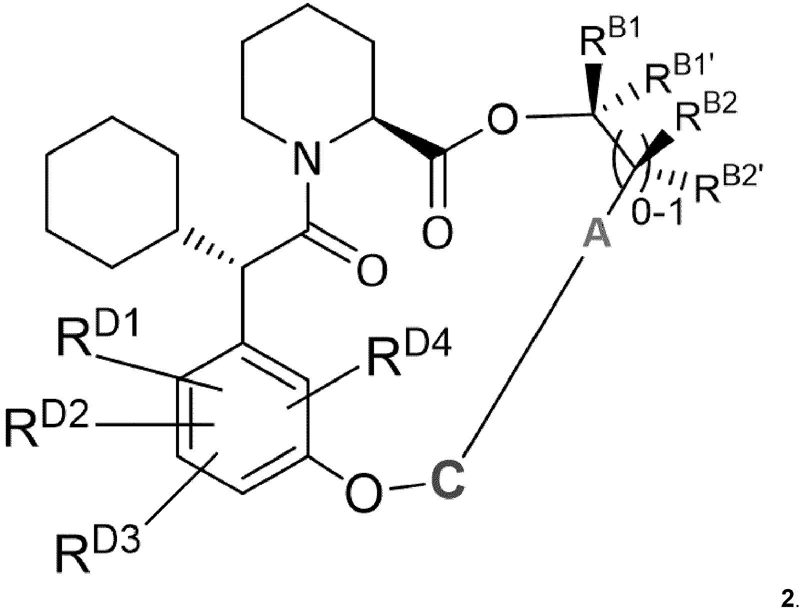

4. The compound of any of the previous claims according to formula 3:

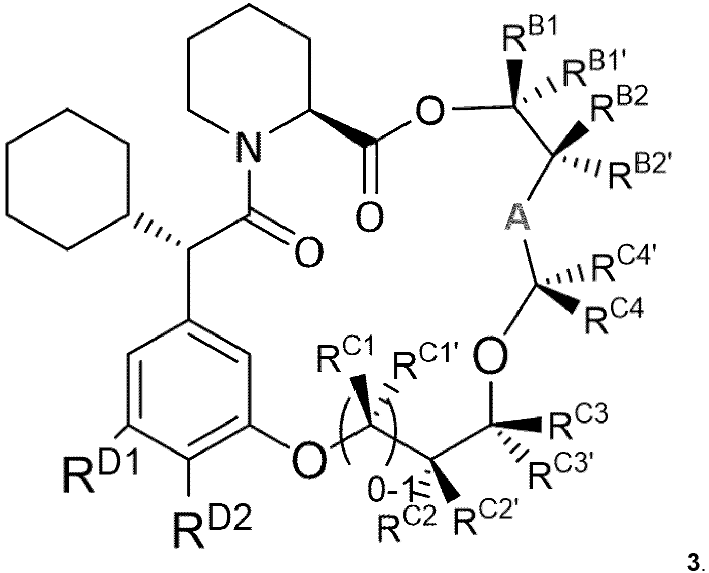

5. The compound according to any of the previous claims, wherein the compound has a K_{d} [nM] to FKBP51 of 500 nM or less

6. The compound according to any of the previous claims, wherein the compound has a IC50 [nM] measured by nanoBRET to FKBP51 of 800 or less.

7. The compound according to any of the previous claims, wherein the compound binds selectively to FKBP51 in the presence of other FKBPs, such as FKBP52.

8. Pharmaceutical composition comprising at least one compound according to claims 1 to 7 together with at least one pharmaceutically acceptable carrier, solvent or excipient or together with at least one pharmaceutically acceptable carrier, solvent or excipient and at least one active agent selected from the group consisting of an anti-depressant and other psychotropic drugs, as well as diabetes drugs, pain drugs and/or antibiotics.

9. Pharmaceutical composition according to any of the previous claims further comprising at least one active agent selected from the group consisting of an anti-depressant and other psychotropic drugs.

10. Pharmaceutical composition according to any of the previous claims, wherein the anti-depressant is selected from amitriptyline, amioxide clomipramine, doxepine, duloxetine, imipramine trimipramine, mirtazapine, reboxetine, citaloprame, fluoxetine, moclobemide and sertraline.

11. Compound according to any one of the previous claims for use as pharmaceutically active agent in medicine.

12. Compound according to any one of claim 1 to 7, or the pharmaceutical composition according to any of claim 8 to 10, for use in the treatment or prophylaxis of psychiatric disorders, neurological disorders, metabolic diseases, cancers, glucocorticoid hyposensitivity syndromes, peripheral glucocorticoid resistance, infectious diseases, alopecia, abnormally elevated intraocular pressure, macular degeneration, oxidative damage to eye tissues, vision disorder, sleeping disorders, asthma, diabetes, traumatic brain injury, nerve injury, Alzheimer's disease, Huntington's disease, Parkinson's disease, ischemia, memory impairment and for neuroprotection, neuroregeneration, promoting hair growth, stimulating neurite growth, wound healing, antiglaucomatous medications, improving vision, enhancing memory performance, for the use in treatment or prevention of multi-drug resistance, for the use in induced abortion and the inhibition of embryo implantation, for the use in limiting or preventing haemorrhage or neovascularization and for the use in treatment of diseases relating to neurodegeneration.

13. The compound for the use according to claim 12, wherein the psychiatric disorder is an affective disorder or an anxiety disorder;
wherein the affective disorder is selected from the group consisting of depression, bipolar disorder, mania, substance induced mood disorder and seasonal affective disorder (SAD); and
wherein the anxiety disorder is selected from the group consisting of generalized anxiety disorder, panic disorder, panic disorder with agoraphobia, phobias, obsessive-compulsive disorder, post-traumatic stress disorder, separation anxiety and childhood anxiety disorders.

14. Use of a compound according to any one of the previous claims as agents for inducing protein-protein interactions (PPIs) acting as molecular glues.

15. The use according to claim 14, for at least one of the uses selected from the group consisting of targeted protein degradation, enzyme modulation, modulation of protein-protein interactions, immunomodulation, chemical biology research, development of PROTACs, and Pharmacological Chaperones, as well as combinations thereof.
